(19) Europäisches Patentamt
European Patent Office
Office européen des brevets

(11) EP 1 401 841 B1

(12) **EUROPÄISCHE PATENTSCHRIFT**

(45) Veröffentlichungstag und Bekanntmachung des Hinweises auf die Patenterteilung:
**24.08.2005 Patentblatt 2005/34**

(21) Anmeldenummer: **02735422.4**

(22) Anmeldetag: **21.06.2002**

(51) Int Cl.[7]: **C07D 498/10**, A61K 31/438, A61P 29/02

(86) Internationale Anmeldenummer:
**PCT/EP2002/006880**

(87) Internationale Veröffentlichungsnummer:
**WO 2003/000699 (03.01.2003 Gazette 2003/01)**

(54) **SUBSTITUIERTE 1-OXA-2,8-DIAZA-SPIRO[4,5]DEC-2-EN-DERIVATE ALS ARZNEIMITTEL GEGEN SCHMERZ**

SUBSTITUTED 1-OXA-2,8-DIAZA-SPIRO 4,5]DEC-2-ENE DERIVATIVES AS MEDICAMENTS FOR THE TREATMENT OF PAIN

DERIVES DE 1-OXA-2,8-DIAZASPIRO 4,5]DEC-2-ENE SERVANT D'ANTALGIQUES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **25.06.2001 DE 10130020**

(43) Veröffentlichungstag der Anmeldung:
**31.03.2004 Patentblatt 2004/14**

(73) Patentinhaber: **Grünenthal GmbH**
**52078 Aachen (DE)**

(72) Erfinder:
- **BUSCHMANN, Helmut, Heinrich**
  **E-08950 Esplugues de Llobregat (ES)**
- **ENGLBERGER, Werner, Günter**
  **52223 Stolberg (DE)**
- **GERMANN, Tieno**
  **52080 Aachen (DE)**
- **HENNIES, Hagen-Heinrich**
  **52152 Simmerath (DE)**
- **MAUL, Corinna**
  **52066 Aachen (DE)**
- **SUNDERMANN, Bernd**
  **52066 Aachen (DE)**
- **HOLENZ, Jörg**
  **08012 Barcelona (ES)**
- **HAURAND, Michael**
  **52078 Aachen (DE)**

(56) Entgegenhaltungen:
**US-A- 3 399 192**
**US-A- 3 598 828**
**US-A- 5 073 560**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 401 841 B1

Printed by Jouve, 75001 PARIS (FR)

## Beschreibung

Die vorliegende Erfindung betrifft substituierte 1-Oxa-2,8-diaza-spiro[4.5]dec-2-en-derivate, sowie Verfahren zu deren Herstellung, deren Verwendung zur Herstellung von Arzneimitteln und Arzneimittel enthaltend diese Verbindungen.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen, z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Proteingekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs-/Nebenwirkungsprofil als z.B. Morphin verfügen. Weitere pharmakologische Untersuchungen haben inzwischen die Existenz mehrerer Subtypen dieser Opioidrezeptoren ($\mu_1$, $\mu_2$, $\kappa_1$, $\kappa_2$, $\kappa_3$, $\delta_1$ und $\delta_2$) wahrscheinlich gemacht.

Daneben gibt es weitere Rezeptoren und Ionenkanäle, die wesentlich an dem System der Schmerzentstehung und Schmerzweiterleitung beteiligt sind, beispielsweise die sogenannte Batrachotoxin-(BTX-)Bindungsstelle (= Bindungsstelle 2) des Natriumkanals oder der NMDA-Ionenkanal, über den ein wesentlicher Teil der Kommunikation von Synapsen durch Steuerung des Calcium-Ionenaustauschs zwischen neuronaler Zelle und ihrer Umgebung abläuft.

Der vorliegenden Erfindung liegt als eine Aufgabe zugrunde, analgetisch wirksame Verbindungen zur Verfügung zu stellen, die sich zur Schmerztherapie - ggf. auch zur Therapie chronischer und neuropathischer Schmerzen - eignen. Darüber hinaus sollten diese Substanzen möglichst nicht oder in geringerem Maße die Nebenwirkungen, die üblicherweise bei der Anwendung von Opioiden wie Morphin auftreten, wie z.B. Übelkeit, Erbrechen, Abhängigkeit, Atemdepression oder Obstipation, hervorrufen.

Die Aufgabe wird durch die analgetisch wirksamen substituierten 1-Oxa-2,8-diaza-spiro[4.5]dec-2-en-derivate der allgemeinen Formel (I)

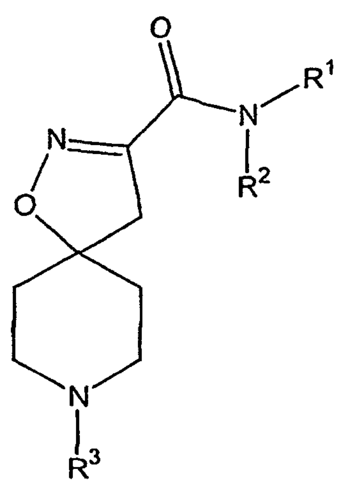

I ,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;

worin

| | |
|---|---|
| $R^1$ und $R^2$ | unabhängig voneinander H, $C_{1-18}$-Alkyl, $C_{3-10}$-Cycloalkyl, ($C_{1-12}$-Alkyl)-$C_{3-10}$-Cycloalkyl, Aryl, ($C_{1-12}$-Alkyl)-Aryl, Heterocyclyl, ($C_{1-12}$-Alkyl)-Heterocyclyl oder NH-C(=O)-Aryl bedeuten, wobei mindestens einer der Reste $R^1$ und $R^2$ nicht H bedeutet, oder gemeinsam für $-(CR^4R^5)_m-(CR^6R^7)_n-Y-(CR^8R^9)_p-(CR^{10}R^{11})_q-$ mit m, n, p und q jeweils = 0, 1, 2, 3, 4 oder 5, unter der Maßgabe, daß $m+n \geq 1$ und $p+q \geq 1$, oder für $-CH_2-CH_2-C(Aryl)=CH-CH_2-$ stehen; |
| $R^3$ | H, $SO_2R^{12}$ oder $COR^{13}$ bedeutet; |
| $R^4$, $R^5$, $R^6$, $R^7$, $R^8$, $R^9$, $R^{10}$ und $R^{11}$ | unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder C(=O)$R^{14}$ bedeuten; |
| Y | $CR^{15}R^{16}$, $NR^{17}$ oder O bedeutet; |
| $R^{12}$ und $R^{13}$ | unabhängig voneinander $C_{1-10}$-Alkyl, $C_{3-10}$-Cycloalkyl, ($C_{1-6}$-Alkyl)-$C_{3-10}$-Cycloalkyl, Aryl, ($C_{1-6}$-Alkyl)-Aryl, Heterocyclyl, ($C_{1-6}$-Alkyl)-Heterocyclyl oder $NR^{18}R^{19}$ bedeuten; |
| $R^{14}$ | H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $OR^{20}$ bedeutet; |
| $R^{15}$ und $R^{16}$ | unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder C(=O)$R^{21}$ bedeuten; |
| $R^{17}$ | H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder C(=O)$R^{22}$ bedeutet; |
| $R^{18}$ und $R^{19}$ | unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl oder ($C_{1-6}$-Alkyl)-Heterocyclyl bedeuten; |
| $R^{20}$ | H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl oder ($C_{1-6}$-Alkyl)-Heterocyclyl bedeutet; |
| $R^{21}$ | H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $OR^{23}$ bedeutet; |
| $R^{22}$ | H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $OR^{24}$ bedeutet; und |
| $R^{23}$ und $R^{24}$ | unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl oder ($C_{1-6}$-Alkyl)-Heterocyclyl bedeuten; |

gelöst.

Diese erfindungsgemäßen Verbindungen der allgemeinen Formel (I) haben sich als analgetisch wirksame Verbindungen erwiesen. Sie können an die BTX-Bindungsstelle des Natriumkanals binden bzw. sind NMDA-Antagonisten mit analgetischer Wirkung.

Die Begriffe "Alkyl", "$C_{1-18}$-Alkyl", "$C_{1-12}$-Alkyl", "$C_{1-10}$-Alkyl", "$C_{1-8}$-Alkyl", "$C_{1-6}$-Alkyl", "$C_{1-4}$-Alkyl", "$C_{1-3}$-Alkyl" bzw. "$C_{1-2}$-Alkyl" umfassen im Sinne dieser Erfindung acyclische gesättigte oder ungesättigte Kohlenwasserstoffreste, die verzweigt oder geradkettig sowie unsubstituiert oder einfach substituiert oder mehrfach gleich oder verschieden substituiert sein können, mit (wie im Fall von $C_{1-18}$-Alkyl) 1 bis 18 (d.h. 1, 2, 3, 4, 5, 6, 7, 8, 9, 10, 11, 12, 13, 14, 15, 16, 17 oder 18) bzw. (wie im Fall von $C_{1-12}$-Alkyl) 1 bis 12 (d.h. 1, 2, 3, 4, 5, 6, 7, 8, 9, 10, 11 oder 12) bzw. (wie im Fall von $C_{1-10}$-Alkyl) 1 bis 10 (d.h. 1, 2, 3, 4, 5, 6, 7, 8, 9 oder 10) bzw. (wie im Fall von $C_{1-8}$-Alkyl) 1 bis 8 (d.h. 1, 2, 3, 4, 5, 6, 7 oder 8) bzw. (wie im Fall von $C_{1-6}$-Alkyl) 1 bis 6 (d.h. 1, 2, 3, 4, 5 oder 6) bzw. (wie im Fall von $C_{1-4}$-Alkyl) 1 bis 4 (d.h. 1, 2, 3 oder 4) bzw. (wie im Fall von $C_{1-3}$-Alkyl) 1 bis 3 (d.h. 1, 2 oder 3) bzw. (wie im Fall von $C_{1-2}$-Alkyl) 1 oder 2 C-Atomen , d.h. $C_{1-18}$-, $C_{1-12}$-, $C_{1-10}$-, $C_{1-8}$-, $C_{1-6}$-, $C_{1-4}$-, $C_{1-3}$- bzw. $C_{1-2}$-Alkanyle, $C_{2-18}$-, $C_{2-12}$-, $C_{2-10}$-, $C_{2-8}$-, $C_{2-6}$-, $C_{2-4}$-, $C_{2-3}$- bzw. $C_2$-Alkenyle und $C_{2-18}$-, $C_{2-12}$-, $C_{2-10}$-, $C_{2-8}$-, $C_{2-6}$-, $C_{2-4}$-, $C_{2-3}$- bzw. $C_2$- Alkinyle. Dabei weisen "Alkenyle" mindestens eine C-C-Doppelbindung und "Alkinyle" mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sek-Butyl, tert-Butyl, n-Pentyl, iso-Pentyl, neo-Pentyl, n-Hexyl, 2-Hexyl, n-Heptyl, n-Octyl, n-Nonyl, n-Decyl, n-Dodecyl; Ethenyl (Vinyl), Ethinyl, Propenyl ($-CH_2CH=CH_2$, $-CH=CH-CH_3$, $-C(=CH_2)-CH_3$), Propinyl ($-CH_2-C\equiv CH$, $-C\equiv C-CH_3$), Butenyl, Butinyl, Pentenyl, Pentinyl, Hexenyl, Hexinyl, Octenyl und Octinyl umfaßt.

"$C_{3-10}$-Cycloalkyl" bzw. "$C_{3-8}$-Cycloalkyl" (bzw. "Cycloalkyl") bedeutet im Sinne dieser Erfindung cyclische

gesättigte oder ungesättigte Kohlenwasserstoff-Reste mit 3, 4, 5, 6, 7. 8,9 oder 10 bzw. 3, 4, 5, 6, 7 oder 8 C-Atomen, wobei jeder Rest unsubstituiert oder einfach substituiert oder mehrfach gleich oder verschieden substituiert und ggf. benzokondensiert sein kann. Ferner kann es sich um ein bi-, tri- oder polycyclisches Ringsystem handeln. Beispielhaft steht Cycloalkyl für Cyclopropyl, Cyclobutyl, Cyclopentyl, Cyclohexyl, Cycloheptanyl und Cyclooctanyl sowie für Adamantyl und Bicyclo[3.1.1]heptan-3-yl.

Unter dem Ausdruck "Aryl" ist für die Zwecke der vorliegenden Erfindung ein Rest zu verstehen, der aus der Gruppe, die Phenyl, Naphthyl, Phenanthrenyl, Anthracenyl und Biphenyl umfaßt, ausgewählt ist und unsubstituiert oder einfach oder mehrfach gleich oder verschieden substituiert ist. Bevorzugt ist Aryl ein unsubstituiertes oder einfach substituiertes oder mehrfach gleich oder verschieden substituiertes Phenyl, 1-Naphthyl oder 2-Naphthyl.

Der Ausdruck "Heterocyclyl" steht für einen monocyclischen oder polycyclischen organischen Rest, in dem mindestens ein Cyclus 1 Heteroatom oder 2, 3, 4 oder 5 gleiche oder verschiedene Heteroatome enthält, das/die aus der Gruppe, die N, O und S enthält, ausgewählt ist/sind, wobei der Rest gesättigt oder ungesättigt ist und unsubstituiert oder einfach substituiert oder mehrfach gleich oder verschieden substituiert ist. Beispiele für Heterocyclyl-Reste im Sinne dieser Erfindung sind monocyclische fünf-, sechs- oder siebengliedrige organische Reste mit 1 Heteroatom oder 2, 3, 4 oder 5 gleichen oder verschiedenen Heteroatomen, bei dem/denen es sich um Stickstoff, Sauerstoff und/oder Schwefel handelt, und deren benzokondensierte Analoga. Eine Untergruppe der Heterocyclyl-Reste bilden die "Heteroaryl"-Reste, bei denen es sich um solche Heterocyclyle handelt, in denen der mindestens eine Cyclus, der das/die Heteroatom/e enthält, heteroaromatisch ist. Jeder Heteroaryl-Rest kann unsubstituiert oder einfach substituiert oder mehrfach gleich oder verschieden substituiert vorliegen. Beispiele für Heterocyclyl-Reste im Sinne der vorliegenden Erfindung sind Pyrrolidinyl, Tetrahydrofuryl, 1,4-Dioxanyl, Piperidinyl, Piperazinyl und insbesondere Morpholinyl. Beispiele für Heteroaryl-Reste sind Pyrrolyl, Furanyl, Thienyl, Pyrazolyl, Imidazolyl, Pyridazinyl, Pyrimidinyl, Pyrazinyl, Isoxazolyl, Thieno[2,3-d]pyrimidinyl, Indolyl und Pyridinyl sowie deren benzokondensierte Analoga. Alle diese Reste können jeweils unsubstituiert oder substituiert vorliegen.

Die Ausdrücke "($C_{1-12}$-Alkyl)-$C_{3-10}$-Cycloalkyl", "($C_{1-6}$-Alkyl)-$C_{3-10}$-Cycloalkyl", "($C_{1-4}$-Alkyl)-$C_{3-10}$-Cycloalkyl", "($C_{1-3}$-Alkyl)-$C_{3-10}$-Cycloalkyl", "($C_{1-12}$-Alkyl)-Heterocyclyl", "($C_{1-6}$-Alkyl)-Heterocyclyl", "($C_{1-4}$-Alkyl)-Heterocyclyl", "($C_{1-3}$-Alkyl)-Heterocyclyl", "($C_{1-12}$-Alkyl)-Aryl", "($C_{1-6}$-Alkyl)-Aryl", "($C_{1-4}$-Alkyl)-Aryl" und "($C_{1-3}$-Alkyl)-Aryl" bedeuten für die Zwecke der vorliegenden Erfindung, daß der Cycloalkyl-, Heterocyclyl- bzw. Aryl-Rest über eine $C_{1-12}$-, $C_{1-6}$-, $C_{1-4}$- bzw. $C_{1-3}$-Alkyl-Gruppe an die mit ihm substituierte Verbindung gebunden ist. Ein besonders bevorzugtes Beispiel für"AlkylCycloalkyl" ist der Cyclopropylmethylrest.

Im Zusammenhang mit "Alkyl", "Alkanyl", "Alkenyl", "Alkinyl" und "Cycloalkyl" versteht man unter dem Begriff "substituiert" - soweit der Ausdruck nicht an anderer Stelle der Beschreibung bzw. in den Ansprüchen definiert ist - im Sinne dieser Erfindung die einfache oder mehrfache Substitution von einem oder mehreren Wasserstoffatomen durch beispielsweise F, Cl, Br, I, -CN, $NH_2$, NH-Alkyl, NH-Aryl, NH-Alkyl-Aryl, NH-Heterocyclyl, N(Alkyl)$_2$, N(Alkyl-Aryl)$_2$, N-Alkyl-N-Aryl, $NO_2$, OH, O-Alkyl, S-Alkyl, O-Aryl, O-Alkyl-Aryl, O-Alkyl-O-Alkyl, C(=O)$C_{1-6}$-Alkyl, C(=O)Aryl, C(=O)$C_{1-6}$-Alkyl-Aryl, C(=O)-Heterocyclyl, $CO_2H$, $CO_2$-Alkyl, $CO_2$-Alkyl-Aryl, C(=O)$NH_2$, C(=O)NH-Alkyl, C(=O)NHAryl, C(=O) NH-Heterocyclyl, C(=O)N(Alkyl)$_2$, C(=O)N(Alkyl-Aryl)$_2$, Cycloalkyl, Aryl oder Heterocyclyl, wobei die mehrfache Substitution entweder an verschiedenen oder an gleichen Atomen mehrfach, z.B. zwei- oder dreifach, erfolgt, beispielsweise dreifach am gleichen C-Atom wie im Falle von $CF_3$ oder -$CH_2CF_3$ oder an verschiedenen Stellen wie im Falle von -CH(OH)-CH=CCl-$CH_2$Cl. Die Mehrfachsubstitution kann mit dem gleichen oder mit verschiedenen Substituenten erfolgen. Besonders bevorzugt für die Zwecke der vorliegenden Erfindung ist $CF_3$ als substituiertes Alkyl.

In Bezug auf "Aryl", "Heterocyclyl" sowie "Heteroaryl" versteht man im Sinne dieser Erfindung unter "substituiert" die ein- oder mehrfache, z.B. zwei-, drei- oder vierfache, Substitution eines oder mehrerer Wasserstoffatome des Ringsystems durch einen geeigneten Substituenten. Soweit die Bedeutung dieser geeigneten Substituenten im Zusammenhang mit "Aryl", "Heterocyclyl" oder "Heteroaryl" nicht an anderer Stelle der Beschreibung oder in den Ansprüchen definiert ist, sind geeignete Substituenten F, Cl, Br, I, -CN, $NH_2$, NH-Alkyl, NH-Aryl, NH-Alkyl-Aryl, NH-Heterocyclyl, N(Alkyl)$_2$, N(Alkyl-Aryl)$_2$, $NO_2$, SH, S-Alkyl, OH, O-Alkyl, O-Cycloalkyl, O-Aryl, O-Alkyl-Aryl, O-Heterocyclyl, CHO, C(=O)$C_{1-6}$-Alkyl, C(=O)Aryl, C(=O)-$C_{1-6}$-Alkyl-Aryl, $CO_2H$, $CO_2$-Alkyl, $CO_2$-Alkyl-Aryl, C(=O)$NH_2$, C(=O)NH-Alkyl, C(=O)NHAryl, C(=O)NH-Heterocyclyl, C(=O)N(Alkyl)$_2$, $SO_2NH_2$, $SO_3H$, $CF_3$, $CHF_2$, $CH_2F$, $OCF_3$, $OCHF_2$, $OCH_2F$, $SCF_3$, $SCHF_2$, $SCH_2F$; Alkyl, Cycloalkyl, Aryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Besonders bevorzugte Substituenten für Aryl und Heterocyclyl sind $C_{1-6}$-Alkyl, F, Cl, Br, I, $CF_3$, OAlkyl, $OCF_3$, Phenyl, CN und/oder $NO_2$.

"Benzokondensiert" bedeutet für die Zwecke der vorliegenden Erfindung, daß ein Benzol-Ring an einen anderen Cyclus ankondensiert ist.

Pharmazeutisch annehmbare bzw. physiologisch verträgliche Salze im Sinne dieser Erfindung sind solche Salze der erfindungsgemäßen Verbindungen gemäß der allgemeinen Formel (I), die bei pharmazeutischer Verwendung physiologisch - insbesondere bei Anwendung am Säugetier und/oder Menschen - verträglich sind, d.h. keine (akute) wesentliche Beeinträchtigung der physiologischen Funktionen der jeweiligen Spezies hervorrufen. Solche pharmazeutisch annehmbaren (physiologisch verträglichen) Salze können beispielsweise mit anorganischen oder organischen Säuren oder für den Fall, daß die erfindungsgemäßen Verbindungen Säuren, insbesondere Carbonsäuren sind, mit Basen gebildet werden.

Vorzugsweise werden die pharmazeutisch annehmbaren Salze der erfindungsgemäßen Verbindungen gemäß der allgemeinen Formel (I) mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Handelt es sich bei den erfindungsgemäßen Verbindungen um Säuren, z.B. Carbonsäuren, können die pharmazeutisch annehmbaren Salze auch durch Umsetzung mit Basen, wie z.B. Natriumhydroxid, Natriumhydrogencarbonat oder Natriumcarbonat, gebildet werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate bzw. um Natrium-Salze. Ebenfalls bevorzugt sind die Hydrate der erfindungsgemäßen Verbindungen, die z.B. durch Kristallisation aus wäßriger Lösung erhalten werden können.

Soweit erfindungsgemäße Verbindungen der Formel (I) mindestens ein Asymmetriezentrum enthalten, können sie in Form ihrer Racemate, in Form der reinen Enantiomeren und/oder (im Falle mehrerer Asymmetriezentren) der Diastereomeren oder in Form von Mischungen dieser Enantiomeren bzw. Diastereomeren vorliegen, und zwar sowohl in Substanz als auch als physiologisch verträgliche Salze dieser Verbindungen. Die Mischungen können in jedem beliebigen Mischungsverhältnis der Stereoisomeren vorliegen. Bevorzugt liegen die Verbindungen der allgemeinen Struktur (I) als enantiomerenreine Verbindungen vor.

Bevorzugte erfindungsgemäße Verbindungen der allgemeinen Formel (I) sind solche, die dadurch gekennzeichnet sind, daß

| | |
|---|---|
| $R^1$ und $R^2$ | unabhängig voneinander H, $C_{1-8}$-Alkyl, $C_{3-8}$-Cycloalkyl, ($C_{1-4}$-Alkyl)-$C_{3-8}$-Cycloalkyl, Aryl, ($C_{1-4}$-Alkyl)-Aryl, Heterocyclyl, ($C_{1-4}$-Alkyl)-Heterocyclyl oder NH-C(=O)-Aryl bedeuten, wobei mindestens einer der Reste $R^1$ und $R^2$ nicht H bedeutet,<br>oder<br>gemeinsam für $-(CR^4R^5)_m-(CR^6R^7)_n-Y-(CR^8R^9)_p-(CR^{10}R^{11})_q-$ mit m = 1, n = 0 oder 1, p = 1 oder 2 und q = 1 oder 2, oder für $-CH_2-CH_2-C(-Aryl)=CH-CH_2-$ stehen; |
| $R^3$ | $SO_2R^{12}$ oder $COR^{13}$ bedeutet; |
| $R^4$, $R^5$, $R^6$, $R^7$, $R^8$, $R^9$, $R^{10}$ und $R^{11}$ | unabhängig voneinander H, $C_{1-8}$-Alkyl oder $C(=O)R^{14}$ bedeuten; |
| Y | $CR^{15}R^{16}$ oder $NR^{17}$ bedeutet; |
| $R^{12}$ | $C_{1-4}$-Alkyl, ($C_{1-4}$-Alkyl)-$C_{3-8}$-Cycloalkyl, Aryl, ($C_{1-4}$-Alkyl)-Aryl, Heterocyclyl oder $NR^{18}R^{19}$ bedeutet; |
| $R^{13}$ | $C_{1-6}$-Alkyl, $C_{3-10}$-Cycloalkyl, Aryl, ($C_{1-4}$-Alkyl)-Aryl oder Heterocyclyl bedeutet; |
| $R^{14}$ | $OR^{20}$ bedeutet; |
| $R^{15}$ und $R^{16}$ | unabhängig voneinander H, Aryl oder ($C_{1-4}$-Alkyl)-Aryl bedeuten; |
| $R^{17}$ | H, $C_{3-8}$-Cycloalkyl, Aryl, ($C_{1-4}$-Alkyl)-Aryl, Heterocyclyl oder $C(=O)R^{22}$ bedeutet; |
| $R^{18}$ und $R^{19}$ | unabhängig voneinander H oder $C_{1-6}$-Alkyl bedeuten; |
| $R^{20}$ | $C_{1-6}$-Alkyl bedeutet; |
| $R^{22}$ | Aryl, ($C_{1-4}$-Alkyl)-Aryl, Heterocyclyl oder $OR^{24}$ bedeutet; und |
| $R^{24}$ | $C_{1-6}$-Alkyl oder ($C_{1-4}$-Alkyl)-Aryl bedeutet. |

Eine besonders bevorzugte Untergruppe dieser bevorzugten Verbindungen der Formel (I) wird von Verbindungen gebildet, in denen

$R^1$ $C_{1-6}$-Alkyl, $C_{3-8}$-Cycloalkyl, ($C_{1-3}$-Alkyl)-$C_{3-8}$-Cycloalkyl, Aryl, ($C_{1-3}$-Alkyl)-Aryl, Heterocyclyl, ($C_{1-3}$-Alkyl)-Heterocyclyl oder NH-C(=O)-Aryl bedeutet; und

$R^2$ H, $C_{1-4}$-Alkyl, ($C_{1-3}$-Alkyl)-Aryl oder ($C_{1-3}$-Alkyl)-Heterocyclyl bedeutet;

wobei ganz besonders bevorzugte Verbindungen solche sind, in denen

$R^1$ unsubstituiertes oder mit F, Cl, Br, I, -CN, N-Alkyl-N-Arylamin, N,N-Dialkylamin, Amid, Carboxyalkyl, Carboxybenzyl substituiertes Methyl, Ethyl, n-Propyl, iso-Propyl, 2-Methylpropyl, n-Butyl, tert.-Butyl, n-Pentyl, 3-Methylbutyl oder $CH_2-C(CH_3)=CH_2$, insbesondere Methyl, Ethyl, $CH_2C(CH_3)=CH_2$, $CH(C(=O)OCH_2CH=CH_2)-CH_2C(=O)O$-tert.-Butyl, 2-Cyanoethyl, $CH_2-CH_2-NH-C(=O)CH_3$, 2-(N-Ethyl-N-(3-methylphenyl)amino)-ethyl, 2-(N,N-Dimethylamino)-ethyl, 2-(C(=O)-NH-(β-Naphthyl)-ethyl, 1,2-(Di-(C(=O)O-tert.-butyl)ethyl, 3-(N-Methyl-N-phenylamino)-propyl, 1-(C(=O)O-Benzyl)-3-methyl-butyl, 1-(C(=O)O-Butyl)-3-methyl-butyl, $CH_2CO_2$Ethyl, $CH_2$-$CH_2CO_2$Ethyl, $CH_2$-$CH_2$-OPhenyl, $CH_2$-$CH_2$-S-$CH_2$-$CH_3$;

unsubstituiertes oder mit F, Cl, Br, I, -CN, Alkyl, Aryl, Carboxyalkyl, Carboxybenzyl, O-Alkyl, O-Benzyl substituiertes Cyclopropyl, Cyclobutyl, Cyclopentyl, Cyclohexyl, Cycloheptyl, Cyclooctyl, Bicyclo[3.1.1]heptan-3-yl, insbesondere 2-Phenylcyclopropyl, 2-(O-Benzyl)-cyclopentyl, 2-(Carboxyethyl)cyclohexyl, 7,7-Dimethyl-2-methylbicyclo[3.1.1]heptan-3-yl;

Cyclopropylmethyl, 7,7-Dimethyl-2-oxo-bicyclo[2.2.1]hept-1-ylmethyl;

unsubstituiertes oder mit Phenoxy, -$CH_2$-P(=O)(OEthyl)$_2$ substiuiertes Phenyl, 1-Naphthyl oder 2-Naphthyl;

$CH_2$-Aryl, $CH_2$-$CH_2$-Aryl, $CH_2$-$CH_2$-$CH_2$-Aryl, $CH_2$-$CH_2$-$CH_2$-$CH_2$-Aryl CH($CH_3$)-Aryl, CH($CH_3$)-$CH_2$-Aryl, $CH_2$-CH-(Aryl)$_2$, CH($CO_2$Alkyl)-$CH_2$-Aryl, $CH_2$-$CH_2$-CH-(Aryl)$_2$, wobei Aryl unsubstituiertes Phenyl, 1-Naphthyl oder 2-Naphthyl oder mit F, Cl, Br, I, -CN, -$NO_2$, Alkyl, $CF_3$, Alkoxy, Alkylendioxy substituiertes Phenyl ist, insbesondere Benzyl, -$CH_2$-Naphth-1-yl, 2-Fluorbenzyl, 3-Fluorbenzyl, 3-Chlorbenzyl, 3-Methoxybenzyl, 2-Ethoxybenzyl, 2,4-Difluorbenzyl, 3,5-Dichlorbenzyl, 3-Fluor-5-trifluormethylbenzyl, 3-Fluor-4-trifluormethylbenzyl, 2-Chlor-6-fluorbenzyl, 2,5-Dimethoxybenzyl, 2-Chlor-6-methyl-benzyl, 3,4-Dimethoxybenzyl, 3,4-Dioxymethylenbenzyl, CH($CH_3$)-Phenyl, CH($CH_3$)-(4-$CH_3$-Phenyl), CH($CH_3$)-(4-Nitrophenyl), CH($CH_3$)-(2,3-Dioxyethylenphenyl), $CH_2$-$CH_2$-Phenyl, $CH_2$-$CH_2$-(2-Fluorphenyl), $CH_2$-$CH_2$-(3-Fluorphenyl), $CH_2$-$CH_2$-(4-Fluorphenyl), $CH_2$-$CH_2$-(4-Chlorphenyl), $CH_2$-$CH_2$-(3,4-Dichlorphenyl), $CH_2$-$CH_2$-(3-Methoxyphenyl), $CH_2$-$CH_2$-(2,5-Dimethoxyphenyl), CH($CO_2$-tert.-Butyl)-$CH_2$-Phenyl, CH($CO_2$-Methyl)-$CH_2$-(4-Chlorphenyl), $CH_2$-CH(Phenyl)$_2$, CH ($CH_3$)-$CH_2$-(4-Chlorphenyl), $CH_2$-$CH_2$-CH(Phenyl)$_2$, $CH_2$-$CH_2$-$CH_2$-Phenyl;

unsubstituiertes oder mit Aryl, Alkylaryl oder Carboxyethyl substituiertes Pyrrolidin oder Piperidin, insbesondere Pyrrolidin-3-yl, N-(4-Trifluorbenzyl)-pyrrolidin-3-yl, N-(3-Methoxybenzyl)-pyrrolidin-3-yl, N-($CH_2$-(β-Naphthyl)-pyrrolidin-3-yl oder N-(Carboxyethyl)-piperidin-4-yl;

unsubstituiertes oder mit Alkyl, F, Cl, Br, I, -CN, Aryl, Alkylaryl substituiertes $(CH_2)_{1-3}$-Heterocyclyl, wobei Heterocyclyl für Furanyl, Benzofuranyl, 1,4-Dioxanyl, Benzo-1,4-dioxanyl, Thienyl, Pyridinyl, Pyrrolidinyl, 1H-Indolyl, Imidazolyl, Piperidinyl, Tetrahydrofuranyl steht, insbesondere $CH_2$-Furan-2-yl, 5-Methylfuran-2-yl $CH_2$-Benzofuran-2-yl,

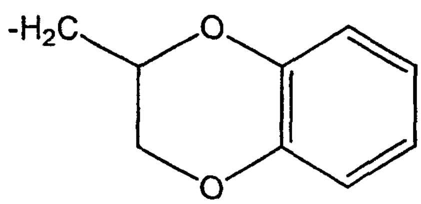

,

$CH_2$-Thien-2-yl, $CH_2$-Pyridin-3-yl, $CH_2$-Pyridin-4-yl, $CH_2$-$CH_2$-Pyridin-2-yl, $CH_2$-$CH_2$-(1H-Indol-3-yl), $CH_2$-$CH_2$-Pyrrolidin-1-yl, $CH_2$-(N-2,6-Dichiorbenzylpyrrolidin-3-yl), $CH_2$-$CH_2$-(N-Methyl-pyrrolidin-2-yl), -$(CH_2)_3$-Imidazol-1-yl, $CH_2$-(Tetrahydrofuran-2-yl) oder

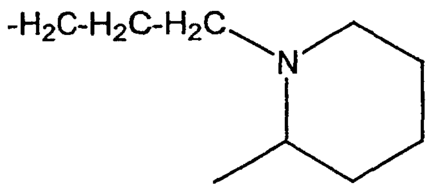

,

oder CH($CO_2$Methyl)-$CH_2$-(1H-Indol-3-yl);

NH-C(=O)-(4-Diethylaminophenyl) bedeutet; und

$R^2$ H;

unsubstituiertes oder mit F, Cl, Br, I, -CN substituiertes Methyl, Ethyl oder $CH_2$-C($CH_3$)=$CH_2$, insbesondere Methyl, Ethyl, 2-Cyanoethyl, $CH_2$-C($CH_3$)=$CH_2$;

unsubstituiertes oder mit F, Cl, Br, I, -CN, Methoxy, Ethoxy substituiertes Benzyl oder Phenethyl, insbesondere Benzyl, 4-Fluorbenzyl, 2-Chlor-6-Fluorbenzyl, 2,5-Dimethoxybenzyl, Phenethyl; oder

$CH_2$-Furanyl, insbesondere $CH_2$-Furan-2-yl, $CH_2$-Benzofuranyl, insbesondere $CH_2$-Benzofuran-2-yl, $CH_2$-Pyri-

dinyl, insbesondere $CH_2$-Pyridin-3-yl, $CH_2$-Tetrahydrofuranyl, insbesondere $CH_2$-Tetrahydrofuran-2-yl, $CH_2$-$CH_2$-Pyridinyl, insbesondere $CH_2$-$CH_2$-Pyridin-2-yl, bedeutet.

Eine weitere Gruppe bevorzugter erfindungsgemäßer Verbindungen der Formel (I) wird von solchen gebildet, die dadurch gekennzeichnet sind, daß

$R^1$ und $R^2$ zusammen für

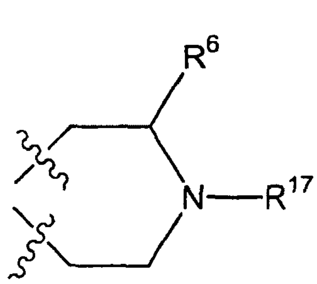
,
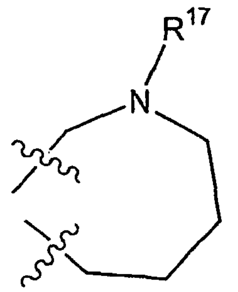
,

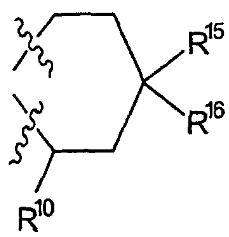
oder
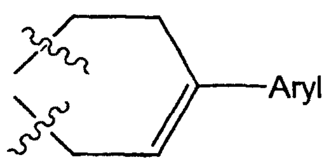
,

wobei Aryl Phenyl oder mit F, Cl, Br, I substituiertes Phenyl, insbesondere 4-Fluorphenyl, bedeutet, stehen;

$R^6$ H oder $C_{1-4}$-Alkyl, inbesondere Methyl, bedeutet;

$R^{10}$ H, C(=O)OMethyl, C(=O)OEthyl, C(=O)O-n-Propyl, C(=O)O-iso-Propyl, C(=O)O-n-Butyl, C(=O)O-tert.-Butyl bedeutet;

$R^{15}$ H oder $CH_2$-Aryl bedeutet;

$R^{16}$ H bedeutet;

$R^{17}$ H;

$C_{3-8}$-Cycloalkyl, insbesondere Cycloheptyl; Aryl, insbesondere unsubstituiertes oder mit Methyl, Ethyl, n-Propyl, iso-Propyl, n-Butyl, tert.-Butyl, $CF_3$, F, Cl, Br, I, -CN, Methoxy, Ethoxy, n-Propoxy, iso-Propoxy, n-Butoxy, tert.-Butoxy substituiertes Phenyl oder Naphthyl;

$(CH_2)_{1-3}$-Alkyl)-Aryl oder $CH(CH_3)$-Aryl, wobei Aryl unsubstituiertes oder mit Alkyl, $CF_3$, F, Cl, Br, I, -CN, Alkoxy substituiertes Phenyl oder Naphthyl bedeutet; unsubstituiertes oder mit mit Alkyl, $CF_3$, F, Cl, Br, I, -CN, Alkoxy substituiertes Pyridinyl, Pyrazinyl oder Thieno[2,3-d]pyrimidinyl bedeutet; oder

$C(=O)R^{22}$ bedeutet; und

$R^{22}$ Phenyl oder Alkoxy-substituiertes Phenyl, O-Methyl, O-Ethyl, O-n-Propyl, O-iso-Propyl, O-n-Butyl, O-tert.-Butyl, O-Benzyl, unsubstituiertes Benzyl, mit F substituiertes Benzyl, unsubstituiertes Pyrazinyl oder mit Alkyl substituiertes Pyrazinyl bedeutet;

wobei unter diesen Verbindungen solche ganz besonders bevorzugte Verbindungen sind, in denen

$R^6$ H oder Methyl bedeutet;

$R^{10}$ H oder C(=O)OEthyl bedeutet;

$R^{15}$ H oder Benzyl bedeutet;

$R^{16}$ H bedeutet; und

$R^{17}$ H;

Cycloheptyl;

Phenyl, 2-Methylphenyl, 3-Methylphenyl, 2,4-Dimethylphenyl, 2-Ethylphenyl, 3-Trifluormethyl, 4-Fluorphenyl, 4-Chlorphenyl, 2-Methoxyphenyl, 3,5-Dimethoxyphenyl, 3-Chlor-6-methylphenyl;
Benzyl, $CH_2$-(4-tert.-Butylphenyl), $CH_2$-(β-Naphthyl), $CH(CH_3)$-Phenyl, $(CH_2)_3$-Phenyl;
Pyridin-2-yl, (4-Trifluormethyl)-pyridin-2-yl, Thieno[2,3-d]pyrimidin-4-yl; oder
C(=O)-(4-Methoxyphenyl), C(=O)-Benzyl, C(=O)-$CH_2$-(3,4-Difluorphenyl), C(=O)-(2-Methylpyrazin-5-yl), C (=O)O-tert.-Butyl oder O-Benzyl bedeutet.

Weiterhin ist es bevorzugt, daß, wenn in den erfindungsgemäßen Verbindungen der allgemeinen Formel (I) $R^3$ $SO_2R^{12}$ bedeutet,

$R^{12}$ Methyl, Ethyl, n-Propyl, iso-Propyl, insbesondere n-Propyl;
7,7-Dimethyl-2-oxo-bicyclo[2.2.1]hept-1-ylmethyl; Phenyl oder mit Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert. -Butoxy, $OCF_3$, $CO_2$Methyl substituiertes Phenyl, insbesondere 4-Methylphenyl, 3-Trifluormethylphenyl, 4-Fluorphenyl, 2-Chlorphenyl, 3-Chlorphenyl, 4-Chlorphenyl, 4-Methoxyphenyl, 4-Trifluormethoxyphenyl, 4-Nitrophenyl, 2-$CO_2$Methyl-phenyl, 2,5-Dichlorphenyl, 3-Fluor-6-methylphenyl, 3-Brom-6-methoxyphenyl, 2-Methyl-5-nitrophenyl, 2,4,6-Trimethylphenyl;
Benzyl oder mit Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy, $OCF_3$ substituiertes Benzyl;
unsubstituiertes oder mit Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy substituiertes Furanyl oder Thienyl, insbesondere Thien-2-yl, 5-Chlorthien-2-yl; oder $NR^{18}R^{19}$ bedeutet; und

$R^{18}$ und $R^{19}$ unabhängig voneinander H, Methyl oder Ethyl bedeuten.

Ferner ist es bevorzugt, daß, wenn in den erfindungsgemäßen Verbindungen der allgemeinen Formel (I) $R^3$ $COR^{13}$ bedeutet,

$R^{13}$ unsubstituiertes oder mit O-Methyl, O-Ethyl, O-$(CH_2)_2$-$OCH_3$, O-Benzyl, O-Phenyl, wobei Phenyl unsubstituiert oder mit F, Cl, Br, I, -CN substituiert ist, O-C(=O)-Methyl, O-C(=O)-Ethyl substituiertes Methyl, Ethyl, C(=O) OMethyl, n-Propyl, iso-Propyl, 2-Methylpropyl, n-Butyl, tert.-Butyl, n-Pentyl oder 3-Methylbutyl, insbesondere Methyl, Ethyl, n-Propyl, n-Butyl, tert.-Butyl, n-Pentyl, $CH_2$-O-$CH_2$-$CH_2$-$OCH_3$, $CH(CH_3)$-O-Phenyl, $CH_2$-$CH_2$-C (=O)$OCH_3$, $C(CH_3)_2$-OC(=O)$CH_3$, $CH_2$-O-Benzyl, $CH_2$-O-(3-Chlorphenyl), $CH_2$-$CH_2$-$CH_2$-O-Phenyl, CH(OC (=O)Methyl)$CH_3$;
Cyclopropyl, 2-Phenylcyclopropyl, 1-Adamantyl;
unsubstituiertes oder mit F, Cl, Br, I, -CN, Phenyl, Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy, $OCF_3$, $OCHF_2$, $OCH_2F$, $SCF_3$, $SCHF_2$, $SCH_2F$, $SCH_3$, $CH_2OC$(=O)Phenyl, -$N(CH_3)$ substituiertes Phenyl oder Naphthyl, insbesondere 4-Fluorphenyl, 2-Chlorphenyl, 3-Chlorphenyl, 4-Bromphenyl, 4-Phenylphenyl (4-Biphenyl), 4-Ethylphenyl, 4-$CF_3$-Phenyl, 4-Methoxyphenyl, 2-Ethoxyphenyl, 4-tert.-Butyl, 3-$OCF_3$-Phenyl, 4-$OCF_3$-Phenyl, 4-$SCF_3$-Phenyl, 3-$SCF_2$-Phenyl, 2-$CH_2$-OC(=O)Phenyl, 3-Dimethylaminophenyl, 2,3-Dichlorphenyl, 2,4-Difluorphenyl, 3-Chlor-4-fluorphenyl, 3-Chlor-2-fluorphenyl, 4-$CF_3$-3-Fluorphenyl, 3-$CF_3$-6-Fluorphenyl, 4-Brom-3-methylphenyl, 2-Chlor-4-nitrophenyl, 2,3,4,5,6-Pentafluorphenyl, 2,6-Difluor-3-methylphenyl, 2,3-Difluor-4-methylphenyl, 2-Chlor-5-methyl-6-fluorphenyl, 1-Naphthyl, 2-Naphthyl;
unsubstituiertes oder substituiertes ($C_{1-2}$-Alkyl)-Aryl, insbesondere Benzyl, Phenethyl, CH($C_2H_5$)-Phenyl, CH (NH-$SO_2$-(4-Methylphenyl))-$CH_2$-Phenyl, CH=CH-Phenyl, CH=CH-(3-Trifluorphenyl); oder
unsubstituiertes oder Alkyl-substituiertes Furanyl, Benzofuranyl, unsubstiuiertes oder mit Alkyl, $CF_3$, Aryl, O-Phenyl, Chlor, S-Methyl, S-Ethyl substituiertes Thienyl, Pyridinyl, Pyrazolyl, Benzodihydropyranyl, Isooxazolyl, insbesondere 1,5-Diemthylfuran-3-yl, 2-Methyl-5-tert.-butyl-furan-3-yl, 3-Chlorthien-2-yl 1-(4-Chlorphenyl)-5-trifluormethyl-pyrazol-4-yl, 1-Methyl-3-tert.-butyl-pyrazol-5-yl,

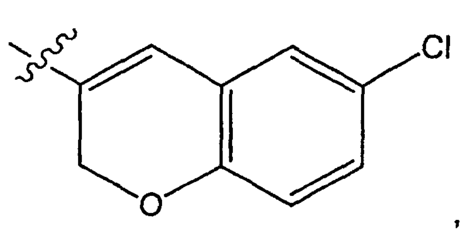

,

Pyridin-4-yl, 2-Methylthiopyridin-3-yl, 2-Ethylthiopyridin-3-yl, 2-Phenoxypyridin-3-yl, 2-Chlorpyridin-3-yl, 5-Methyl-3-(2,6-Dichlorphenyl)-isoxazol-4-yl, 5-Methyl-3-(2-Chlor-6-fluorphenyl)-isoxazol-4-yl bedeutet.

Ausgewählte und besonders bevorzugte erfindungsgemäße Verbindungen der Formel (I) sind:

- 3-(1H-Indol-3-yl)-2-{[8-(4-trifluoromethoxy-benzolsulfonyl)-1-oxa-2,8-diaza-spiro[4.5]dec-2-en-3-carbonyl]-amino}-propionsäuremethylester
- {4-[(8-Phenylmethanesulfonyl-1-oxa-2,8-diaza-spiro[4.5]dec-2-ene-3-carbonyl)-amino]-benzyl}-phosphonsäurediethylester
- (4-Cycloheptyl-piperazin-1-yl)-[8-(thiophene-2-sulfonyl)-1-oxa-2,8-diaza-spiro[4.5]dec-2-en-3-yl]-methanon
- [8-(5-Chloro-thiophen-2-sulfonyl)-1-oxa-2,8-diaza-spiro[4.5]dec-2-en-3-yl]-(4-phenyl-piperazin-1-yl)-methanon
- 8-(2-Chloro-4-nitro-benzoyl)-1-oxa-2,8-diaza-spiro[4.5]dec-2-en-3-carbonsäurecyclopentylamid
- (4-Naphthalin-2-ylmethyl-piperazin-1-yl)-(8-phenylmethansulfonyl-1-oxa-2,8-diaza-spiro[4.5]dec-2-en-3-yl)-methanon
- 8-Dimethylsulfamoyl-1-oxa-2,8-diaza-spiro[4.5]dec-2-ene-3-carbonsäurephenethylamid
- 8-(Thiophen-2-sulfonyl)-1-oxa-2,8-diaza-spiro[4.5]dec-2-ene-3-carbonsäure(1-phenyl-ethyl)-amid
- 8-(Toluol-4-sulfonyl)-1-oxa-2,8-diaza-spiro[4.5]dec-2-ene-3-carbonsäure-[2-(3,4-Dichloro-phenyl)-ethyl]-amid
- 4-Diethylamino-benzoesäure N'-[8-(thiophen-2-sulfonyl)-1-oxa-2,8-diaza-spiro[4.5]dec-2-en-3-carbonyl]-hydrazide
- 8-(3-Chloro-4-fluoro-benzolsulfonyl)-1-oxa-2,8-diaza-spiro[4.5]dec-2-ene-3-carbonsäure(2-pyrrolidin-1-yl-ethyl)-amid
- 8-Phenylmethansulfonyl-1-oxa-2,8-diaza-spiro[4.5]dec-2-en-3-carbonsäurephenethyl-amid
- 8-Benzolsulfonyl-1-oxa-2,8-diaza-spiro[4.5]dec-2-ene-3-carbonsäure-(2-dimethylamino-ethyl)-amid
- [4-(3-Phenyl-propyl)-piperazin-1-yl]-[8-(thiophen-2-sulfonyl)-1-oxa-2,8-diaza-spiro[4.5]dec-2-en-3-yl]-methanon
- 8-(2,5-Dichloro-benzolsulfonyl)-1-oxa-2,8-diaza-spiro[4.5]dec-2-en-3-carbonsäure-[3-(2-methyl-piperidin-1-yl)-propyl]-amid
- 8-(4-Trifluoromethoxy-benzolsulfonyl)-1-oxa-2,8-diaza-spiro[4.5]dec-2-en-3-carbonsäure-[2-(1H-indol-3-yl)-ethyl]-methyl-amid
- 8-(5-Fluoro-2-methyl-benzolsulfonyl)-1-oxa-2,8-diaza-spiro[4.5]dec-2-ene-3-carbonsäure-(2-acetylamino-ethyl)-amid
- 8-(Toluol-4-sulfonyl)-1-oxa-2,8-diaza-spiro[4.5]dec-2-ene-3-carbonsäurebenzyl-phenethyl-amid
- 8-(2-Methyl-5-nitro-benzolsulfonyl)-1-oxa-2,8-diaza-spiro[4.5]dec-2-en-3-carbonsäure-(2-dimethylamino-ethyl)-amid
- 8-Phenylmethansulfonyl-1-oxa-2,8-diaza-spiro[4.5]dec-2-ene-3-carbonsäure-(2-benzyloxy-cyclopentyl)-amid
- 8-(Thiophen-2-sulfonyl)-1-oxa-2,8-diaza-spiro[4.5]dec-2-ene-3-carbonsäure-(2,3-dihydro-benzo[1,4]dioxin-2-ylmethyl)-amid
- 8-(Toluol-4-sulfonyl)-1-oxa-2,8-diaza-spiro[4.5]dec-2-ene-3-carbonsäure-(4-phenoxy-phenyl)-amid
- 8-(Thiophen-2-sulfonyl)-1-oxa-2,8-diaza-spiro[4.5]dec-2-ene-3-carbonsäure-(1-p-tolyl-ethyl)-amid
- 8-(Thiophen-2-sulfonyl)-1-oxa-2,8-diaza-spiro[4.5]dec-2-ene-3-carbonsäure-3-fluoro-benzylamid
- 2-Phenyl-1-{4-[8-(toluol-4-sulfonyl)-1-oxa-2,8-diaza-spiro[4.5]dec-2-ene-3-carbonyl]-piperazin-1-yl}-ethanone
- 8-(Thiophen-2-sulfonyl)-1-oxa-2,8-diaza-spiro[4.5]dec-2-ene-3-carbonsäure-[1-(4-trifluoromethyl-benzyl)-pyrrolidin-3-yl]-amid
- [8-(Thiophen-2-sulfonyl)-1-oxa-2,8-diaza-spiro[4.5]dec-2-en-3-yl]-(4-o-tolyl-piperazin-1-yl)-methanon
- 8-(Toluol-4-sulfonyl)-1-oxa-2,8-diaza-spiro[4.5]dec-2-ene-3-carbonsäure-benzyl-(2-cyano-ethyl)-amid
- [8-(2,5-Dichloro-benzolsulfonyl)-1-oxa-2,8-diaza-spiro[4.5]dec-2-en-3-yl]-(4-thieno[2,3-d]pyrimidin-4-yl-piperazin-1-yl)-methanon
- 8-(5-Chloro-thiophen-2-sulfonyl)-1-oxa-2,8-diaza-spiro[4.5]dec-2-ene-3-carbonsäure-(benzo[1,3]dioxol-5-ylmethyl)-amid
- (3-Methyl-4-m-tolyl-piperazin-1-yl)-[8-(thiophen-2-sulfonyl)-1-oxa-2,8-diaza-spiro[4.5]dec-2-en-3-yl]-methanon
- [4-(1-Phenyl-ethyl)-piperazin-1-yl]-[8-(toluol-4-sulfonyl)-1-oxa-2,8-diaza-spiro[4.5]dec-2-en-3-yl]-methanon
- [4-(2,4-Dimethyl-phenyl)-piperazin-1-yl]-[8-(toluol-4-sulfonyl)-1-oxa-2,8-diaza-spiro[4.5]dec-2-en-3-yl]-methanon
- 8-(2-Chloro-pyridine-3-carbonyl)-1-oxa-2,8-diaza-spiro[4.5]dec-2-ene-3-carbonsäure-cyclopentylamid
- (4-Naphthalen-2-ylmethyl-piperazin-1-yl)-[8-(3-trifluoromethyl-benzolsulfonyl)-1-oxa-2,8-diaza-spiro[4.5]dec-2-en-3-yl]-methanon
- 8-[3-Phenyl-2-(toluol-4-sulfonylamino)-propionyl]-1-oxa-2,8-diaza-spiro[4.5]dec-2-ene-3-carbonsäure-(pyridin-3-ylmethyl)-amid
- 8-(5-Fluoro-2-methyl-benzolsulfonyl)-1-oxa-2,8-diaza-spiro[4.5]dec-2-ene-3-carbonsäure-(2-dimethylamino-ethyl)-amid

- 8-(4-Nitro-benzolsulfonyl)-1-oxa-2,8-diaza-spiro[4.5]dec-2-ene-3-carbonsäure(thiophen-2-ylmethyl)-amid
- 8-(2,5-Dichloro-benzolsulfonyl)-1-oxa-2,8-diaza-spiro[4.5]dec-2-ene-3-carbonsäure-3-fluoro-5-trifluoromethyl-benzylamid
- 8-(5-Chloro-thiophen-2-sulfonyl)-1-oxa-2,8-diaza-spiro[4.5]dec-2-ene-3-carbonsäurebenzhydryl-amid
- 8-[3-Phenyl-2-(toluol-4-sulfonylamino)-propionyl]-1-oxa-2,8-diaza-spiro[4.5]dec-2-ene-3-carbonsäure-(2-cyano-ethyl)-amid • [(8-Phenylmethanesulfonyl-1-oxa-2,8-diaza-spiro[4.5]dec-2-ene-3-carbonyl)-amino]-essigsäureethylester
- 8-(3-Dimethylamino-benzoyl)-1-oxa-2,8-diaza-spiro[4.5]dec-2-ene-3-carbonsäure(2-cyano-ethyl)-amid
- 8-(2,5-Dichloro-benzolsulfonyl)-1-oxa-2,8-diaza-spiro[4.5]dec-2-ene-3-carbonsäure-[1-(naphthalin-2-ylcarbamoyl)-ethyl]-amid
- 8-(Toluol-4-sulfonyl)-1-oxa-2,8-diaza-spiro[4.5]dec-2-ene-3-carbonsäure-(1-p-tolyl-ethyl)-amid
- [4-(4-Chloro-phenyl)-piperazin-1-yl]-(8-phenylmethanesulfonyl-1-oxa-2,8-diaza-spiro[4.5]dec-2-en-3-yl)-methanon
- 8-(Thiophen-2-sulfonyl)-1-oxa-2,8-diaza-spiro[4.5]dec-2-ene-3-carbonsäure-(2-benzyloxy-cyclopentyl)-amid
- 8-Acetyl-1-oxa-2,8-diaza-spiro[4.5]dec-2-ene-3-carbonsäurebenzylamid
- 8-(5-Chloro-thiophen-2-sulfonyl)-1-oxa-2,8-diaza-spiro[4.5]dec-2-ene-3-carbonsäure-2,5-difluoro-benzylamid
- 8-(5-Chloro-thiophen-2-sulfonyl)-1-oxa-2,8-diaza-spiro[4.5]dec-2-ene-3-carbonsäure-(1-naphthalin-2-ylmethyl-pyrrolidin-3-yl)-amid
- 8-Phenylmethanesulfonyl-1-oxa-2,8-diaza-spiro[4.5]dec-2-ene-3-carbonsäure-2-ethoxy-benzylamid
- 8-(4-Ethyl-benzoyl)-1-oxa-2,8-diaza-spiro[4.5]dec-2-ene-3-carbonsäure-(thiophen-2-ylmethyl)-amid
- 2-{[8-(2,5-Dichloro-benzolsulfonyl)-1-oxa-2,8-diaza-spiro[4.5]dec-2-ene-3-carbonyl]-amino}-bernsteinsäure-1-allylester-4-tert-butylester
- 8-(4-Trifluoromethoxy-benzolsulfonyl)-1-oxa-2,8-diaza-spiro[4.5]dec-2-ene-3-carbonsäure-naphthalin-2-ylamid
- 4-{[8-(2-Phenyl-cyclopropanecarbonyl)-1-oxa-2,8-diaza-spiro[4.5]dec-2-ene-3-carbonyl]-amino}-piperidine-1-carbonsäureethylester
- 4-{[8-(2,4-Difluoro-benzoyl)-1-oxa-2,8-diaza-spiro[4.5]dec-2-ene-3-carbonyl]-amino}-piperidine-1-carbonsäureethylester
- 8-(Toluol-4-sulfonyl)-1-oxa-2,8-diaza-spiro[4.5]dec-2-ene-3-carbonsäure(2-dimethylamino-ethyl)-amid
- 8-Dimethylsulfamoyl-1-oxa-2,8-diaza-spiro[4.5]dec-2-ene-3-carbonsäure[2-(4-chloro-phenyl)-ethyl]-amid
- 8-(4-Methoxy-benzoyl)-1-oxa-2,8-diaza-spiro[4.5]dec-2-ene-3-carbonsäure(5-methyl-furan-2-ylmethyl)-amid
- 8-(Thiophen-2-sulfonyl)-1-oxa-2,8-diaza-spiro[4.5]dec-2-ene-3-carbonsäure-[2-(4-chloro-phenyl)-ethyl]-amid
- 8-(5-Chloro-thiophen-2-sulfonyl)-1-oxa-2,8-diaza-spiro[4.5]dec-2-ene-3-carbonsäure-[2-(1-methyl-pyrrolidin-2-yl)-ethyl]-amid
- 4-{[8-(4-Bromo-benzoyl)-1-oxa-2,8-diaza-spiro[4.5]dec-2-ene-3-carbonyl]-amino}-piperidine-1-carbonsäureethylester
- 8-(Toluol-4-sulfonyl)-1-oxa-2,8-diaza-spiro[4.5]dec-2-ene-3-carbonsäure-3,5-Dichloro-benzylamid
- 8-(2,5-Dichloro-benzolsulfonyl)-1-oxa-2,8-diaza-spiro[4.5]dec-2-ene-3-carbonsäure-[1-(3-methoxy-benzyl)-pyrrolidin-3-yl]-amid
- 3-(4-Naphthalin-2-ylmethyl-piperazine-1-carbonyl)-1-oxa-2,8-diaza-spiro[4.5]dec-2-ene-8-sulfonsäure dimethylamid
- 3-{[8-(7,7-Dimethyl-2-oxo-bicyclo[2.2.1]hept-1-ylmethanesulfonyl)-1-oxa-2,8-diaza-spiro[4.5]dec-2-ene-3-carbonyl]-amino}-propionsäureethylester
- 8-(Toluol-4-sulfonyl)-1-oxa-2,8-diaza-spiro[4.5]dec-2-ene-3-carbonsäure(2-benzyloxy-cyclopentyl)-amid
- 8-(2,5-Dichloro-benzolsulfonyl)-1-oxa-2,8-diaza-spiro[4.5]dec-2-ene-3-carbonsäure3,4-dimethoxy-benzylamid
- (8-Phenylmethanesulfonyl-1-oxa-2,8-diaza-spiro[4.5]dec-2-en-3-yl)-[4-(3-phenyl-propyl)-piperazin-1-yl]-methanon
- 3-(4-Thieno[2,3-d]pyrimidin-4-yl-piperazine-1-carbonyl)-1-oxa-2,8-diaza-spiro[4.5]dec-2-ene-8-sulfonsäuredimethylamid
- [8-(5-Chloro-thiophen-2-sulfonyl)-1-oxa-2,8-diaza-spiro[4.5]dec-2-en-3-yl]-[4-(3,5-dimethoxy-phenyl)-piperazin-1-yl]-methanon
- 8-Dimethylsulfamoyl-1-oxa-2,8-diaza-spiro[4.5]dec-2-ene-3-carbonsäure-3-fluoro-4-trifluoromethyl-benzylamid
- 8-(Toluol-4-sulfonyl)-1-oxa-2,8-diaza-spiro[4.5]dec-2-ene-3-carbonsäure-(2-benzyloxy-cyclopentyl)-amid
- 4-[(8-Butyryl-1-oxa-2,8-diaza-spiro[4.5]dec-2-ene-3-carbonyl)-amino]-piperidine-1-carbonsäureethylester
- 8-(Toluol-4-sulfonyl)-1-oxa-2,8-diaza-spiro[4.5]dec-2-ene-3-carbonsäure-(2-phenyl-cyclopropyl)-amid
- [8-(4-Fluoro-benzolsulfonyl)-1-oxa-2,8-diaza-spiro[4.5]dec-2-en-3-yl]-[4-(5-methyl-pyrazine-2-carbonyl)-piperazin-1-yl]-methanon
- 8-Dimethylsulfamoyl-1-oxa-2,8-diaza-spiro[4.5]dec-2-ene-3-carbonsäure-2,4-difluoro-benzylamid
- 8-Phenylmethanesulfonyl-1-oxa-2,8-diaza-spiro[4.5]dec-2-ene-3-carbonsäure-[2-(3-fluoro-phenyl)-ethyl]-amid

- 8-(5-Chloro-thiophen-2-sulfonyl)-1-oxa-2,8-diaza-spiro[4.5]dec-2-ene-3-carbonsäure-(2,2-diphenyl-ethyl)-amid
- 3-[4-(3-Phenyl-propyl)-piperazine-1-carbonyl]-1-oxa-2,8-diaza-spiro[4.5]dec-2-ene-8-sulfonsäuredimethylamid
- 3-{[8-(3,5-Difluoro-benzoyl)-1-oxa-2,8-diaza-spiro[4.5]dec-2-ene-3-carbonyl]-amino}-propionsäureethyl ester
- 8-(Propane-1-sulfonyl)-1-oxa-2,8-diaza-spiro[4.5]dec-2-ene-3-carbonsäureisobutyl-amid
- [4-(3-Phenyl-propyl)-piperazin-1-yl]-[8-(3-trifluoromethyl-benzolsulfonyl)-1-oxa-2,8-diaza-spiro[4.5]dec-2-en-3-yl]-methanon
- 8-(Thiophen-2-sulfonyl)-1-oxa-2,8-diaza-spiro[4.5]dec-2-ene-3-carbonsäure-[1-(2,3-dihydro-benzo[1,4]dioxin-5-yl)-ethyl]-amid
- 8-Butyryl-1-oxa-2,8-diaza-spiro[4.5]dec-2-ene-3-carbonsäurephenylamid
- 8-(4-Trifluoromethoxy-benzolsulfonyl)-1-oxa-2,8-diaza-spiro[4.5]dec-2-ene-3-carbonsäurecyclooctylamid
- 8-[2-(2-Methoxy-ethoxy)-acetyl]-1-oxa-2,8-diaza-spiro[4.5]dec-2-ene-3-carbonsäurebenzylamid
- 8-(4-Bromo-3-methyl-benzoyl)-1-oxa-2,8-diaza-spiro[4.5]dec-2-ene-3-carbonsäureisobutyl-amid
- 8-(5-Chloro-thiophen-2-sulfonyl)-1-oxa-2,8-diaza-spiro[4.5]dec-2-ene-3-carbonsäure-[3-(methyl-phenyl-amino)-propyl]-amid
- 4-{[8-(2-Methyl-5-nitro-benzolsulfonyl)-1-oxa-2,8-diaza-spiro[4.5]dec-2-ene-3-carbonyl]-amino}-piperidine-1-carbonsäureethylester
- [8-(5-Chloro-thiophen-2-sulfonyl)-1-oxa-2,8-diaza-spiro[4.5]dec-2-en-3-yl]-[4-(4-fluoro-phenyl)-piperazin-1-yl]-methanon
- 8-(2,5-Dichloro-benzolsulfonyl)-1-oxa-2,8-diaza-spiro[4.5]dec-2-ene-3-carbonsäure-methyl-pyridin-3-ylmethyl-amid
- 2-(3,4-Difluoro-phenyl)-1-{4-[8-(3-trifluoromethyl-benzolsulfonyl)-1-oxa-2,8-diaza-spiro[4.5]dec-2-ene-3-carbonyl]-piperazin-1-yl}-ethanon
- 8-(3-Trifluoromethyl-benzolsulfonyl)-1-oxa-2,8-diaza-spiro[4.5]dec-2-ene-3-carbonsäurecyclopropylmethyl-amid
- 3-{[8-(2-Methyl-5-nitro-benzolsulfonyl)-1-oxa-2,8-diaza-spiro[4.5]dec-2-ene-3-carbonyl]-amino}-propionsäureethylester
- 8-[2-(3-Chloro-phenoxy)-acetyl]-1-oxa-2,8-diaza-spiro[4.5]dec-2-ene-3-carbonsäure-(thiophen-2-ylmethyl)-amid
- 8-(4-Trifluoromethoxy-benzolsulfonyl)-1-oxa-2,8-diaza-spiro[4.5]dec-2-ene-3-carbonsäurebenzyl-phenethyl-amid
- 8-(2,5-Dichloro-benzolsulfonyl)-1-oxa-2,8-diaza-spiro[4.5]dec-2-ene-3-carbonsäurebenzyl-phenethyl-amid
- 8-(2,5-Dichloro-benzolsulfonyl)-1-oxa-2,8-diaza-spiro[4.5]dec-2-ene-3-carbonsäure-(2-cyano-ethyl)-pyridin-3-yl-methyl-amid
- 8-(2,5-Dichloro-benzolsulfonyl)-1-oxa-2,8-diaza-spiro[4.5]dec-2-ene-3-carbonsäure-(2-cyano-ethyl)-(tetrahydro-furan-2-ylmethyl)-amid
- 8-(3-Trifluoromethyl-benzolsulfonyl)-1-oxa-2,8-diaza-spiro[4.5]dec-2-ene-3-carbonsäure-methyl-pyridin-3-ylmethyl-amid
- 8-(Pyridine-4-carbonyl)-1-oxa-2,8-diaza-spiro[4.5]dec-2-ene-3-carbonsäure-benzylamid
- 8-(4-Trifluoromethoxy-benzolsulfonyl)-1-oxa-2,8-diaza-spiro[4.5]dec-2-ene-3-carbonsäureethyl-(2-methyl-allyl)-amid
- 8-(4-Methoxy-benzolsulfonyl)-1-oxa-2,8-diaza-spiro[4.5]dec-2-ene-3-carbonsäure-[2-(2-fluoro-phenyl)-ethyl]-amid
- 8-(5-tert-Butyl-2-methyl-furan-3-carbonyl)-1-oxa-2,8-diaza-spiro[4.5]dec-2-ene-3-carbonsäureisobutyl-amid
- 8-(3-Phenyl-acryloyl)-1-oxa-2,8-diaza-spiro[4.5]dec-2-ene-3-carbonsäure-(thiophen-2-ylmethyl)-amid
- 8-(4-Trifluoromethyl-benzoyl)-1-oxa-2,8-diaza-spiro[4.5]dec-2-ene-3-carbonsäurecyclopropylmethyl-amid
- 8-(4-Trifluoromethylsulfanyl-benzoyl)-1-oxa-2,8-diaza-spiro[4.5]dec-2-ene-3-carbonsäurecyclopropylmethyl-amid
- 8-(4-Bromo-benzoyl)-1-oxa-2,8-d iaza-spiro[4.5]dec-2-ene-3-carbonsäure-(5-methyl-furan-2-ylmethyl)-amid
- 8-(3-Fluoro-4-trifluoromethyl-benzoyl)-1-oxa-2,8-diaza-spiro[4.5]dec-2-ene-3-carbonsäurecyclopropylmethyl-amid
- 8-(7,7-Dimethyl-2-oxo-bicyclo[2.2.1]hept-1-ylmethanesulfonyl)-1-oxa-2,8-diaza-spiro[4.5]dec-2-ene-3-carbonsäure-(2-ethylsulfanyl-ethyl)-amid
- 8-(2,4-Difluoro-benzoyl)-1-oxa-2,8-diaza-spiro[4.5]dec-2-ene-3-carbonsäure-(tetrahydro-furan-2-ylmethyl)-amid
- {[8-(3-Chloro-thiophen-2-carbonyl)-1-oxa-2,8-diaza-spiro[4.5]dec-2-ene-3-carbonyl]-amino}-essigsäureethylester
- 4-Oxo-4-{3-[(thiophen-2-ylmethyl)-carbamoyl]-1-oxa-2,8-diaza-spiro[4.5]dec-2-en-8-yl}-buttersäuremethylester
- 8-(2-Ethylsulfanyl-pyridine-3-carbonyl)-1-oxa-2,8-diaza-spiro[4.5]dec-2-ene-3-carbonsäurebenzylamid
- 3-{[8-(2-Chloro-benzolsulfonyl)-1-oxa-2,8-diaza-spiro[4.5]dec-2-ene-3-carbonyl]-amino}-propionsäureethylester
- 8-(4-Trifluoromethoxy-benzoyl)-1-oxa-2,8-diaza-spiro[4.5]dec-2-ene-3-carbonsäurecyclopropylmethyl-amid
- 8-(4-Phenoxy-butyryl)-1-oxa-2,8-diaza-spiro[4.5]dec-2-ene-3-carbonsäurecyclopropylmethyl-amid

- [8-(3-Trifluoromethyl-benzolsulfonyl)-1-oxa-2,8-diaza-spiro[4.5]dec-2-en-3-yl]-[4-(4-trifluoromethyl-pyridin-2-yl)-piperazin-1-yl]-methanon
- 8-(2-Chloro-5-trifluoromethyl-benzoyl)-1-oxa-2,8-diaza-spiro[4.5]dec-2-ene-3-carbonsäurecyclopropylmethyl-amid
- 8-(4-Methoxy-benzolsulfonyl)-1-oxa-2,8-diaza-spiro[4.5]dec-2-ene-3-carbonsäure(2-benzyloxy-cyclopentyl)-amid
- 4-{[8-(3-Trifluoromethyl-benzolsulfonyl)-1-oxa-2,8-diaza-spiro[4.5]dec-2-ene-3-carbonyl]-amino}-piperidine-1-carbonsäureethylester
- 8-(2-Phenoxy-propionyl)-1-oxa-2,8-diaza-spiro[4.5]dec-2-ene-3-carbonsäurecyclopropylmethyl-amid
- 8-(4-Fluoro-benzolsulfonyl)-1-oxa-2,8-diaza-spiro[4.5]dec-2-ene-3-carbonsäure-[2-(3-methoxy-phenyl)-ethyl]-amid
- 8-(2-Methylsulfanyl-pyridine-3-carbonyl)-1-oxa-2,8-diaza-spiro[4.5]dec-2-ene-3-carbonsäureisobutyl-amid
- 8-(5-tert-Butyl-2-methyl-furan-3-carbonyl)-1-oxa-2,8-diaza-spiro[4.5]dec-2-ene-3-carbonsäurecyclopentylamid
- 8-(4-Chloro-benzolsulfonyl)-1-oxa-2,8-diaza-spiro[4.5]dec-2-ene-3-carbonsäure-(thiophen-2-ylmethyl)-amid
- 8-(5-Chloro-thiophen-2-sulfonyl)-1-oxa-2,8-diaza-spiro[4.5]dec-2-ene-3-carbonsäure-(naphthalin-1-ylmethyl)-amid
- 8-(4-Trifluoromethoxy-benzolsulfonyl)-1-oxa-2,8-diaza-spiro[4.5]dec-2-ene-3-carbonsäure-[2-(4-fluoro-phenyl)-ethyl]-amid
- 8-(4-Bromo-3-methyl-benzoyl)-1-oxa-2,8-diaza-spiro[4.5]dec-2-ene-3-carbonsäure-(pyridin-4-ylmethyl)-amid
- 8-(4-Methoxy-benzoyl)-1-oxa-2,8-diaza-spiro[4.5]dec-2-ene-3-carbonsäurebenzylamid
- 8-(3-Trifluoromethyl-benzolsulfonyl)-1-oxa-2,8-diaza-spiro[4.5]dec-2-ene-3-carbonsäure-3-chloro-benzylamid
- (8-Phenylmethanesulfonyl-1-oxa-2,8-diaza-spiro[4.5]dec-2-en-3-yl)-(4-o-tolyl-piperazin-1-yl)-methanon
- (8-Phenylmethanesulfonyl-1-oxa-2,8-diaza-spiro[4.5]dec-2-en-3-yl)-(4-pyridin-2-yl-piperazin-1-yl)-methanon
- 8-(4-Trifluoromethylsulfanyl-benzoyl)-1-oxa-2,8-diaza-spiro[4.5]dec-2-ene-3-carbonsäure-(thiophen-2-ylmethyl)-amid
- 8-(Thiophen-2-sulfonyl)-1-oxa-2,8-diaza-spiro[4.5]dec-2-ene-3-carbonsäurebenzylamid
- 8-(4-Fluoro-benzoyl)-1-oxa-2,8-diaza-spiro[4.5]dec-2-ene-3-carbonsäureisobutyl-amid
- 2-(3-Isobutylcarbamoyl-1-oxa-2,8-diaza-spiro[4.5]dec-2-ene-8-sulfonyl)-benzoesäuremethylester
- 2-[(8-Dimethylsulfamoyl-1-oxa-2,8-diaza-spiro[4.5]dec-2-ene-3-carbonyl)-amino]-3-phenyl-propionsäure-tert-butylester
- 8-(5-Chloro-thiophen-2-sulfonyl)-1-oxa-2,8-diaza-spiro[4.5]dec-2-ene-3-carbonsäure-[1-(2,3-dihydro-benzo[1,4]dioxin-5-yl)-ethyl]-amid
- 8-(4-Bromo-benzoyl)-1-oxa-2,8-diaza-spiro[4.5]dec-2-ene-3-carbonsäure-(thiophen-2-ylmethyl)-amid
- 8-(4-Fluoro-benzolsulfonyl)-1-oxa-2,8-diaza-spiro[4.5]dec-2-ene-3-carbonsäure-(3-imidazol-1-yl-propyl)-amid
- 4-[8-(4-Fluoro-benzolsulfonyl)-1-oxa-2,8-diaza-spiro[4.5]dec-2-ene-3-carbonyl]-piperazine-1-carbonsäurebenzylester
- 3-(4-Cycloheptyl-piperazine-1-carbonyl)-1-oxa-2,8-diaza-spiro[4.5]dec-2-ene-8-sulfonsäuredimethylamid
- 4-Methyl-2-{[8-(thiophen-2-sulfonyl)-1-oxa-2,8-diaza-spiro[4.5]dec-2-ene-3-carbonyl]-amino}-pentansäure-tert-butylester
- 8-(3-Chloro-2-fluoro-benzoyl)-1-oxa-2,8-diaza-spiro[4.5]dec-2-ene-3-carbonsäure-(thiophen-2-ylmethyl)-amid
- 2-{[8-(4-Methoxy-benzolsulfonyl)-1-oxa-2,8-diaza-spiro[4.5]dec-2-ene-3-carbonyl]-amino}-4-methyl-pentansäure-tert-butylester
- 3-{[8-(6-Chloro-2-fluoro-3-methyl-benzoyl)-1-oxa-2,8-diaza-spiro[4.5]dec-2-ene-3-carbonyl]-amino}-propionsäureethylester
- 8-(2-Phenoxy-propionyl)-1-oxa-2,8-diaza-spiro[4.5]dec-2-ene-3-carbonsäurebenzylamid
- 8-(5-Fluoro-2-methyl-benzolsulfonyl)-1-oxa-2,8-diaza-spiro[4.5]dec-2-ene-3-carbonsäure-(thiophen-2-ylmethyl)-amid
- [8-(5-Chloro-thiophen-2-sulfonyl)-1-oxa-2,8-diaza-spiro[4.5]dec-2-en-3-yl]-[4-(2-methoxy-phenyl)-piperidin-1-yl]-methanon
- 8-(2-Chloro-benzoyl)-1-oxa-2,8-diaza-spiro[4.5]dec-2-ene-3-carbonsäure-(thiophen-2-ylmethyl)-amid
- 8-(2-Phenoxy-pyridine-3-carbonyl)-1-oxa-2,8-diaza-spiro[4.5]dec-2-ene-3-carbonsäurecyclopentylamid
- 8-(4-Bromo-benzoyl)-1-oxa-2,8-diaza-spiro[4.5]dec-2-ene-3-carbonsäure-(2-ethylsulfanyl-ethyl)-amid
- 1-[8-(5-Chloro-thiophen-2-sulfonyl)-1-oxa-2,8-diaza-spiro[4.5]dec-2-ene-3-carbonyl]-piperidine-2-carbonsäureethylester
- 8-(2-Chloro-benzolsulfonyl)-1-oxa-2,8-diaza-spiro[4.5]dec-2-ene-3-carbonsäure-(thiophen-2-ylmethyl)-amid
- 8-(2,3-Dichloro-benzoyl)-1-oxa-2,8-diaza-spiro[4.5]dec-2-ene-3-carbonsäurecyclopropylmethyl-amid
- 8-Hexanoyl-1-oxa-2,8-diaza-spiro[4.5]dec-2-ene-3-carbonsäure-cyclopropylmethyl-amid
- 8-(2,3-Difluoro-4-methyl-benzoyl)-1-oxa-2,8-diaza-spiro[4.5]dec-2-ene-3-carbonsäurecyclopropylmethyl-amid

- 8-(4-Fluoro-benzolsulfonyl)-1-oxa-2,8-diaza-spiro[4.5]dec-2-ene-3-carbonsäure-(2,5-dimethoxy-benzyl)-furan-2-ylmethyl-amid
- 8-Dimethylsulfamoyl-1-oxa-2,8-diaza-spiro[4.5]dec-2-ene-3-carbonsäure-(2-ethylsulfanyl-ethyl)-amid
- 8-(4-Trifluoromethoxy-benzolsulfonyl)-1-oxa-2,8-diaza-spiro[4.5]dec-2-ene-3-carbonsäure-3-methoxy-benzyiamid
- 8-Dimethylsulfamoyl-1-oxa-2,8-diaza-spiro[4.5]dec-2-ene-3-carbonsäure-(2-benzyloxy-cyclopentyl)-amid
- 8-(4-Methoxy-benzolsulfonyl)-1-oxa-2,8-diaza-spiro[4.5]dec-2-ene-3-carbonsäure-[2-(4-chloro-phenyl)-1-methyl-ethyl]-amid
- 8-(4-Trifluoromethoxy-benzolsulfonyl)-1-oxa-2,8-diaza-spiro[4.5]dec-2-ene-3-carbonsäure-[2-(3,4-Dichloro-phenyl)-ethyl]-amid
- 8-[2-(3-Chloro-phenoxy)-acetyl]-1-oxa-2,8-diaza-spiro[4.5]dec-2-ene-3-carbonsäurebenzylamid
- 8-(4-Fluoro-benzolsulfonyl)-1-oxa-2,8-diaza-spiro[4.5]dec-2-ene-3-carbonsäure-(2-cyano-ethyl)-(2-pyridin-2-yl-ethyl)-amid
- 8-(5-Chloro-thiophen-2-sulfonyl)-1-oxa-2,8-diaza-spiro[4.5]dec-2-ene-3-carbonsäure-[2-(4-chloro-phenyl)-1-methyl-ethyl]-amid
- 8-(4-Methoxy-benzolsulfonyl)-1-oxa-2,8-diaza-spiro[4.5]dec-2-ene-3-carbonsäure-(2-benzyloxy-cyclopentyl)-amid
- 8-(2,6-Difluoro-3-methyl-benzoyl)-1-oxa-2,8-diaza-spiro[4.5]dec-2-ene-3-carbonsäure-(thiophen-2-ylmethyl)-amid
- 8-(2-Benzyloxy-acetyl)-1-oxa-2,8-diaza-spiro[4.5]dec-2-ene-3-carbonsäure-benzylamid
- 8-(2,3-Dichloro-benzoyl)-1-oxa-2,8-diaza-spiro[4.5]dec-2-ene-3-carbonsäurephenylamid
- 8-(5-Bromo-2-methoxy-benzolsulfonyl)-1-oxa-2,8-diaza-spiro[4.5]dec-2-ene-3-carbonsäurecyclopentylamid
- Essigsäure-2-(3-benzylcarbamoyl-1-oxa-2,8-diaza-spiro[4.5]dec-2-en-8-yl)-1,1-dimethyl-2-oxo-ethylester
- 8-[3-(2-Chloro-6-fluoro-phenyl)-5-methyl-isoxazole-4-carbonyl]-1-oxa-2,8-diaza-spiro[4.5]dec-2-ene-3-carbonsäure-(2-ethylsulfanyl-ethyl)-amid
- [4-(3-Phenyl-propyl)-piperazin-1-yl]-[8-(4-trifluoromethoxy-benzolsulfonyl)-1-oxa-2,8-diaza-spiro[4.5]dec-2-en-3-yl]-methanon
- 8-[3-(2-Chloro-6-fluoro-phenyl)-5-methyl-isoxazole-4-carbonyl]-1-oxa-2,8-diaza-spiro[4.5]dec-2-ene-3-carbonsäurebenzylamid
- 8-(Naphthalincarbonyl)-1-oxa-2,8-diaza-spiro[4.5]dec-2-ene-3-carbonsäureisobutyl-amid
- 1-[8-(5-tert-Butyl-2-methyl-furan-3-carbonyl)-1-oxa-2,8-diaza-spiro[4.5]dec-2-ene-3-carbonyl]-piperidine-2-carbonsäureethylester
- 8-(3-Difluoromethylsulfanyl-benzoyl)-1-oxa-2,8-diaza-spiro[4.5]dec-2-ene-3-carbonsäurebenzylamid
- 8-(4-Bromo-benzoyl)-1-oxa-2,8-diaza-spiro[4.5]dec-2-ene-3-carbonsäurephenylamid
- 8-(4-Methoxy-benzolsulfonyl)-1-oxa-2,8-diaza-spiro[4.5]dec-2-ene-3-carbonsäure(2-phenyl-cyclopropyl)-amid
- 8-(4-Phenoxy-butyryl)-1-oxa-2,8-diaza-spiro[4.5]dec-2-ene-3-carbonsäure(thiophen-2-ylmethyl)-amid
- 8-(3-Chloro-4-fluoro-benzoyl)-1-oxa-2,8-diaza-spiro[4.5]dec-2-ene-3-carbonsäure(thiophen-2-ylmethyl)-amid
- 8-(5-tert-Butyl-2-methyl-2H-pyrazole-3-carbonyl)-1-oxa-2,8-diaza-spiro[4.5]dec-2-ene-3-carbonsäure-(2-ethylsulfanyl-ethyl)-amid
- [8-(4-Fluoro-benzolsulfonyl)-1-oxa-2,8-diaza-spiro[4.5]dec-2-en-3-yl]-[4-(3-phenyl-propyl)-piperazin-1-yl]-methanon
- 8-(4-Trifluoromethoxy-benzoyl)-1-oxa-2,8-diaza-spiro[4.5]dec-2-ene-3-carbonsäure-(pyridin-4-ylmethyl)-amid
- Benzoe säure 2-(3-cyclopentylcarbamoyl-1-oxa-2,8-diaza-spiro[4.5]dec-2-ene-8-carbonyl)-benzyl ester
- 3-[4-(4-tert-Butyl-benzyl)-piperazine-1-carbonyl]-1-oxa-2,8-diaza-spiro[4.5]dec-2-ene-8-sulfonsäuredimethylamid
- {[8-(2-Ethoxy-benzoyl)-1-oxa-2,8-diaza-spiro[4.5]dec-2-ene-3-carbonyl]-amino}-essigsäureethylester
- 8-(5-Chloro-thiophen-2-sulfonyl)-1-oxa-2,8-diaza-spiro[4.5]dec-2-ene-3-carbonsäure-(2-benzyloxy-cyclopentyl)-amid
- 8-(Naphthalin-1-carbonyl)-1-oxa-2,8-diaza-spiro[4.5]dec-2-ene-3-carbonsäure-(thiophen-2-ylmethyl)-amid
- 8-(4-Fluoro-benzolsulfonyl)-1-oxa-2,8-diaza-spiro[4.5]dec-2-ene-3-carbonsäure-(3-phenyl-propyl)-amid
- 8-(5-Chloro-thiophen-2-sulfonyl)-1-oxa-2,8-d iaza-spiro[4.5]dec-2-ene-3-carbonsäure-(2,3-dihydro-benzo[1,4]dioxin-2-ylmethyl)-amid
- 1-[8-(4-tert-Butyl-benzoyl)-1-oxa-2,8-diaza-spiro[4.5]dec-2-ene-3-carbonyl]-piperidine-2-carbonsäure-ethyl ester
- 8-(4-Fluoro-benzolsulfonyl)-1-oxa-2,8-diaza-spiro[4.5]dec-2-ene-3-carbonsäure-[2-(3-trifluoromethyl-phenyl)-ethyl]-amid
- 8-[3-(2,6-Dichloro-phenyl)-5-methyl-isoxazole-4-carbonyl]-1-oxa-2,8-diaza-spiro[4.5]dec-2-ene-3-carbonsäure-(thiophen-2-ylmethyl)-amid
- 8-(4-Fluoro-benzolsulfonyl)-1-oxa-2,8-diaza-spiro[4.5]dec-2-ene-3-carbonsäure-[1-(naphthalin-2-ylcarbamoyl)-ethyl]-amid

- 8-(4-Fluoro-benzolsulfonyl)-1-oxa-2,8-diaza-spiro[4.5]dec-2-ene-3-carbonsäure-(2-benzyloxy-cyclopentyl)-amid
- 3-(4-Chloro-phenyl)-2-{[8-(4-methoxy-benzolsulfonyl)-1-oxa-2,8-diaza-spiro[4.5]dec-2-ene-3-carbonyl]-amino}-propionic säure methyl ester
- 8-(3-Trifluoromethyl-benzolsulfonyl)-1-oxa-2,8-diaza-spiro[4.5]dec-2-ene-3-carbonsäure-(2-benzyloxy-cyclopentyl)-amid
- 8-(3-Trifluoromethyl-benzolsulfonyl)-1-oxa-2,8-diaza-spiro[4.5]dec-2-ene-3-carbonsäure-(2-phenoxy-ethyl)-amid
- 8-(2-Phenoxy-pyridine-3-carbonyl)-1-oxa-2,8-diaza-spiro[4.5]dec-2-ene-3-carbonsäure-benzylamid
- 8-(Naphthalin-2-carbonyl)-1-oxa-2,8-diaza-spiro[4.5]dec-2-ene-3-carbonsäure-cyclopropylmethyl-amid
- 8-(3-Chloro-4-fluoro-benzoyl)-1-oxa-2,8-diaza-spiro[4.5]dec-2-ene-3-carbonsäure-benzylamid
- 8-(2-Phenyl-butyryl)-1-oxa-2,8-diaza-spiro[4.5]dec-2-ene-3-carbonsäure-(2-cyano-ethyl)-amid
- 8-(4-Fluoro-benzolsulfonyl)-1-oxa-2,8-diaza-spiro[4.5]dec-2-ene-3-carbonsäure-2-fluoro-benzylamid
- 8-(3-Chloro-benzoyl)-1-oxa-2,8-diaza-spiro[4.5]dec-2-ene-3-carbonsäure-(thiophen-2-ylmethyl)-amid
- (4-Benzyl-piperazin-1-yl)-[8-(2,5-Dichloro-benzolsulfonyl)-1-oxa-2,8-diaza-spiro[4.5]dec-2-en-3-yl]-methanon
- 8-(3-Chloro-benzolsulfonyl)-1-oxa-2,8-diaza-spiro[4.5]dec-2-ene-3-carbonsäure-benzylamid
- (4-Benzyl-piperidin-1-yl)-[8-(3-trifluoromethyl-benzolsulfonyl)-1-oxa-2,8-diaza-spiro[4.5]dec-2-en-3-yl]-methanon
- 8-(4-Bromo-benzoyl)-1-oxa-2,8-diaza-spiro[4.5]dec-2-ene-3-carbonsäure-benzylamid
- 8-(4-Fluoro-benzolsulfonyl)-1-oxa-2,8-diaza-spiro[4.5]dec-2-ene-3-carbonsäure-[1-(4-nitro-phenyl)-ethyl]-amid
- 4-Methyl-2-{[8-(3-trifluoromethyl-benzolsulfonyl)-1-oxa-2,8-diaza-spiro[4.5]dec-2-ene-3-carbonyl]-amino}-pentanoic säure tert-butyl ester
- [4-(4-Fluoro-phenyl)-3,6-dihydro-2H-pyridin-1-yl]-(8-phenylmethanesulfonyl-1-oxa-2,8-diaza-spiro[4.5]dec-2-en-3-yl)-methanon
- 8-Dimethylsulfamoyl-1-oxa-2,8-diaza-spiro[4.5]dec-2-ene-3-carbonsäure-[2-(ethyl-m-tolyl-amino)-ethyl]-amid
- 8-(2,5-Dimethyl-furan-3-carbonyl)-1-oxa-2,8-diaza-spiro[4.5]dec-2-ene-3-carbonsäure-benzylamid
- (4-Cycloheptyl-piperazin-1-yl)-[8-(4-fluoro-benzolsulfonyl)-1-oxa-2,8-diaza-spiro[4.5]dec-2-en-3-yl]-methanon
- 8-(2-Benzyloxy-acetyl)-1-oxa-2,8-diaza-spiro[4.5]dec-2-en-3-carbonsäure benzylamid
- *R,R*-8-(5-Chlor-thiophen-2-sulfonyl)-1-oxa-2,8-diaza-spiro[4.5]dec-2-en-3-carbonsäure (2-benzyloxy-cyclopentyl)-amid;

sowie ihre Hydrochloride.

Ein weiterer Gegenstand der vorliegenden Erfindung ist ein Verfahren zur Herstellung erfindungsgemäßer 1-Oxa-2,8-diaza-spiro[4.5]dec-2-en-derivate der allgemeinen Formel (I). Dieses Verfahren ist dadurch gekennzeichnet, daß

(a) die Verbindung der Formel (II)

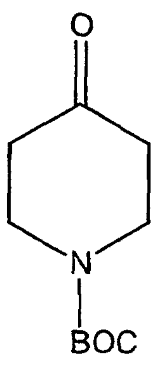

II ,

die käuflich erhältlich oder aus Piperidin-4-on und einem BOCylierungsmittel in einfacher Weise zugänglich ist, mit einem Methylenierungsmittel, bevorzugt $Ph_3PCH_3Br$ in Gegenwart von Kalium-tert.-Butylat in THF, zu Verbindung (III)

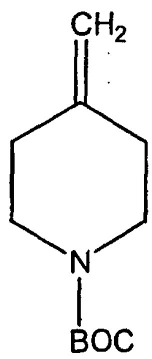

III

umgesetzt wird;

(b) Verbindung (III) einer Umsetzung mit Ethylchloroximidoacetat (IV)

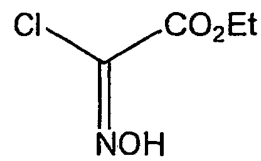

IV

in Gegenwart einer Base, bevorzugt Natriumhydrogencarbonat oder Lithiumhydroxid, bevorzugt in einem organischen Lösungsmittel, insbesondere Methanol, Dichlormethan oder THF, unter Bildung des 1-Oxa-2,8-diaza-spiro[4.5]dec-2-en-derivats der Formel (V)

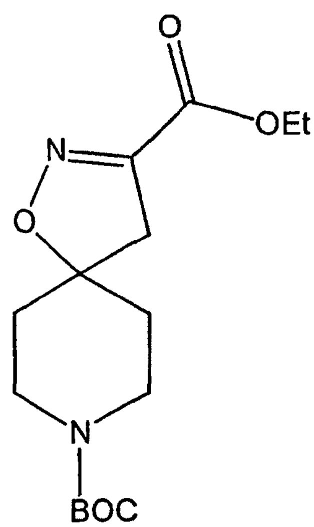

V

unterworfen wird;

(c) Verbindung (V) entweder direkt oder nach vorheriger Verseifung der Carbonsäureethylesterfunktion der Verbindung (V) und gegebenenfalls unter Aktivierung der so gebildeten Carbonsäurefunktion mit einem Amin der Formel $HNR^1R^2$, worin $R^1$ und $R^2$ wie in irgendeinem der Ansprüche 1 bis 8 definiert sind, zu dem 1-Oxa-2,8-diaza-spiro[4.5]dec-2-en-Derivat der Formel (VI)

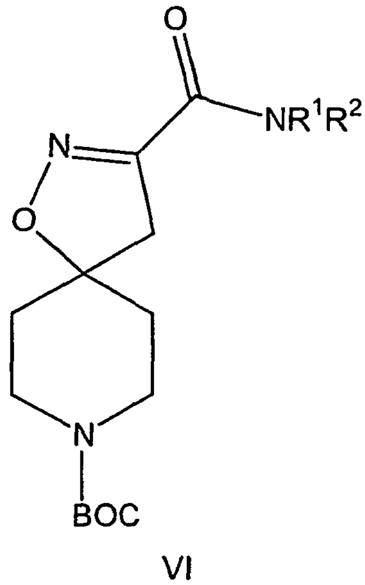

VI

umgesetzt wird;

(d) durch Entfernen der BOC-Schutzgruppe aus Verbindung (VI) Verbindung (I) mit $R^3$ = H

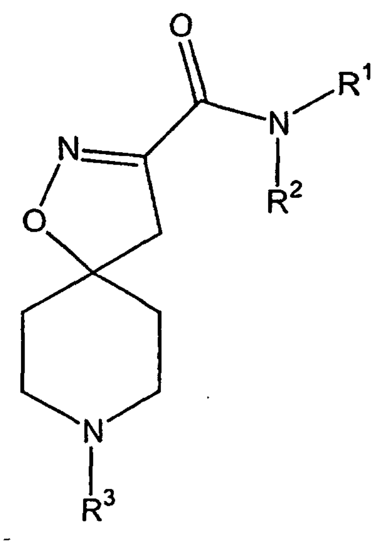

I

erhalten wird; und

(e) gegebenenfalls Verbindung (I) mit $R^3$ = H mit einem Säurechlorid der Formel $R^{12}SO_2Cl$ in eine Verbindung (I) mit $R^3$ = $SO_2R^{12}$, worin $R^{12}$ wie in irgendeinem der Ansprüche 1 bis 8 definiert ist, oder mit einem Carbonsäurechlorid der Formel $R^{13}COCl$ in eine Verbindung (I) mit $R^3$ = $COR^{13}$, worin $R^{13}$ wie in irgendeinem der Ansprüche 1 bis 8 definiert ist, umgewandelt wird.

Dabei erfolgt die Einführung der BOC-Schutzgruppe (BOC = tert.-Butyloxycarbonyl) vor Schritt (a) sowie die Bildung der exo-Methylengruppe in Schritt (a) und die 1,3- dipolare Cycloaddition in Schritt (b) des erfindungsgemäßen Verfahrens in Analogie zu einer literaturbekannten Vorschrift gemäß WO 97/33887, S. 78 bis 82. Die abschließenden Reaktionsschritte (c), (d) und gegebenenfalls (e) - ausgehend vom Spiroester (V) unter Amidbildung mittels Umsetzung mit einem primären oder sekundären Amins ((c)) (gegebenenfalls nach vorheriger Spaltung des Ethylesters und gegebenenfalls unter Aktivierung der freien Carbonsäurefunktion, z.B. mit Dicyclohexylcarbodiimid (DCC), 1-Hydroxybenzotriazol und Triethylamin bzw. N-Methylmorpholin und Castro-Reagenz (BOP-Reagenz) in z.B. DMF), Entfernung

der BOC-Schutzgruppe aus Amid (VI) ((d)) und unter gegebenenfalls erfolgender Umsetzung mit Säurechloriden oder Sulfonsäurechloriden ((e)) - wird nach dem Fachmann allgemein bekannten Methoden, wie sie z.B. in "Peptide Chemistry", M. Bodansky, Springer-Verlag, 1993, angegeben sind, durchgeführt. Das nachfolgende Reaktionschema faßt die erfindungsgemäße Synthesesequenz zusammen.

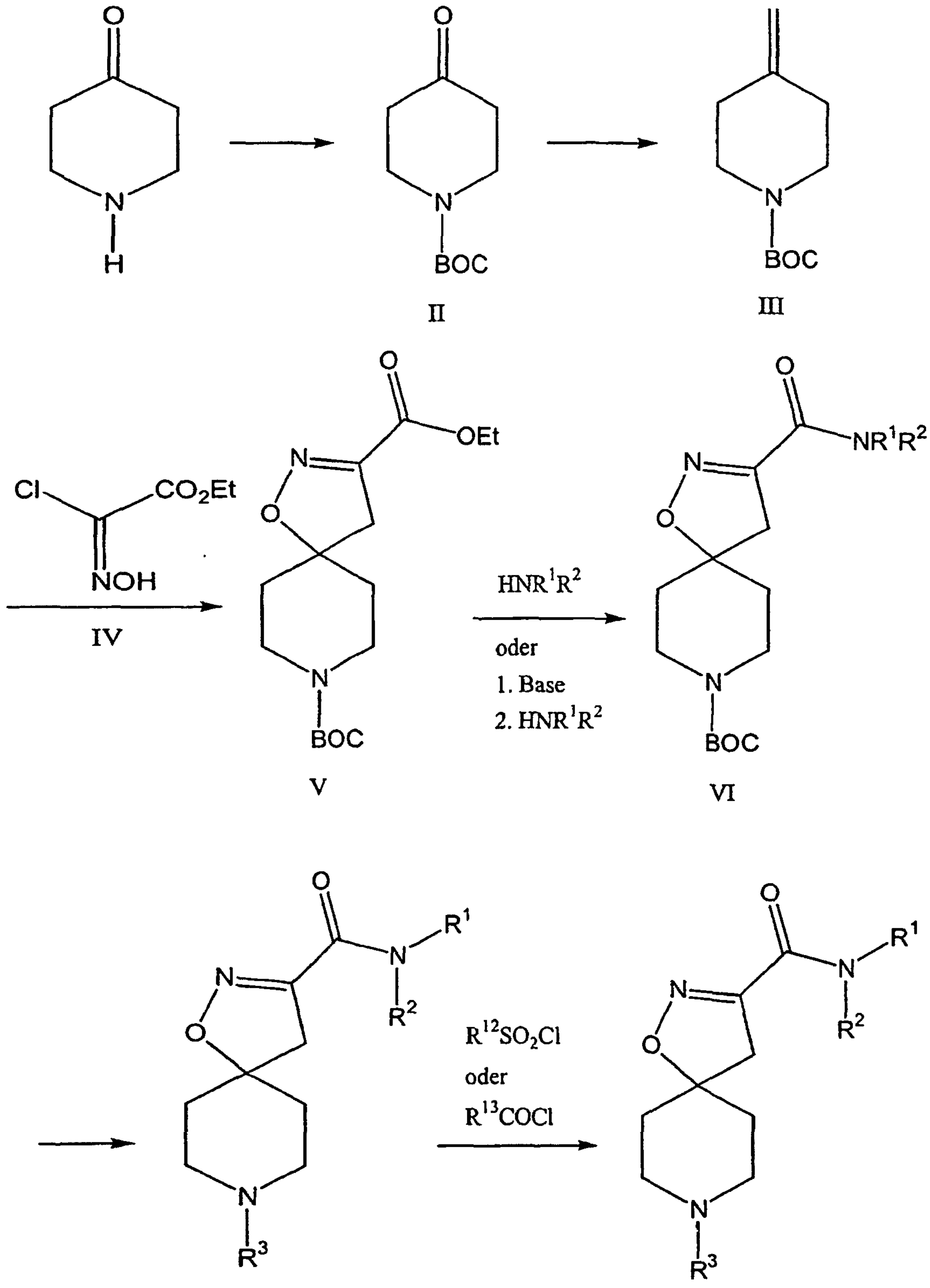


II III IV V VI

I mit $R^3$ = H

I mit $R^3$ = $SO_2R^{12}$; $COR^{13}$

Die in dem erfindungsgemäßen Verfahren eingesetzten Verbindungen und Reagenzien sind, sofern sie nicht kommerziell erhältlich sind, mittels dem Fachmann aus dem Stand der Technik bekannten Vorgehensweisen zugänglich.

Die erfindungsgemäßen 1-Oxa-2,8-diaza-spiro[4.5]dec-2-en-derivate sind toxikologisch unbedenklich, so daß sie sich als pharmazeutische Wirkstoffe in Arzneimitteln eignen.

Ein weiterer Erfindungsgegenstand ist daher auch ein Arzneimittel enthaltend mindestens ein substituiertes 1-Oxa-2,8-diaza-spiro[4.5]dec-2-en-derivat der wie oben definierten allgemeinen Formel (I) in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate.

Die erfindungsgemäßen Arzneimittel können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden und enthalten neben mindestens einem erfindungsgemäßen 1-Oxa-2,8-diaza-spiro[4.5] dec-2-en-derivate der Formel (I) je nach galenischer Form gegebenenfalls Trägermaterialien Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße 1-Oxa-2,8-diaza-spiro[4.5]dec-2-en-derivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Tetrahydrochinolinderivate verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblichweise werden 2 bis 500 mg/kg wenigstens eines erfindungsgemäßen 1-Oxa-2,8-diaza-spiro[4.5]dec-2-en-derivate der Formel (I) appliziert.

Vorzugsweise werden die erfindungsgemäßen 1-Oxa-2,8-diaza-spiro[4.5]dec-2-en-derivate zur Schmerzbehandlung, insbesondere chronischer und neuropathischer Schmerzen, aber auch bei Migräne eingesetzt, so daß ein weiterer Erfindungsgegenstand die Verwendung mindestens eines erfindungsgemäßen 1-Oxa-2,8-diaza-spiro[4.5] dec-2-en-derivats gemäß Formel I auch in Form seiner Racemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, zur Herstellung eines Arzneimittels zur Behandlung von Schmerz, insbesondere des neuropathischen und/oder chronischen Schmerzes, und/oder zur Behandlung und/oder Vorbeugung von Migräne ist.

Überraschenderweise hat es sich herausgestellt, daß die erfindungsgemäßen 1-Oxa-2,8-diaza-spiro[4.5]dec-2-en-derivate der allgemeinen Formel (I) auch für weitere Indikationen, insbesondere zur Behandlung von Harninkontinenz, Juckreiz, Tinnitus aurium und/oder Diarrhoe sehr geeignet sind. Ein weiterer Gegenstand der Anmeldung ist daher die Verwendung mindestens eines erfindungsgemäßen 1-Oxa-2,8-diaza-spiro[4.5]dec-2-en-derivats gemäß Formel (I), auch in Form seiner Racemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochloridsalzes, zur Herstellung eines Arzneimittels zur Behandlung und/oder Prophylaxe von Harninkontinenz und/oder Juckreiz und/oder Tinnitus aurium und/oder Diarrhoe.

Aus der Affinität zur BTX-Bindungsstelle ergeben sich weitere therapeutische Anwendungsgebiete, so daß die erfindungsgemäßen substituierten 1-Oxa-2,8-diaza-spiro[4.5]dec-2-en-derivate der allgemeinen Formel (I) in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, ganz besonders bevorzugt in Form ihrer Hydrochloride, oder in Form ihrer Solvate, insbesondere der Hydrate; zur Herstellung eines Arzneimittels zur Anästhesie, insbesondere Lokalanästhesie, und/oder zur Behandlung und/oder Prophylaxe von Arrhythmien und/oder Emesis und/oder cardiovaskulären Erkrankungen und/oder cerebralen Ischämien und/oder Alkoholabhängigkeit und/oder Drogenabhängigkeit und/oder Medikamentenabhängigkeit und/oder Entzündungen und/oder Vertigo und/oder als Nootropikum (Neurotropikum) und/oder Muskelrelaxanz Verwendung finden.

Ferner ergeben sich aus der Affinität an den NMDA-Rezeptor weitere Anwendungsgebiete, da NMDA-Antagonisten bekanntermaßen u.a. eine neuroprotektive Wirkung haben und daher auch gut bei mit Neurodegeneration und -schädigung einhergehenden Krankheitsbildern, wie Morbus Parkinson und Morbus Huntington etc. eingesetzt werden können. Weitere Indikationen der erfindungsgemäßen NMDA-Antagonisten sind Epilepsie, Glaukom, Osteoporose, Ototoxizität, die mit Alkohol- und/oder Drogenmißbrauch einhergehenden Entzugserscheinungen, der Schlaganfall, sowie damit zusammenhängend cerebrale Ischämien, cerebrale Infarkten, Hirnödem, Hypoxie, Anoxie, sowie

auch der Einsatz zur Anxiolyse und in der Anästhesie. Ein weiterer Gegenstand der Erfindung ist daher die Verwendung mindestens eines erfindungsgemäßen substituierten 1-Oxa-2,8-diaza-spiro[4.5]dec-2-en-derivats gemäß Formel (I), auch in Form seiner Racemate; Enantiomere, Diastereomere, insbesondere Mischungen seiner Enantiomere oder Diastereomere oder eines einzelnen Enantiomers oder Diastereomers; seiner Basen und/oder Salze physiologisch verträglicher Säuren, insbesondere des Hydrochforidsalzes, zur Herstellung eines Arzneimittels zur Behandlung/Prophylaxe von/bei Epilepsie, Morbus Parkinson, Morbus Huntington, Glaukom, Ototoxizität, Entzugserscheinungen bei Alkohol- und/oder Drogenmißbrauch, Schlaganfall, cerebralen Ischämien, cerebralen Infarkten, Hirnödem, Hypoxie, Anoxie und/oder zur Anxiolyse und/oder Anästhesie.

Erfindungsgemäße Verbindungen der Formel (I) binden darüberhinaus auch wirksam an den $\alpha_{2A}$-Rezeptor bzw. haben sich als NA-Uptake/5HT-Uptake-Hemmer erwiesen. Somit ist ein weiterer Gegenstand der Erfindung die Verwendung eines substituierten 1-Oxa-2,8-diaza-spiro[4.5]dec-2-en-derivats der allgemeinen Formel (I) in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Behandlung und/oder Prophylaxe von inflammatorischen und/oder allergischen Reaktionen und/oder Gastritis und/oder Ulcera und/oder Depressionen und/oder Schockzuständen und/oder Narkolepsie und/oder Epilepsie und/oder Übergewicht und/oder Asthma und/oder Glaukom und/oder hyperkinetischem Syndrom; von Antriebslosigkeit und/oder Bulimie und/oder Anorexie und/oder Katalepsie und/oder zur Anxiolyse und/oder zur Vigilanz- und/oder Libidosteigerung; von bipolaren Störungen und/oder postmenopausalen Hitzewallungen und/oder Amyotropischer Lateraler Sklerose (ALS) und/oder Reflex-sympathetischer Dystrophie (RSD) und/oder spastischer Lähmung und/oder Restless Leg Syndrom und/oder erworbenem Nystagmus und/oder Multipler Sklerose und/oder Morbus Parkinson und/oder Morbus Alzheimer und/oder Morbus Huntington.

Ein weiterer Gegenstand der Erfindung ist ein Verfahren zur Behandlung eines nichthumanen Säugetiers oder eines Menschen, das/der eine Behandlung medizinisch relevanter Symptome benötigt, durch Verabreichung einer therpeutisch wirksamen Dosis eines erfindungsgemäßen substituierten 1-Oxa-2,8-diaza-spiro[4.5]dec-2-en-derivats der allgemeinen Formel (I), auch in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochforide, oder in Form ihrer Solvate, insbesondere der Hydrate; oder eines erfindungsgemäßen Arzneimittels. Die Erfindung betrifft insbesondere entsprechende Verfahren zur Behandlung von Schmerz, insbesondere neuropathischem Schmerz und/oder chronischem Schmerz, und/oder Migräne und/oder Harninkontinenz und/oder Juckreiz und/oder Tinnitus aurium und/oder Diarrhoe und/oder Arrhythmien und/oder Emesis und/oder cardiovaskulären Erkrankungen und/oder cerebralen Ischämien und/oder Alkoholabhängigkeit und/oder Drogenabhängigkeit und/oder Medikamentenabhängigkeit und/oder Entzündungen und/oder Vertigo und/oder inflammatorischen Reaktionen und/oder allergischen Reaktionen und/oder Gastritis und/oder Ulcera und/oder Depressionen und/oder Schockzuständen und/oder Narkolepsie und/oder Epilepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanz- und/oder Libidosteigerung und/oder Prophylaxe von bipolaren Störungen und/oder postmenopausalen Hitzewallungen und/oder Amyotropischer Lateraler Sklerose (ALS) und/oder Reflex-sympathetischer Dystrophie (RSD) und/oder spastischer Lähmung und/oder Restless Leg Syndrom und/oder erworbenem Nystagmus und/oder Multipler Sklerose und/oder Morbus Parkinson und/oder Morbus Alzheimer und/oder Morbus Huntington.

Im folgenden wird die Erfindung durch Beispiele weiter erläutert, ohne sie darauf zu beschränken

**Beispiele**

Die eingesetzten Chemikalien und Lösungsmittel wurden kommerziell von einem der folgenden Anbieter erworben: Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan; oder nach allgemeinen im Stand der Technik bekannten Verfahren hergestellt.

Die dünschichtchromatographischen Untersuchungen wurden mit HPTLC-Fertigplatten, Kieselgel 60 F 254, der Firma E. Merck, Darmstadt, durchgeführt.

Jede Probe wurde mit ESI-MS und/oder NMR analysiert. Massenspektrometrische Untersuchungen (ESI-MS) wurden mit einem Massenspektrometer der Fa. Finnegan, LCQ Classic durchgeführt. $^1$H-NMR-Untersuchungen der erfindungsgemäßen Verbindungen wurden mit einem 300 MHz DPX Advance NMR-Gerät der Fa. Bruker durchgeführt.

**Herstellung erfindungsgemäßer substituierter 1-Oxa-2,8-diaza-spiro[4.5]dec-2-en-derivate**

**4-Methylen-piperidin-1-carbonsäure *tert*-butylester**

Zu einer Suspension von 5,34 g (15 mmol) Methyltriphenylphosphoniumbromid in 50 ml Diethylether wurden unter Rühren bei 0°C (Eisbad) 1,6 g (14 mmol) Kalium *tert*-butylat zugegeben. Nach 15 min Rühren wurde eine Lösung von 2,00 g (10 mmol) 1-Boc-4-Piperidon (von Merck KGaA) in 15 ml Diethylether langsam zugegeben. Man ließ die Suspension weitere 30 min bei 0°C rühren. Nach Zugabe von 60 ml 10%iger wäßriger $NH_4Cl$-Lösung wurde die organische Phase abgetrennt, über Magnesiumsulfat getrocknet und im Vakuum vom Lösungsmittel befreit. Nach Chromatographie an Kieselgel (Hexan:Essigester = 5:1) erhielt man 1,71 g (89%) 4-Methylen-piperidin-1-carbonsäure*tert*-butylester als farblose Flüssigkeit.

$^1$H-NMR-Spektrum (d$_6$-DMSO/TMS$_{ext.}$): $\delta$ = 1,47 ppm (s, 9H, $C(CH_3)_3$); 2,16-2.19 ppm (m, 4H, $CH_2$); 3,40-3,44 ppm (m, 4H, $CH_2$); 4,74 (s, 2H, $C{=}CH_2$).

**1-Oxa-2,8-diaza-spiro[4.5]dec-2-en-3,8-dicarbonsäure 8-*tert*-butylester 3-ethylester**

Zu einer Mischung von 0,50 g (2,6 mmol) 4-Methylen-piperidin-1-carbonsäure*tert*-butylester und 0,60 g (3,9 mmol) 2-Chlor-2-hydroxyiminoessigsäureethylester in 10 ml Dichlormethan wurden bei 0°C (Eisbad) 0,55 ml (3,9 mmol) an zuvor frisch destilliertem Triethylamin langsam hinzugegeben. Nach 12 h Rühren bei RT wurden 0.79 g (5,1 mmol) an 2-Chlor-2-hydroxyiminoessigsäureethylester und 0,72 ml (5,1 mmol) an Triethylamin bei 0°C nachdosiert und erneut für 24 h rühren gelassen. Nach Waschen mit 10%iger wäßriger Zitronensäure und gesättigter wäßriger NaCl erhielt man nach Trocknen der organischen Phase ($MgSO_4$) und Entfernen des Lösungsmittels im Vakuum ein gelbes Öl. Nach Säulenchromatographie an Kieselgel (Hexan:Diethylether = 4:1) erhielt man 320 mg (39%) an 1-Oxa-2,8-diaza-spiro[4.5]dec-2-en-3,8-dicarbonsäure 8-*tert*-butylester 3-ethylester in Form eines leicht gelblich gefärbten Öles.

$^1$H-NMR-Spektrum (d$_6$-DMSO/TMS$_{ext.}$): $\delta$ = 1,37 ppm (t, J = 6.0 Hz, 3H, $CH_3$); 1,46 ppm (s, 9H, $C(CH_3)_3$); 1,67-1,75 ppm (m, 2H, $CH_2$); 1,85-1,92 ppm (m, 2H, $CH_2$); 2,96 ppm (s, 2H, $CH_2$); 3.39-3,49 ppm (m, 2H, $CH_2$); 3,60-3,70 ppm (m, 2H, $CH_2$); 4,35 (q, *J* = 6.0 Hz, 2H, $CH_2$).

**1-Oxa-2,8-diaza-spiro[4.5]dec-2-en-3,8-dicarbonsäure 8-*tert*-butylester**

Eine Mischung von 320 mg (1 mmol) an 4-Methylen-piperidin-1-carbonsäure-*tert*-butylester-3-ethylester in 2 ml MeOH und 70 mg (1,5 mmol) an Lithiumhydroxidmonohydrat in 1,3 ml $H_2O$ wurde 1,5 h bei RT Rühren gelassen. Nach Entfernen des Lösungsmittelgemisches im Vakuum wurde der Rückstand in Wasser und Essigester aufgenommen und partitioniert, wobei die wäßrige Phase mit Zitronensäure auf pH = 4 eingestellt wurde. Die organische Phase wurde getrocknet ($MgSO_4$) und im Vakuum vom Lösungsmittel befreit. Man erhielt 280 mg (98%) der freien Säure in Form eines farblosen Feststoffes.

$^1$H-NMR-Spektrum (d$_6$-DMSO/TMS$_{ext.}$): $\delta$ = 1,47 ppm (s, 9H, $C(CH_3)_3$); 1,73-1,78 ppm (m, 2H, $CH_2$); 1,88-1,93 ppm (m, 2H, $CH_2$); 2,97 ppm (s, 2H, $CH_2$); 3.39-3,48 ppm (m, 2H, $CH_2$); 3,65-3,74 ppm (m, 2H, $CH_2$); 9,35 (s, 1H, COOH).

**Allgemeine Vorschrift zur Umsetzung von 1-Oxa-2,8-diaza-spiro[4.5]dec-2-en-3,8-dicarbonsäure-8-*tert*-butylester mit primären oder sekundären Aminen**

Eine Mischung von 1 Äquivalent an 1-Oxa-2,8-diaza-spiro[4.5]dec-2-en-3,8-dicarbonsäure 8-*tert*-butylester, 1 Äquivalent des jeweiligen Amins, 2,7 Äquivalenten an N-Methylmorpholin und 1,8 Äquivalenten Castro-Reagenz (BOP-Reagenz) in DMF wurde 12 h bei RT rühren gelassen. Nach Entfernen des DMF im Vakuum wurde der Rückstand mit $H_2O$ und Essigester versetzt und partitioniert. Die organische Phase wurde mit $H_2O$, 10%iger Zitronensäure, gesättigter $Na_2CO_3$-Lösung und gesättigter NaCl-Lösung gewaschen, getrocknet ($MgSO_4$) und im Vakuum vom Lösungsmittel befreit. Nach Säulenchromatographie (Kieselgel, Diethylether:Hexan = 10:1) erhielt man die jeweiligen Kupplungsprodukte.

In einigen Fällen wurde die Kupplung mit DCC (1 Äquivalent), 1-Hydroxybenzotriazol (1 Äquivalent) und Triethylamin (1 Äquivalent) in DMF bei 0°C durchgeführt. Nach 1 h bei 0°C ließ man auf RT erwärmen und weitere 12 h rühren. Dann wurde abfiltriert und das Filtrat zwischen wäßriger gesättigter $NaHCO_3$-Lösung und Diethylether partitioniert. Die organische Phase wurde mit 10%iger Zitronensäure, gesättigter $NaHCO_3$-Lösung und gesättigter NaCl-Lösung gewaschen, getrocknet ($MgSO_4$) und im Vakuum vom Lösungsmittel befreit. Nach Säulenchromatographie (Kieselgel, Diethylether:Hexan = 10:1) erhielt man die jeweiligen Kupplungsprodukte.

**3-Benzylcarbamoyl-1-oxa-2,8-diaza-spiro[4.5]dec-2-en-8-carbonsäure-tert-butylester**

Analog der o.g. allgemeinen Vorschrift (mit Castro-Reagenz) erhielt man 250 mg (56%) an 3-Benzylcarbamoyl-1-oxa-2,8-diaza-spiro[4.5]dec-2-en-8-carbonsäure-*tert*-butylester in Form eines farblosen Feststoffes aus 340 mg (1,2 mmol) 1-Oxa-2,8-diaza-spiro[4.5]dec-2-en-3,8-dicarbonsäure 8-*tert*-butylester und 130 mg (1,2 mmol) Benzylamin.

$^1$H-NMR-Spektrum ($d_6$-DMSO/$TMS_{ext.}$): δ = 1,46 ppm (s, 9H, $C(CH_3)_3$); 1,63-1,70 ppm (m, 2H, $CH_2$); 1,79-1,89 ppm (m, 2H, $CH_2$); 2,98 ppm (s, 2H, $CH_2$); 3.35-3,45 ppm (m, 2H, $CH_2$); 3,55-3,65 ppm (m, 2H, $CH_2$); 4,51 ppm (d, *J* = 6 Hz, 2H, N-$CH_2$); 7,15-7,20 ppm (m, 1H, NH); 7,26-7,33 ppm (m, 5 H, Aryl-H).

***R,R*-3-(2-Benzyloxy-cyclopentylcarbamoyl)-1-oxa-2,8-diaza-spiro[4.5]dec-2-en-8-carbonsäure-*tert*-butylester**

Analog der oben angegebenen allgemeinen Vorschrift (mit DCC) erhielt man 700 mg (55%) an *R*,*R*-3-(2-Benzyloxy-cyclopentylcarbamoyl)-1-oxa-2,8-diaza-spiro[4.5]dec-2-en-8-carbonsäure-*tert*-butylester in Form eines farblosen Feststoffes aus 800 mg (2,8 mmol) 1-Oxa-2,8-diaza-spiro[4.5]dec-2-en-3,8-dicarbonsäure 8-*tert*-butylester und 540 mg (2,8 mmol) *R,R*-2-Benzyloxycyclopentylamin.

$^1$H-NMR-Spektrum ($d_6$-DMSO/$TMS_{ext.}$): δ = 1,46 ppm (s, 9H, $C(CH_3)_3$); 1,60-2,00 ppm (m, 9H, $CH_2$); 2,15-2,30 ppm (m, 1H, $CH_2$); 2,99 ppm (s, 2H, $CH_2$); 3.40-3,50 ppm (m, 2H, $CH_2$); 3,59-3,65 ppm (m, 2H, $CH_2$); 3,80-3,90 ppm (m, 1H, CH); 4,25-4,35 ppm (m, 1H, CH); 4,61 ppm (m, 2H, O-$CH_2$); 6,51-6,53 ppm (m, 1H, NH); 7,25-7,34 ppm (m, 5H, Aryl-H).

**Allgemeine Vorschrift zur Abspaltung der Boc-Gruppe**

Das entsprechende N-Boc-Piperidin wurde mit einem Überschuss einer 4M methanolischen HCl-Lösung bei RT versetzt und rühren gelassen (DC Kontrolle). Nach vollständiger Reaktion wurde die Lösung bis zur ersten Trübung eingeengt, dann mit Diethylether versetzt und zur Vervollständigung der Fällung bei 4°C über Nacht gelagert. Der ausgefallene Feststoff wurde abfiltriert, mit kleinen Portionen an Diethylether gewaschen und im Vakuum getrocknet.

**1-Oxa-2,8-diaza-spiro[4,5]dec-2-en-3-carbonsäure benzylamid-Hydrochlorid**

Analog der zuvor beschriebenen Vorschrift wurden aus 250 mg (0,7 mmol) 3-Benzylcarbamoyl-1-oxa-2,8-diaza-spiro[4.5]dec-2-en-8-carbonsäure-*tert*-butylester 130 mg (60%) 1-Oxa-2,8-diaza-spiro[4.5]dec-2-en-3-carbonsäure benzylamid als Hydrochlorid in Form eines farblosen Feststoffes erhalten.

$^1$H-NMR-Spektrum ($d_6$-DMSO/$TMS_{ext.}$): δ = 1,92-2,06 ppm (m, 4H, $CH_2$); 3,10-3,18 ppm (m, 6H, $CH_2$); 4,31-4,40 ppm (m, 2H, $CH_2$); 7,15-7,33 ppm (m, 5H, Aryl-H); 8,99-9,13 ppm (m, 3H, NH).

***R,R*-1-Oxa-2,8-diaza-spiro[4.5]dec-2-en-3-carbonsäure (2-benzyloxy-cyclopentyl)-amid-Hydrochlorid**

Analog der allgemeinen Vorschrift wurden aus 700 mg (2,5 mmol) R,R-3-(2-Benzyloxy-cyclopentylcarbamoyl)-1-oxa-2,8-diaza-spiro[4.5]dec-2-en-8-carbonsäure-*tert*-butylester 490 mg (50%) *R,R*-1-Oxa-2,8-diaza-spiro[4.5]dec-2-en-3-carbonsäure (2-benzyloxy-cyclopentyl)-amid als Hydrochlorid in Form eines farblosen Feststoffes erhalten.

$^1$H-NMR-Spektrum ($d_6$-DMSO/$TMS_{ext.}$): δ = 1,50-1,75 ppm (m, 4H, $CH_2$); 1,92-2,11 ppm (m, 6H, $CH_2$); 3,06-3,20 ppm (m, 6H, $CH_2$); 3.82-3,95 ppm (m, 1H, CH); 4,10-4,20 ppm (m, 1H, CH); 4,42-4,60 ppm (m, 2H, O-$CH_2$); 7,20-7,34 ppm (m, 5H, Aryl-H); 8,47-8,54 ppm (m, 1H, NH); 9,05-9,15 ppm (m, 2H, NH).

**Allgemeine Vorschrift zur Umsetzung der Piperidine mit Carbonsäurehalogeniden oder Sulfonsäurehalogeniden**

Zu einer Lösung des entsprechenden Säurehalogenids (1,5 Äquivalente), Triethylamin (2 Äquivalente) und *N*,*N*-Dimethyl-4-aminopyridin (DMAP; katalytische Mengen) in Dichlormethan wurde bei 0°C das jeweilige Piperidin (1 Äquivalent) hinzugegeben. Man ließ auf RT erwärmen und über Nacht rühren. Nach Hydrolyse mit 10%iger wäßriger $NH_4Cl$-Lösung wurde die organische Phase getrocknet ($MgSO_4$) und im Vakuum vom Lösungsmittel befreit. Nach Säulenchromatographie an Kieselgel (Essigester-Hexan-Gemische variabler Zusammensetzung) erhielt man die Zielverbindungen.

**8-(2-Benzyloxy-acetyl)-1-oxa-2,8-diaza-spiro[4.5]dec-2-en-3-carbonsäure benzylamid (Beispiel 219)**

In Analogie zu o.g. allgemeiner Vorschrift wurden 120 mg (0,42 mmol) 1-Oxa-2,8-diaza-spiro[4.5]dec-2-en-3-carbonsäurebenzylamid (Base, mit wäßriger NaOH freigesetzt) mit 116 mg (0,63 mmol) Benzyloxyacetylchlorid zu 60 mg (34%) 8-(2-Benzyloxy-acetyl)-1-oxa-2,8-diaza-spiro[4.5]dec-2-en-3-carbonsäurebenzylamid umgesetzt. Man erhielt ein farbloses Öl, welches bei längerem Stehenlassen bei Raumtemperatur langsam kristallisierte.

$^1$H-NMR-Spektrum ($d_6$-DMSO/$TMS_{ext.}$): δ = 1,55-1,69 ppm (m, 2H, $CH_2$); 1,75-1,85 ppm (m, 2H, $CH_2$); 2,92 ppm (s, 2H, $CH_2$); 3,20-3,32 ppm (m, 1H, $CH_2$); 3,35-3,48 ppm (m, 1H, $CH_2$); 3,50-3,60 (m, 1H, $CH_2$); 3,94-4,05 ppm (m, 1H, $CH_2$); 6,95-7,05 ppm (m, 1H, NH); 7,15-7,35 ppm (m, 10H, Aryl-H).

***R,R*-8-(5-Chlor-thiophen-2-sulfonyl)-1-oxa-2,8-diaza-spiro[4.5]dec-2-en-3-carbonsäure (2-benzyloxy-cyclopentyl)-amid (Beispiel 220)**

Wie oben beschrieben wurden 490 mg (1,24 mmol) an *R*,*R*-1-Oxa-2,8-diaza-spiro[4.5]dec-2-en-3-carbonsäure (2-benzyloxy-cyclopentyl)-amid (Base, mit wäßriger NaOH freigesetzt) mit 410 mg (1,86 mmol) 5-Chlorthiophen-2-sulfonylchlorid zu 480 mg (72%) 8-(5-Chlor-thiophen-2-sulfonyl)-1-oxa-2,8-diaza-spiro[4.5]dec-2-en-3-carbonsäure (2-benzyloxy-cyclopentyl)-amid umgesetzt. Man erhielt einen farblosen kristallinen Feststoff.

$^1$H-NMR-Spektrum ($d_6$-DMSO/$TMS_{ext.}$): δ = 1,40-1,55 ppm (m, 1H, $CH_2$); 1,60-2,05 ppm (m, 8H, $CH_2$); 2,10-2,30 ppm (m, 1H, $CH_2$); 2,93-3,05 ppm (m, 2H, $CH_2$); 2,00 ppm (m, 2H, $CH_2$); 3.41-3,55 ppm (m, 2H, $CH_2$); 3,75-3,88 ppm (m, 1H, CH); 4,20-4,32 ppm (m, 1H, CH); 4,42-4,60 ppm (m, 2H, O-$CH_2$); 6,42-6,48 (d, 1H, Aryl-H); 7,00 ppm (d, *J* = 3 Hz, 1H, Aryl-H); 7,22-7,40 ppm (m, 5H, Aryl-H).

Nach diesem Verfahren werden die in Tabelle 1 aufgeführten Verbindungen hergestellt; alternativ ist die Herstellung auch parallelsynthetisch in halbautomatisierter Weise möglich.

Tabelle 1

| Beispiel | Verbindung |
|---|---|
| 1 | 3-(1H-Indol-3-yl)-2-{[8-(4-trifluormethoxy-benzolsulfonyl)-1-oxa-2,8-diaza-spiro[4.5]dec-2-en-3-carbonyl]-amino}-propionsäure methyl ester |
| 2 | {4-[(8-Phenylmethansulfonyl-1-oxa-2,8-diaza-spiro[4.5]dec-2-en-3-carbonyl)-amino]-benzyl}-phosphonsäurediethylester |
| 3 | (4-Cycloheptyl-piperazin-1-yl)-[8-(thiophen-2-sulfonyl)-1-oxa-2,8-diaza-spiro[4.5]dec-2-en-3-yl]-methanon |
| 4 | [8-(5-Chlor-thiophen-2-sulfonyl)-1-oxa-2,8-diaza-spiro[4.5]dec-2-en-3-yl]-(4-phenyl-piperazin-1-yl)-methanon |
| 5 | 8-(2-Chlor-4-nitro-benzoyl)-1-oxa-2,8-diaza-spiro[4.5]dec-2-en-3-carbonsäure cyclopentylamid |
| 6 | (4-Naphthalen-2-ylmethyl-piperazin-1-yl)-(8-phenylmethansulfonyl-1-oxa-2,8-diaza-spiro[4.5]dec-2-en-3-yl)-methanon |
| 7 | 8-Dimethylsulfamoyl-1-oxa-2,8-diaza-spiro[4.5]dec-2-en-3-carbonsäure phenethyl-amid |
| 8 | 8-(Thiophen-2-sulfonyl)-1-oxa-2,8-diaza-spiro[4.5]dec-2-en-3-carbonsäure (1-phenyl-ethyl)-amid |
| 9 | 8-(Toluol-4-sulfonyl)-1-oxa-2,8-diaza-spiro[4.5]dec-2-en-3-carbonsäure [2-(3,4-Dichlor-phenyl)-ethyl]-amid |
| 10 | 4-Diethylamino-benzoic säure N'-[8-(thiophen-2-sulfonyl)-1-oxa-2,8-diaza-spiro[4.5]dec-2-en-3-carbonyl]-hydrazid |
| 11 | 8-(3-Chlor-4-fluor-benzolsulfonyl)-1-oxa-2,8-diaza-spiro[4.5]dec-2-en-3-carbonsäure (2-pyrrolidin-1-yl-ethyl)-amid |
| 12 | 8-Phenylmethansulfonyl-1-oxa-2,8-diaza-spiro[4.5]dec-2-en-3-carbonsäure phenethyl-amid |
| 13 | 8-Benzolsulfonyl-1-oxa-2,8-diaza-spiro[4.5]dec-2-en-3-carbonsäure (2-dimethylamino-ethyl)-amid |
| 14 | [4-(3-Phenyl-propyl)-piperazin-1-yl]-[8-(thiophen-2-sulfonyl)-1-oxa-2,8-diaza-spiro[4.5]dec-2-en-3-yl]-methanon |

Tabelle 1 (fortgesetzt)

| Beispiel | Verbindung |
|---|---|
| 15 | 8-(2,5-Dichlor-benzolsulfonyl)-1-oxa-2,8-diaza-spiro[4.5]dec-2-en-3-carbonsäure [3-(2-methyl-piperidin-1-yl)-propyl]-amid |
| 16 | 8-(4-Trifluormethoxy-benzolsulfonyl)-1-oxa-2.8-diaza-spiro[4.5]dec-2-en-3-carbonsäure [2-(1H-indol-3-yl)-ethyl]-methyl-amid |
| 17 | 8-(5-Fluor-2-methyl-benzolsulfonyl)-1-oxa-2,8-diaza-spiro[4.5]dec-2-en-3-carbonsäure (2-acetylamino-ethyl)-amid |
| 18 | 8-(Toluol-4-sulfonyl)-1-oxa-2,8-diaza-spiro[4.5]dec-2-en-3-carbonsäure benzyl-phenethyl-amid |
| 19 | 8-(2-Methyl-5-nitro-benzolsulfonyl)-1-oxa-2,8-diaza-spiro[4.5]dec-2-en-3-carbonsäure (2-dimethylamino-ethyl)-amid |
| 20 | 8-Phenylmethansulfonyl-1-oxa-2,8-diaza-spiro[4.5]dec-2-en-3-carbonsäure (2-benzyloxy-cyclopentyl)-amid |
| 21 | 8-(Thiophen-2-sulfonyl)-1-oxa-2,8-diaza-spiro[4.5]dec-2-en-3-carbonsäure (2,3-dihydro-benzo[1,4] dioxin-2-ylmethyl)-amid |
| 22 | 8-(Toluol-4-sulfonyl)-1-oxa-2,8-diaza-spiro[4.5]dec-2-en-3-carbonsäure (4-phenoxy-phenyl)-amid |
| 23 | 8-(Thiophen-2-sulfonyl)-1-oxa-2,8-diaza-spiro[4.5]dec-2-en-3-carbonsäure (1-p-tolyl-ethyl)-amid |
| 24 | 8-(Thiophen-2-sulfonyl)-1-oxa-2,8-diaza-spiro[4.5]dec-2-en-3-carbonsäure 3-fluor-benzylamid |
| 25 | 2-Phenyl-1-{4-[8-(toluol-4-sulfonyl)-1-oxa-2,8-diaza-spiro[4.5]dec-2-en-3-carbonyl]-piperazin-1-yl}-ethanone |
| 26 | 8-(Thiophen-2-sulfonyl)-1-oxa-2,8-diaza-spiro[4.5]dec-2-en-3-carbonsäure [1-(4-trifluormethyl-benzyl)-pyrrolidin-3-yl]-amid |
| 27 | [8-(Thiophen-2-sulfonyl)-1-oxa-2,8-diaza-spiro[4.5]dec-2-en-3-yl]-(4-o-tolyl-piperazin-1-yl)-methanon |
| 28 | 8-(Toluol-4-sulfonyl)-1-oxa-2,8-diaza-spiro[4.5]dec-2-en-3-carbonsäure benzyl-(2-cyano-ethyl)-amid |
| 29 | [8-(2,5-Dichlor-benzolsulfonyl)-1-oxa-2,8-diaza-spiro[4.5]dec-2-en-3-yl]-(4-thieno[2,3-d]pyrimidin-4-yl-piperazin-1-yl)-methanon |
| 30 | 8-(5-Chlor-thiophen-2-sulfonyl)-1-oxa-2,8-diaza-spiro[4.5]dec-2-en-3-carbonsäure (benzo[1,3]dioxol-5-ylmethyl)-amid |
| 31 | (3-Methyl-4-m-tolyl-piperazin-1-yl)-[8-(thiophen-2-sulfonyl)-1-oxa-2,8-diaza-spiro[4.5]dec-2-en-3-yl]-methanon |
| 32 | [4-(1-Phenyl-ethyl)-piperazin-1-yl]-[8-(toluol-4-sulfonyl)-1-oxa-2,8-diaza-spiro[4.5]dec-2-en-3-yl]-methanon |
| 33 | [4-(2,4-Dimethyl-phenyl)-piperazin-1-yl]-[8-(toluol-4-sulfonyl)-1-oxa-2,8-diaza-spiro[4.5]dec-2-en-3-yl]-methanon |
| 34 | 8-(2-Chlor-pyridine-3-carbonyl)-1-oxa-2,8-diaza-spiro[4.5]dec-2-en-3-carbonsäure cyclopentylamid |
| 35 | (4-Naphthalen-2-ylmethyl-piperazin-1-yl)-[8-(3-trifluormethyl-benzolsulfonyl)-1-oxa-2,8-diaza-spiro [4.5]dec-2-en-3-yl]-methanon |
| 36 | 8-[3-Phenyl-2-(toluol-4-sulfonylamino)-propionyl]-1-oxa-2,8-diaza-spiro[4.5]dec-2-en-3-carbonsäure (pyridin-3-ylmethyl)-amid |
| 37 | 8-(5-Fluor-2-methyl-benzolsulfonyl)-1-oxa-2,8-diaza-spiro[4.5]dec-2-en-3-carbonsäure (2-dimethylamino-ethyl)-amid |
| 38 | 8-(4-Nitro-benzolsulfonyl)-1-oxa-2,8-diaza-spiro[4.5]dec-2-en-3-carbonsäure (thiophen-2-ylmethyl)-amid |
| 39 | 8-(2,5-Dichlor-benzolsulfonyl)-1-oxa-2,8-diaza-spiro[4.5]dec-2-en-3-carbonsäure 3-fluor-5-trifluormethyl-benzylamid |

Tabelle 1 (fortgesetzt)

| Beispiel | Verbindung |
|---|---|
| 40 | 8-(5-Chlor-thiophen-2-sulfonyl)-1-oxa-2,8-diaza-spiro[4.5]dec-2-en-3-carbonsäure benzhydryl-amid |
| 41 | 8-[3-Phenyl-2-(toluol-4-sulfonylamino)-propionyl]-1-oxa-2,8-diaza-spiro[4.5]dec-2-en-3-carbonsäure (2-cyano-ethyl)-amid |
| 42 | [(8-Phenylmethansulfonyl-1-oxa-2,8-diaza-spiro[4.5]dec-2-en-3-carbonyl)-amino]-essigsäureethylester |
| 43 | 8-(3-Dimethylamino-benzoyl)-1-oxa-2,8-diaza-spiro[4.5]dec-2-en-3-carbonsäure (2-cyano-ethyl)-amid |
| 44 | 8-(2,5-Dichlor-benzolsulfonyl)-1-oxa-2,8-diaza-spiro[4.5]dec-2-en-3-carbonsäure [1-(naphthalen-2-ylcarbamoyl)-ethyl]-amid |
| 45 | 8-(Toluol-4-sulfonyl)-1-oxa-2,8-diaza-spiro[4.5]dec-2-en-3-carbonsäure (1-p-tolyl-ethyl)-amid |
| 46 | [4-(4-Chlor-phenyl)-piperazin-1-yl]-(8-phenylmethansulfonyl-1-oxa-2,8-diaza-spiro[4.5]dec-2-en-3-yl)-methanon |
| 47 | 8-(Thiophen-2-sulfonyl)-1-oxa-2,8-diaza-spiro[4.5]dec-2-en-3-carbonsäure (2-benzyloxy-cyclopentyl)-amid |
| 48 | 8-Acetyl-1-oxa-2,8-diaza-spiro[4.5]dec-2-en-3-carbonsäure benzylamid |
| 49 | 8-(5-Chlor-thiophen-2-sulfonyl)-1-oxa-2,8-diaza-spiro[4.5]dec-2-en-3-carbonsäure 2,5-difluor-benzylamid |
| 50 | 8-(5-Chlor-thiophen-2-sulfonyl)-1-oxa-2,8-diaza-spiro[4.5]dec-2-en-3-carbonsäure (1-naphthalin-2-ylmethyl-pyrrolidin-3-yl)-amid |
| 51 | 8-Phenylmethansulfonyl-1-oxa-2,8-diaza-spiro[4.5]dec-2-en-3-carbonsäure 2-ethoxy-benzylamid |
| 52 | 8-(4-Ethyl-benzoyl)-1-oxa-2,8-diaza-spiro[4.5]dec-2-en-3-carbonsäure (thiophen-2-ylmethyl)-amid |
| 53 | 2-{[8-(2,5-Dichlor-benzolsulfonyl)-1-oxa-2,8-diaza-spiro[4.5]dec-2-en-3-carbonyl]-amino}-succinic säure 1-allyl ester 4-tert-butyl ester |
| 54 | 8-(4-Trifluormethoxy-benzolsulfonyl)-1-oxa-2,8-diaza-spiro[4.5]dec-2-en-3-carbonsäure naphthalen-2-ylamid |
| 55 | 4-{[8-(2-Phenyl-cyclopropanecarbonyl)-1-oxa-2,8-diaza-spiro[4.5]dec-2-en-3-carbonyl]-amino}-piperidine-1-carbonsäure ethyl ester |
| 56 | 4-{[8-(2,4-Difluor-benzoyl)-1-oxa-2,8-diaza-spiro[4.5]dec-2-en-3-carbonyl]-amino}-piperidine-1-carbonsäure ethyl ester |
| 57 | 8-(Toluol-4-sulfonyl)-1-oxa-2,8-diaza-spiro[4.5]dec-2-en-3-carbonsäure (2-dimethylamino-ethyl)-amid |
| 58 | 8-Dimethylsulfamoyl-1-oxa-2,8-diaza-spiro[4.5]dec-2-en-3-carbonsäure [2-(4-chlor-phenyl)-ethyl]-amid |
| 59 | 8-(4-Methoxy-benzoyl)-1-oxa-2,8-diaza-spiro[4.5]dec-2-en-3-carbonsäure (5-methyl-furan-2-ylmethyl)-amid |
| 60 | 8-(Thiophen-2-sulfonyl)-1-oxa-2,8-diaza-spiro[4.5]dec-2-en-3-carbonsäure [2-(4-chlor-phenyl)-ethyl]-amid |
| 61 | 8-(5-Chlor-thiophen-2-sulfonyl)-1-oxa-2,8-diaza-spiro[4.5]dec-2-en-3-carbonsäure [2-(1-methyl-pyrrolidin-2-yl)-ethyl]-amid |
| 62 | 4-{[8-(4-Brom-benzoyl)-1-oxa-2,8-diaza-spiro[4.5]dec-2-en-3-carbonyl]-amino}-piperidine-1-carbonsäure ethyl ester |
| 63 | 8-(Toluol-4-sulfonyl)-1-oxa-2,8-diaza-spiro[4.5]dec-2-en-3-carbonsäure 3,5-dichlor-benzylamid |
| 64 | 8-(2,5-Dichlor-benzolsulfonyl)-1-oxa-2,8-diaza-spiro[4.5]dec-2-en-3-carbonsäure [1-(3-methoxy-benzyl)-pyrrolidin-3-yl]-amid |

Tabelle 1 (fortgesetzt)

| Beispiel | Verbindung |
|---|---|
| 65 | 3-(4-Naphthalin-2-ylmethyl-piperazine-1-carbonyl)-1-oxa-2,8-diaza-spiro[4.5]dec-2-en-8-sulfon säure dimethylamid |
| 66 | 3-{[8-(7,7-Dimethyl-2-oxo-bicyclo[2.2.1]hept-1-ylmethansulfonyl)-1-oxa-2,8-diaza-spiro[4.5]dec-2-en-3-carbonyl]-amino}-propionsäure ethyl ester |
| 67 | 8-(Toluol-4-sulfonyl)-1-oxa-2,8-diaza-spiro[4.5]dec-2-en-3-carbonsäure (2-benzyloxy-cyclopentyl)-amid |
| 68 | 8-(2,5-Dichlor-benzolsulfonyl)-1-oxa-2,8-diaza-spiro[4.5]dec-2-en-3-carbonsäure 3,4-dimethoxy-benzylamid |
| 69 | (8-Phenylmethansulfonyl-1-oxa-2,8-diaza-spiro[4.5]dec-2-en-3-yl)-[4-(3-phenyl-propyl)-piperazin-1-yl]-methanon |
| 70 | 3-(4-Thieno[2,3-d]pyrimidin-4-yl-piperazine-1-carbonyl)-1-oxa-2,8-diaza-spiro[4.5]dec-2-en-8-sulfon säure dimethylamid |
| 71 | [8-(5-Chlor-thiophen-2-sulfonyl)-1-oxa-2,8-diaza-spiro[4.5]dec-2-en-3-yl]-[4-(3,5-dimethoxy-phenyl)-piperazin-1-yl]-methanon |
| 72 | 8-Dimethylsulfamoyl-1-oxa-2,8-diaza-spiro[4.5]dec-2-en-3-carbonsäure 3-fluor-4-trifluormethyl-benzylamid |
| 73 | 8-(Toluol-4-sulfonyl)-1-oxa-2,8-diaza-spiro[4.5]dec-2-en-3-carbonsäure (2-benzyloxy-cyclopentyl)-amid |
| 74 | 4-[(8-Butyryl-1-oxa-2,8-diaza-spiro[4.5]dec-2-en-3-carbonyl)-amino]-piperidin-1-carbonsäure ethyl ester |
| 75 | 8-(Toluol-4-sulfonyl)-1-oxa-2,8-diaza-spiro[4.5]dec-2-en-3-carbonsäure (2-phenyl-cyclopropyl)-amid |
| 76 | [8-(4-Fluor-benzolsulfonyl)-1-oxa-2,8-diaza-spiro[4.5]dec-2-en-3-yl]-[4-(5-methyl-pyrazine-2-carbonyl)-piperazin-1-yl]-methanon |
| 77 | 8-Dimethylsulfamoyl-1-oxa-2,8-diaza-spiro[4.5]dec-2-en-3-carbonsäure 2,4-difluor-benzylamid |
| 78 | 8-Phenylmethansulfonyl-1-oxa-2,8-diaza-spiro[4.5]dec-2-en-3-carbonsäure [2-(3-fluor-phenyl)-ethyl]-amid |
| 79 | 8-(5-Chlor-thiophen-2-sulfonyl)-1-oxa-2,8-diaza-spiro[4.5]dec-2-en-3-carbonsäure (2,2-diphenyl-ethyl)-amid |
| 80 | 3-[4-(3-Phenyl-propyl)-piperazine-1-carbonyl]-1-oxa-2,8-diaza-spiro[4.5]dec-2-en-8-sulfonsäure dimethylamid |
| 81 | 3-{[8-(3,5-Difluor-benzoyl)-1-oxa-2,8-diaza-spiro[4.5]dec-2-en-3-carbonyl]-amino}-propionsäure ethyl ester |
| 82 | 8-(Propane-1-sulfonyl)-1-oxa-2,8-diaza-spiro[4.5]dec-2-en-3-carbonsäure isobutyl-amid |
| 83 | [4-(3-Phenyl-propyl)-piperazin-1-yl]-[8-(3-trifluormethyl-benzolsulfonyl)-1-oxa-2,8-diaza-spiro[4.5]dec-2-en-3-yl]-methanon |
| 84 | 8-(Thiophen-2-sulfonyl)-1-oxa-2,8-diaza-spiro[4.5]dec-2-en-3-carbonsäure [1-(2.3-dihydro-benzo[1,4] dioxin-5-yl)-ethyl]-amid |
| 85 | 8-Butyryl-1-oxa-2,8-diaza-spiro[4.5]dec-2-en-3-carbonsäure phenylamid |
| 86 | 8-(4-Trifluormethoxy-benzolsulfonyl)-1-oxa-2,8-diaza-spiro[4.5]dec-2-en-3-carbonsäure cyclooctylamid |
| 87 | 8-[2-(2-Methoxy-ethoxy)-acetyl]-1-oxa-2,8-diaza-spiro[4.5]dec-2-en-3-carbonsäure benzylamid |
| 88 | 8-(4-Bromo-3-methyl-benzoyl)-1-oxa-2,8-diaza-spiro[4.5]dec-2-en-3-carbonsäure isobutyl-amid |
| 89 | 8-(5-Chlor-thiophen-2-sulfonyl)-1-oxa-2,8-diaza-spiro[4.5]dec-2-en-3-carbonsäure [3-(methyl-phenyl-amino)-propyl]-amid |

Tabelle 1 (fortgesetzt)

| Beispiel | Verbindung |
|---|---|
| 90 | 4-{[8-(2-Methyl-5-nitro-benzolsulfonyl)-1-oxa-2,8-diaza-spiro[4.5]dec-2-en-3-carbonyl]-amino}-piperidine-1-carbonsäure ethyl ester |
| 91 | [8-(5-Chlor-thiophen-2-sulfonyl)-1-oxa-2,8-diaza-spiro[4.5]dec-2-en-3-yl]-[4-(4-fluor-phenyl)-piperazin-1-yl]-methanon |
| 92 | 8-(2,5-Dichlor-benzolsulfonyl)-1-oxa-2,8-diaza-spiro[4.5]dec-2-en-3-carbonsäure methyl-pyridin-3-ylmethyl-amid |
| 93 | 2-(3,4-Difluor-phenyl)-1-{4-[8-(3-trifluormethyl-benzolsulfonyl)-1-oxa-2,8-diaza-spiro[4.5]dec-2-en-3-carbonyl]-piperazin-1-yl}-ethanon |
| 94 | 8-(3-Trifluormethyl-benzolsulfonyl)-1-oxa-2,8-diaza-spiro[4.5]dec-2-en-3-carbonsäure cyclopropylmethyl-amid |
| 95 | 3-{[8-(2-Methyl-5-nitro-benzolsulfonyl)-1-oxa-2,8-diaza-spiro[4.5]dec-2-en-3-carbonyl]-amino}-propionsäure ethyl ester |
| 96 | 8-[2-(3-Chlor-phenoxy)-acetyl]-1-oxa-2,8-diaza-spiro[4.5]dec-2-en-3-carbonsäure (thiophen-2-ylmethyl)-amid |
| 97 | 8-(4-Trifluormethoxy-benzolsulfonyl)-1-oxa-2,8-diaza-spiro[4.5]dec-2-en-3-carbonsäure benzyl-phenethyl-amid |
| 98 | 8-(2,5-Dichlor-benzolsulfonyl)-1-oxa-2,8-diaza-spiro[4.5]dec-2-en-3-carbonsäure benzyl-phenethyl-amid |
| 99 | 8-(2,5-Dichlor-benzolsulfonyl)-1-oxa-2,8-diaza-spiro[4.5]dec-2-en-3-carbonsäure (2-cyano-ethyl)-pyridin-3-ylmethyl-amid |
| 100 | 8-(2,5-Dichlor-benzolsulfonyl)-1-oxa-2,8-diaza-spiro[4.5]dec-2-en-3-carbonsäure (2-cyano-ethyl) -(tetrahydro-furan-2-ylmethyl)-amid |
| 101 | 8-(3-Trifluormethyl-benzolsulfonyl)-1-oxa-2,8-diaza-spiro[4.5]dec-2-en-3-carbonsäure methyl-pyridin-3-ylmethyl-amid |
| 102 | 8-(Pyridin-4-carbonyl)-1-oxa-2,8-diaza-spiro[4.5]dec-2-en-3-carbonsäure benzylamid |
| 103 | 8-(4-Trifluormethoxy-benzolsulfonyl)-1-oxa-2,8-diaza-spiro[4.5]dec-2-en-3-carbonsäure ethyl-(2-methyl-allyl)-amid |
| 104 | 8-(4-Methoxy-benzolsulfonyl)-1-oxa-2,8-diaza-spiro[4.5]dec-2-en-3-carbonsäure [2-(2-fluor-phenyl)-ethyl]-amid |
| 105 | 8-(5-tert-Butyl-2-methyl-furan-3-carbonyl)-1-oxa-2,8-diaza-spiro[4.5]dec-2-en-3-carbonsäure isobutyl-amid |
| 106 | 8-(3-Phenyl-acryloyl)-1-oxa-2,8-diaza-spiro[4.5]dec-2-en-3-carbonsäure (thiophen-2-ylmethyl)-amid |
| 107 | 8-(4-Trifluormethyl-benzoyl)-1-oxa-2,8-diaza-spiro[4.5]dec-2-en-3-carbonsäure cyclopropylmethyl-amid |
| 108 | 8-(4-Trifluormethylsulfanyl-benzoyl)-1-oxa-2,8-diaza-spiro[4.5]dec-2-en-3-carbonsäure cyclopropylmethyl-amid |
| 109 | 8-(4-Bromo-benzoyl)-1-oxa-2,8-diaza-spiro[4.5]dec-2-en-3-carbonsäure (5-methyl-furan-2-ylmethyl)-amid |
| 110 | 8-(3-Fluor-4-trifluormethyl-benzoyl)-1-oxa-2,8-diaza-spiro[4.5]dec-2-en-3-carbonsäure cyclopropylmethyl-amid |
| 111 | 8-(7,7-Dimethyl-2-oxo-bicyclo[2.2.1]hept-1-ylmethansulfonyl)-1-oxa-2,8-diaza-spiro[4.5]dec-2-en-3-carbonsäure (2-ethylsulfanyl-ethyl)-amid |
| 112 | 8-(2,4-Difluor-benzoyl)-1-oxa-2,8-diaza-spiro[4.5]dec-2-en-3-carbonsäure (tetrahydro-furan-2-ylmethyl)-amid |

Tabelle 1 (fortgesetzt)

| Beispiel | Verbindung |
|---|---|
| 113 | {[8-(3-Chlor-thiophen-2-carbonyl)-1-oxa-2,8-diaza-spiro[4.5]dec-2-en-3-carbonyl]-amino}-essig säure ethyl ester |
| 114 | 4-Oxo-4-{3-[(thiophen-2-ylmethyl)-carbamoyl]-1-oxa-2,8-diaza-spiro[4.5]dec-2-en-8-yl}-butter säure methyl ester |
| 115 | 8-(2-Ethylsulfanyl-pyridine-3-carbonyl)-1-oxa-2,8-diaza-spiro[4.5]dec-2-en-3-carbonsäure benzylamid |
| 116 | 3-{[8-(2-Chlor-benzolsulfonyl)-1-oxa-2,8-diaza-spiro[4.5]dec-2-en-3-carbonyl]-amino}-propionsäure ethyl ester |
| 117 | 8-(4-Trifluormethoxy-benzoyl)-1-oxa-2,8-diaza-spiro[4.5]dec-2-en-3-carbonsäure cyclopropylmethyl-amid |
| 118 | 8-(4-Phenoxy-butyryl)-1-oxa-2,8-diaza-spiro[4.5]dec-2-en-3-carbonsäure cyclopropylmethyl-amid |
| 119 | [8-(3-Trifluormethyl-benzolsulfonyl)-1-oxa-2,8-diaza-spiro[4.5]dec-2-en-3-yl]-[4-(4-trifluormethyl-pyridin-2-yl)-piperazin-1-yl]-methanon |
| 120 | 8-(2-Chlor-5-trifluormethyl-benzoyl)-1-oxa-2,8-diaza-spiro[4.5]dec-2-en-3-carbonsäure cyclopropylmethyl-amid |
| 121 | 8-(4-Methoxy-benzolsulfonyl)-1-oxa-2,8-diaza-spiro[4.5]dec-2-en-3-carbonsäure (2-benzyloxy-cyclopentyl)-amid |
| 122 | 4-{[8-(3-Trifluormethyl-benzolsulfonyl)-1-oxa-2,8-diaza-spiro[4.5]dec-2-en-3-carbonyl]-amino}-piperidin-1-carbonsäure ethyl ester |
| 123 | 8-(2-Phenoxy-propionyl)-1-oxa-2,8-diaza-spiro[4.5]dec-2-en-3-carbonsäure cyclopropylmethyl-amid |
| 124 | 8-(4-Fluor-benzolsulfonyl)-1-oxa-2,8-diaza-spiro[4.5]dec-2-en-3-carbonsäure [2-(3-methoxy-phenyl)-ethyl]-amid |
| 125 | 8-(2-Methylsulfanyl-pyridine-3-carbonyl)-1-oxa-2,8-diaza-spiro[4.5]dec-2-en-3-carbonsäure isobutyl-amid |
| 126 | 8-(5-*tert*-Butyl-2-methyl-furan-3-carbonyl)-1-oxa-2,8-diaza-spiro[4.5]dec-2-en-3-carbonsäure cyclopentylamid |
| 127 | 8-(4-Chlor-benzolsulfonyl)-1-oxa-2,8-diaza-spiro[4.5]dec-2-en-3-carbonsäure (thiophen-2-ylmethyl)-amid |
| 128 | 8-(5-Chlor-thiophen-2-sulfonyl)-1-oxa-2,8-diaza-spiro[4.5]dec-2-en-3-carbonsäure (naphthalin-1-ylmethyl)-amid |
| 129 | 8-(4-Trifluormethoxy-benzolsulfonyl)-1-oxa-2,8-diaza-spiro[4.5]dec-2-en-3-carbonsäure [2-(4-fluor-phenyl)-ethyl]-amid |
| 130 | 8-(4-Bromo-3-methyl-benzoyl)-1-oxa-2,8-diaza-spiro[4.5]dec-2-en-3-carbonsäure (pyridin-4-ylmethyl)-amid |
| 131 | 8-(4-Methoxy-benzoyl)-1-oxa-2,8-diaza-spiro[4.5]dec-2-en-3-carbonsäure benzylamid |
| 132 | 8-(3-Trifluormethyl-benzolsulfonyl)-1-oxa-2,8-diaza-spiro[4.5]dec-2-en-3-carbonsäure 3-chlor-benzylamid |
| 133 | (8-Phenylmethansulfonyl-1-oxa-2,8-diaza-spiro[4.5]dec-2-en-3-yl)-(4-o-tolyl-piperazin-1-yl)-methanon |
| 134 | (8-Phenylmethansulfonyl-1-oxa-2,8-diaza-spiro[4.5]dec-2-en-3-yl)-(4-pyridin-2-yl-piperazin-1-yl)-methanon |
| 135 | 8-(4-Trifluormethylsulfanyl-benzoyl)-1-oxa-2,8-diaza-spiro[4.5]dec-2-en-3-carbonsäure (thiophen-2-ylmethyl)-amid |
| 136 | 8-(Thiophen-2-sulfonyl)-1-oxa-2,8-diaza-spiro[4.5]dec-2-en-3-carbonsäure benzylamid |
| 137 | 8-(4-Fluor-benzoyl)-1-oxa-2,8-diaza-spiro[4.5]dec-2-en-3-carbonsäure isobutylamid |

Tabelle 1 (fortgesetzt)

| Beispiel | Verbindung |
|---|---|
| 138 | 2-(3-Isobutylcarbamoyl-1-oxa-2,8-diaza-spiro[4.5]dec-2-en-8-sulfonyl)-benzoesäure methyl ester |
| 139 | 2-[(8-Dimethylsulfamoyl-1-oxa-2,8-diaza-spiro[4.5]dec-2-en-3-carbonyl)-amino]-3-phenyl-propionsäure tert-butyl ester |
| 140 | 8-(5-Chlor-thiophen-2-sulfonyl)-1-oxa-2,8-diaza-spiro[4.5]dec-2-en-3-carbonsäure [1-(2,3-dihydro-benzo[1,4]dioxin-5-yl)-ethyl]-amid |
| 141 | 8-(4-Brom-benzoyl)-1-oxa-2,8-diaza-spiro[4.5]dec-2-en-3-carbonsäure (thiophen-2-ylmethyl)-amid |
| 142 | 8-(4-Fluor-benzolsulfonyl)-1-oxa-2,8-diaza-spiro[4.5]dec-2-en-3-carbonsäure (3-imidazol-1-yl-propyl)-amid |
| 143 | 4-[8-(4-Fluor-benzolsulfonyl)-1-oxa-2,8-diaza-spiro[4.5]dec-2-en-3-carbonyl]-piperazine-1-carbonsäure benzyl ester |
| 144 | 3-(4-Cycloheptyl-piperazine-1-carbonyl)-1-oxa-2,8-diaza-spiro[4.5]dec-2-en-8-sulfon säure dimethylamid |
| 145 | 4-Methyl-2-{[8-(thiophen-2-sulfonyl)-1-oxa-2,8-diaza-spiro[4.5]dec-2-en-3-carbonyl]-amino}-pentansäure tert-butyl ester |
| 146 | 8-(3-Chlor-2-fluor-benzoyl)-1-oxa-2,8-diaza-spiro[4.5]dec-2-en-3-carbonsäure (thiophen-2-ylmethyl)-amid |
| 147 | 2-{[8-(4-Methoxy-benzolsulfonyl)-1-oxa-2,8-diaza-spiro[4.5]dec-2-en-3-carbonyl]-amino}-4-methyl-pentanoic säure tert-butyl ester |
| 148 | 3-{[8-(6-Chlor-2-fluor-3-methyl-benzoyl)-1-oxa-2,8-diaza-spiro[4.5]dec-2-en-3-carbonyl]-amino}-propionsäure ethyl ester |
| 149 | 8-(2-Phenoxy-propionyl)-1-oxa-2,8-diaza-spiro[4.5]dec-2-en-3-carbonsäure benzylamid |
| 150 | 8-(5-Fluor-2-methyl-benzolsulfonyl)-1-oxa-2,8-diaza-spiro[4.5]dec-2-en-3-carbonsäure (thiophen-2-ylmethyl)-amid |
| 151 | [8-(5-Chlor-thiophen-2-sulfonyl)-1-oxa-2,8-diaza-spiro[4.5]dec-2-en-3-yl]-[4-(2-methoxy-phenyl)-piperidin-1-yl]-methanon |
| 152 | 8-(2-Chlor-benzoyl)-1-oxa-2,8-diaza-spiro[4.5]dec-2-en-3-carbonsäure (thiophen-2-ylmethyl)-amid |
| 153 | 8-(2-Phenoxy-pyridine-3-carbonyl)-1-oxa-2,8-diaza-spiro[4.5]dec-2-en-3-carbonsäure cyclopentylamid |
| 154 | 8-(4-Bromo-benzoyl)-1-oxa-2,8-diaza-spiro[4.5]dec-2-en-3-carbonsäure (2-ethylsulfanyl-ethyl)-amid |
| 155 | 1-[8-(5-Chlor-thiophen-2-suffonyl)-1-oxa-2,8-diaza-spiro[4.5]dec-2-en-3-carbonyl]-piperidin-2-carbonsäure ethyl ester |
| 156 | 8-(2-Chlor-benzolsulfonyl)-1-oxa-2,8-diaza-spiro[4.5]dec-2-en-3-carbonsäure (thiophen-2-ylmethyl)-amid |
| 157 | 8-(2,3-Dichlor-benzoyl)-1-oxa-2,8-diaza-spiro[4.5]dec-2-en-3-carbonsäure cyclopropylmethyl-amid |
| 158 | 8-Hexanoyl-1-oxa-2,8-diaza-spiro[4.5]dec-2-en-3-carbonsäure cyclopropylmethyl-amid |
| 159 | 8-(2,3-Difluor-4-methyl-benzoyl)-1-oxa-2,8-diaza-spiro[4.5]dec-2-en-3-carbonsäure cyclopropylmethyl-amid |
| 160 | 8-(4-Fluor-benzolsulfonyl)-1-oxa-2,8-diaza-spiro[4.5]dec-2-en-3-carbonsäure (2,5-dimethoxy-benzyl)-furan-2-ylmethyl-amid |
| 161 | 8-Dimethylsulfamoyl-1-oxa-2,8-diaza-spiro[4.5]dec-2-en-3-carbonsäure (2-ethylsulfanyl-ethyl)-amid |
| 162 | 8-(4-Trifluormethoxy-benzolsulfonyl)-1-oxa-2,8-diaza-spiro[4.5]dec-2-en-3-carbonsäure 3-methoxy-benzylamid |

Tabelle 1 (fortgesetzt)

| Beispiel | Verbindung |
|---|---|
| 163 | 8-Dimethylsulfamoyl-1-oxa-2,8-diaza-spiro[4.5]dec-2-en-3-carbonsäure (2-benzyloxy-cyclopentyl)-amid |
| 164 | 8-(4-Methoxy-benzolsulfonyl)-1-oxa-2,8-diaza-spiro[4.5]dec-2-en-3-carbonsäure [2-(4-Chlor-phenyl)-1-methyl-ethyl]-amid |
| 165 | 8-(4-Trifluormethoxy-benzolsulfonyl)-1-oxa-2,8-diaza-spiro[4.5]dec-2-en-3-carbonsäure [2-(3,4-Dichlor-phenyl)-ethyl]-amid |
| 166 | 8-[2-(3-Chlor-phenoxy)-acetyl]-1-oxa-2,8-diaza-spiro[4.5]dec-2-en-3-carbonsäure benzylamid |
| 167 | 8-(4-Fluor-benzolsulfonyl)-1-oxa-2,8-diaza-spiro[4.5]dec-2-en-3-carbonsäure (2-cyano-ethyl)-(2-pyridin-2-yl-ethyl)-amid |
| 168 | 8-(5-Chlor-thiophen-2-sulfonyl)-1-oxa-2,8-diaza-spiro[4.5]dec-2-en-3-carbonsäure [2-(4-Chlor-phenyl)-1-methyl-ethyl]-amid |
| 169 | 8-(4-Methoxy-benzolsulfonyl)-1-oxa-2,8-diaza-spiro[4.5]dec-2-en-3-carbonsäure (2-benzyloxy-cyclopentyl)-amid |
| 170 | 8-(2,6-Difluor-3-methyl-benzoyl)-1-oxa-2,8-diaza-spiro[4.5]dec-2-en-3-carbonsäure (thiophen-2-ylmethyl)-amid |
| 171 | 8-(2-Benzyloxy-acetyl)-1-oxa-2,8-diaza-spiro[4.5]dec-2-en-3-carbonsäure benzylamid |
| 172 | 8-(2,3-Dichlor-benzoyl)-1-oxa-2,8-diaza-spiro[4.5]dec-2-en-3-carbonsäure phenylamid |
| 173 | 8-(5-Bromo-2-methoxy-benzolsulfonyl)-1-oxa-2,8-diaza-spiro[4.5]dec-2-en-3-carbonsäure cyclopentylamid |
| 174 | Essig säure 2-(3-benzylcarbamoyl-1-oxa-2,8-diaza-spiro[4.5]dec-2-en-8-yl)-1,1-dimethyl-2-oxo-ethyl ester |
| 175 | 8-[3-(2-Chlor-6-fluor-phenyl)-5-methyl-isoxazole-4-carbonyl]-1-oxa-2,8-diaza-spiro[4.5]dec-2-en-3-carbonsäure (2-ethylsulfanyl-ethyl)-amid |
| 176 | [4-(3-Phenyl-propyl)-piperazin-1-yl]-[8-(4-trifluormethoxy-benzolsulfonyl)-1-oxa-2,8-diaza-spiro[4.5]dec-2-en-3-yl]-methanon |
| 177 | 8-[3-(2-Chlor-6-fluor-phenyl)-5-methyl-isoxazole-4-carbonyl]-1-oxa-2,8-diaza-spiro[4.5]dec-2-en-3-carbonsäure benzylamid |
| 178 | 8-(Naphthalin-1-carbonyl)-1-oxa-2,8-diaza-spiro[4.5]dec-2-en-3-carbonsäure isobutyl-amid |
| 179 | 1-[8-(5-tert-Butyl-2-methyl-furan-3-carbonyl)-1-oxa-2,8-diaza-spiro[4.5]deo-2-en-3-carbonyl]-piperidin-2-carbonsäure ethyl ester |
| 180 | 8-(3-Difluormethylsulfanyl-benzoyl)-1-oxa-2,8-diaza-spiro[4.5]dec-2-en-3-carbonsäure benzylamid |
| 181 | 8-(4-Bromo-benzoyl)-1-oxa-2,8-diaza-spiro[4.5]dec-2-en-3-carbonsäure phenylamid |
| 182 | 8-(4-Methoxy-benzolsulfonyl)-1-oxa-2,8-diaza-spiro[4.5]dec-2-en-3-carbonsäure (2-phenyl-cyclopropyl)-amid |
| 183 | 8-(4-Phenoxy-butyryl)-1-oxa-2,8-diaza-spiro[4.5]dec-2-en-3-carbonsäure (thiophen-2-ylmethyl)-amid |
| 184 | 8-(3-Chlor-4-fluor-benzoyl)-1-oxa-2,8-diaza-spiro[4.5]dec-2-en-3-carbonsäure (thiophen-2-ylmethyl)-amid |
| 185 | 8-(5-tert-Butyl-2-methyl-2H-pyrazole-3-carbonyl)-1-oxa-2,8-diaza-spiro[4.5]dec-2-en-3-carbonsäure (2-ethylsulfanyl-ethyl)-amid |
| 186 | [8-(4-Fluor-benzolsulfonyl)-1-oxa-2,8-diaza-spiro[4.5]dec-2-en-3-yl]-[4-(3-phenylpropyl)-piperazin-1-yl]-methanon |
| 187 | 8-(4-Trifluormethoxy-benzoyl)-1-oxa-2,8-diaza-spiro[4.5]dec-2-en-3-carbonsäure (pyridin-4-ylmethyl)-amid |

Tabelle 1 (fortgesetzt)

| Beispiel | Verbindung |
|---|---|
| 188 | Benzoesäure 2-(3-cyclopentylcarbamoyl-1-oxa-2,8-diaza-spiro[4.5]dec-2-en-8-carbonyl)-benzyl ester |
| 189 | 3-[4-(4-tert-Butyl-benzyl)-piperazine-1-carbonyl]-1-oxa-2,8-diaza-spiro[4.5]dec-2-en-8-sulfon säure dimethylamid |
| 190 | {[8-(2-Ethoxy-benzoyl)-1-oxa-2,8-diaza-spiro[4.5]dec-2-en-3-carbonyl]-amino}-essig säure ethyl ester |
| 191 | 8-(5-Chlor-thiophen-2-sulfonyl)-1-oxa-2,8-diaza-spiro[4.5]dec-2-en-3-carbonsäure (2-benzyloxy-cyclopentyl)-amid |
| 192 | 8-(Naphthalin-1-carbonyl)-1-oxa-2,8-diaza-spiro[4.5]dec-2-en-3-carbonsäure (thiophen-2-ylmethyl)-amid |
| 193 | 8-(4-Fluor-benzolsulfonyl)-1-oxa-2,8-diaza-spiro[4.5]dec-2-en-3-carbonsäure (3-phenyl-propyl)-amid |
| 194 | 8-(5-Chlor-thiophen-2-sulfonyl)-1-oxa-2,8-diaza-spiro[4.5]dec-2-en-3-carbonsäure (2,3-dihydro-benzo [1,4]dioxin-2-ylmethyl)-amid |
| 195 | 1-[8-(4-tert-Butyl-benzoyl)-1-oxa-2,8-diaza-spiro[4.5]dec-2-en-3-carbonyl]-piperidin-2-carbonsäure ethyl ester |
| 196 | 8-(4-Fluor-benzolsulfonyl)-1-oxa-2,8-diaza-spiro[4.5]dec-2-en-3-carbonsäure [2-(3-trifluormethyl-phenyl)-ethyl]-amid |
| 197 | 8-[3-(2,6-Dichior-phenyl)-5-methyl-isoxazole-4-carbonyl]-1-oxa-2,8-diaza-spiro[4.5]dec-2-en-3-carbonsäure (thiophen-2-ylmethyl)-amid |
| 198 | 8-(4-Fluor-benzolsulfonyl)-1-oxa-2,8-diaza-spiro[4.5]dec-2-en-3-carbonsäure [1-(naphthalen-2-ylcarbamoyl)-ethyl]-amid |
| 199 | 8-(4-fluor-benzolsulfonyl)-1-oxa-2,8-diaza-spiro[4.5]dec-2-en-3-carbonsäure (2-beneoxy-cyclopentyl)-amid |
| 200 | 3-(4-Chlor-phenyl)-2-{[8-(4-methoxy-benzolsulfonyl)-1-oxa-2,8-diaza-spiro[4.5]dec-2-en-3-carbonyl]-amino}-propionsäure methyl ester |
| 201 | 8-(3-Trifluormethyl-benzolsulfonyl)-1-oxa-2,8-diaza-spiro[4.5]dec-2-en-3-carbonsäure (2-benzyloxy-cyclopentyl)-amid |
| 202 | 8-(3-Trifluormethyl-benzolsulfonyl)-1-oxa-2,8-diaza-spiro[4.5]dec-2-en-3-carbonsäure (2-phenoxy-ethyl)-amid |
| 203 | 8-(2-Phenoxy-pyridin-3-carbonyl)-1-oxa-2,8-diaza-spiro[4.5]dec-2-en-3-carbonsäure benzylamid |
| 204 | 8-(Naphthalin-2-carbonyl)-1-oxa-2,8-diaza-spiro[4.5]dec-2-en-3-carbonsäure cyctopropylmethyl-amid |
| 205 | 8-(3-Chlor-4-fluor-benzoyl)-1-oxa-2,8-diaza-spiro[4.5]dec-2-en-3-carbonsäure benzylamid |
| 206 | 8-(2-Phenyl-butyryl)-1-oxa-2,8-diaza-spiro[4.5]dec-2-en-3-carbonsäure (2-cyano-ethyl)-amid |
| 207 | 8-(4-Fluor-benzolsulfonyl)-1-oxa-2,8-diaza-spiro[4.5]dec-2-en-3-carbonsäure 2-fluor-benzylamid |
| 208 | 8-(3-Chlor-benzoyl)-1-oxa-2,8-diaza-spiro[4.5]dec-2-en-3-carbonsäure (thiophen-2-ylmethyl)-amid |
| 209 | (4-Benzyl-piperazin-1-yl)-[8-(2,5-Dichlor-benzolsulfonyl)-1-oxa-2,8-diaza-spiro[4.5]dec-2-en-3-yl]-methanon |
| 210 | 8-(3-Chlor-benzolsulfonyl)-1-oxa-2,8-diaza-spiro[4.5]dec-2-en-3-carbonsäure benzylamid |
| 211 | (4-Benzyl-piperidin-1-yl)-[8-(3-trifluormethyl-benzolsulfonyl)-1-oxa-2,8-diaza-spiro[4.5]dec-2-en-3-yl]-methanon |
| 212 | 8-(4-Bromo-benzoyl)-1-oxa-2,8-diaza-spiro[4.5]dec-2-en-3-carbonsäure benzylamid |
| 213 | 8-(4-Fluor-benzolsulfonyl)-1-oxa-2,8-diaza-spiro[4.5]dec-2-en-3-carbonsäure [1-(4-nitro-phenyl)-ethyl]-amid |
| 214 | 4-Methyl-2-{[8-(3-trifluormethyl-benzolsulfonyl)-1-oxa-2,8-diaza-spiro[4.5]dec-2-en-3-carbonyl]-amino}-pentansäure tert-butyl ester |

Tabelle 1 (fortgesetzt)

| Beispiel | Verbindung |
|---|---|
| 215 | [4-(4-Fluor-phenyl)-3,6-dihydro-2H-pyridin-1-yl]-(8-phenylmethansulfonyl-1-oxa-2,8-diaza-spiro[4.5]dec-2-en-3-yl)-methanon |
| 216 | 8-Dimethylsulfamoyl-1-oxa-2,8-diaza-spiro[4.5]dec-2-en-3-carbonsäure [2-(ethyl-m-tolyl-amino)-ethyl]-amid |
| 217 | 8-(2,5-Dimethyl-furan-3-carbonyl)-1-oxa-2,8-diaza-spiro[4.5]dec-2-en-3-carbonsäure benzylamid |
| 218 | (4-Cycloheptyl-piperazin-1-yl)-[8-(4-fluor-benzolsulfonyl)-1-oxa-2,8-diaza-spiro[4.5]dec-2-en-3-yl]-methanon |
| 219 | 8-(2-Benzyloxy-acetyl)-1-oxa-2,8-diaza-spiro[4.5]dec-2-en-3-carbonsäure benzylamid |
| 220 | R,R-8-(5-Chlor-thiophen-2-sulfonyl)-1-oxa-2,8-diaza-spiro[4.5]dec-2-en-3-carbonsäure (2-benzyloxy-cyclopentyl)-amid |

**Pharmakologische Testung**

Erfindungsgemäße substituierte 1-Oxa-2,8-diaza-spiro[4.5]dec-2-en-derivate wurden folgenden pharmakologischen Tests unterworfen:

**Bindungsuntersuchungen am Natriumkanal**
**Bindungsstelle 2 (BTX-Bindung):**

Die Bindungsstelle 2 des Natriumkanals ist die sogenannte Batrachotoxin-(BTX) Bindungsstelle. Als Ligand wurde [$^3$H]-Batrachotoxinin A20 $\alpha$-Benzoat (10 nM im Ansatz) eingesetzt. Diese Ionenkanal-Partikel (Synaptosomen) wurden aus dem Ratten-Cerebrocortex nach Gray und Whittaker (E.G. Gray und V.P. Whittaker (1962) J. Anat. 76, 79-88) angereichert. Als unspezifische Bindung ist die Radioaktivität definiert, die in Gegenwart von Veratridin gemessen wird. Inkubation bei 37°C für 120 min. Die Assaybedingungen sind nach der Veröffentlichung von Pauwels, Leysen und Laduron (P.J. Pauwels, J.E. Leysen und P.M. Laduron (1986) Eur. J. Pharmacol. 124, 291-298) durchgeführt worden.

Erfindungsgemäße Verbindungen wurden in diesem Assay getestet; die Meßergebnisse sind in Tabelle 2 wiedergegeben:

Tabelle 2

| Beispiel | BTX-Bindung, 10$\mu$M, %Hemmung |
|---|---|
| 1 | 68 |
| 2 | 55 |
| 3 | 78 |
| 5 | 64 |
| 6 | 49 |
| 8 | 63 |
| 11 | 69 |
| 14 | 79 |
| 15 | 88 |
| 16 | 60 |
| 18 | 53 |
| 19 | 66 |
| 20 | 69 |

Tabelle 2 (fortgesetzt)

| Beispiel | BTX-Bindung, 10μM, %Hemmung |
|---|---|
| 21 | 63 |
| 23 | 60 |
| 24 | 41 |
| 26 | 84 |
| 27 | 74 |
| 28 | 52 |
| 30 | 56 |
| 31 | 80 |
| 32 | 66 |
| 33 | 67 |
| 35 | 50 |
| 37 | 70 |
| 38 | 65 |
| 40 | 47 |
| 45 | 49 |
| 47 | 71 |
| 48 | 43 |
| 50 | 87 |
| 52 | 63 |
| 53 | 47 |
| 55 | 47 |
| 57 | 45 |
| 61 | 79 |
| 62 | 49 |
| 63 | 38 |
| 64 | 91 |
| 65 | 67 |
| 67 | 80 |
| 69 | 50 |
| 71 | 54 |
| 73 | 75 |
| 74 | 47 |
| 79 | 83 |
| 80 | 44 |
| 83 | 72 |
| 84 | 58 |
| 85 | 62 |

Tabelle 2 (fortgesetzt)

| Beispiel | BTX-Bindung, 10μM, %Hemmung |
|---|---|
| 87 | 42 |
| 88 | 45 |
| 89 | 81 |
| 91 | 72 |
| 92 | 65 |
| 93 | 52 |
| 96 | 72 |
| 97 | 79 |
| 98 | 56 |
| 99 | 50 |
| 102 | 43 |
| 103 | 53 |
| 106 | 62 |
| 108 | 43 |
| 115 | 48 |
| 119 | 72 |
| 121 | 42 |
| 124 | 54 |
| 126 | 56 |
| 127 | 70 |
| 133 | 47 |
| 135 | 85 |
| 136 | 44 |
| 140 | 60 |
| 141 | 61 |
| 144 | 53 |
| 146 | 67 |
| 149 | 40 |
| 150 | 86 |
| 152 | 56 |
| 153 | 41 |
| 155 | 48 |
| 156 | 70 |
| 160 | 75 |
| 163 | 40 |
| 164 | 52 |
| 166 | 58 |

Tabelle 2 (fortgesetzt)

| Beispiel | BTX-Bindung, 10µM, %Hemmung |
|---|---|
| 168 | 69 |
| 169 | 48 |
| 170 | 59 |
| 171 | 44 |
| 172 | 60 |
| 173 | 62 |
| 176 | 76 |
| 177 | 45 |
| 179 | 55 |
| 180 | 57 |
| 181 | 50 |
| 182 | 47 |
| 183 | 68 |
| 184 | 73 |
| 186 | 56 |
| 188 | 77 |
| 189 | 69 |
| 191 | 75 |
| 192 | 87 |
| 193 | 66 |
| 194 | 68 |
| 195 | 85 |
| 196 | 51 |
| 197 | 70 |
| 199 | 67 |
| 201 | 61 |
| 205 | 53 |
| 207 | 56 |
| 208 | 74 |
| 209 | 61 |
| 210 | 62 |
| 211 | 48 |
| 212 | 58 |
| 214 | 61 |
| 216 | 64 |
| 217 | 51 |
| 218 | 79 |

**Untersuchungen zur Noradrenalin-Wiederaufnahmehemmung (NA-Uptakehemmung)**

Um diese in vitro-Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Folgende Kenndaten wurden für den NA-Transporter ermittelt:

$$\text{NA-Uptake : Km} = 0{,}32 \pm 0{,}11\ \mu\text{M}$$

(Jeweils N = 4, d.h. Mittelwerte ± SEM aus 4 unabhängigen Versuchsreihen, die in Dreifach-Parallelversuchen durchgeführt wurden).

Eine detaillierte Methodenbeschreibung kann der Litaratur entnommen werden (M.Ch. Frink, H.-H. Hennies, W. Englberger, M. Haurand und B. Wilffert (1996) Arzneim.-Forsch./Drug Res. 46 (III), 11, 1029-1036).

Erfindungsgemäße Verbindungen wurden in diesem Assay getestet; die Meßergebnisse sind in Tabelle 3 wiedergegeben:

Tabelle 3

| Beispiel | NA-Uptake, 10μM, % Hemmung |
|---|---|
| 3 | 59 |
| 4 | 45 |
| 5 | 53 |
| 7 | 50 |
| 8 | 48 |
| 9 | 77 |
| 11 | 41 |
| 12 | 54 |
| 13 | 43 |
| 14 | 70 |
| 15 | 41 |
| 18 | 64 |
| 20 | 70 |
| 21 | 61 |
| 22 | 42 |
| 23 | 49 |
| 27 | 80 |
| 30 | 48 |
| 31 | 66 |
| 32 | 43 |
| 33 | 42 |
| 37 | 52 |
| 38 | 44 |
| 42 | 47 |
| 44 | 48 |

Tabelle 3 (fortgesetzt)

| Beispiel | NA-Uptake, 10µM, % Hemmung |
|---|---|
| 45 | 59 |
| 47 | 92 |
| 50 | 50 |
| 55 | 50 |
| 64 | 61 |
| 65 | 63 |
| 67 | 59 |
| 69 | 56 |
| 73 | 93 |
| 75 | 65 |
| 78 | 42 |
| 79 | 58 |
| 80 | 60 |
| 84 | 67 |
| 89 | 68 |
| 96 | 49 |
| 97 | 83 |
| 98 | 82 |
| 101 | 43 |
| 102 | 48 |
| 103 | 47 |
| 104 | 49 |
| 105 | 53 |
| 106 | 47 |
| 107 | 42 |
| 108 | 42 |
| 109 | 40 |
| 110 | 45 |
| 111 | 40 |
| 112 | 45 |
| 113 | 50 |
| 114 | 42 |
| 115 | 55 |
| 116 | 60 |
| 117 | 46 |
| 118 | 46 |
| 119 | 43 |
| 120 | 40 |

Tabelle 3 (fortgesetzt)

| Beispiel | NA-Uptake, 10µM, % Hemmung |
|---|---|
| 121 | 55 |
| 122 | 41 |
| 123 | 42 |
| 124 | 49 |
| 125 | 40 |
| 126 | 67 |
| 127 | 51 |
| 128 | 45 |
| 129 | 43 |
| 130 | 61 |
| 131 | 57 |
| 132 | 46 |
| 133 | 48 |
| 134 | 51 |
| 135 | 56 |
| 136 | 53 |
| 137 | 49 |
| 138 | 49 |
| 139 | 51 |
| 140 | 83 |
| 141 | 61 |
| 142 | 49 |
| 143 | 47 |
| 144 | 56 |
| 145 | 46 |
| 146 | 61 |
| 149 | 50 |
| 150 | 48 |
| 153 | 45 |
| 155 | 41 |
| 156 | 60 |
| 163 | 62 |
| 164 | 80 |
| 165 | 55 |
| 166 | 53 |
| 167 | 63 |
| 168 | 79 |
| 169 | 64 |

Tabelle 3 (fortgesetzt)

| Beispiel | NA-Uptake, 10μM, % Hemmung |
|---|---|
| 170 | 43 |
| 171 | 73 |
| 172 | 41 |
| 173 | 60 |
| 176 | 52 |
| 177 | 47 |
| 178 | 51 |
| 179 | 49 |
| 180 | 61 |
| 181 | 51 |
| 182 | 73 |
| 183 | 67 |
| 184 | 62 |
| 185 | 45 |
| 186 | 63 |
| 187 | 44 |
| 188 | 49 |
| 189 | 45 |
| 191 | 74 |
| 192 | 41 |
| 193 | 47 |
| 194 | 65 |
| 195 | 41 |
| 196 | 40 |
| 197 | 41 |
| 198 | 52 |
| 199 | 78 |
| 200 | 43 |
| 201 | 58 |
| 203 | 58 |
| 204 | 48 |
| 205 | 46 |
| 207 | 50 |
| 208 | 52 |
| 209 | 51 |
| 210 | 42 |
| 211 | 49 |
| 212 | 55 |

Tabelle 3 (fortgesetzt)

| Beispiel | NA-Uptake, 10µM, % Hemmung |
|---|---|
| 213 | 46 |
| 214 | 45 |
| 215 | 42 |
| 216 | 40 |
| 217 | 40 |
| 218 | 44 |

**Untersuchungen zur Serotonin-Wiederaufnahmehemmung (5HT-Uptakehemmung)**

Um diese in vitro-Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

Erfindungsgemäße Verbindungen wurden in diesem Assay getestet; die Meßergebnisse sind in Tabelle 4 wiedergegeben:

Tabelle 4

| Beispiel | 5HT-Uptake, 10µM, % Hemmung |
|---|---|
| 1 | 47 |
| 2 | 55 |
| 3 | 51 |
| 4 | 54 |
| 5 | 59 |
| 6 | 54 |
| 9 | 75 |
| 10 | 40 |
| 14 | 78 |
| 15 | 55 |
| 16 | 60 |
| 17 | 46 |
| 19 | 80 |
| 24 | 40 |
| 25 | 51 |
| 26 | 65 |
| 27 | 49 |
| 28 | 43 |
| 29 | 42 |
| 31 | 69 |
| 33 | 57 |
| 35 | 44 |

Tabelle 4 (fortgesetzt)

| Beispiel | 5HT-Uptake, 10μM, % Hemmung |
|---|---|
| 37 | 70 |
| 39 | 65 |
| 40 | 45 |
| 41 | 70 |
| 43 | 41 |
| 44 | 80 |
| 46 | 40 |
| 48 | 41 |
| 49 | 50 |
| 50 | 57 |
| 51 | 49 |
| 52 | 60 |
| 53 | 53 |
| 54 | 43 |
| 55 | 65 |
| 56 | 52 |
| 57 | 41 |
| 58 | 55 |
| 59 | 45 |
| 60 | 65 |
| 61 | 72 |
| 62 | 73 |
| 63 | 55 |
| 64 | 61 |
| 65 | 81 |
| 66 | 40 |
| 67 | 58 |
| 68 | 52 |
| 69 | 63 |
| 70 | 54 |
| 71 | 43 |
| 72 | 42 |
| 73 | 46 |
| 74 | 43 |
| 76 | 42 |
| 77 | 41 |
| 81 | 46 |
| 82 | 42 |

Tabelle 4 (fortgesetzt)

| Beispiel | 5HT-Uptake, 10µM, % Hemmung |
|---|---|
| 83 | 52 |
| 84 | 68 |
| 85 | 41 |
| 86 | 46 |
| 87 | 40 |
| 88 | 46 |
| 90 | 48 |
| 91 | 45 |
| 92 | 51 |
| 93 | 42 |
| 94 | 57 |
| 95 | 54 |
| 99 | 44 |
| 100 | 43 |
| 101 | 46 |
| 104 | 41 |
| 109 | 56 |
| 130 | 64 |
| 144 | 41 |
| 147 | 67 |
| 148 | 47 |
| 150 | 70 |
| 152 | 41 |
| 154 | 48 |
| 156 | 41 |
| 157 | 52 |
| 158 | 42 |
| 159 | 40 |
| 160 | 50 |
| 161 | 44 |
| 162 | 40 |
| 164 | 54 |
| 165 | 41 |

**$\alpha_{2A}$-Assay**

Hier wird über die Verdrängung eines markierten Liganden die Bindung gemessen. Der Test wurde durchgeführt nach einer Vorschrift von J.P. Devlin, "High throughput screening - the discovery of bioactive substances", S. 275 - 453, Marcel Dekker, New York, 1997. Membranen, die Alpha-Rezeptoren der Subtypen (human) enthalten wurden käuflich erworben.

Erfindungsgemäße Verbindungen wurden in diesem Assay getestet; die Meßergebnisse sind in Tabelle 5 wiedergegeben:

Tabelle 5

| Beispiel | alpha2A human, 1 μM, % Hemmung |
|---|---|
| 21 | 31 |
| 27 | 37 |
| 151 | 33 |

**Rezeptorbindung (Glycin-Bindungsstelle des NMDA-Rezeptorkanals)**

Die Untersuchungen zur Bestimmung der Affinität der erfindungsgemäßen Verbindungen zur Glycin-Bindungsstelle des NMDA-Rezeptorkanals wurde an Himmembran-homogenaten (Homogenat von Cortex- und Hippocampus-Areal aus dem Hirn von männlichen Ratten, Stamm Wistar) durchgeführt [B.M. Baron , B.W. Siegel, B.L. Harrison, R.S. Gross, C. Hawes and P.Towers, Journal of Pharmacology and Experimental Therapeutics, Vol. 279, S. 62, 1996].

Hierzu wurde Cortex und Hippocampus aus frisch entnommenen Rattengehirnen freipräpariert und in 5 mmol/l TRIS-Acetatpuffer, 0,32 mol/l Sacharose pH 7,4 (10 ml/g Frischgewicht) mit einem Potter-Homogenisator (Fa. Braun/Melsungen 10 Kolbenhübe bei 500 Upm) unter Eiskühlung homogenisiert und anschließend für 10 Minuten bei 1.000 g und 4°C zentrifugiert. Der erste Überstand wurde gesammelt und das Sediment erneut mit 5 mmol/l TRIS-Acetatpuffer, 0,32 mol/l Saccharose pH 7,4 (5 ml/g ursprüngliches Frischgewicht) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membran-sediment mit 5 mmol/l TRIS-Acetatpuffer pH 8,0 (20 ml/g ursprüngliches Frischgewicht) aufgenommen und mit 10 Kolbenhüben bei 500 Upm homogenisiert.

Anschließend wurde das Membranhomogenat für 1 Stunde bei 4°C inkubiert für 30 Minuten bei 50.000 g und 4°C zentrifugiert. Der Überstand wurde verworfen und das Zentrifugen-röhrchen mit dem Membransediment mit Parafilm verschlossen und für 24 Stunden bei -20°C eingefroren. Am folgenden Tag wurde das Membransediment aufgetaut und mit eiskaltem 5 mmol/l TRIS-Acetatpuffer, 0,1 % Saponin (w/v) pH 7,0 (10 ml/g ursprüngliches Frischgewicht) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Testung eingefroren.

Für den Rezeptorbindungstest wurden Aliquote aufgetaut 1:10 mit 5 mmol/l TRIS-Acetatpuffer pH 7,0 verdünnt, mit 10 Kolbenhüben bei 500 Upm mit dem Potter-Homogenisator (10 Kolbenhübe bei 500 Upm) unter Eiskühlung homogenisiert und für 60 Minuten bei 55.000 g bei 4°C zentrifugiert. Der Überstand wurde dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Dieses Membranhomogenat wurde im Rezeptorbindungstest eingesetzt.

Im Bindungstest wurde als Puffer 50 mmol/l TRIS-Acetatpuffer pH 7,0 sowie als radioaktiver Ligand 1 nmol/l ($^3H$)-MDL 105.519 (B.M. Baron et al. 1996) eingesetzt. Der Anteil an unspezifischer Bindung wurde in Anwesenheit von 1 mmol/l Glycin bestimmt.

In weiteren Ansätzen wurden die erfindungsgemäßen Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die Ansätze wurden über 120 Minuten bei 4°C inkubiert und anschließend zur Bestimmung des an das Membranhomogenat gebundenen radioaktiven Liganden mittels Filtration durch Glasfaser-Filtermatten (GF/B) geerntet. Die auf den Glasfaser-Filtern zurückgehaltene Radioaktivität wurde nach Zugabe von Szintillator im β-Counter gemessen.

Erfindungsgemäße Verbindungen wurden in diesem Assay getestet; die Meßergebnisse sind in Tabelle 6 wiedergegeben:

Tabelle 6

| Beispiel | GlyB-Bindung, 10 μM, %Hemmung |
|---|---|
| 34 | 37 |
| 36 | 38 |
| 137 | 36 |
| 174 | 41 |
| 175 | 91 |
| 190 | 89 |
| 202 | 59 |
| 206 | 39 |

**Pharmazeutische Formulierung eines erfindungsgemäßen Arzneimittels**

1 g des Hydrochlorids von 8-(2,5-Dichlorbenzolsulfonyl)-1-oxa-2,8-diaza-spiro[4.5]dec-2-en-3-carbonsäure [1-(3-methoxybenzyl)-pyrrolidin-3-yl]amid (Beispiel 64) wurde in 1 l Wasser für Injektionszwecke bei Raumtemperatur gelöst und anschließend durch Zugabe von Natriumchlorid auf isotone Bedingungen eingestellt.

**Patentansprüche**

1. Substituiertes 1-Oxa-2,8-diaza-spiro[4.5]dec-2-en-derivat der allgemeinen Formel (I)

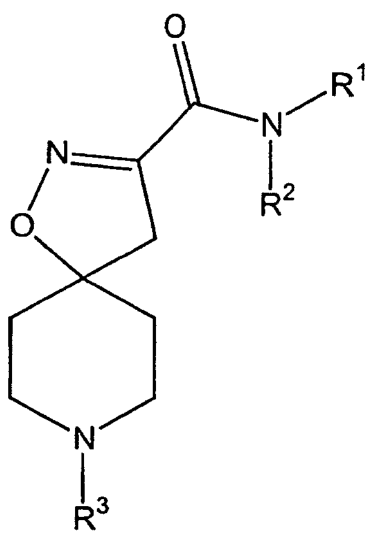

I ,

in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form ihrer Solvate, insbesondere der Hydrate;
worin

$R^1$ und $R^2$ unabhängig voneinander H, $C_{1-18}$-Alkyl, $C_{3-10}$-Cycloalkyl, ($C_{1-12}$-Alkyl)

-$C_{3-10}$-Cycloalkyl, Aryl, ($C_{1-12}$-Alkyl)-Aryl, Heterocyclyl, ($C_{1-12}$-Alkyl)-Heterocyclyl oder NH-C(=O)-Aryl bedeuten, wobei mindestens einer der Reste $R^1$ und $R^2$ nicht H bedeutet,
oder
gemeinsam für $-(CR^4R^5)_m-(CR^6R^7)_n-Y-(CR^8R^9)_p-(CR^{10}R^{11})_q-$ mit m, n, p und q jeweils = 0, 1, 2, 3, 4 oder 5, unter der Maßgabe, daß m+n ≥ 1 und p+q ≥ 1, oder für $-CH_2-CH_2-C(-Aryl)=CH-CH_2-$ stehen;

$R^3$ H, $SO_2R^{12}$ oder $COR^{13}$ bedeutet;

$R^4$, $R^5$, $R^6$, $R^7$, $R^8$, $R^9$, $R^{10}$ und $R^{11}$ unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $C(=O)R^{14}$ bedeuten;

Y $CR^{15}R^{16}$, $NR^{17}$ oder O bedeutet;

$R^{12}$ und $R^{13}$ unabhängig voneinander $C_{1-10}$-Alkyl, $C_{3-10}$-Cycloalkyl, ($C_{1-6}$-Alkyl)-$C_{3-10}$-Cycloalkyl, Aryl, ($C_{1-6}$-Alkyl)-Aryl, Heterocyclyl, ($C_{1-6}$-Alkyl)-Heterocyclyl oder $NR^{18}R^{19}$ bedeuten;

$R^{14}$ H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $OR^{20}$ bedeutet;

$R^{15}$ und $R^{16}$ unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $C(=O)R^{21}$ bedeuten;

$R^{17}$ H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $C(=O)R^{22}$ bedeutet;

$R^{18}$ und $R^{19}$ unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl oder ($C_{1-6}$-Alkyl)-Heterocyclyl bedeuten;

$R^{20}$ H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl oder ($C_{1-6}$-Alkyl)-Heterocyclyl bedeutet;

$R^{21}$ H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $OR^{23}$ bedeutet;

$R^{22}$ H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl, ($C_{1-6}$-Alkyl)-Heterocyclyl oder $OR^{24}$ bedeutet; und

$R^{23}$ und $R^{24}$ unabhängig voneinander H, $C_{1-10}$-Alkyl, $C_{3-8}$-Cycloalkyl, ($C_{1-6}$-Alkyl)-$C_{3-8}$-Cycloalkyl, Aryl, ($C_{1-6}$-Alkyl)-Aryl, Heterocyclyl oder ($C_{1-6}$-Alkyl)-Heterocyclyl bedeuten.

**2.** Substituiertes 1-Oxa-2,8-diaza-spiro[4.5]dec-2-en-derivat gemäß Anspruch 1, **dadurch gekennzeichnet, daß**

$R^1$ und $R^2$ unabhängig voneinander H, $C_{1-8}$-Alkyl, $C_{3-8}$-Cycloalkyl, ($C_{1-4}$-Alkyl)-$C_{3-8}$-Cycloalkyl, Aryl, ($C_{1-4}$-Alkyl)-Aryl, Heterocyclyl, ($C_{1-4}$-Alkyl)-Heterocyclyl oder NH-C(=O)-Aryl bedeuten, wobei mindestens einer der Reste $R^1$ und $R^2$ nicht H bedeutet,
oder
gemeinsam für $-(CR^4R^5)_m-(CR^6R^7)_n-Y-(CR^8R^9)_p-(CR^{10}R^{11})_q-$ mit m = 1, n = 0 oder 1, p = 1 oder 2 und q = 1 oder 2, oder für $-CH_2-CH_2-C(-Aryl)=CH-CH_2-$stehen;

$R^3$ $SO_2R^{12}$ oder $COR^{13}$ bedeutet;

| | |
|---|---|
| $R^4$, $R^5$, $R^6$, $R^7$, $R^8$, $R^9$, $R^{10}$ und $R^{11}$ | unabhängig voneinander H, $C_{1-8}$-Alkyl oder C(=O)$R^{14}$ bedeuten; |
| Y | $CR^{15}R^{16}$ oder $NR^{17}$ bedeutet; |
| $R^{12}$ | $C_{1-4}$-Alkyl, ($C_{1-4}$-Alkyl)-$C_{3-8}$-Cycloalkyl, Aryl, ($C_{1-4}$-Alkyl)-Aryl, Heterocyclyl oder $NR^{18}R^{19}$ bedeutet; |
| $R^{13}$ | $C_{1-6}$-Alkyl, $C_{3-10}$-Cycloalkyl, Aryl, ($C_{1-4}$-Alkyl)-Aryl oder Heterocyclyl bedeutet; |
| $R^{14}$ | $OR^{20}$ bedeutet; |
| $R^{15}$ und $R^{16}$ | unabhängig voneinander H, Aryl oder ($C_{1-4}$-Alkyl)-Aryl bedeuten; |
| $R^{17}$ | H, $C_{3-8}$-Cycloalkyl, Aryl, ($C_{1-4}$-Alkyl)-Aryl, Heterocyclyl oder C(=O)$R^{22}$ bedeutet; |
| $R^{18}$ und $R^{19}$ | unabhängig voneinander H oder $C_{1-6}$-Alkyl bedeuten; |
| $R^{20}$ | $C_{1-6}$-Alkyl bedeutet; |
| $R^{22}$ | Aryl, ($C_{1-4}$-Alkyl)-Aryl, Heterocyclyl oder $OR^{24}$ bedeutet; und |
| $R^{24}$ | $C_{1-6}$-Alkyl oder ($C_{1-4}$-Alkyl)-Aryl bedeutet. |

**3.** Substituiertes 1-Oxa-2,8-diaza-spiro[4.5]dec-2-en-derivat gemäß Anspruch 1 oder 2, **dadurch gekennzeichnet, daß**

$R^1$ $C_{1-6}$-Alkyl, $C_{3-8}$-Cycloalkyl, ($C_{1-3}$-Alkyl)-$C_{3-8}$-Cycloalkyl, Aryl, ($C_{1-3}$-Alkyl)-Aryl, Heterocyclyl, ($C_{1-3}$-Alkyl)-Heterocyclyl oder NH-C(=O)-Aryl bedeutet; und

$R^2$ H, $C_{1-4}$-Alkyl, ($C_{1-3}$-Alkyl)-Aryl oder ($C_{1-3}$-Alkyl)-Heterocyclyl bedeutet.

**4.** Substituiertes 1-Oxa-2,8-diaza-spiro[4.5]dec-2-en-derivat gemäß Anspruch 3, **dadurch gekennzeichnet, daß**

$R^1$ unsubstituiertes oder mit F, Cl, Br, I, -CN, N-Alkyl-N-Arylamin, N,N-Dialkylamin, Amid, Carboxyalkyl, Carboxybenzyl substituiertes Methyl, Ethyl, n-Propyl, iso-Propyl, 2-Methylpropyl, n-Butyl, tert.-Butyl, n-Pentyl, 3-Methylbutyl oder $CH_2$-C($CH_3$)=$CH_2$, insbesondere Methyl, Ethyl, $CH_2$-C($CH_3$)=$CH_2$, CH(C(=O)$OCH_2CH$=$CH_2$)-$CH_2$C(=O)O-tert.-Butyl, 2-Cyanoethyl, $CH_2$-$CH_2$-NH-C(=O)$CH_3$, 2-(N-Ethyl-N-(3-methylphenyl)amino)-ethyl, 2-(N,N-Dimethylamino)-ethyl, 2-(C(=O)-NH-(β-Naphthyl)-ethyl, 1,2-(Di-(C(=O)O-tert.-butyl)ethyl, 3-(N-Methyl-N-phenylamino)-propyl, 1-(C(=O)O-Benzyl)-3-methyl-butyl, 1-(C(=O)O-Butyl)-3-methyl-butyl, $CH_2CO_2$Ethyl, $CH_2$-$CH_2CO_2$Ethyl, $CH_2$-$CH_2$-OPhenyl, $CH_2$-$CH_2$-S-$CH_2$-$CH_3$;
unsubstituiertes oder mit F, Cl, Br, I, -CN, Alkyl, Aryl, Carboxyalkyl, Carboxybenzyl, O-Alkyl, O-Benzyl substituiertes Cyclopropyl, Cyclobutyl, Cyclopentyl, Cyclohexyl, Cycloheptyl, Cyclooctyl, Bicyclo[3.1.1]heptan-3-yl, insbesondere 2-Phenylcyclopropyl, 2-(O-Benzyl)-cyclopentyl, 2-(Carboxyethyl)cyclohexyl, 7,7-Dimethyl-2-methylbicyclo[3.1.1]heptan-3-yl;
Cyclopropylmethyl, 7,7-Dimethyl-2-oxo-bicyclo[2.2.1]hept-1-ylmethyl;
unsubstituiertes oder mit Phenoxy, -$CH_2$-P(=O)(OEthyl)$_2$ substiuiertes Phenyl, 1-Naphthyl oder 2-Naphthyl;
$CH_2$-Aryl, $CH_2$-$CH_2$-Aryl, $CH_2$-$CH_2$-$CH_2$-Aryl, $CH_2$-$CH_2$-$CH_2$-$CH_2$-Aryl CH($CH_3$)-Aryl, CH($CH_3$)-$CH_2$-Aryl, $CH_2$-CH-(Aryl)$_2$, CH($CO_2$Alkyl)-$CH_2$-Aryl, $CH_2$-$CH_2$-CH-(Aryl)$_2$, wobei Aryl unsubstituiertes Phenyl, 1-Naphthyl oder 2-Naphthyl oder mit F, Cl, Br, I, -CN, -$NO_2$, Alkyl, $CF_3$, Alkoxy, Alkylendioxy substituiertes Phenyl ist, insbesondere Benzyl, -$CH_2$-Naphth-1-yl, 2-Fluorbenzyl, 3-Fluorbenzyl, 3-Chlorbenzyl, 3-Methoxybenzyl, 2-Ethoxybenzyl, 2,4-Difluorbenzyl, 3,5-Dichlorbenzyl, 3-Fluor-5-trifluormethylbenzyl, 3-Fluor-4-trifluormethylbenzyl, 2-Chlor-6-fluorbenzyl, 2,5-Dimethoxybenzyl, 2-Chlor-6-methyl-benzyl, 3,4-Dimethoxybenzyl, 3,4-Dioxymethylenbenzyl, CH($CH_3$)-Phenyl, CH($CH_3$)-(4-$CH_3$-Phenyl), CH($CH_3$)-(4-Nitrophenyl), CH($CH_3$)-(2,3-Dioxyethylenphenyl), $CH_2$-$CH_2$-Phenyl, $CH_2$-$CH_2$-(2-Fluorphenyl), $CH_2$-$CH_2$-(3-Fluorphenyl), $CH_2$-$CH_2$-(4-Fluorphenyl), $CH_2$-$CH_2$-(4-Chlorphenyl), $CH_2$-$CH_2$-(3,4-Dichlorphenyl), $CH_2$-$CH_2$-(3-Methoxyphenyl), $CH_2$-$CH_2$-(2,5-Dimethoxyphenyl), CH($CO_2$-tert.-Butyl)-$CH_2$-Phenyl, CH($CO_2$-Methyl)-$CH_2$-(4-Chlorphenyl), $CH_2$-CH(Phenyl)$_2$, CH($CH_3$)-$CH_2$-(4-Chlorphenyl), $CH_2$-$CH_2$-CH(Phenyl)$_2$, $CH_2$-$CH_2$-$CH_2$-Phenyl;
unsubstituiertes oder mit Aryl, Alkylaryl oder Carboxyethyl substituiertes Pyrrolidin oder Piperidin, insbesonderePyrrolidin-3-yl,N-(4-Trifluorbenzyl)-pyrrolidin-3-yl,N-(3-Methoxybenzyl)-pyrrolidin-3-yl,N-($CH_2$-(β-Naphthyl)-pyrrolidin-3-yl oder N-(Carboxyethyl)-piperidin-4-yl;
unsubstituiertes oder mit Alkyl, F, Cl, Br, I, -CN, Aryl, Alkylaryl substituiertes ($CH_2$)$_{1-3}$-Heterocyclyl, wobei Heterocyclyl für Furanyl, Benzofuranyl, 1,4-Dioxanyl, Benzo-1,4-dioxanyl, Thienyl, Pyridinyl, Pyrrolidinyl, 1H-Indolyl, Imidazolyl, Piperidinyl, Tetrahydrofuranyl steht, insbesondere $CH_2$-Furan-2-yl, 5-Methylfuran-2-yl $CH_2$-Benzofuran-2-yl,

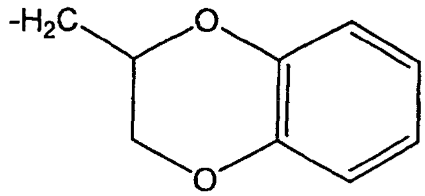,

$CH_2$-Thien-2-yl, $CH_2$-Pyridin-3-yl, $CH_2$-Pyridin-4-yl, $CH_2$-$CH_2$-Pyridin-2-yl, $CH_2$-$CH_2$-(1H-Indol-3-yl), $CH_2$-$CH_2$-Pyrrolidin-1-yl, $CH_2$-(N-2,6-Dichlorbenzylpyrrolidin-3-yl), $CH_2$-$CH_2$-(N-Methyl-pyrrolidin-2-yl), -$(CH_2)_3$-Imidazol-1-yl, $CH_2$-(Tetrahydrofuran-2-yl) oder

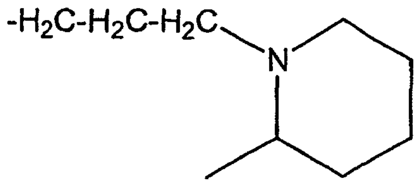,

oder $CH(CO_2$Methyl)-$CH_2$-(1H-Indol-3-yl);
NH-C(=O)-(4-Diethylaminophenyl) bedeutet; und

$R^2$ H;
unsubstituiertes oder mit F, Cl, Br, I, -CN substituiertes Methyl, Ethyl oder $CH_2$-$C(CH_3)$=$CH_2$, insbesondere Methyl, Ethyl, 2-Cyanoethyl, $CH_2$-$C(CH_3)$=$CH_2$;
unsubstituiertes oder mit F, Cl, Br, I, -CN, Methoxy, Ethoxy substituiertes Benzyl oder Phenethyl, insbesondere Benzyl, 4-Fluorbenzyl, 2-Chlor-6-Fluorbenzyl, 2,5-Dimethoxybenzyl, Phenethyl; oder $CH_2$-Furanyl, insbesondere $CH_2$-Furan-2-yl, $CH_2$-Benzofuranyl, insbesondere $CH_2$-Benzofuran-2-yl, $CH_2$-Pyridinyl, insbesondere $CH_2$-Pyridin-3-yl, $CH_2$-Tetrahydrofuranyl, insbesondere $CH_2$-Tetrahydrofuran-2-yl, $CH_2$-$CH_2$-Pyridinyl, insbesondere $CH_2$-$CH_2$-Pyridin-2-yl, bedeutet.

**5.** Substituiertes 1-Oxa-2,8-diaza-spiro[4.5]dec-2-en-derivat gemäß Anspruch 1 oder 2, **dadurch gekennzeichnet, daß**

$R^1$ und $R^2$ zusammen für

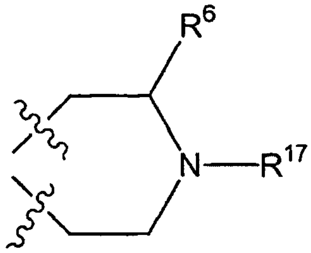, 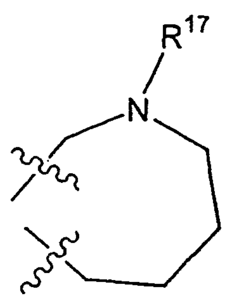,

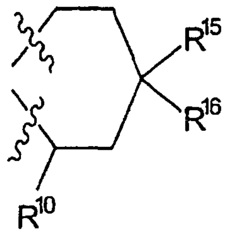
oder
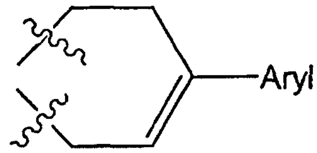
,

wobei Aryl Phenyl oder mit F, Cl, Br, I substituiertes Phenyl, insbesondere 4-Fluorphenyl, bedeutet, stehen;

$R^6$ H oder $C_{1-4}$-Alkyl, inbesondere Methyl, bedeutet;

$R^{10}$ H, C(=O)OMethyl, C(=O)OEthyl, C(=O)O-n-Propyl, C(=O)O-iso-Propyl, C(=O)O-n-Butyl, C(=O)O-tert.-Butyl bedeutet;

$R^{15}$ H oder $CH_2$-Aryl bedeutet;

$R^{16}$ H bedeutet;

$R^{17}$ H;

$C_{3-8}$-Cycloalkyl, insbesondere Cycloheptyl; Aryl, insbesondere unsubstituiertes oder mit Methyl, Ethyl, n-Propyl, iso-Propyl, n-Butyl, tert.-Butyl, $CF_3$, F, Cl, Br, I, -CN, Methoxy, Ethoxy, n-Propoxy, iso-Propoxy, n-Butoxy, tert.-Butoxy substituiertes Phenyl oder Naphthyl;

$((CH_2)_{1-3}$-Alkyl)-Aryl oder $CH(CH_3)$-Aryl, wobei Aryl unsubstituiertes oder mit Alkyl, $CF_3$, F, Cl, Br, I, -CN, Alkoxy substituiertes Phenyl oder Naphthyl bedeutet; unsubstituiertes oder mit mit Alkyl, $CF_3$, F, Cl, Br, I, -CN, Alkoxy substituiertes Pyridinyl, Pyrazinyl oder Thieno[2,3-d]pyrimidinyl bedeutet; oder $C(=O)R^{22}$ bedeutet; und

$R^{22}$ Phenyl oder Alkoxy-substituiertes Phenyl, O-Methyl, O-Ethyl, O-n-Propyl, O-iso-Propyl, O-n-Butyl, O-tert.-Butyl, O-Benzyl, unsubstituiertes Benzyl, mit F substituiertes Benzyl, unsubstituiertes Pyrazinyl oder mit Alkyl substituiertes Pyrazinyl bedeutet.

**6.** Substituiertes 1-Oxa-2,8-diaza-spiro[4.5]dec-2-en-derivat gemäß Anspruch 5, **dadurch gekennzeichnet, daß**

$R^6$ H oder Methyl bedeutet;

$R^{10}$ H oder C(=O)OEthyl bedeutet;

$R^{15}$ H oder Benzyl bedeutet;

$R^{16}$ H bedeutet; und

$R^{17}$ H;

Cycloheptyl;

Phenyl, 2-Methylphenyl, 3-Methylphenyl, 2,4-Dimethylphenyl, 2-Ethylphenyl, 3-Trifluormethyl, 4-Fluorphenyl, 4-Chlorphenyl, 2-Methoxyphenyl, 3,5-Dimethoxyphenyl; 3-Chlor-6-methylphenyl;

Benzyl, $CH_2$-(4-tert.-Butylphenyl), $CH_2$-(β-Naphthyl), $CH(CH_3)$-Phenyl, $(CH_2)_3$-Phenyl;

Pyridin-2-yl, (4-Trifluormethyl)-pyridin-2-yl, Thieno[2,3-d]pyrimidin-4-yl; oder

C(=O)-(4-Methoxyphenyl), C(=O)-Benzyl, C(=O)-$CH_2$-(3,4-Difluorphenyl), C(=O)-(2-Methylpyrazin-5-yl), C(=O)O-tert.-Butyl oder O-Benzyl bedeutet.

**7.** Substituiertes 1-Oxa-2,8-diaza-spiro[4.5]dec-2-en-derivat gemäß irgendeinem der Ansprüche 1 bis 6, **dadurch gekennzeichnet, daß**

$R^3$ $SO_2R^{12}$ bedeutet;

$R^{12}$ Methyl, Ethyl, n-Propyl, iso-Propyl, insbesondere n-Propyl;

7,7-Dimethyl-2-oxo-bicyclo[2.2.1]hept-1-ylmethyl;

Phenyl oder mit Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy, $OCF_3$, $CO_2$Methyl substituiertes Phenyl, insbesondere 4-Methylphenyl, 3-Trifluormethylphenyl, 4-Fluorphenyl, 2-Chlorphenyl, 3-Chlorphenyl, 4-Chlorphenyl, 4-Methoxyphenyl, 4-Trifluormethoxyphenyl, 4-Nitrophenyl, 2-$CO_2$Methyl-phenyl, 2,5-Dichlorphenyl, 3-Fluor-6-methylphenyl, 3-Brom-6-methoxyphenyl, 2-Methyl-5-nitrophenyl, 2,4,6-Trimethylphenyl;

Benzyl oder mit Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy, $OCF_3$ substituiertes Benzyl;

unsubstituiertes oder mit Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy substituiertes Furanyl oder Thienyl, insbesondere Thien-2-yl, 5-Chlorthien-2-yl; oder $NR^{18}R^{19}$ bedeutet; und

$R^{18}$ und $R^{19}$ unabhängig voneinander H, Methyl oder Ethyl bedeuten.

**8.** Substituiertes 1-Oxa-2,8-diaza-spiro[4.5]dec-2-en-derivat gemäß irgendeinem der Ansprüche 1 bis 6, **dadurch gekennzeichnet, daß**

$R^3$ $COR^{13}$ bedeutet; und

$R^{13}$ unsubstituiertes oder mit O-Methyl, O-Ethyl, O-$(CH_2)_2$-$OCH_3$, O-Benzyl, O-Phenyl, wobei Phenyl unsubstituiert oder mit F, Cl, Br, I, -CN substituiert ist, O-C(=O)-Methyl, O-C(=O)-Ethyl substituiertes Methyl, Ethyl, C(=O)OMethyl, n-Propyl, iso-Propyl, 2-Methylpropyl, n-Butyl, tert.-Butyl, n-Pentyl oder 3-Methylbutyl, insbesondere Methyl, Ethyl, n-Propyl, n-Butyl, tert.-Butyl, n-Pentyl, $CH_2$-O-$CH_2$-$CH_2$-$OCH_3$, $CH(CH_3)$-O-Phenyl, $CH_2$-$CH_2$-C(=O)$OCH_3$, $C(CH_3)_2$-OC(=O)$CH_3$, $CH_2$-O-Benzyl, $CH_2$-O-(3-Chlorphenyl), $CH_2$-$CH_2$-$CH_2$-O-Phenyl, CH(OC(=O)Methyl)$CH_3$; Cyclopropyl, 2-Phenylcyclopropyl, 1-Adamantyl;

unsubstituiertes oder mit F, Cl, Br, I, -CN, Phenyl, Methyl, Ethyl, n-Propyl, iso-Propyl, n-Butyl, tert.-Butyl, $CF_3$, F, Cl, Br, I, -CN, $NO_2$, Methoxy, Ethoxy, n-Propoxy, iso-Propoxy, n-Butoxy, tert.-Butoxy, $OCF_3$, $OCHF_2$, $OCH_2F$, $SCF_3$, $SCHF_2$, $SCH_2F$, $SCH_3$. $CH_2OC$(=O)Phenyl, -N($CH_3$) substituiertes Phenyl oder Naphthyl, insbesondere 4-Fluorphenyl, 2-Chlorphenyl, 3-Chlorphenyl, 4-Bromphenyl, 4-Phenylphenyl (4-Biphenyl), 4-Ethylphenyl, 4-$CF_3$-Phenyl, 4-Methoxyphenyl, 2-Ethoxyphenyl, 4-tert.-Butyl, 3-$OCF_3$-Phenyl, 4-$OCF_3$-Phenyl, 4-$SCF_3$-Phenyl, 3-$SCF_2$-Phenyl, 2-$CH_2$-OC(=O)Phenyl, 3-Dimethylaminophenyl, 2,3-Dichlorphenyl, 2,4-Difluorphenyl, 3-Chlor-4-fluorphenyl, 3-Chlor-2-fluorphenyl, 4-$CF_3$-3-Fluorphenyl, 3-$CF_3$-6-Fluorphenyl, 4-Brom-3-methylphenyl, 2-Chlor-4-nitrophenyl, 2,3,4,5,6-Pentafluorphenyl, 2,6-Difluor-3-methylphenyl, 2,3-Difluor-4-methylphenyl, 2-Chlor-5-methyl-6-fluorphenyl, 1-Naphthyl, 2-Naphthyl;

unsubstituiertes oder substituiertes ($C_{1-2}$-Alkyl)-Aryl, insbesondere Benzyl, Phenethyl, CH($C_2H_5$)-Phenyl, CH(NH-$SO_2$-(4-Methylphenyl))-$CH_2$-Phenyl, CH=CH-Phenyl, CH=CH-(3-Trifluorphenyl); oder

unsubstituiertes oder Alkyl-substituiertes Furanyl, Benzofuranyl, unsubstiuiertes oder mit Alkyl, $CF_3$, Aryl, O-Phenyl, Chlor, S-Methyl, S-Ethyl substituiertes Thienyl, Pyridinyl, Pyrazolyl, Benzodihydropyranyl, Isooxazolyl, insbesondere 1,5-Diemthylfuran-3-yl, 2-Methyl-5-tert.-butyl-furan-3-yl, 3-Chlorthien-2-yl 1-(4-Chlorphenyl)-5-trifluormethyl-pyrazol-4-yl, 1-Methyl-3-tert.-butyl-pyrazol-5-yl,

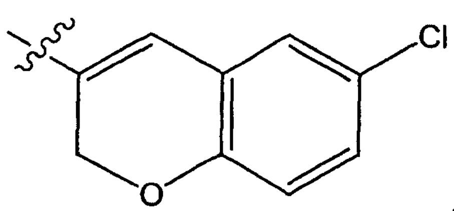

,

Pyridin-4-yl, 2-Methylthiopyridin-3-yl, 2-Ethylthiopyridin-3-yl, 2-Phenoxypyridin-3-yl, 2-Chlorpyridin-3-yl, 5-Methyl-3-(2,6-Dichlorphenyl)-isoxazol-4-yl, 5-Methyl-3-(2-Chlor-6-fluorphenyl)-isoxazol-4-yl bedeutet.

**9.** Substituiertes 1-Oxa-2,8-diaza-spiro[4.5]dec-2-en-derivat gemäß Anspruch 1, **dadurch gekennzeichnet, daß** es aus der Gruppe ausgewählt ist, die besteht aus:

- 3-(1H-Indol-3-yl)-2-{[8-(4-trifluoromethoxy-benzolsulfonyl)-1-oxa-2,8-diaza-spiro[4.5]dec-2-en-3-carbonyl]-amino}-propionsäuremethylester
- {4-[(8-Phenylmethanesulfonyl-1-oxa-2,8-diaza-spiro[4.5]dec-2-en-3-carbonyl)-amino]-benzyl}-phosphonsäurediethylester
- (4-Cycloheptyl-piperazin-1-yl)-[8-(thiophene-2-sulfonyl)-1-oxa-2,8-diaza-spiro[4.5]dec-2-en-3-yl]-methanon
- [8-(5-Chloro-thiophen-2-sulfonyl)-1-oxa-2,8-diaza-spiro[4.5]dec-2-en-3-yl]-(4-phenyl-piperazin-1-yl)-methanon
- 8-(2-Chloro-4-nitro-benzoyl)-1-oxa-2,8-diaza-spiro[4.5]dec-2-en-3-carbonsäurecyclopentylamid
- (4-Naphthalin-2-ylmethyl-piperazin-1-yl)-(8-phenylmethansulfonyl-1-oxa-2,8-diaza-spiro[4.5]dec-2-en-3-yl)-methanon
- 8-Dimethylsulfamoyl-1-oxa-2,8-diaza-spiro[4.5]dec-2-en-3-carbonsäurephenethylamid
- 8-(Thiophen-2-sulfonyl)-1-oxa-2,8-diaza-spiro[4.5]dec-2-en-3-carbonsäure(1-phenyl-ethyl)-amid

- 8-(Toluol-4-sulfonyl)-1-oxa-2,8-diaza-spiro[4.5]dec-2-en-3-carbonsäure-[2-(3,4-Dichloro-phenyl)-ethyl]-amid
- 4-Diethylamino-benzoesäure N'-[8-(thiophen-2-sulfonyl)-1-oxa-2,8-diaza-spiro[4.5]dec-2-en-3-carbonyl]-hydrazide
- 8-(3-Chloro-4-fluoro-benzolsulfonyl)-1-oxa-2,8-diaza-spiro[4.5]dec-2-en-3-carbonsäure(2-pyrrolidin-1-yl-ethyl)-amid
- 8-Phenylmethansulfonyl-1-oxa-2,8-diaza-spiro[4.5]dec-2-en-3-carbonsäurephenethyl-amid
- 8-Benzolsulfonyl-1-oxa-2,8-diaza-spiro[4.5]dec-2-en-3-carbonsäure-(2-dimethylamino-ethyl)-amid
- [4-(3-Phenyl-propylypiperazin-1-yl]-[8-(thiophen-2-sulfonyl)-1-oxa-2,8-diaza-spiro[4.5]dec-2-en-3-yl]-methanon
- 8-(2,5-Dichloro-benzolsulfonyl)-1-oxa-2,8-diaza-spiro[4.5]dec-2-en-3-carbonsäure-[3-(2-methyl-piperidin-1-yl)-propyl]-amid
- 8-(4-Trifluoromethoxy-benzolsulfonyl)-1-oxa-2,8-diaza-spiro[4.5]dec-2-en-3-carbonsäure-[2-(1H-indol-3-yl)-ethyl]-methyl-amid
- 8-(5-Fluoro-2-methyl-benzolsulfonyl)-1-oxa-2,8-diaza-spiro[4.5]dec-2-en-3-carbonsäure-(2-acetylamino-ethyl)-amid
- 8-(Toluol-4-sulfonyl)-1-oxa-2,8-diaza-spiro[4.5]dec-2-en-3-carbonsäurebenzyl-phenethyl-amid
- 8-(2-Methyl-5-nitro-benzolsulfonyl)-1-oxa-2,8-diaza-spiro[4.5]dec-2-en-3-carbonsäure-(2-dimethylamino-ethyl)-amid
- 8-Phenylmethansulfonyl-1-oxa-2,8-diaza-spiro[4.5]dec-2-en-3-carbonsäure-(2-benzyloxy-cyclopentyl)-amid
- 8-(Thiophen-2-sulfonyl)-1-oxa-2,8-diaza-spiro[4.5]dec-2-en-3-carbonsäure-(2,3-dihydro-benzo[1,4]dioxin-2-ylmethyl)-amid
- 8-(Toluol-4-sulfonyl)-1-oxa-2,8-diaza-spiro[4.5]dec-2-en-3-carbonsäure-(4-phenoxy-phenyl)-amid
- 8-(Thiophen-2-sulfonyl)-1-oxa-2,8-diaza-spiro[4.5]dec-2-en-3-carbonsäure-(1-p-tolyl-ethyl)-amid
- 8-(Thiophen-2-sulfonyl)-1-oxa-2,8-diaza-spiro[4.5]dec-2-en-3-carbonsäure-3-fluoro-benzylamid
- 2-Phenyl-1-{4-[8-(toiuol-4-sulfonyl)-1-oxa-2,8-diaza-spiro[4.5]dec-2-en-3-carbonyl]-piperazin-1-yl}-ethanone
- 8-(Thiophen-2-sulfonyl)-1-oxa-2,8-diaza-spiro[4.5]dec-2-en-3-carbonsäure-[1-(4-trifluoromethyl-benzyl)-pyrrolidin-3-yl]-amid
- [8-(Thiophen-2-sulfonyl)-1-oxa-2,8-diaza-spiro[4.5]dec-2-en-3-yl]-(4-o-tolyl-piperazin-1-yl)-methanon
- 8-(Toluol-4-sulfonyl)-1-oxa-2,8-diaza-spiro[4.5]dec-2-en-3-carbonsäure-benzyl-(2-cyano-ethyl)-amid
- [8-(2,5-Dichloro-benzolsulfonyl)-1-oxa-2,8-diaza-spiro[4.5]dec-2-en-3-yl]-(4-thieno[2,3-d]pyrimidin-4-yl-piperazin-1-yl)-methanon
- 8-(5-Chloro-thiophen-2-sulfonyl)-1-oxa-2,8-diaza-spiro[4.5]dec-2-en-3-carbonsäure-(benzo[1,3]dioxol-5-ylmethyl)-amid
- (3-Methyl-4-m-tolyl-piperazin-1-yl)-[8-(thiophen-2-sulfonyl)-1-oxa-2,8-diaza-spiro[4.5]dec-2-en-3-yl]-methanon
- [4-(1-Phenyl-ethyl)-piperazin-1-yl]-[8-(toluol-4-sulfonyl)-1-oxa-2,8-diaza-spiro[4.5]dec-2-en-3-yl]-methanon
- [4-(2,4-Dimethyl-phenyl)-piperazin-1-yl]-[8-(toluol-4-sulfonyl)-1-oxa-2,8-diaza-spiro[4.5]dec-2-en-3-yl]-methanon
- 8-(2-Chloro-pyridine-3-carbonyl)-1-oxa-2,8-diaza-spiro[4.5]dec-2-en-3-carbonsäure-cyclopentylamid
- (4-Naphthalen-2-ylmethyl-piperazin-1-yl)-[8-(3-trifluoromethyl-benzolsulfonyl)-1-oxa-2,8-diaza-spiro[4.5]dec-2-en-3-yl]-methanon
- 8-[3-Phenyl-2-(toluol-4-sulfonylamino)-propionyl]-1-oxa-2,8-diaza-spiro[4.5]dec-2-en-3-carbonsäure-(pyridin-3-ylmethyl)-amid
- 8-(5-Fluoro-2-methyl-benzolsulfonyl)-1-oxa-2,8-diaza-spiro[4.5]dec-2-en-3-carbonsäure-(2-dimethylamino-ethyl)-amid
- 8-(4-Nitro-benzolsulfonyl)-1-oxa-2,8-diaza-spiro[4.5]dec-2-en-3-carbonsäure(thiophen-2-ylmethyl)-amid
- 8-(2,5-Dichloro-benzolsulfonyl)-1-oxa-2,8-diaza-spiro[4.5]dec-2-en-3-carbonsäure-3-fluoro-5-trifluoromethyl-benzylamid
- 8-(5-Chloro-thiophen-2-sulfonyl)-1-oxa-2,8-diaza-spiro[4.5]dec-2-en-3-carbonsäurebenzhydryl-amid
- 8-[3-Phenyl-2-(toluol-4-sulfonylamino)-propionyl]-1-oxa-2,8-diaza-spiro[4.5]dec-2-en-3-carbonsäure-(2-cyano-ethyl)-amid
- [(8-Phenylmethanesulfonyl-1-oxa-2,8-diaza-spiro[4.5]dec-2-en-3-carbonyl)-amino]-essigsäureethylester
- 8-(3-Dimethylamino-benzoyl)-1-oxa-2,8-diaza-spiro[4.5]dec-2-en-3-carbonsäure(2-cyano-ethyl)-amid
- 8-(2,5-Dichloro-benzolsulfonyl)-1-oxa-2,8-diaza-spiro[4.5]dec-2-en-3-carbonsäure-[1-(naphthalin-2-ylcarbamoyl)-ethyl]-amid
- 8-(Toluol-4-sulfonyl)-1-oxa-2,8-diaza-spiro[4.5]dec-2-en-3-carbonsäure-(1-p-tolyl-ethyl)-amid
- [4-(4-Chloro-phenyl)-piperazin-1-yl]-(8-phenylmethanesulfonyl-1-oxa-2,8-diaza-spiro[4.5]dec-2-en-3-yl)-methanon

- 8-(Thiophen-2-sulfonyl)-1-oxa-2,8-diaza-spiro[4.5]dec-2-en-3-carbonsäure-(2-benzyloxy-cyclopentyl)-amid
- 8-Acetyl-1-oxa-2,8-diaza-spiro[4.5]dec-2-en-3-carbonsäurebenzylamid
- 8-(5-Chloro-thiophen-2-sulfonyl)-1-oxa-2,8-diaza-spiro[4.5]dec-2-en-3-carbonsäure-2,5-difluoro-benzylamid
- 8-(5-Chloro-thiophen-2-sulfonyl)-1-oxa-2,8-diaza-spiro[4.5]dec-2-en-3-carbonsäure-(1-naphthalin-2-ylmethyl-pyrrolidin-3-yl)-amid
- 8-Phenylmethanesulfonyl-1-oxa-2,8-diaza-spiro[4.5]dec-2-en-3-carbonsäure-2-ethoxy-benzylamid
- 8-(4-Ethyl-benzoyl)-1-oxa-2,8-diaza-spiro[4.5]dec-2-en-3-carbonsäure-(thiophen-2-ylmethyl)-amid
- 2-{[8-(2,5-Dichloro-benzolsulfonyl)-1-oxa-2,8-diaza-spiro[4.5]dec-2-en-3-carbonyl]-amino}-bernsteinsäure-1-allylester-4-tert-butylester
- 8-(4-Trifluoromethoxy-benzolsulfonyl)-1-oxa-2,8-diaza-spiro[4.5]dec-2-en-3-carbonsäure-naphthalin-2-ylamid
- 4-{[8-(2-Phenyl-cyclopropanecarbonyl)-1-oxa-2,8-diaza-spiro[4.5]dec-2-en-3-carbonyl]-amino}-piperidin-1-carbonsäureethylester
- 4-{[8-(2,4-Difluoro-benzoyl)-1-oxa-2,8-diaza-spiro[4.5]dec-2-en-3-carbonyl]-amino}-piperidin-1-carbonsäureethylester
- 8-(Toluol-4-sulfonyl)-1-oxa-2,8-diaza-spiro[4.5]dec-2-en-3-carbonsäure(2-dimethylamino-ethyl)-amid
- 8-Dimethylsulfamoyl-1-oxa-2,8-diaza-spiro[4.5]dec-2-en-3-carbonsäure[2-(4-chloro-phenyl)-ethyl]-amid
- 8-(4-Methoxy-benzoyl)-1-oxa-2,8-diaza-spiro[4.5]dec-2-en-3-carbonsäure(5-methyl-furan-2-ylmethyl)-amid
- 8-(Thiophen-2-sulfonyl)-1-oxa-2,8-diaza-spiro[4.5]dec-2-en-3-carbonsäure-[2-(4-chloro-phenyl)-ethyl]-amid
- 8-(5-Chloro-thiophen-2-sulfonyl)-1-oxa-2,8-diaza-spiro[4.5]dec-2-en-3-carbonsäure-[2-(1-methyl-pyrrolidin-2-yl)-ethyl]-amid
- 4-{[8-(4-Bromo-benzoyl)-1-oxa-2,8-diaza-spiro[4.5]dec-2-en-3-carbonyl]-amino}-piperidin-1-carbonsäureethylester
- 8-(Toluol-4-sulfonyl)-1-oxa-2,8-diaza-spiro[4.5]dec-2-en-3-carbonsäure-3,5-Dichloro-benzylamid
- 8-(2,5-Dichloro-benzolsulfonyl)-1-oxa-2,8-diaza-spiro[4.5]dec-2-en-3-carbonsäure-[1-(3-methoxy-benzyl)-pyrrolidin-3-yl]-amid
- 3-(4-Naphthalin-2-ylmethyl-piperazine-1-carbonyl)-1-oxa-2,8-diaza-spiro[4.5]dec-2-en-8-sulfonsäure dimethylamid
- 3-{[8-(7,7-Dimethyl-2-oxo-bicyclo[2.2.1]hept-1-ylmethanesulfonyl)-1-oxa-2,8-diaza-spiro[4.5]dec-2-en-3-carbonyl]-amino}-propionsäureethylester
- 8-(Toluol-4-sulfonyl)-1-oxa-2,8-diaza-spiro[4.5]dec-2-en-3-carbonsäure(2-benzyloxy-cyclopentyl)-amid
- 8-(2,5-Dichloro-benzolsulfonyl)-1-oxa-2,8-diaza-spiro[4.5]dec-2-en-3-carbonsäure3,4-dimethoxy-benzylamid
- (8-Phenylmethanesulfonyl-1-oxa-2,8-diaza-spiro[4.5]dec-2-en-3-yl)-[4-(3-phenyl-propyl)-piperazin-1-yl]-methanon
- 3-(4-Thieno[2,3-d]pyrimidin-4-yl-piperazine-1-carbonyl)-1-oxa-2,8-diaza-spiro[4.5]dec-2-en-8-sulfonsäuredimethylamid
- [8-(5-Chloro-thiophen-2-sulfonyl)-1-oxa-2,8-diaza-spiro[4.5]dec-2-en-3-yl]-[4-(3,5-dimethoxy-phenyl)-piperazin-1-yl]-methanon
- 8-Dimethylsulfamoyl-1-oxa-2,8-diaza-spiro[4.5]dec-2-en-3-carbonsäure-3-fluoro-4-trifluoromethyl-benzylamid
- 8-(Toluol-4-sulfonyl)-1-oxa-2,8-diaza-spiro[4.5]dec-2-en-3-carbonsäure-(2-benzyloxy-cyclopentyl)-amid
- 4-[(8-Butyryl-1-oxa-2,8-diaza-spiro[4.5]dec-2-en-3-carbonyl)-amino]-piperidin-1-carbonsäureethylester
- 8-(Toluol-4-sulfonyl)-1-oxa-2,8-diaza-spiro[4.5]dec-2-en-3-carbonsäure-(2-phenyl-cyclopropyl)-amid
- [8-(4-Fluoro-benzolsulfonyl)-1-oxa-2,8-diaza-spiro[4.5]dec-2-en-3-yl]-[4-(5-methyl-pyrazine-2-carbonyl)-piperazin-1-yl]-methanon
- 8-Dimethylsulfamoyl-1-oxa-2,8-diaza-spiro[4.5]dec-2-en-3-carbonsäure-2,4-difluoro-benzylamid
- 8-Phenylmethanesulfonyl-1-oxa-2,8-diaza-spiro[4.5]dec-2-en-3-carbonsäure-[2-(3-fluoro-phenyl)-ethyl]-amid
- 8-(5-Chloro-thiophen-2-sulfonyl)-1-oxa-2,8-diaza-spiro[4.5]dec-2-en-3-carbonsäure-(2,2-diphenyl-ethyl)-amid
- 3-[4-(3-Phenyl-propyl)-piperazine-1-carbonyl]-1-oxa-2,8-diaza-spiro[4.5]dec-2-en-8-sulfonsäuredimethylamid
- 3-{[8-(3,5-Difluoro-benzoyl)-1-oxa-2,8-diaza-spiro[4.5]dec-2-en-3-carbonyl]-amino}-propionsäureethyl ester
- 8-(Propane-1-sulfonyl)-1-oxa-2,8-diaza-spiro[4.5]dec-2-en-3-carbonsäureisobutyl-amid
- [4-(3-Phenyl-propyl)-piperazin-1 -yl]-[8-(3-trifluoromethyl-benzolsulfonyl)-1-oxa-2,8-diaza-spiro[4.5]dec-2-en-3-yl]-methanon
- 8-(Thiophen-2-sulfonyl)-1-oxa-2,8-diaza-spiro[4.5]dec-2-en-3-carbonsäure-[1-(2,3-dihydro-benzo[1,4]dioxin-5-yl)-ethyl]-amid

- 8-Butyryl-1-oxa-2,8-diaza-spiro[4.5]dec-2-en-3-carbonsäurephenylamid
- 8-(4-Trifluoromethoxy-benzolsulfonyl)-1-oxa-2,8-diaza-spiro[4.5]dec-2-en-3-carbonsäurecyclooctylamid
- 8-[2-(2-Methoxy-ethoxy)-acetyl]-1-oxa-2,8-diaza-spiro[4.5]dec-2-en-3-carbonsäurebenzylamid
- 8-(4-Bromo-3-methyl-benzoyl)-1-oxa-2,8-diaza-spiro[4.5]dec-2-en-3-carbonsäureisobutyl-amid
- 8-(5-Chloro-thiophen-2-sulfonyl)-1-oxa-2,8-diaza-spiro[4.5]dec-2-en-3-carbonsäure-[3-(methyl-phenyl-amino)-propyl]-amid
- 4-{[8-(2-Methyl-5-nitro-benzolsulfonyl)-1-oxa-2,8-diaza-spiro[4.5]dec-2-en-3-carbonyl]-amino}-piperidin-1-carbonsäureethylester
- [8-(5-Chloro-thiophen-2-sulfonyl)-1-oxa-2,8-diaza-spiro[4.5]dec-2-en-3-yl]-[4-(4-fluoro-phenyl)-piperazin-1-yl]-methanon
- 8-(2,5-Dichloro-benzolsulfonyl)-1-oxa-2,8-diaza-spiro[4.5]dec-2-en-3-carbonsäure-methyl-pyridin-3-ylmethyl-amid
- 2-(3,4-Difluoro-phenyl)-1-(4-[8-(3-trifluoromethyl-benzolsulfonylyl-oxa-2,8-diaza-spiro[4.5]dec-2-en-3-carbonyl]-piperazin-1-yl}-ethanon
- 8-(3-Trifluoromethyl-benzolsulfonyl)-1-oxa-2,8-diaza-spiro[4.5]dec-2-en-3-carbonsäurecyclopropylmethyl-amid
- 3-{[8-(2-Methyl-5-nitro-benzolsulfonyl)-1-oxa-2,8-diaza-spiro[4.5]dec-2-en-3-carbonyl]-amino}-propionsäureethylester
- 8-[2-(3-Chloro-phenoxy)-acetyl]-1-oxa-2,8-diaza-spiro[4.5]dec-2-en-3-carbonsäure-(thiophen-2-ylmethyl)-amid
- 8-(4-Trifluoromethoxy-benzolsulfonyl)-1-oxa-2,8-diaza-spiro[4.5]dec-2-en-3-carbonsäurebenzyl-phenethyl-amid
- 8-(2,5-Dichloro-benzolsulfonyl)-1-oxa-2,8-diaza-spiro[4.5]dec-2-en-3-carbonsäurebenzyl-phenethyl-amid
- 8-(2,5-Dichloro-benzolsulfonyl)-1-oxa-2,8-diaza-spiro[4.5]dec-2-en-3-carbonsäure-(2-cyano-ethyl)-pyridin-3-ylmethyl-amid
- 8-(2,5-Dichloro-benzolsulfonyl)-1-oxa-2,8-diaza-spiro[4.5]dec-2-en-3-carbonsäure-(2-cyano-ethyl)-(tetrahydro-furan-2-ylmethyl)-amid
- 8-(3-Trifluoromethyl-benzolsulfonyl)-1-oxa-2,8-diaza-spiro[4.5]dec-2-en-3-carbonsäure-methyl-pyridin-3-ylmethyl-amid
- 8-(Pyridine-4-carbonyl)-1-oxa-2,8-diaza-spiro[4.5]dec-2-en-3-carbonsäure-benzylamid
- 8-(4-Trifluoromethoxy-benzolsulfonyl)-1-oxa-2,8-diaza-spiro[4.5]dec-2-en-3-carbonsäureethyl-(2-methyl-allyl)-amid
- 8-(4-Methoxy-benzolsulfonyl)-1-oxa-2,8-diaza-spiro[4.5]dec-2-en-3-carbonsäure-[2-(2-fluoro-phenyl)-ethyl]-amid
- 8-(5-tert-Butyl-2-methyl-furan-3-carbonyl)-1-oxa-2,8-diaza-spiro[4.5]dec-2-en-3-carbonsäureisobutyl-amid
- 8-(3-Phenyl-acryloyl)-1-oxa-2,8-diaza-spiro[4.5]dec-2-en-3-carbonsäure-(thiophen-2-ylmethyl)-amid
- 8-(4-Trifluoromethyl-benzoyl)-1-oxa-2,8-diaza-spiro[4.5]dec-2-en-3-carbonsäurecyclopropylmethyl-amid
- 8-(4-Trifluoromethylsulfanyl-benzoyl)-1-oxa-2,8-diaza-spiro[4.5]dec-2-en-3-carbonsäurecyclopropylmethyl-amid
- 8-(4-Bromo-benzoyl)-1-oxa-2,8-diaza-spiro[4.5]dec-2-en-3-carbonsäure-(5-methyl-furan-2-ylmethyl)-amid
- 8-(3-Fluoro-4-trifluoromethyl-benzoyl)-1-oxa-2,8-diaza-spiro[4.5]dec-2-en-3-carbonsäurecyclopropylmethyl-amid
- 8-(7,7-Dimethyl-2-oxo-bicyclo[2.2.1]hept-1-ylmethanesulfonyl)-1-oxa-2,8-diaza-spiro[4.5]dec-2-en-3-carbonsäure-(2-ethylsulfanyl-ethyl)-amid
- 8-(2,4-Difluoro-benzoyl)-1-oxa-2,8-diaza-spiro[4.5]dec-2-en-3-carbonsäure-(tetrahydro-furan-2-ylmethyl)-amid
- {[8-(3-Chloro-thiophen-2-carbonyl)-1-oxa-2,8-diaza-spiro[4.5]dec-2-en-3-carbonyl]-amino}-essigsäureethylester
- 4-Oxo-4-{3-[(thiophen-2-ylmethyl)-carbamoyl]-1-oxa-2,8-diaza-spiro[4.5]dec-2-en-8-yl}-buttersäuremethylester
- 8-(2-Ethylsulfanyl-pyridine-3-carbonyl)-1-oxa-2,8-diaza-spiro[4.5]dec-2-en-3-carbonsäurebenzylamid
- 3-{[8-(2-Chloro-benzolsulfonyl)-1-oxa-2,8-diaza-spiro[4.5]dec-2-en-3-carbonyl]-amino}-propionsäureethylester
- 8-(4-Trifluoromethoxy-benzoyl)-1-oxa-2,8-diaza-spiro[4.5]dec-2-en-3-carbonsäurecyclopropylmethyl-amid
- 8-(4-Phenoxy-butyryl)-1-oxa-2,8-diaza-spiro[4.5]dec-2-en-3-carbonsäurecyclopropylmethyl-amid
- [8-(3-Trifluoromethyl-benzolsulfonyl)-1-oxa-2,8-diaza-spiro[4.5]dec-2-en-3-yl]-[4-(4-trifluoromethyl-pyridin-2-yl)-piperazin-1-yl]-methanon

- 8-(2-Chloro-5-trifluoromethyl-benzoyl)-1-oxa-2,8-diaza-spiro[4.5]dec-2-en-3-carbonsäurecyclopropylmethyl-amid
- 8-(4-Methoxy-benzolsulfonyl)-1-oxa-2,8-diaza-spiro[4.5]dec-2-en-3-carbonsäure(2-benzyloxy-cyclopentyl)-amid
- 4-{[8-(3-Trifluoromethyl-benzolsulfonyl)-1-oxa-2,8-diaza-spiro[4.5]dec-2-en-3-carbonyl]-amino}-piperidin-1-carbonsäureethylester
- 8-(2-Phenoxy-propionyl)-1-oxa-2,8-diaza-spiro[4.5]dec-2-en-3-carbonsäurecyclopropylmethyl-amid
- 8-(4-Fluoro-benzolsulfonyl)-1-oxa-2,8-diaza-spiro[4.5]dec-2-en-3-carbonsäure-[2-(3-methoxy-phenyl)-ethyl]-amid
- 8-(2-Methylsulfanyl-pyridine-3-carbonyl)-1-oxa-2,8-d iaza-spiro[4.5]dec-2-en-3-carbonsäureisobutyl-amid
- 8-(5-tert-Butyl-2-methyl-furan-3-carbonyl)-1-oxa-2,8-diaza-spiro[4.5]dec-2-en-3-carbonsäurecyclopentylamid
- 8-(4-Chloro-benzolsulfonyl)-1-oxa-2,8-diaza-spiro[4.5]dec-2-en-3-carbonsäure-(thiophen-2-ylmethyl)-amid
- 8-(5-Chloro-thiophen-2-sulfonyl)-1-oxa-2,8-diaza-spiro[4.5]dec-2-en-3-carbonsäure-(naphthalin-1-ylmethyl)-amid
- 8-(4-Trifluoromethoxy-benzolsulfonyl)-1-oxa-2,8-diaza-spiro[4.5]dec-2-en-3-carbonsäure-[2-(4-fluoro-phenyl)-ethyl]-amid
- 8-(4-Bromo-3-methyl-benzoyl)-1-oxa-2,8-diaza-spiro[4.5]dec-2-en-3-carbonsäure-(pyridin-4-ylmethyl)-amid
- 8-(4-Methoxy-benzoyl)-1-oxa-2,8-diaza-spiro[4.5]dec-2-en-3-carbonsäurebenzylamid
- 8-(3-Trifluoromethyl-benzolsulfonyl)-1-oxa-2,8-diaza-spiro[4.5]dec-2-en-3-carbonsäure-3-chloro-benzylamid
- (8-Phenylmethanesulfonyl-1-oxa-2,8-diaza-spiro[4.5]dec-2-en-3-yl)-(4-o-tolyl-piperazin-1-yl)methanon
- (8-Phenylmethanesuffonyl-1-oxa-2,8-diaza-spiro[4.5]dec-2-en-3-yl)-(4-pyridin-2-yl-piperazin-1-yl)-methanon
- 8-(4-Trifluoromethylsulfanyl-benzoyl)-1-oxa-2,8-diaza-spiro[4.5]dec-2-en-3-carbonsäure-(thiophen-2-ylmethyl)-amid
- 8-(Thiophen-2-sulfonyl)-1-oxa-2,8-diaza-spiro[4.5]dec-2-en-3-carbonsäurebenzylamid
- 8-(4-Fluoro-benzoyl)-1-oxa-2,8-diaza-spiro[4.5]dec-2-en-3-carbonsäureisobutyl-amid
- 2-(3-Isobutylcarbamoyl-1-oxa-2,8-diaza-spiro[4.5]dec-2-en-8-sulfonyl)-benzoesäuremethylester
- 2-[(8-Dimethylsulfamoyl-1-oxa-2,8-diaza-spiro[4.5]dec-2-en-3-carbonyl)-amino]-3-phenyl-propionsäure-tert-butylester
- 8-(5-Chloro-thiophen-2-sulfonyl)-1-oxa-2,8-diaza-spiro[4.5]dec-2-en-3-carbonsäure-[1-(2,3-dihydro-benzo [1,4]dioxin-5-yl)-ethyl]-amid
- 8-(4-Bromo-benzoyl)-1-oxa-2,8-diaza-spiro[4.5]dec-2-en-3-carbonsäure-(thiophen-2-ylmethyl)-amid
- 8-(4-Fluoro-benzolsulfonyl)-1-oxa-2,8-diaza-spiro[4.5]dec-2-en-3-carbonsäure-(3-imidazol-1-yl-propyl)-amid
- 4-[8-(4-Fluoro-benzolsulfonyl)-1-oxa-2,8-diaza-spiro[4.5]dec-2-en-3-carbonyl]-piperazine-1-carbonsäurebenzylester
- 3-(4-Cycloheptyl-piperazine-1-carbonyl)-1-oxa-2,8-diaza-spiro[4.5]dec-2-en-8-sulfonsäuredimethyfamid
- 4-Methyl-2-{[8-(thiophen-2-sulfonyl)-1-oxa-2,8-diaza-spiro[4.5]dec-2-en-3-carbonyl]-amino}-pentansäure-tert-butylester
- 8-(3-Chloro-2-fluoro-benzoyl)-1-oxa-2,8-diaza-spiro[4.5]dec-2-en-3-carbonsäure-(thiophen-2-ylmethyl)-amid
- 2-{[8-(4-Methoxy-benzolsulfonyl)-1-oxa-2,8-diaza-spiro[4.5]dec-2-en-3-carbonyl]-amino}-4-methyl-pentansäure-tert-butylester
- 3-{[8-(6-Chloro-2-fluoro-3-methyl-benzoyf)-1-oxa-2,8-diaza-spiro[4.5]dec-2-en-3-carbonyl]-amino}-propionsäureethylester
- 8-(2-Phenoxy-propionyl)-1-oxa-2,8-diaza-spiro[4.5]dec-2-en-3-carbonsäurebenzylamid
- 8-(5-Fluoro-2-methyl-benzolsulfonyl)-1-oxa-2,8-diaza-spiro[4.5]dec-2-en-3-carbonsäure-(thiophen-2-ylmethyl)-amid
- [8-(5-Chloro-thiophen-2-sulfonyl)-1-oxa-2,8-diaza-spiro[4.5]dec-2-en-3-yl]-[4-(2-methoxy-phenyl)-piperidin-1-yl]-methanon
- 8-(2-Chloro-benzoyl)-1-oxa-2,8-diaza-spiro[4.5]dec-2-en-3-carbonsäure-(thiophen-2-ylmethyl)-amid
- 8-(2-Phenoxy-pyridine-3-carbonyl)-1-oxa-2,8-diaza-spiro[4.5]dec-2-en-3-carbonsäurecyclopentylamid
- 8-(4-Bromo-benzoyl)-1-oxa-2,8-diaza-spiro[4.5]dec-2-en-3-carbonsäure-(2-ethylsulfanyl-ethyl)-amid
- 1-[8-(5-Chloro-thiophen-2-sulfonyl)-1-oxa-2,8-diaza-spiro[4.5]dec-2-en-3-carbonyl]-piperidin-2-carbonsäureethylester
- 8-(2-Chloro-benzolsulfonyl)-1-oxa-2,8-diaza-spiro[4.5]dec-2-en-3-carbonsäure-(thiophen-2-ylmethyl)-amid
- 8-(2,3-Dichloro-benzoyl)-1-oxa-2,8-diaza-spiro[4.5]dec-2-en-3-carbonsäurecyclopropylmethyl-amid
- 8-Hexanoyl-1-oxa-2,8-diaza-spiro[4.5]dec-2-en-3-carbonsäurecyclopropylmethyl-amid
- 8-(2,3-Difluoro-4-methyl-benzoyl)-1-oxa-2,8-diaza-spiro[4.5]dec-2-en-3-carbonsäurecyclopropylmethyl-amid
- 8-(4-Fluoro-benzolsulfonyl)-oxa-2,8-diaza-spiro[4.5]dec-2-en-3-carbonsäure-(2, 5-dimethoxy-benzyl)-furan-2-ylmethyl-amid

- 8-Dimethylsulfamoyl-1-oxa-2,8-diaza-spiro[4.5]dec-2-en-3-carbonsäure-(2-ethylsulfanyl-ethyl)-amid
- 8-(4-Trifluoromethoxy-benzolsulfonyl)-1-oxa-2,8-diaza-spiro[4.5]dec-2-en-3-carbonsäure-3-methoxy-benzylamid
- 8-Dimethylsulfamoyl-1-oxa-2,8-diaza-spiro[4.5]dec-2-en-3-carbonsäure-(2-benzyloxy-cyclopentyl)-amid
- 8-(4-Methoxy-benzolsulfonyl)-1-oxa-2,8-diaza-spiro[4.5]dec-2-en-3-carbonsäure-[2-(4-chloro-phenyl)-1-methyl-ethyl]-amid
- 8-(4-Trifluoromethoxy-benzolsulfonyl)-1-oxa-2,8-diaza-spiro[4.5]dec-2-en-3-carbonsäure-[2-(3,4-Dichloro-phenyl)-ethyl]-amid
- 8-[2-(3-Chloro-phenoxy)-acetyl]-1-oxa-2,8-diaza-spiro[4.5]dec-2-en-3-carbonsäurebenzylamid
- 8-(4-Fluoro-benzolsulfonyl)-1-oxa-2,8-diaza-spiro[4.5]dec-2-en-3-carbonsäure-(2-cyano-ethyl)-(2-pyridin-2-yl-ethyl)-amid
- 8-(5-Chloro-thiophen-2-sulfonyl)-1-oxa-2,8-diaza-spiro[4.5]dec-2-en-3-carbonsäure-[2-(4-chloro-phenyl)-1-methyl-ethyl]-amid
- 8-(4-Methoxy-benzolsulfonyl)-1-oxa-2,8-diaza-spiro[4.5]dec-2-en-3-carbonsäure-(2-benzyloxy-cyclopentyl)-amid
- 8-(2,6-Difluoro-3-methyl-benzoyl)-1-oxa-2,8-diaza-spiro[4.5]dec-2-en-3-carbonsäure-(thiophen-2-ylmethyl)-amid
- 8-(2-Benzyloxy-acetyl)-1-oxa-2,8-diaza-spiro[4.5]dec-2-en-3-carbonsäure-benzylamid
- 8-(2,3-Dichloro-benzoyl)-1-oxa-2,8-diaza-spiro[4.5]dec-2-en-3-carbonsäurephenylamid
- 8-(5-Bromo-2-methoxy-benzolsulfonyl)-1-oxa-2,8-diaza-spiro[4.5]dec-2-en-3-carbonsäurecyclopentylamid
- Essigsäure-2-(3-benzylcarbamoyl-1-oxa-2,8-diaza-spiro[4.5]dec-2-en-8-yl)-1,1-dimethyl-2-oxo-ethylester
- 8-[3-(2-Chloro-6-fluoro-phenyl)-5-methyl-isoxazole-4-carbonyl]-1-oxa-2,8-diaza-spiro[4.5]dec-2-en-3-carbonsäure-(2-ethylsulfanyl-ethyl)-amid
- [4-(3-Phenyl-propyl)-piperazin-1-yl]-[8-(4-trifluoromethoxy-benzolsulfonyl)-1-oxa-2,8-diaza-spiro[4.5]dec-2-en-3-yl]-methanon
- 8-[3-(2-Chloro-6-fluoro-phenyl)-5-methyl-isoxazole-4-carbonyl]-1-oxa-2,8-diaza-spiro[4.5]dec-2-en-3-carbonsäurebenzylamid
- 8-(Naphthalincarbonyl)-1-oxa-2,8-diaza-spiro[4.5]dec-2-en-3-carbonsäureisobutyl-amid
- 1-[8-(5-tert-Butyl-2-methyl-furan-3-carbonyl)-1-oxa-2,8-diaza-spiro[4.5]dec-2-en-3-carbonyl]-piperidin-2-carbonsäureethylester
- 8-(3-Difluoromethylsulfanyl-benzoyl)-1-oxa-2,8-diaza-spiro[4.5]dec-2-en-3-carbonsäurebenzylamid
- 8-(4-Bromo-benzoyl)-1-oxa-2,8-diaza-spiro[4.5]dec-2-en-3-carbonsäurephenylamid
- 8-(4-Methoxy-benzolsulfonyl)-1-oxa-2,8-diaza-spiro[4.5]dec-2-en-3-carbonsäure(2-phenyl-cyclopropyl)-amid
- 8-(4-Phenoxy-butyryl)-1-oxa-2,8-diaza-spiro[4.5]dec-2-en-3-carbonsäure(thiophen-2-ylmethyl)-amid
- 8-(3-Chloro-4-fluoro-benzoyl)-1-oxa-2,8-diaza-spiro[4.5]dec-2-en-3-carbonsäure(thiophen-2-ylmethyl)-amid
- 8-(5-tert-Butyl-2-methyl-2H-pyrazole-3-carbonyl)-1-oxa-2,8-diaza-spiro[4.5]dec-2-en-3-carbonsäure-(2-ethylsulfanyl-ethyl)-amid
- [8-(4-Fluoro-benzolsulfonyl)-1-oxa-2,8-diaza-spiro[4.5]dec-2-en-3-yl]-[4-(3-phenyl-propyl)-piperazin-1-yl]-methanon
- 8-(4-Trifluoromethoxy-benzoyl)-1-oxa-2,8-diaza-spiro[4.5]dec-2-en-3-carbonsäure-(pyridin-4-ylmethyl)-amid
- Benzoe säure 2-(3-cyclopentylcarbamoyl-1-oxa-2,8-diaza-spiro[4.5]dec-2-en-8-carbonyl)-benzyl ester
- 3-[4-(4-tert-Butyl-benzyl)-piperazine-1-carbonyl]-1-oxa-2,8-diaza-spiro[4.5]dec-2-en-8-sulfonsäuredimethylamid
- {[8-(2-Ethoxy-benzoyl)-1-oxa-2,8-diaza-spiro[4.5]dec-2-en-3-carbonyl]-amino}-essigsäureethylester
- 8-(5-Chloro-thiophen-2-sulfonyl)-1-oxa-2,8-diaza-spiro[4.5]dec-2-en-3-carbonsäure-(2-benzyloxy-cyclopentyl)-amid
- 8-(Naphthalin-1-carbonyl)-1-oxa-2,8-diaza-spiro[4.5]dec-2-en-3-carbonsäure-(thiophen-2-ylmethyl)-amid
- 8-(4-Fluoro-benzolsulfonyl)-1-oxa-2,8-diaza-spiro[4.5]dec-2-en-3-carbonsäure-(3-phenyl-propyl)-amid
- 8-(5-Chloro-thiophen-2-sulfonyl)-1-oxa-2,8-diaza-spiro[4.5]dec-2-en-3-carbonsäure-(2,3-dihydro-benzo[1,4]dioxin-2-ylmethyl)-amid
- 1-[8-(4-tert-Butyl-benzoyl)-1-oxa-2,8-diaza-spiro[4.5]dec-2-en-3-carbonyl]-piperidin-2-carbonsäure-ethyl ester
- 8-(4-Fluoro-benzolsulfonyl)-1-oxa-2,8-diaza-spiro[4.5]dec-2-en-3-carbonsäure-[2-(3-trifluoromethyl-phenyl)-ethyl]-amid
- 8-[3-(2,6-Dichloro-phenyl)-5-methyl-isoxazole-4-carbonyl]-1-oxa-2,8-diaza-spiro[4.5]dec-2-en-3-carbonsäure-(thiophen-2-ylmethyl)-amid

- 8-(4-Fluoro-benzolsulfonyl)-1-oxa-2,8-diaza-spiro[4.5]dec-2-en-3-carbonsäure-[1-(naphthalin-2-ylcarbamoyl)-ethyl]-amid
- 8-(4-Fluoro-benzolsulfonyl)-1-oxa-2,8-diaza-spiro[4.5]dec-2-en-3-carbonsäure-(2-benzyloxy-cyclopentyl)-amid
- 3-(4-Chloro-phenyl)-2-{[8-(4-methoxy-benzolsulfonyl)-1-oxa-2,8-diaza-spiro[4.5]dec-2-en-3-carbonyl]-amino}-propionic säure methyl ester
- 8-(3-Trifluoromethyl-benzolsulfonyl)-1-oxa-2,8-diaza-spiro[4.5]dec-2-en-3-carbonsäure-(2-benzyloxy-cyclopentyl)-amid
- 8-(3-Trifluoromethyl-benzolsulfonyl)-1-oxa-2,8-diaza-spiro[4.5]dec-2-en-3-carbonsäure-(2-phenoxy-ethyl)-amid
- 8-(2-Phenoxy-pyridine-3-carbonyl)-1-oxa-2,8-diaza-spiro[4.5]dec-2-en-3-carbonsäure-benzylamid
- 8-(Naphthalin-2-carbonyl)-1-oxa-2,8-diaza-spiro[4.5]dec-2-en-3-carbonsäure-cyclopropylmethyl-amid
- 8-(3-Chloro-4-fluoro-benzoyl)-1-oxa-2,8-diaza-spiro[4.5]dec-2-en-3-carbonsäure-benzylamid
- 8-(2-Phenyl-butyryl)-1-oxa-2,8-diaza-spiro[4.5]dec-2-en-3-carbonsäure-(2-cyano-ethyl)-amid
- 8-(4-Fluoro-benzolsulfonyl)-1-oxa-2,8-diaza-spiro[4.5]dec-2-en-3-carbonsäure-2-fluoro-benzylamid
- 8-(3-Chloro-benzoyl)-1-oxa-2,8-diaza-spiro[4.5]dec-2-en-3-carbonsäure-(thiophen-2-ylmethyl)-amid
- (4-Benzyl-piperazin-1-yl)-[8-(2,5-Dichloro-benzolsulfonyl)-1-oxa-2,8-diaza-spiro[4.5]dec-2-en-3-yl]-methanon
- 8-(3-Chloro-benzolsulfonyl)-1-oxa-2,8-diaza-spiro[4.5]dec-2-en-3-carbonsäure-benzylamid
- (4-Benzyl-piperidin-1-yl)-[8-(3-trifluoromethyl-benzolsulfonyl)-1-oxa-2,8-diaza-spiro[4.5]dec-2-en-3-yl]-methanon
- 8-(4-Bromo-benzoyl)-1-oxa-2,8-diaza-spiro[4.5]dec-2-en-3-carbonsäure-benzylamid
- 8-(4-Fluoro-benzolsulfonyl)-1-oxa-2,8-diaza-spiro[4.5]dec-2-en-3-carbonsäure-[1-(4-nitro-phenyl)-ethyl]-amid
- 4-Methyl-2-{[8-(3-trifluoromethyl-benzolsulfonyl)-1-oxa-2,8-diaza-spiro[4.5]dec-2-en-3-carbonyl]-amino}-pentanoic säure tert-butyl ester
- [4-(4-Fluoro-phenyl)-3,6-dihydro-2H-pyridin-1-yl]-(8-phenylmethanesulfonyl-1-oxa-2,8-diaza-spiro[4.5]dec-2-en-3-yl)-methanon
- 8-Dimethylsulfamoyl-1-oxa-2,8-diaza-spiro[4.5]dec-2-en-3-carbonsäure-[2-(ethyl-m-tolyl-amino)-ethyl]-amid
- 8-(2,5-Dimethyl-furan-3-carbonyl)-1-oxa-2,8-diaza-spiro[4.5]dec-2-en-3-carbonsäure-benzylamid
- (4-Cycloheptyl-piperazin-1-yl)-[8-(4-fluoro-benzolsulfonyl)-1-oxa-2,8-diaza-spiro[4.5]dec-2-en-3-yl]-methanon
- 8-(2-Benzyloxy-acetyl)-1-oxa-2,8-diaza-spiro[4.5]dec-2-en-3-carbonsäure benzylamid
- *R*,*R*-8-(5-Chlor-thiophen-2-sulfonyl)-1-oxa-2,8-diaza-spiro[4.5]dec-2-en-3-carbonsäure (2-benzyloxy-cyclopentyl)-amid;

sowie ihre Hydrochloride.

**10.** Verfahren zur Herstellung eines substituierten 1-Oxa-2,8-diaza-spiro[4.5]dec-2-en-derivats der allgemeinen Formel (I)

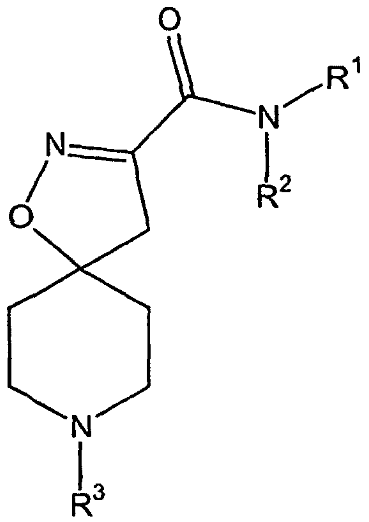

I ,

worin $R^1$, $R^2$ und $R^3$ wie in irgendeinem der Ansprüche 1 bis 8 definiert sind,
**dadurch gekennzeichnet, daß**

(a) die Verbindung der Formel (II)

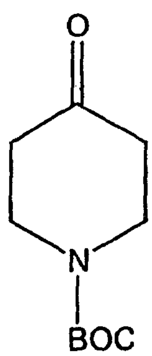

II

mit einem Methylenierungsmittel, bevorzugt $Ph_3PCH_3Br$ in Gegenwart von Kalium-tert.-Butylat in THF, zu Verbindung (III)

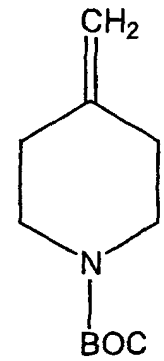

III

umgesetzt wird;
(b) Verbindung (III) einer Umsetzung mit Ethylchloroximidoacetat (IV)

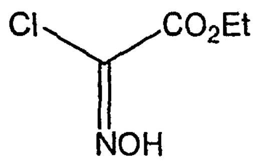

IV

in Gegenwart einer Base, bevorzugt Natriumhydrogencarbonat oder Lithiumhydroxid, bevorzugt in einem organischen Lösungsmittel, insbesondere Methanol, Dichlormethan oder THF, unter Bildung des 1-Oxa-2,8-diaza-spiro[4.5]dec-2-en-derivats der Formel (V)

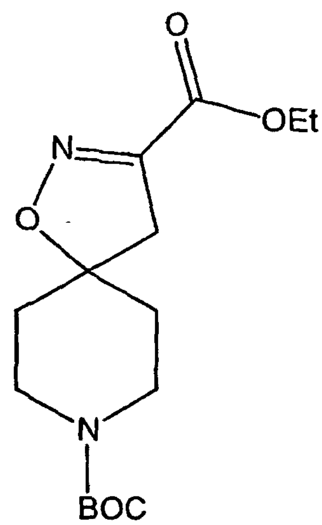

V

unterworfen wird;
(c) Verbindung (V) entweder direkt oder nach vorheriger Verseifung der Carbonsäureethylesterfunktion der Verbindung (V) und gegebenenfalls unter Aktivierung der so gebildeten Carbonsäurefunktion mit einem Amin der Formel $HNR^1R^2$, worin $R^1$ und $R^2$ wie in irgendeinem der Ansprüche 1 bis 8 definiert sind, zu dem 1-Oxa-2,8-diaza-spiro[4.5]dec-2-en-Derivat der Formel (VI)

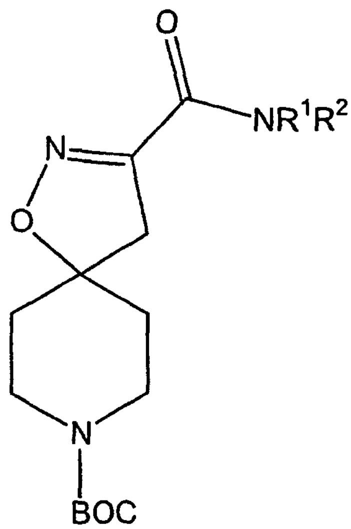

VI

umgesetzt wird;
(d) durch Entfernen der BOC-Schutzgruppe aus Verbindung (VI) Verbindung (I) mit $R^3$ = H

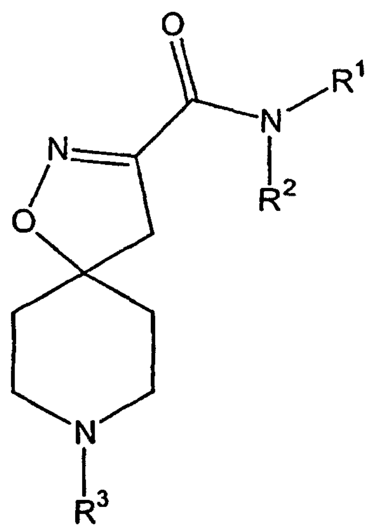

I

erhalten wird; und
(e) gegebenenfalls Verbindung (I) mit $R^3$ = H mit einem Säurechlorid der Formel $R^{12}SO_2Cl$ in eine Verbindung (I) mit $R^3 = SO_2R^{12}$, worin $R^{12}$ wie in irgendeinem der Ansprüche 1 bis 8 definiert ist, oder mit einem Carbonsäurechlorid der Formel $R^{13}COCl$ in eine Verbindung (I) mit $R^3 = COR^{13}$, worin $R^{13}$ wie in irgendeinem der Ansprüche 1 bis 8 definiert ist, umgewandelt wird.

**11.** Arzneimittel, enthaltend mindestens ein substituiertes 1-Oxa-2,8-diaza-spiro[4.5]dec-2-en-derivat der allgemeinen Formel (I) gemäß Anspruch 1 in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate.

**12.** Verwendung mindenstens eines substituierten 1-Oxa-2,8-diaza-spiro[4.5]dec-2-en-derivats der allgemeinen Formel (I) gemäß Anspruch 1 in Form seiner Racemate, seiner reinen Stereoisomeren, insbesondere Enantiomeren

oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Behandlung von Schmerz, insbesondere neuropathischem Schmerz und/oder chronischem Schmerz, und/oder zur Behandlung und/oder Vorbeugung von Migräne.

**13.** Verwendung mindestens eines substituierten 1-Oxa-2,8-diaza-spiro[4.5]dec-2-en-derivats der allgemeinen Formel (I) gemäß Anspruch 1 in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Behandlung und/oder Prophylaxe von Harninkontinenz und/oder Juckreiz und/oder Tinnitus aurium und/oder Diarrhoe.

**14.** Verwendung mindestens eines substituierten 1-Oxa-2,8-diaza-spiro[4.5]dec-2-en-derivats der allgemeinen Formel (I) gemäß Anspruch 1 in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Anästhesie, insbesondere Lokalanästhesie, und/oder zur Behandlung und/oder Prophylaxe von Arrhythmien und/oder Emesis und/oder cardiovaskulären Erkrankungen und/oder cerebralen Ischämien und/oder Alkoholabhängigkeit und/oder Drogenabhängigkeit und/oder Medikamentenabhängigkeit und/oder Entzündungen und/oder Vertigo und/oder als Nootropikum (Neurotropikum) und/oder Muskelrelaxanz.

**15.** Verwendung mindestens eines substituierten 1-Oxa-2,8-diaza-spiro[4.5]dec-2-en-derivats der allgemeinen Formel (I) gemäß Anspruch 1 in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Behandlung und/oder Prophylaxe von inflammatorischen und/oder allergischen Reaktionen und/oder Gastritis und/oder Ulcera und/oder Depressionen und/oder Schockzuständen und/oder Narkolepsie und/oder Epilepsie und/oder Übergewicht und/oder Asthma und/oder Glaukom und/oder hyperkinetischem Syndrom.

**16.** Verwendung mindestens eines substituierten 1-Oxa-2,8-diaza-spiro[4.5]dec-2-en-derivats der allgemeinen Formel (I) gemäß Anspruch 1 in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Behandlung und/oder Prophylaxe von Antriebslosigkeit und/oder Bulimie und/oder Anorexie und/oder Katalepsie und/oder zur Anxiolyse und/oder zur Vigilanz- und/oder Libidosteigerung.

**17.** Verwendung mindestens eines substituierten 1-Oxa-2,8-diaza-spiro[4.5]dec-2-en-derivats der allgemeinen Formel (I) gemäß Anspruch 1 in Form seiner Racemate, seiner reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze, ganz besonders bevorzugt in Form seiner Hydrochloride, oder in Form seiner Solvate, insbesondere der Hydrate; zur Herstellung eines Arzneimittels zur Behandlung und/oder Prophylaxe von bipolaren Störungen und/oder postmenopausalen Hitzewallungen und/oder Amyotropischer Lateraler Sklerose (ALS) und/oder Reflex-sympathetischer Dystrophie (RSD) und/oder spastischer Lähmung und/oder Restless Leg Syndrom und/oder erworbenem Nystagmus und/oder Multipler Sklerose und/oder Morbus Parkinson und/oder Morbus Alzheimer und/oder Morbus Huntington.

## Claims

**1.** Substituted 1-oxa-2,8-diaza-spiro[4.5]dec-2-ene derivative of general formula

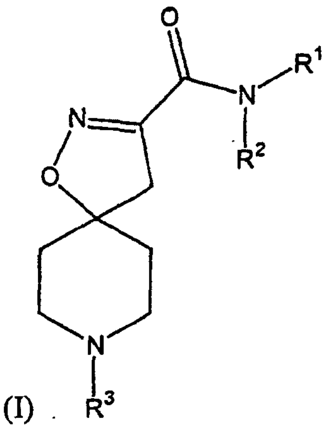

(I) .

I

in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its solvates, in particular the hydrates,
wherein

| | |
|---|---|
| $R^1$ and $R^2$ | independently of one another represent H, $C_{1-18}$-alkyl, $C_{3-10}$-cycloalkyl, ($C_{1-12}$-alkyl)-$C_{3-10}$-cycloalkyl, aryl, ($C_{1-12}$-alkyl)-aryl, heterocyclyl, ($C_{1-12}$-alkyl)-heterocyclyl or NH-C(=O)-aryl, wherein at least one of the radicals $R^1$ and $R^2$ does not represent H, or together represent $-(CR^4R^5)_m-(CR^6R^7)_n-Y-(CR^8R^9)_p-(CR^{10}R^{11})_q-$ where m, n, p and q respectively = 0, 1, 2, 3, 4 or 5, with the proviso that $m+n \geq 1$ and $p+q \geq 1$, or $-CH_2-CH_2-C(-aryl)=CH-CH_2-$; |
| $R^3$ | represents H, $SO_2R^{12}$ or $COR^{13}$; |
| $R^4$, $R^5$, $R^6$, $R^7$, $R^8$, $R^9$, $R^{10}$ and $R^{11}$ | independently of one another represent H, $C_{1-10}$-alkyl, $C_{3-8}$-cycloalkyl, ($C_{1-6}$-alkyl)-$C_{3-8}$-cycloalkyl, aryl, ($C_{1-6}$-alkyl)-aryl, heterocyclyl, ($C_{1-6}$-alkyl)-heterocyclyl or $C(=O)R^{14}$; |
| Y | represents $CR^{15}R^{16}$, $NR^{17}$ or O; |
| $R^{12}$ and $R^{13}$ | independently of one another represent $C_{1-10}$-alkyl, $C_{3-10}$-cycloalkyl, ($C_{1-6}$-alkyl)-$C_{3-10}$-cycloalkyl, aryl, ($C_{1-6}$-alkyl)-aryl, heterocyclyl, ($C_{1-6}$-alkyl)-heterocyclyl or $NR^{18}R^{19}$; |
| $R^{14}$ | represents H, $C_{1-10}$-alkyl, $C_{3-8}$-cycloalkyl, ($C_{1-6}$-alkyl)-$C_{3-8}$-cyaoalkyl, aryl, ($C_{1-6}$-alkyl)-aryl, heterocyclyl, ($C_{1-6}$-alkyl)-heterocyclyl or $OR^{20}$; |
| $R^{15}$ and $R^{16}$ | independently of one another represent H, $C_{1-10}$-alkyl, $C_{3-8}$-cycloalkyl, ($C_{1-6}$-alkyl)-$C_{3-8}$-cycloalkyl, aryl, ($C_{1-6}$-alkyl)-aryl, heterocyclyl, ($C_{1-6}$-alkyl)-heterocyclyl or $C(=O)R^{21}$; |
| $R^{17}$ | represents H, $C_{1-10}$-alkyl, $C_{3-8}$-cycloalkyl, ($C_{1-6}$-alkyl)-$C_{3-8}$-cycloalkyl, aryl, ($C_{1-6}$-alkyl)-aryl, heterocyclyl, ($C_{1-6}$-alkyl)-heterocyclyl or $C(=O)R^{22}$; |

$R^{18}$ and $R^{19}$ independently of one another represent H, $C_{1-10}$-alkyl, $C_{3-8}$-cycloalkyl, $C_{1-6}$-alkyl)-$C_{3-8}$-cycloalkyl, aryl, ($C_{1-6}$-alkyl)-aryl, heterocyclyl or ($C_{1-6}$-alkyl)-heterocyclyl;

$R^{20}$ represents H, $C_{1-10}$-alkyl, $C_{3-8}$-cycloalkyl, ($C_{1-6}$-alkyl)-$C_{3-8}$-cycloalkyl, aryl, ($C_{1-6}$-alkyl)-aryl, heterocyclyl or ($C_{1-6}$-alkyl)-heterocyclyl;

$R^{21}$ represents H, $C_{1-10}$-alkyl, $C_{3-8}$-cycloalkyl, ($C_{1-6}$-alkyl)-$C_{3-8}$ cycloalkyl, aryl, ($C_{1-6}$-alkyl)-aryl, heterocyclyl, ($C_{1-6}$-alkyl)-heterocyclyl or $OR^{23}$;

$R^{22}$ represents H, $C_{1-10}$-alkyl, $C_{3-8}$-cycloalkyl, ($C_{1-6}$-alkyl)-$C_{3-8}$-cycloalkyl, aryl, ($C_{1-6}$-alkyl)-aryl, heterocyclyl, ($C_{1-6}$-alkyl)-heterocyclyl or $OR^{24}$; and

$R^{23}$ and $R^{24}$ independently of one another represent H, $C_{1-10}$-alkyl, $C_{3-8}$-cycloalkyl, ($C_{1-6}$-alkyl)-$C_{3-8}$-cycloalkyl, aryl, ($C_{1-6}$-alkyl)-aryl, heterocyclyl or ($C_{1-6}$-alkyl)-heterocyclyl.

2. Substituted 1-oxa-2,8-diaza-spiro[4.5]dec-2-ene derivative according to claim 1, **characterised in that**

$R^1$ and $R^2$ independently of one another represent H, $C_{1-8}$-alkyl, $C_{3-8}$-cycloalkyl, ($C_{1-4}$-alkyl)-$C_{3-8}$-cycloalkyl, aryl, ($C_{1-4}$-alkyl)-aryl, heterocyclyl, ($C_{1-4}$-alkyl)-heterocyclyl or NH-C(=O)-aryl , wherein at least one of the radicals $R^1$ and $R^2$ does not represent H,
or
together represent $-(CR^4R^5)m-(CR^6R^7)n-Y-(CR^8R^9)p(CR^{10}R^{11})_q-$ where m = 1, n = 0 or 1, p = 1 or 2 and q = 1 or 2, or $-CH_2-CH_2-C(-aryl)=CH-CH_2$;

$R^3$ represents $SO_2R^{12}$ or $COR^{13}$;

$R^4$, $R^5$, $R^6$, $R^7$ $R^8$, $R^9$, $R^{10}$ and $R^{11}$ independently of one another represent H, $C_{1-8}$-alkyl or $C(=O)R^{14}$;

Y represents $CR^{15}R^{16}$ or $NR^{17}$;

$R^{12}$ represents $C_{14}$-alkyl, ($C_{1-4}$-alkyl)-$C_{3-8}$-cycloalkyl, aryl, ($C_{1-4}$-alkyl)-aryl, heterocyclyl or $NR^{18}R^{19}$;

$R^{13}$ represents $C_{1-6}$-alkyl, $C_{3-10}$-cycloalkyl, aryl, ($C_{1-4}$-alkyl)-aryl or heterocyclyl;

$R^{14}$ represents $OR^{20}$;

$R^{15}$ and $R^{16}$ independently of one another represent H, aryl or ($C_{1-4}$-alkyl)-aryl;

$R^{17}$ represents H, $C_{3-8}$-cycloalkyl, aryl, ($C_{1-4}$-alkyl)-aryl, heterocyclyl or C(=O) $R^{22}$;

$R^{18}$ and $R^{19}$ independently of one another represent H or $C_{1-6}$-alkyl;

$R^{20}$ represents $C_{1-6}$-alkyl;

$R^{22}$ represents aryl, ($C_{1-4}$-alkyl)-aryl, heterocyclyl or $OR^{24}$;
and

$R^{24}$ represents $C_{1-6}$-alkyl or ($C_{1-4}$-alkyl)-aryl.

3. Substituted 1-oxa-2,8-diaza-spiro[4.5]dec-2-ene derivative according to claim 1 or 2, **characterised in that**

$R^1$ represents $C_{1-6}$-alkyl, $C_{3-8}$-cycloalkyl, ($C_{1-3}$-alkyl)-$C_{3-8}$-cycloalkyl, aryl, ($C_{1-3}$-alkyl)-aryl, heterocyclyl, ($C_{1-3}$-alkyl-)heterocyclyl or NH-C(=O)-aryl; and

$R^2$ represents H, $C_{1-4}$-alkyl, ($C_{1-3}$-alkyl)-aryl or ($C_{1-3}$-alkyl)-heterocyclyl.

**4.** Substituted 1-oxa-2,8-diaza-spiro[4.5]dec-2-ene derivative according to claim 3, **characterised in that**

$R^1$ represents methyl, ethyl, n-propyl, iso-propyl, 2-methylpropyl, n-butyl, tert.-butyl, n-phenyl, 3-methylbutyl or $CH_2$-$C(CH_3)$=$CH_2$, in particular methyl, ethyl, $CH_2$-$C(CH_3)$=$CH_2$, CH(C(=O)O$CH_2$=$CH_2$)-$CH_2$C(=O)O-tert.-butyl, 2-cyanoethyl, $CH_2$-$CH_2$-NH-C(=O)$CH_3$, 2-(N-ethyl-N-(3-methylphenyl)amino)-ethyl, 2-(N,N-dimethylamino)-ethyl, 2-(C(=O)-NH-(β-naphthyl)-ethyl, 1,2-(di(C(=O)O-tert.-butyl)ethyl, 3-(N-methyl-N-phenylamino)-propyl, 1-(C(=O)O-benzyl)-3-methylbutyl, 1-(C(=O)O-butyl)-3-methyl-butyl, $CH_2CO_2$ethyl, $CH_2$-$CH_2CO_2$ethyl, $CH_2$-$CH_2$-Ophenyl, $CH_2$-$CH_2$-S-$CH_2$-$CH_3$, unsubstituted or substituted by F, Cl, Br, I, -CN, N-alkyl-N-arylamine, N,N-dialkylamine, amide, carboxyalkyl, carboxybenzyl; cyclopropyl, cyclobutyl, cyclopentyl, cyclohexyl, cycloheptyl, cyclooctyl, bicyclo[3.1.1]heptan-3-yl, in particular 2-phenylcyclopropyl, 2-(O-benzyl)-cyclopentyl, 2-(carboxyethyl)cyclohexyl, 7,7-dimethyl-2-methylbicyclo[3.1.1]-heptan-3-yl; cyclopropylmethyl, 7,7-dimethyl-2-oxo-bicyclo[2.2.1]hept-1-ylmethyl, unsubstituted or substituted by F, Cl, Br, I, -CN, alkyl, aryl, carboxyalkyl, carboxybenzyl, O-alkyl, O-benzyl;
phenyl, 1-naphthyl or 2-naphthyl, unsubstituted or substituted by phenoxy, -$CH_2$- P(=O)(Oethyl)$_2$;
$CH_2$-aryl, $CH_2$-$CH_2$-aryl, $CH_2$-$CH_2$-$CH_2$-aryl, $CH_2$-$CH_2$-$CH_2$-$CH_2$-aryl CH($CH_3$)-aryl, CH($CH_3$)-$CH_2$-aryl, $CH_2$CH-(aryl)$_2$, CH($CO_2$alkyl)-$CH_2$-aryl, $CH_2$-$CH_2$-CH-(aryl)$_2$, wherein aryl is unsubstituted phenyl, 1-naphthyl or 2-naphthyl or phenyl, in particular benzyl, -$CH_2$-naphth-1-yl, 2-fluorobenzyl, 3-fluorobenzyl, 3-chlorobenzyl, 3-methoxybenzyl, 2-ethoxybenzyl, 2,4-difluorobenzyl, 3,5-dichlorobenzyl, 3-fluoro-5-trifluoromethylbenzyl, 3-fluoro-4-trifluoromethylbenzyl, 2-chloro-6-fluorobenzyl, 2,5dimethoxybenzyl, 2-chloro-6-methylbenzyl, 3,4-dimethoxybenzyl, 3,4-dioxymethylenebenzyl, CH($CH_3$)phenyl, CH($CH_3$)-(4-$CH_3$-phenyl), CH ($CH_3$)-(4-nitrophenyl), CH($CH_3$)-(2,3-dioxyethylenephenyl), $CH_2CH_2$-phenyl, $CH_2$-$CH_2$-(2-fluorophenyl), $CH_2$-$CH_2$-(3-fluorophenyl),$CH_2$-$CH_2$-(4-fluorophenyl),$CH_2$-$CH_2$-(4-chlorophenyl),$CH_2$-$CH_2$-(3,4-dichlorophenyl), $CH_2$-$CH_2$(3-methoxyphenyl), $CH_2$-$CH_2$-(2,5-dimethoxyphenyl), CH($CO_2$-tert.-butyl)-$CH_2$-phenyl, CH ($CO_2$-methyl)-$CH_2$(4-chlorophenyl), $CH_2$-CH(phenyl)$_2$, CH($CH_3$)-$CH_2$-(4-chlorophenyl), $CH_2$-$CH_2$-CH(phenyl)$_2$, $CH_2$-$CH_2$-$CH_2$-phenyl substituted by F, Cl, Br, I, -CN, -$NO_2$, alkyl, $CF_3$, alkoxy, alkylene dioxy;
pyrrolidine or piperidine, in particular pyrrolidin-3-yl, N-(4-trifluorobenzyl)-pyrrolidin-3-yl, N-(3-methoxybenzyl)-pyrrolidin-3-yl, N($CH_2$-(β-naphthyl)-pyrrolidin-3-yl or N-(carboxyethyl)-piperidin-4-yl, unsubstituted or substituted by aryl, alkylaryl or carboxyethyl;
$(CH_2)_{1-3}$-heterocyclyl, unsubstituted or substituted by alkyl, F, Cl, Br, I, -CN, aryl, alkylaryl, wherein heterocyclyl represents furanyl, benzofuranyl, 1,4-dioxanyl, benzo-1,4-dioxanyl, thienyl, pyridinyl, pyrrolidinyl, 1H-indolyl, imidazolyl, piperidinyl, tetrahydrofuranyl, in particular $CH_2$-furan-2-yl, 5-methylfuran-2-yl $CH_2$-benzofuran-2-yl,

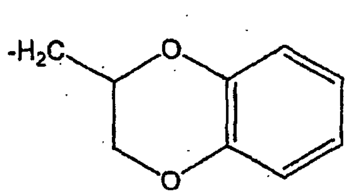

,

$CH_2$-thien-2-yl, $CH_2$-pyridin-3-yl, $CH_2$-pyridin-4-yl, $CH_2CH_2$-pyridin-2-yl, $CH_2$-$CH_2$-(1H-indol-3-yl), $CH_2$-$CH_2$-pyrrolidin-1-yl, $CH_2$-(N-2,6-dichlorobenzyl-pyrrolidin-3-yl), $CH_2$-$CH_2$-(N-methyl-pyrrolidin-2-yl), -$(CH_2)_3$-imidazol-1-yl, $CH_2$-(tetrahydrofuran-2-yl) or

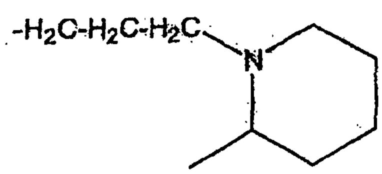

,

or CH($CO_2$methyl)-$CH_2$-(1H-indol-3-yl); NH-C(=O)-(4-diethylaminophenyl); and

$R^2$ represents H;
methyl, ethyl or $CH_2$-C($CH_3$)=$CH_2$, in particular methyl, ethyl, 2-cyanoethyl, $CH_2$-C($CH_3$)= $CH_2$, unsubstituted or substituted by F, Cl, Br, I, -CN;
benzyl or phenethyl, in particular benzyl, 4-fluorobenzyl, 2-chloro-6-fluorobenzyl, 2,5-dimethoxybenzyl, phenethyl, unsubstituted or substituted by F, Cl, Br, I, -CN, methoxy, ethoxy; or $CH_2$-furanyl, in particular $CH_2$-furan-2-yl, $CH_2$-benzofuranyl, in particular $CH_2$-benzofuran-2-yl, $CH_2$-pyridinyl, in particular $CH_2$-pyridin-3-yl, $CH_2$-tetrahydrofuranyl, in particular $CH_2$-tetrahydrofuran-2-yl, $CH_2$-$CH_2$-pyridinyl, in particular $CH_2$-$CH_2$-pyridin-2-yl.

**5.** Substituted 1-oxa-2,8-diaza-spiro[4.5]dec-2-ene derivative according to claim 1 or 2, **characterised in that**

$R^1$ and $R^2$ together represent

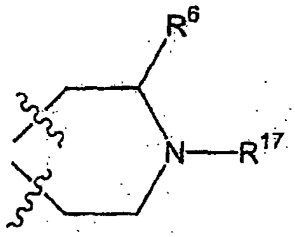 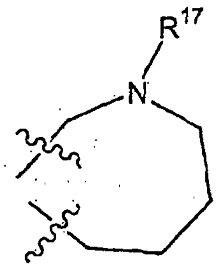 ,

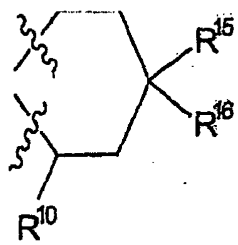 or 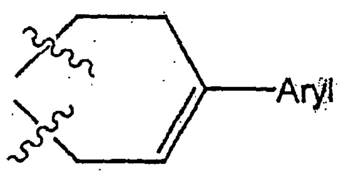 ,

wherein aryl
represents phenyl, or phenyl, in particular 4-fluorophenyl, substituted by F, Cl, Br, I;

$R^6$ represents H or $C_{1-4}$ alkyl, in particular methyl;

$R^{10}$ represents H, C(=O)Omethyl, C(=O)Oethyl, C(=O)O-n-propyl, C(=O)O-iso-propyl, C(=O)O-n-butyl, C(=O)O-tert.-butyl;

$R^{15}$ represents H or $CH_2$-aryl;

$R^{16}$ represents H;

$R^{17}$ represents H;
$C_{3-8}$-cycloalkyl, in particular cycloheptyl;
aryl, in particular phenyl or naphthyl, unsubstituted or substituted by methyl, ethyl, n-propyl, isopropyl, n-butyl, tert.-butyl, $CF_3$, F, Cl, Br, I, -CN, methoxy, ethoxy, n-propoxy, isopropoxy, n-butoxy, tert.-butoxy;
(($CH_2$)$_{1-3}$-alkyl)-aryl or CH($CH_3$)-aryl, wherein aryl represents phenyl or naphthyl, unsubstituted or substituted by alkyl, $CF_3$, F, Cl, Br, I, -CN, alkoxy;
pyridinyl, pyrazinyl or thieno[2,3-d]pyrimidinyl, unsubstituted or substituted by alkyl, $CF_3$, F, Cl, Br, I, - CN, alkoxy; or
C(=O)$R^{22}$; and

$R^{22}$ represents phenyl or alkoxy-substituted phenyl, O-methyl, O-ethyl, O-n-propyl, O-iso-propyl, O-n-butyl, O-tert.butyl, O-benzyl, unsubstituted benzyl, benzyl substituted by F, unsubstituted pyrazinyl or pyrazinyl substituted by alkyl.

**6.** Substituted 1-oxa-2,8-diaza-spiro[4.5]dec-2-ene derivative according to claim 5, **characterised in that**

$R^6$ represents H or methyl;

$R^{10}$ represents H or C(=O)Oethyl;

$R^{15}$ represents H or benzyl;

$R^{16}$ represents H; and

$R^{17}$ represents H;
cycloheptyl;
phenyl, 2-methylphenyl, 3-methylphenyl, 2,4-dimethylphenyl, 2-ethylphenyl, 3-trifluoromethyl, 4-fluorophenyl, 4-chlorophenyl, 2-methoxyphenyl, 3,5-dimethoxyphenyl, 3-chloro-6-methylphenyl;
benzyl, $CH_2$-(4-tert.-butylphenyl), $CH_2$-(β-naphthyl), $CH(CH_3)$-phenyl, $(CH_2)_3$-phenyl;
pyridin-2-yl, (4-trifluoromethyl)-pyridin-2-yl, thieno[2,3-d]pyrimidin-4-yl; or
C(=O)-(4-methoxyphenyl), C(=O)-benzyl, C(=O)-$CH_2$-(3,4-difluorophenyl), C(=O)-(2-methylpyrazin-5-yl), C(=O)O-tert.-butyl or O-benzyl.

**7.** Substituted 1-oxa-2,8-diaza-spiro[4.5]dec-2-ene derivative according to any of claims 1 to 6, **characterised in that**

$R^3$ represents $SO_2R^{12}$;
$R^{12}$ represents methyl, ethyl, n-propyl, iso-propyl, in particular n-propyl;
7,7-dimethyl-2-oxo-bicyclo[2.2.1]hept-1-ylmethyl;
phenyl, or phenyl, in particular 4-methylphenyl, 3-trifluoromethylphenyl, 4-fluorophenyl, 2-chlorophenyl, 3-chlorophenyl, 4-chlorophenyl, 4-methoxyphenyl, 4-trifluoromethoxyphenyl, 4-nitrophenyl, 2-$CO_2$-methyl-phenyl, 2,5-dichlorophenyl, 3-fluoro-6-methylphenyl, 3-bromo-6-methoxyphenyl, 2-methyl-5-nitrophenyl, 2,4,6-trimethylphenyl substituted by methyl, ethyl, n-propyl, iso-propyl, n-butyl, tert.-butyl, $CF_3$, F, Cl, Br, I, -CN, $NO_2$, methoxy, ethoxy, n-propoxy, iso-propoxy, n-butoxy, tert.-butoxy, $OCF_3$, $CO_2$-methyl; benzyl or benzyl substituted by methyl, ethyl, n-propyl, iso-propyl, n-butyl, tert.-butyl, $CF_3$, F, Cl, Br, I, -CN, $NO_2$, methoxy, ethoxy, n-propoxy, iso-propoxy, n-butoxy, tert.-butoxy, $OCF_3$; furanyl or thienyl, in particular thien-2-yl, 5-chlorothien-2-yl, unsubstituted or substituted by methyl, ethyl, n-propyl, iso-propyl, n-butyl, tert.-butyl, $CF_3$, F, Cl, Br, I, -CN, $NO_2$, methoxy, ethoxy, n-propoxy, iso-propoxy, n-butoxy, tert.-butoxy; or $NR^{13}R^{19}$; and
$R^{18}$ and $R^{19}$ independently of one another represent H, methyl or ethyl.

**8.** Substituted 1-oxa-2,8-diaza-spiro[4.5]dec-2-ene derivative according to any of claims 1 to 6, **characterised in that**

$R^3$ represents $COR^{13}$; and

$R^{13}$ represents methyl, ethyl, C(=O)Omethyl, n-propyl, iso-propyl, 2-methylpropyl, n-butyl, tert.-butyl, n-pentyl or 3-methylbutyl, in particular methyl, ethyl, n-propyl, n-butyl, tert.-butyl, n-pentyl, $CH_2$-O-$CH_2$-$CH_2$-$OCH_3$, $CH(CH_3)$-O-phenyl, $CH_2$-$CH_2$-C(=O)$OCH_3$, $C(CH_3)_2$-OC(=O)$CH_3$, $CH_2$-O-benzyl, $CH_2$-O-(3-chlorophenyl), $CH_2$-$CH_2$-$CH_2$-O-phenyl, CH(OC(=O)methyl)$CH_3$; cyclopropyl, 2-phenylcyclopropyl, 1-adamantyl, unsubstituted or substituted by O-methyl, O-ethyl, O-$(CH_2)_2OCH_3$, O-benzyl, O-phenyl, wherein phenyl is unsubstituted or substituted by F, Cl, Br, I, -CN, O-C(=O)-methyl, O-C(=O)-ethyl;
phenyl or naphthyl, in particular 4-fluorophenyl, 2-chlorophenyl, 3-chlorophenyl, 4-bromophenyl, 4-phenylphenyl (4-biphenyl), 4-ethylphenyl, 4-$CF_3$-phenyl, 4-methoxyphenyl, 2-ethoxyphenyl, 4-tert.-butyl, 3-$OCF_3$-phenyl, 4-$OCF_3$-phenyl, 4-$SCF_3$-phenyl, 3-$SCF_2$-phenyl, 2-$CH_2$-OC(=O)phenyl, 3-dimethylaminophenyl, 2,3-dichlorophenyl, 2,4-difluorophenyl, 3-chloro-4-fluorophenyl, 3-chloro-2-fluorophenyl, 4-$CF_3$-3-fluorophenyl, 3-$CF_3$-6-fluorophenyl, 4-bromo-3-methylphenyl, 2-chloro-4-nitrophenyl, 2,3,4,5,6-pentafluorophenyl, 2,6-difluoro-3-methylphenyl, 2,3-difluoro-4-methylphenyl, 2-chloro-5-methyl-6-fluorophenyl, 1-naphthyl, 2-naphthyl, unsubstituted or substituted by F, Cl, Br, I, -CN, phenyl, methyl, ethyl, n-propyl, iso-propyl, n-butyl, tert.-butyl, $CF_3$, F, Cl, Br, I, -CN, $NO_2$, methoxy, ethoxy, n-propoxy, iso-propoxy, n-butoxy, tert.-butoxy,

$OCF_3$, $OCHF_2$, $OCH_2F$, $SCF_3$, $SCHF_2$, $SCH_2F$, $SCH_3$, $CH_2$ OC(=O)phenyl, -N($CH_3$); unsubstituted or substituted ($C_{1-2}$-alkyl)-aryl, in particular benzyl, phenethyl, CH($C_2H_5$)-phenyl, CH (NH-$SO_2$-(4-methylphenyl))-$CH_2$-phenyl, CH=CH-phenyl, CH=CH-(3-trifluorophenyl); or unsubstituted or alkyl-substituted furanyl, benzofuranyl, or thienyl, pyridinyl, pyrazolyl, benzodihydropyranyl, isooxazolyl, in particular 1,5-dimethylfuran-3-yl, 2-methyl-5-tert.-butyl-furan-3-yl, 3-chlorothien-2-yl 1-(4-chlorophenyl)-5-trifluoromethyl-pyrazol-4-yl, 1-methyl-3-tert.-butyl-pyrazol-5-yl,

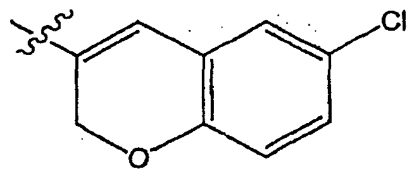

,

pyridin-4-yl, 2-methylthiopyridin-3-yl, 2-ethylthiopyridin-3-yl, 2-phenoxypyridin-3-yl, 2-chloropyridin-3-yl, 5-methyl-3-(2,6-dichlorophenyl)-isoxazol-4-yl, 5-methyl-3-(2-chloro-6-fluorophenyl)-isoxazol-4-yl, unsubstituted or substituted by alkyl, $CF_3$, aryl, O-phenyl, chlorine, S-methyl, S-ethyl.

**9.** Substituted 1-oxa-2,8-diaza-spiro[4.5]dec-2-ene derivative according to claim 1, **characterised in that** it is selected from the group consisting of:

- 3-(1H-indol-3-yl)-2-{[8-(4-trifluoromethoxy-benzenesulphonyl)-1-oxa-2,8-diaza-spiro[4.5]dec-2-ene-3-carbonyl]-amino}-propionic acid methylester
- {4-[(8-phenylmethanesulphonyl-1-oxa-2,8-diaza-spiro[4.5]dec-2-ene-3-carbonyl)-amino]-benzyl}-phosphonic acid diethylester
- (4-cycloheptyl-piperazin-1-yl)-[8-(thiophene-2-sulphonyl)-1-oxa-2,8-diaza-spiro[4.5]dec-2-en-3-yl]-methanone
- (8-(5-chloro-thiophene-2-sulphonyl)-1-oxa-2,8-diaza-spiro[4.5]dec-2-en-3-yl]-(4-phenyl-piperazin-1-yl)-methanone
- 8-(2-chloro-4-nitro-benzoyl)-1-oxa-2,8-diaza-spiro[4.5]dec-2-ene-3-carboxylic acid cyclopentylamide
- (4-naphthalen-2-ylmethyl-piperazin-1-yl)-(8-phenylmethanesulphonyl-1-oxa-2,8-diaza-spiro[4.5]dec-2-en-3-yl)-methanone
- 8-dimethylsulphamoyl-1-oxa-2,8-diaza-spiro[4.5]dec-2-ene-3-carboxylic acid phenethylamide
- 8-(thiophene-2-sulphonyl)-1-oxa-2,8-diaza-spiro[4.5]dec-2-ene-3-carboxylic acid (1-phenyl-ethyl) amide
- 8-(toluene-4-sulphonyl)-1-oxa-2,8-diaza-spiro[4.5]dec-2-ene-3-carboxylic acid [2-(3,4-dichloro-phenyl)-ethyl] amide
- 4-diethylamino-benzoic acid N'-[8-(thiophene-2-sulphonyl)-1-oxa-2,8-diaza-spiro[4.5]dec-2-ene-3-carbonyl]-hydrazides
- 8-(3-chloro-4-fluoro-benzenesulphonyl)-1-oxa-2,8-diaza-spiro[4.5]dec-2-ene-3-carboxylic acid 2-pyrrolidin-1-yl-ethyl) amide
- 8-phenylmethanesulphonyl-1-oxa-2,8-diaza-spiro[4.5]dec-2-ene-3-carboxylic acid phenethyl amide
- 8-benzenesulphonyl-1-oxa-2,8-diaza-spiro[4.5]dec-2-ene-3-carboxylic acid (2-dimethylamino-ethyl) amide
- [4-(3-phenyl-propyl)-piperazin-1-yl]-[8-(thiophene-2-sulphonyl)-1-oxa-2,8-diaza-spiro[4.5]dec-2-en-3-yl]-methanone
- 8-(2,5-dichloro-benzenesulphonyl)-1-oxa-2,8-diaza-spiro[4.5]dec-2-ene-3-carboxylic acid [3-(2-methyl-piperidin-1-yl)-propyl] amide
- 8-(4-trifluoromethoxy-benzenesulphonyl)-1-oxa-2,8-diaza-spiro[4.5]dec-2-ene-3-carboxylic acid [2-(1H-indol-3-yl)-ethyl]-methyl amide
- 8-(5-fluoro-2-methyl-benzenesulphonyl)-1-oxa-2,8-diaza-spiro[4.5]dec-2-ene-3-carboxylic acid (2-acetylamino-ethyl) amide
- 8-(toluene-4-sulphonyl)-1-oxa-2,8-diaza-spiro[4.5]dec-2-ene-3-carboxylic acid benzyl-phenethyl amide
- 8-(2-methyl-5-nitro-benzenesulphonyl)-1-oxa-2,8-diaza-spiro[4.5]dec-2-ene-3-carboxylic acid (2-dimethylamino-ethyl) amide
- 8-phenylmethanesulphonyl-1-oxa-2,8-diaza-spiro[4.5]dec-2-ene-3-carboxylic acid (2-benzyloxy-cyclopentyl) amide
- 8-(thiophene-2-sulphonyl)-1-oxa-2,8-diaza-spiro[4.5]dec-2-ene-3-carboxylic acid (2,3-dihydro-benzo[1,4]dioxin-2-ylmethyl) amide

- 8-(toluene-4-sulphonyl)-1-oxa-2,8-diaza-spiro[4.5]dec-2-ene-3-carboxylic acid (4-phenoxy-phenyl) amide
- 8-(thiophene-2-sulphonyl)-1-oxa-2,8-diaza-spiro[4.5]dec-2-ene-3 carboxylic acid (1-p-tolyl-ethyl) amide
- 8-(thiophene-2-sulphonyl)-1-oxa-2,8-diaza-spiro[4.5]dec-2-ene-3-carboxylic acid 3-fluoro-benzyl amide
- 2-phenyl-1-{4-[8-(toluene-4-sulphonyl)-1-oxa-2,8-diaza-spiro[4.5]dec-2-ene-3-carbonyl]-piperazin-1-yl}-ethanone
- 8-(thiophene-2-sulphonyl)-1-oxa-2,8-diaza-spiro[4.5]dec-2-ene-3-carboxylic acid [1-(4-trifluoromethyl-benzyl)-pyrrolidin-3-yl] amide
- [8-(thiophene-2-sulphonyl)-1-oxa-2,8-diaza-spiro[4.5]dec-2-en-3-yl]-(4-o-tolyl-piperazin-1-yl)-methanone
- 8-(toluene-4-sulphonyl)-1-oxa-2,8-diaza-spiro[4.5]dec-2-ene-3-carboxylic acid benzyl-(2-cyano-ethyl) amide
- [8-(2,5-dichloro-benzenesulphonyl)-1-oxa-2,8-diaza-spiro[4.5]dec-2-en-3-yl]-(4-thieno[2,3-d]pyrimidin-4-yl-piperazin-1-yl)-methanone
- 8-(5-chloro-thiophene-2-sulphonyl)-1-oxa-2,8-diaza-spiro[4.5]dec-2-ene-3-carboxylic acid (benzo[1,3]dioxol-5-yl-methyl) amide
- (3-methyl-4-m-tolyl-piperazin-1-yl)-[8-(thiophene-2-sulphonyl)-1-oxa-2,8-diaza-spiro[4.5]dec-2-en-3-yl]-methanone
- [4-(1-phenyl-ethyl)-piperazin-1-yl]-[8-(toluene-4-sulphonyl)-1-oxa-2,8-diaza-spiro[4.5]dec-2-en-3-yl]-methanone
- [4-(2,4-dimethyl-phenyl)-piperazin-1-yl]-[8-(toluene-4-sulphonyl)-1-oxa-2,8-diaza-spiro[4.5]dec-2-en-3-yl]-methanone
- 8-(2-chloro-pyridine-3-carbonyl)-1-oxa-2,8-diaza-spiro[4.5]dec-2-ene-3-carboxylic acid cyclopentylamide
- (4-naphthalen-2-ylmethyl-piperazin-1-yl)-[8-(3-trifluoromethyl-benzenesulphonyl)-1-oxa-2,8-diaza-spiro[4.5]dec-2-en-3-yl]-methanone
- 8-[3-phenyl-2-(toluene-4-sulphonylamino)-propionyl]-1-oxa-2,8-diaza-spiro[4.5]dec-2-ene-3-carboxylic acid (pyridin-3-ylmethyl) amide
- 8-(5-fluoro-2-methyl-benzenesulphonyl)-1-oxa-2,8-diaza-spiro[4.5]dec-2-ene-3-carboxylic acid (2-dimethylamino-ethyl) amide
- 8-(4-nitro-benzenesulphonyl)-1-oxa-2,8-diaza-spiro[4.5]dec-2-ene-3-carboxylic acid (thiophen-2-ylmethyl) amide
- 8-(2,5-dichloro-benzenesulphonyl)-1-oxa-2,8-diaza-spiro[4.5]dec-2-ene-3-carboxylic acid 3-fluoro-5-trifluoromethyl-benzyl amide
- 8-(5-chloro-thiophene-2-sulphonyl)-1-oxa-2,8-diaza-spiro[4.5]dec-2-ene-3-carboxylic acid benzhydryl amide
- 8-[3-phenyl-2-(toluene-4-sulphonylamino)-propionyl]-1-oxa-2,8-diaza-spiro[4.5]dec-2-ene-3-carboxylic acid (2-cyano-ethyl) amide
- [(8-phenylmethanesulphonyl-1-oxa-2,8-diaza-spiro[4.5]dec-2-ene-3-carbonyl)-amino]-ethyl acetate
- 8-(3-dimethylamino-benzoyl)-1-oxa-2,8-diaza-spiro[4.5]dec-2-ene-3-carboxylic acid (2-cyano-ethyl) amide
- 8-(2,5-dichloro-benzenesulphonyl)-1-oxa-2,8-diaza-spiro[4.5]dec-2-ene-3-carboxylic acid [1-(naphthalen-2-ylcarbamoyl)-ethyl] amide
- 8-(toluene-4-sulphonyl)-I-oxa-2,8-diaza-spiro[4.5]dec-2-ene-3-carboxylic acid (1-p-tolyl-ethyl) amide
- [4-(4-chloro-phenyl)-piperazin-1-yl]-(8-phenylmethanesulphonyl-1-oxa-2,8-diaza-spiro[4.5]dec-2-en-3-yl)-methanone
- 8-(thiophene-2-sulphonyl)-1-oxa-2,8-diaza-spiro[4.5]dec-2-ene-3-carboxylic acid (2-benzyloxy-cyclopentyl) amide
- 8-acetyl-1-oxa-2,8-diaza-spiro[4.5]dec-2-ene-3-carboxylic acid benzyl amide
- 8-(5-chloro-thiophene-2-sulphonyl)-1-oxa-2,8-diaza-spiro[4.5]dec-2-ene-3-carboxylic acid 2,5-difluoro-benzyl amide
- 8-(5-chloro-thiophene-2-sulphonyl)-1-oxa-2,8-diaza-spiro[4.5]dec-2-ene-3-carboxylic acid (1-naphthalen-2-ylmethyl-pyrrolidin-3-yl) amide
- 8-phenylmethanesulphonyl-1-oxa-2,8-diaza-spiro[4.5]dec-2-ene-3-carboxylic acid 2-ethoxy-benzyl amide
- 8-(4-ethyl-benzoyl)-1-oxa-2,8-diaza-spiro[4.5]dec-2-ene-3-carboxylic acid (thiophen-2-ylmethyl) amide
- 2-{[8-(2,5-dichloro-benzenesulphonyl)-1-oxa-2,8-diaza-spiro[4.5]dec-2-ene-3-carbonyl]-amino}-succinic acid 1-allylester-4-tert-butylester
- 8-(4-trifluoromethoxy-benzenesulphonyl)-1-oxa-2,8-diaza-spiro[4.5]dec-2-ene-3-carboxylic acid naphthalen-2-ylamide
- 4-{[8-(2-phenyl-cyclopropanecarbonyl)-1-oxa-2,8-diaza-spiro[4.5]dec-2-ene-3-carbonyl]-amino}-piperidine-1-carboxylic acid ethylester
- 4-([8-(2,4-difluoro-benzoyl)-1-oxa-2,8-diaza-spiro[4.5]dec-2-ene-3-carbonyl]-amino}-piperidine-1-carboxylic acid ethylester
- 8-(toluene-4-sulphonyl)-1-oxa-2,8-diaza-spiro[4.5]dec-2-ene-3-carboxylic acid (2-dimethylamino-ethyl)

amide

- 8-dimethylsulphamoyl-1-oxa-2,8-diaza-spiro[4.5]dec-2-ene-3-carboxylic acid[2-(4-chloro-phenyl)-ethyl] amide
- 8-(4-methoxy-benzoyl)-1-oxa-2,8-diaza-spiro[4.5]dec-2-ene-3-carboxylic acid (5-methyl-furan-2-ylmethyl) amide
- 8-(thiophene-2-sulphonyl)-1-oxa-2,8-diaza-spiro[4.5]dec-2-ene-3-carboxylic acid [2-(4-chloro-phenyl)-ethyl] amide
- 8-(5-chloro-thiophene-2-sulphonyl)-1-oxa-2,8-diaza-spiro[4.5]dec-2-ene-3-carboxylic acid [2-(1-methyl-pyrrolidin-2-yl)-ethyl] amide
- 4-{[8-(4-bromo-benzoyl)-1-oxa-2,8-diaza-spiro[4.5]dec-2-ene-3-carbonyl]-amino}-piperidine-1-carboxylic acid ethylester
- 8-(toluene-4-sulphonyl)-1-oxa-2,8-diaza-spiro[4.5]dec-2-ene-3-carboxylic acid 3,5-dichloro-benzyl amide
- 8-(2,5-dichloro-benzenesulphonyl)-1-oxa-2,8-diaza-spiro[4.5]dec-2-ene-3-carboxylic acid [1-(3-methoxy-benzyl)-pyrrolidin-3-yl] amide
- 3-(4-naphthalen-2-ylmethyl-piperazine-1-carbonyl)-1-oxa-2,8-diaza-spiro[4.5]dec-2-ene-8-sulphonacid dimethylamide
- 3-{[8-(7,7-dimethyl-2-oxo-bicyclo[2.2.1]hept-1-ylmethanesulphonyl)-1-oxa-2,8-diaza-spiro[4.5]dec-2-ene-3-carbonyl]-amino}propionic acid ethylester
- 8-(toluene-4-sulphonyl)-1-oxa-2,8-diaza-spiro[4.5]dec-2-ene-3-carboxylic acid (2-benzyloxy-cyclopentyl) amide
- 8-(2,5-dichloro-benzenesulphonyl)-1-oxa-2,8-diaza-spiro[4.5]dec-2-ene-3-
- carboxylic acid 3,4-dimethoxy-benzyl amide
- (8-phenylmethanesulphonyl-1-oxa-2,8-diaza-spiro[4.5]dec-2-en-3-yl)-[4(3-phenyl-propyl)-piperazin-1-yl]-methanone
- 3-(4-thieno[2,3-d]pyrimidin-4-yl-piperazine-1-carbonyl)-1-oxa-2,8-diaza-spiro[4.5]dec-2-ene-8-sulphonic acid dimethylamide
- [8-(5-chloro-thiophene-2-sulphonyl)-1-oxa-2,8-diaza-spiro[4.5]dec-2-en-3-yl]-[4-(3,5-dimethoxy-phenyl)-piperazin-1-yl]-methanone
- 8-dimethylsulphamoyl-1-oxa-2,8-diaza-spiro[4.5]dec-2-ene-3-carboxylic acid 3-fluoro-4-trifluoromethyl-benzyl amide
- 8-(toluene-4-sulphonyl)-1-oxa-2,8-diaza-spiro[4.5]dec-2-ene-3-carboxylic acid (2-benzyloxy-cyclopentyl) amide
- 4-[(8-Butyryl-1-oxa-2,8-diaza-spiro[4.5]dec-2-ene-3-carbonyl)-amino]-piperidine-1-carboxylic acid ethylester
- 8-(toluene-4-sulphonyl)-1-oxa-2,8-diaza-spiro[4.5]dec-2-ene-3-carboxylic acid (2-phenyl-cyclopropyl) amide
- [8-(4-fluoro-benzenesulphonyl)-1-oxa-2,8-diaza-spiro[4.5]dec-2-en-3-yl]-[4(5-methyl-pyrazine-2-carbonyl)-piperazin-1-yl]-methanone
- 8-dimethylsulphamoyl-1-oxa-2,8-diaza-spiro[4.5]dec-2-ene-3-carboxylic acid 2,4-difluoro-benzyl amide
- 8-phenylmethanesulphonyl-1-oxa-2,8-diaza-spiro[4.5]dec-2-ene-3-carboxylic acid [2-(3-fluoro-phenyl)-ethyl] amide
- 8-(5-chloro-thiophene-2-sulphonyl)-1-oxa-2,8-diaza-spiro[4.5]dec-2-ene-3-carboxylic acid (2,2-diphenyl-ethyl) amide
- 3-[4-(3-phenyl-propyl)-piperazine-1-carbonyl]-1-oxa-2,8-diaza-spiro[4.5]dec-2-ene-8-sulphonic acid dimethylamide
- 3- {[8-(3,5-difluoro-benzoyl)-1-oxa-2,8-diaza-spiro[4.5]dec-2-ene-3-carbonyl]-amino}-propionic acid ethyl ester
- 8-(propane-1-sulphonyl)-1-oxa-2,8-diaza-spiro[4.5]dec-2-ene-3-carboxylic acid isobutyl amide
- [4-(3-phenyl-propyl)-piperazin-1-yl]-[8-(3-trifluoromethyl-benzenesulphonyl) 1-oxa-2,8-diaza-spiro[4.5]dec-2-en-3-yl]-methanone
- 8-(thiophene-2-sulphonyl)-1-oxa-2,8-diaza-spiro[4.5]dec-2-ene-3-carboxylic acid [1-(2,3-dihydro-benzo[1,4] dioxin-5-yl)-ethyl] amide
- 8-butyryl-1-oxa-2,8-diaza-spiro[4.5]dec-2-ene-3-carboxylic acid phenylamide
- 8-(4-trifluoromethoxy-benzenesulphonyl)-1-oxa-2,8-diaza-spiro[4.5]dec-2-ene-3-carboxylic acid cyclooctylamide
- 8-[2-(2-methoxy-ethoxy)-acetyl]-1-oxa-2,8-diaza-spiro[4.5]dec-2-ene-3-carboxylic acid benzyl amide
- 8-(4-bromo-3-methyl-benzoyl)-1-oxa-2,8-diaza-spiro[4.5]dec-2-ene-3-carboxylic acid isobutyl amide
- 8-(5-chloro-thiophene-2-sulphonyl)-1-oxa-2,8-diaza-spiro[4.5]dec-2-ene-3-carboxylic acid [3-(methyl-phenyl-amino)-propyl] amide
- 4-{[-8-(2-methyl-5-nitro-benzenesulphonyl)-1-oxa-2,8-diaza-spiro[4.5]dec-2-ene-3-carbonyl]-amino}-piperidine-1-carboxylic acid ethylester
- [8-(5-chloro-thiophene-2-sulphonyl)-1-oxa-2,8-diaza-spiro[4.5]dec-2-ene-3-yl]-[4-(4-fluoro-phenyl)-piperazin-1-yl]-methanone

- 8-(2,5-dichloro-benzenesulphonyl)-1-oxa-2,8-diaza-spiro[4.5]dec-2-ene-3-carboxylic acid methyl-pyridin-3-ylmethyl amide
- 2-(3,4-difluoro-phenyl)-1-{4-[8-(3-trifluoromethyl-benzenesulphonyl)-1-oxa-2,8-diaza-spiro[4.5]dec-2-ene-3-carbonyl]-piperazin-1-yl}-ethanone
- 8-(3 -trifluoromethyl-benzenesulphonyl)-1-oxa-2,8-diaza-spiro[4.5]dec-2-ene-3-carboxylic acid cyclopropylmethyl amide
- 3- {[8-(2-methyl-5-nitro-benzenesulphonyl)-1-oxa-2,8-diaza-spiro[4.5]dec-2-ene-3-carbonyl]-amino}-propionic acid ethylester
- 8-[2-(3-chloro-phenoxy)-acetyl]-1-oxa-2,8-diaza-spiro[4.5]dec-2-ene-3-carboxylic acid (thiophen-2-ylmethyl) amide
- 8-(4-trifluoromethoxy-benzenesulphonyl)-1-oxa-2,8-diaza-spiro[4.5]dec-2-ene-3-carboxylic acid benzyl-phenethyl amide
- 8-(2,5-dichloro-benzenesulphonyl)-1-oxa-2,8-diaza-spiro[4.5]dec-2-ene-3-carboxylic acid benzyl-phenethyl amide
- 8-(2,5-dichloro-benzenesulphonyl)-1-oxa-2,8-diaza-spiro[4.5]dec-2-ene-3-carboxylic acid (2-cyano-ethyl)-pyridin-3-ylmethyl amide
- 8-(2,5-dichloro-benzenesulphonyl)-1-oxa-2,8-diaza-spiro[4.5]dec-2-ene-3-carboxylic acid (2-cyano-ethyl) -(tetrahydrofuran-2-ylmethyl) amide
- 8-(3-trifluoromethyl-benzenesulphonyl)-1-oxa-2,8-diaza-spiro[4.5]dec-2-ene-3-carboxylic acid methyl-pyridin-3-ylmethyl amide
- 8-(pyridine-4-carbonyl)-1-oxa-2,8-diaza-spiro[4.5]dec-2-ene-3-carboxylic acid benzyl amide
- 8-(4-trifluoromethoxy-benzenesulphonyl)-1-oxa-2,8-diaza-spiro[4.5]dec-2-ene-3-carboxylic acid ethyl-(2-methyl-allyl) amide
- 8-(4-methoxy-benzenesulphonyl)-1-oxa-2,8-diaza-spiro[4.5]dec-2-ene-3-carboxylic acid [2-(2-fluoro-phenyl)-ethyl] amide
- 8-(5-tert-butyl-2-methyl-furan-3-carbonyl)-1-oxa-2,8-diaza-spiro[4.5]dec-2-ene-3-carboxylic acid isobutyl ainide
- 8-(3-phenyl-acryloyl)-1-oxa-2,8-diaza-spiro[4.5]dec-2-ene-3-carboxylic acid (thiophen-2-ylmethyl) amide
- 8-(4-trifluoromethyl-benzoyl)-1-oxa-2,8-diaza-spiro[4.5]dec-2-ene-3-carboxylic acid cyclopropylmethyl amide
- 8-(4-trifluoromethylsulphanyl-benzoyl)-1-oxa-2,8-diaza-spiro[4.5]dec-2-ene-3-carboxylic acid cyclopropylmethyl amide
- 8-(4-bromo-benzoyl)-1-oxa-2,8-diaza-spiro[4.5]dec-2-ene-3-carboxylic acid (5-methyl-furan-2-ylmethyl) amide
- 8-(3-fluoro-4-trifluoromethyl-benzoyl)-1-oxa-2,8-diaza-spiro[4.5]dec-2-ene-3-carboxylic acid cyclopropylmethyl amide
- 8-(7,7-dimethyl-2-oxo-bicyclo[2.2.1]hept-1-ylmethanesulphonyl)-1-oxa-2,8-diaza-spiro[4.5]dec-2-ene-3-carboxylic acid (2-ethylsulphanyl-ethyl) amide
- 8-(2,4-difluoro-benzoyl)-1-oxa-2,8-diaza-spiro[4.5]dec-2-ene-3-carboxylic acid (tetrahydrofuran-2-ylmethyl) amide
- {[8-(3-chloro-thiophene-2-carbonyl)-1-oxa-2,8-diaza-spiro[4.5]dec-2-ene-3-carbonyl]-amino}-acetic acid ethylester
- 4-oxo-4-{3-[(thiophen-2-ylmethyl)-carbamoyl]-1-oxa-2,8-diaza-spiro[4.5]dec-2-ene-8-yl}-butyric acid methylester
- 8-(2-ethylsulphanyl-pyridine-3-carbonyl)-1-oxa-2,8-diaza-spiro[4.5]dec-2-ene-3-carboxylic acid benzyl amide
- 3-{[8-(2-chloro-benzenesulphonyl)-1-oxa-2,8-diaza-spiro[4.5]dec-2-ene-3-carbonyl]-amino}-propionic acid ethylester
- 8-(4-trifluoromethoxy-benzoyl)-1-oxa-2,8-diaza-spiro[4.5]dec-2-ene-3-carboxylic acid cyclopropylmethyl amide
- 8-(4-phenoxy-butyryl)-1-oxa-2,8-diaza-spiro[4.5]dec-2-ene-3-carboxylic acid cyclopropylmethyl amide
- [8-(3-trifluoromethyl-benzenesulphonyl)-1-oxa-2,8-diaza-spiro[4.5]dec-2-en-3-yl]-[4-(4-trifluoromethyl-pyridin-2-yl)-piperazin-1-yl]-methanone
- 8-(2-chloro-5-trifluoromethyl-benzoyl)-1-oxa-2,8-diaza-spiro[4.5]dec-2-ene-3-carboxylic acid cyclopropylmethyl amide
- 8-(4-methoxy-benzenesulphonyl)-1-oxa-2,8-diaza-spiro[4.5]dec-2-ene-3-carboxylic acid (2-benzyloxy-cyclopentyl) amide
- 4-{[8-(3-trifluoromethyl-benzenesulphonyl)-1-oxa-2,8-diaza-spiro[4.5]dec-2-ene-3-carbonyl]-amino}-piperidine-1-carboxylic acid ethylester

- 8-(2-phenoxy-propionyl)-1-oxa-2,8-diaza-spiro[4.5]dec-2-ene-3-carboxylic acid cyclopropylmethyl amide
- 8-(4-fluoro-benzenesulphonyl)-1-oxa-2,8-diaza-spiro[4.5]dec-2-ene-3-carboxylic acid [2-(3-methoxy-phenyl)-ethyl] amide
- 8-(2-methylsulphanyl-pyridine-3-carbonyl)-1-oxa-2,8-diaza-spiro[4.5]dec-2-ene-3-carboxylic acid isobutyl amide
- 8-(5-tert-butyl-2-methyl-furan-3-carbonyl)-1-oxa-2,8-diaza-spiro[4.5]dec-2-ene-3-carboxylic acid cyclopentyl amide
- 8-(4-chloro-benzenesulphonyl)-1-oxa-2,8-diaza-spiro[4.5]dec-2-ene-3-carboxylic acid (thiophen-2-ylmethyl) amide
- 8-(5-chloro-thiophene-2-sulphonyl)-1-oxa-2,8-diaza-spiro[4.5]dec-2-ene-3-carboxylic acid (naphthalen-1-yl-methyl) amide
- 8-(4-trifluoromethoxy-benzenesulphonyl)-1-oxa-2,8-diaza-spiro[4.5]dec-2-ene-3-carboxylic acid [2-(4-fluoro-phenyl)-ethyl] amide
- 8-(4-bromo-3-methyl-benzoyl)-1-oxa-2,8-diaza-spiro[4.5]dec-2-ene-3-carboxylic acid (pyridin-4-ylmethyl) amide
- 8-(4-methoxy-benzoyl)-1-oxa-2,8-diaza-spiro[4.5]dec-2-ene-3-carboxylic acid benzyl amide
- 8-(3-trifluoromethyl-benzenesulphonyl)-1-oxa-2,8-diaza-spiro[4.5]dec-2-ene-3-carboxylic acid 3-chloro-benzyl amide
- (8-phenylmethanesulphonyl-1-oxa-2,8-diaza-spiro[4.5]dec-2-en-3-yl)-(4-o-tolyl-piperazin-1-yl)-methanone
- (8-phenylmethanesulphonyl-1-oxa-2,8-diaza-spiro[4.5]dec-2-en-3-yl)-(4-pyridin-2-yl-piperazin-1-yl)-methanone
- 8-(4-trifluoromethylsulphanyl-benzoyl)-1-oxa-2,8-diaza-spiro[4.5]dec-2-ene-3-carboxylic acid (thiophen-2-yl-methyl) amide
- 8-(thiophene-2-sulphonyl)-1-oxa-2,8-diaza-spiro[4.5]dec-2-ene-3-carboxylic acid benzyl amide
- 8-(4-fluoro-benzoyl)-1-oxa-2,8-diaza-spiro[4.5]dec-2-ene-3-carboxylic acid isobutyl amide
- 2-(3-isobutylcarbamoyl-1-oxa-2,8-diaza-spiro[4.5]dec-2-ene-8-sulphonyl) benzoic acid methylester
- 2-[(8-dimethylsulphamoyl-1-oxa-2,8-diaza-spiro[4.5]dec-2-ene-3-carbonyl) amino]-3-phenyl-propionic acid tert-butylester
- 8-(5-chloro-thiophene-2-sulphonyl)-1-oxa-2,8-diaza-spiro[4.5]dec-2-ene-3-carboxylic acid [1-(2,3-dihydro-benzo[1,4]dioxin-5-yl)-ethyl] amide
- 8-(4-bromo-benzoyl)-1-oxa-2,8-diaza-spiro[4.5]dec-2-ene-3-carboxylic acid (thiophen-2-ylmethyl) amide
- 8-(4-fluoro-benzenesulphonyl)-1-oxa-2,8-diaza-spiro[4.5]dec-2-ene-3-carboxylic acid (3-imidazol-1-yl-propyl) amide
- 4-[8-(4-fluoro-benzenesulphonyl)-1-oxa-2,8-diaza-spiro[4.5]dec-2-ene-3-carbonyl]-piperazine-1-carboxylic acid benzylester
- 3-(4-cycloheptyl-piperazine-1-carbonyl)-1-oxa-2,8-diaza-spiro[4.5]dec-2-ene-8-sulphonic acid dimethylamide
- 4-methyl-2-{[8-(thiophene-2-sulphonyl)-1-oxa-2,8-diaza-spiro[4.5]dec-2-ene-3-carbonyl]-amino}-pentanoic acid tert-butylester
- 8-(3-chloro-2-fluoro-benzoyl)-1-oxa-2,8-diaza-spiro[4.5]dec-2-ene-3-carboxylic acid (thiophen-2-ylmethyl) amide
- 2-{[8-(4-methoxy-benzenesulphonyl)-1-oxa-2,8-diaza-spiro[4.5]dec-2-ene-3-carbonyl]-amino}-4-methyl-pentanoic acid tert-butylester
- 3-{[8-(6-chloro-2-fluoro-3-methyl-benzoyl)-1-oxa-2,8-diaza-spiro[4.5]dec-2-ene-3-carbonyl]-amino}-propionic acid ethylester
- 8-(2-phenoxy-propionyl)-1-oxa-2,8-diaza-spiro[4.5]dec-2-ene-3-carboxylic acid benzyl amide
- 8-(5-fluoro-2-methyl-benzenesulphonyl)-1-oxa-2,8-diaza-spiro[4.5]dec-2- ene-3-carboxylic acid (thiophen-2-ylmethyl) amide
- [8-(5-chloro-thiophene-2-sulphonyl)-1-oxa-2,8-diaza-spiro[4.5]dec-2-en-3-yl]-[4-(2-methoxy-phenyl)-piperidin-1-yl]-methanone
- 8-(2-chloro-benzoyl)-1-oxa-2,8-diaza-spiro[4.5]dec-2-ene-3-carboxylic acid (thiophen-2-ylmethyl) amide
- 8-(2-phenoxy-pyridine-3-carbonyl)-1-oxa-2,8-diaza-spiro[4.5]dec-2-ene-3-carboxylic acid cyclopentylamide
- 8-(4-bromo-benzoyl)-1-oxa-2,8-diaza-spiro[4.5]dec-2-ene-3-carboxylic acid (2-ethylsulphanyl-ethyl) amide
- 1-[8-(5-chloro-thiophene-2-sulphonyl)-1-oxa-2,8-diaza-spiro[4.5]dec-2-ene-3-carbonyl]-piperidin-2-carboxylic acid ethylester
- 8-(2-chloro-benzenesulphonyl)-1-oxa-2,8-diaza-spiro[4.5]dec-2-ene-3-carboxylic acid (thiophen-2-ylmethyl) amide
- 8-(2,3-dichloro-benzoyl)-1-oxa-2,8-diaza-spiro[4.5]dec-2-ene-3-carboxylic acid cyclopropylmethyl amide

- 8-hexanoyl-1-oxa-2,8-diaza-spiro[4.5]dec-2-ene-3-carboxylic acid cyclopropylmethyl amide
- 8-(2,3-difluoro-4-methyl-benzoyl)-1-oxa-2,8-diaza-spiro[4.5]dec-2-ene-3-carboxylic acid cyclopropylmethyl amide
- 8-(4-fluoro-benzenesulphonyl)-1-oxa-2,8-diaza-spiro[4.5]dec-2-ene-3-carboxylic acid (2,5-dimethoxy-benzyl)-furan-2-ylmethyl amide
- 8-dimethylsulphamoyl-1-oxa-2,8-diaza-spiro[4.5]dec-2-ene-3-carboxylic acid (2-ethylsulphanyl-ethyl) amide
- 8-(4-trifluoromethoxy-benzenesulphonyl)-1-oxa-2,8-diaza-spiro[4.5]dec-2-ene-3-carboxylic acid 3-methoxy-benzyl amide
- 8-dimethylsulphamoyl-1-oxa-2,8-diaza-spiro[4.5]dec-2-ene-3-carboxylic acid (2-benzyloxy-cyclopentyl) amide
- 8-(4-methoxy-benzenesulphonyl)-1-oxa-2,8-diaza-spiro[4.5]dec-2-ene-3-carboxylic acid [2-(4-chloro-phenyl)-1-methyl-ethyl] amide
- 8-(4-trifluoromethoxy-benzenesulphonyl)-1-oxa-2,8-diaza-spiro[4.5]dec-2-ene-3-carboxylic acid [2-(3,4-dichloro-phenyl)-ethyl] amide
- 8-[2-(3-chloro-phenoxy)-acetyl]-1-oxa-2,8-diaza-spiro[4.5]dec-2-ene-3-carboxylic acid benzyl amide
- 8-(4-fluoro-benzenesulphonyl)-1-oxa-2,8-diaza-spiro[4.5]dec-2-ene-3-carboxylic acid (2-cyano-ethyl)-(2-pyridin-2-yl-ethyl) amide
- 8-(5-chloro-thiophene-2-sulphonyl)-1-oxa-2,8-diaza-spiro[4.5]dec-2-ene-3-carboxylic acid [2-(4-chloro-phenyl)-1-methyl-ethyl] amide
- 8-(4-methoxy-benzenesulphonyl)-1-oxa-2,8-diaza-spiro[4.5]dec-2-ene-3-carboxylic acid (2-benzyloxy-cyclopentyl) amide
- 8-(2,6-difluoro-3-methyl-benzoyl)-1-oxa-2,8-diaza-spiro[4.5]dec-2-ene-3-carboxylic acid (thiophen-2-ylmethyl) amide
- 8-(2-benzyloxy-acetyl)-1-oxa-2,8-diaza-spiro[4.5]dec-2-ene-3-carboxylic acid benzyl amide
- 8-(2,3-dichloro-benzoyl)-1-oxa-2,8-diaza-spiro[4.5]dec-2-ene-3-carboxylic acid phenylamide
- 8-(5-bromo-2-methoxy-benzenesulphonyl)-1-oxa-2,8-diaza-spiro[4.5]dec-2-ene-3-carboxylic acid cyclopentylamide
- acetic acid 2-(3-benzylcarbamoyl-1-oxa-2,8-diaza-spiro[4.5]dec-2-en-8-yl)-1,1-dimethyl-2-oxo-ethylester
- 8-[3-(2-chloro-6-fluoro-phenyl)-5-methyl-isoxazole-4-carbonyl]-1-oxa-2,8-diaza-spiro[4.5]dec-2-ene-3-carboxylic acid (2-ethylsulphanyl-ethyl) amide
- [4-(3-phenyl-propyl)-piperazin-1-yl]-[8-(4-trifluoromethoxy-benzenesulphonyl)-1-oxa-2,8-diaza-spiro[4.5]dec-2-en-3-yl]-methanone
- 8-[3-(2-chloro-6-fluoro-phenyl)-5-methyl-isoxazole-4-carbonyl]-1-oxa-2,8-diaza-spiro[4.5]dec-2-ene-3-carboxylic acid benzyl amide
- 8-(naphthalene carbonyl)-1-oxa-2,8-diaza-spiro[4.5]dec-2-ene-3-carboxylic acid isobutyl amide
- 1-[8-(5-tert-butyl-2-methyl-furan-3-carbonyl)-1-oxa-2,8-diaza-spiro[4.5]dec-2-ene-3-carbonyl]-piperidin-2-carboxylic acid ethylester
- 8-(3-difluoromethylsulphanyl-benzoyl)-1-oxa-2,8-diaza-spiro[4.5]dec-2-ene-3-carboxylic acid benzyl amide
- 8-(4-bromo-benzoyl)-1-oxa-2,8-diaza-spiro[4.5]dec-2-ene-3-carboxylic acid phenylamide
- 8-(4-methoxy-benzenesulphonyl)-1-oxa-2,8-diaza-spiro[4.5]dec-2-ene-3-carboxylic acid (2-phenyl-cyclopropyl) amide
- 8-(4-phenoxy-butyryl)-1-oxa-2,8-diaza-spiro[4.5]dec-2-ene-3-carboxylic acid (thiophen-2-ylmethyl) amide
- 8-(3-chloro-4-fluoro-benzoyl)-1-oxa-2,8-diaza-spiro[4.5]dec-2-ene-3-carboxylic acid (thiophen-2-ylmethyl) amide
- 8-(5-tert-butyl-2-methyl-2H-pyrazole-3-carbonyl)-1-oxa-2,8-diaza-spiro[4.5]dec-2-ene-3-carboxylic acid (2-ethylsulphanyl-ethyl) amide
- [8-(4-fluoro-benzenesulphonyl)-1-oxa-2,8-diaza-spiro[4.5]dec-2-en-3-yl]-[4(3-phenyl-propyl)-piperazin-1-yl]-methanone
- 8-(4-trifluoromethoxy-benzoyl)-1-oxa-2,8-diaza-spiro[4.5]dec-2-ene-3-carboxylic acid (pyridin-4-ylmethyl) amide
- benzoic acid 2-(3-cyclopentylcarbamoyl-1-oxa-2,8-diaza-spiro[4.5]dec-2-ene-8-carbonyl)-benzyl ester
- 3-[4-(4-tert-butyl-benzyl)-piperazine-1-carbonyl]-1-oxa-2,8-diaza-spiro[4.5]dec-2-ene-8-sulphonic acid dimethylamide
- {[8-(2-ethoxy-benzoyl)-1-oxa-2,8-diaza-spiro[4.5]dec-2-ene-3-carbonyl]- amino}-acetic acid ethylester
- 8-(5-chloro-thiophene-2-sulphonyl)-1-oxa-2,8-diaza-spiro[4.5]dec-2-ene-3-carboxylic acid (2-benzyloxy-cyclopentyl) amide
- 8-(naphthalene-1-carbonyl)-1-oxa-2,8-diaza-spiro[4.5]dec-2-ene-3-carboxylic acid (thiophen-2-ylmethyl) amide

- 8-(4-fluoro-benzenesulphonyl)-1-oxa-2,8-diaza-spiro[4.5]dec-2-ene-3-carboxylic acid (3-phenyl-propyl) amide
- 8-(5-chloro-thiophene-2-sulphonyl)-1-oxa-2,8-diaza-spiro[4.5]dec-2-ene-3-carboxylic acid (2,3-dihydro-benzo[1,4]dioxin-2-ylmethyl) amide
- 1-[8-(4-tert-butyl-benzoyl)-1-oxa-2,8-diaza-spiro[4.5]dec-2-ene-3-carbonyl]-piperidine-2-carboxylic acid ethyl ester
- 8-(4-fluoro-benzenesulphonyl)-1-oxa-2,8-diaza-spiro[4.5]dec-2-ene-3-carboxylic acid [2-(3-trifluoromethyl-phenyl)-ethyl] amide
- 8-[3-(2,6-dichloro-phenyl)-5-methyl-isoxazole-4-carbonyl]-1-oxa-2,8-diaza-spiro[4.5]dec-2-ene-3-carboxylic acid (thiophen-2-ylmethyl) amide
- 8-(4-fluoro-benzenesulphonyl)-1-oxa-2,8-diaza-spiro[4.5]dec-2-ene-3-carboxylic acid [1-(naphthalen-2-ylcarbamoyl)-ethyl] amide
- 8-(4-fluoro-benzenesulphonyl)-1-oxa-2,8-diaza-spiro[4.5]dec-2-ene-3-carboxylic acid (2-benzyloxy-cyclopentyl) amide
- 3-(4-chloro-phenyl)-2-{[8-(4-methoxy-benzenesulphonyl)-1-oxa-2,8-diaza-spirb[4.5]dec-2-ene-3-carbonyl]-amino}-propionic acid methyl ester
- 8-(3-trifluoromethyl-benzenesulphonyl)-1-oxa-2,8-diaza-spiro[4.5]dec-2-ene-3-carboxylic acid (2-benzyloxy-cyclopentyl) amide
- 8-(3-trifluoromethyl-benzenesulphonyl)-1-oxa-2,8-diaza-spiro[4.5]dec-2-ene-3-carboxylic acid (2-phenoxy-ethyl) amide
- 8-(2-phenoxy-pyridine-3-carbonyl)-1-oxa-2,8-diaza-spiro[4.5]dec-2-ene-3-carboxylic acid benzyl amide
- 8-(naphthalene-2-carbonyl)-1-oxa-2,8-diaza-spiro[4.5]dec-2-ene-3-carboxylic acid cyclopropylmethyl amide
- 8-(3-chloro-4-fluoro-benzoyl)-1-oxa-2,8-diaza-spiro[4.5]dec-2-ene-3-carboxylic acid benzyl amide
- 8-(2-phenyl-butyryl)-1-oxa-2,8-diaza-spiro[4.5]dec-2-ene-3-carboxylic acid (2-cyano-ethyl) amide
- 8-(4-fluoro-benzenesulphonyl)-1-oxa-2,8-diaza-spiro[4.5]dec-2-ene-3-carboxylic acid 2-fluoro-benzyl amide
- 8-(3-chloro-benzoyl)-1-oxa-2,8-diaza-spiro[4.5]dec-2-ene-3-carboxylic acid (thiophen-2-ylmethyl) amide
- (4-benzyl-piperazin-1-yl)-[8-(2,5-dichloro-benzenesulphonyl)-1-oxa-2,8-diaza-spiro[4.5]dec-2-en-3-yl]-methanone
- 8-(3-chloro-benzenesulphonyl)-1-oxa-2,8-diaza-spiro[4.5]dec-2-ene-3-carboxylic acid benzyl amide
- 4-benzyl-piperidin-1-yl)-[8-(3-trifluoromethyl-benzenesulphonyl)-1-oxa-2,8-diaza-spiro[4.5]dec-2-en-3-yl]-methanone
- 8-(4-bromo-benzoyl)-1-oxa-2,8-diaza-spiro[4.5]dec-2-ene-3-carboxylic acid benzyl amide
- 8-(4-fluoro-benzenesulphonyl)-1-oxa-2,8-diaza-spiro[4.5]dec-2-ene-3-carboxylic acid [1-(4-nitro-phenyl)-ethyl] amide
- 4-methyl-2-{[8-(3-trifluoromethyl-benzenesulphonyl)-1-oxa-2,8-diaza-spiro[4.5]dec-2-ene-3-carbonyl]-amino)-pentanoic acid tert.-butyl ester
- [4-(4-fluoro-phenyl)-3,6-dihydro-2H-pyridin-1-yl]-(8-phenylmethanesulphonyl-1-oxa-2,8-diaza-spiro[4.5]dec-2-en-3-yl) methanone
- 8-dimethylsulphamoyl-1-oxa-2,8-diaza-spiro[4.5]dec-2-ene-3-carboxylic acid [2-(ethyl-m-tolyl-amino)-ethyl] amide
- 8-(2,5-dimethyl-furan-3-carbonyl)-1-oxa-2,8-diaza-spiro[4.5]dec-2-ene-3-carboxylic acid benzyl amide
- (4-cycloheptyl-piperazin-1-yl)-[8-(4-fluoro-benzenesulphonyl)-1-oxa-2,8-diaza-spiro[4.5]dec-2-en-3-yl]-methanone
- 8-(2-benzyloxy-acetyl)-1-oxa-2,8-diaza-spiro[4.5]dec-2-ene-3-carboxylic acid benzyl amide
- R,R-8-(5-chloro-thiophene-2-sulphonyl)-1-oxa-2,8-diaza-spiro[4.5]dec-2-ene-3-carboxylic acid (2-benzyloxy-cyclopentyl) amide

and the hydrochlorides thereof.

**10.** Process for producing a substituted 1-oxa-2,8-diaza-spiro[4.5]dec-2-ene derivative of general formula (I)

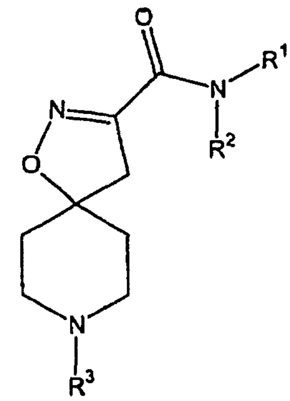

I

wherein $R^1$, $R^2$ and $R^3$ are as defined in any of claims 1 to 8,
**characterised in that**

(a) the compound of formula (II)

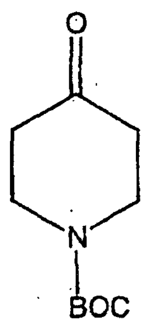

II

is reacted with a methylenation agent, preferably $Ph_3PCH_3Br$ in the presence of potassium-tert.-butylate in THF, to form compound (III)

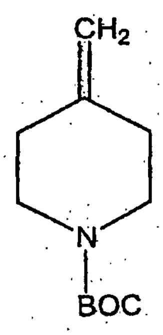

III

(b) compound (III) is subject to a reaction with ethyl chloroximidoacetate (IV)

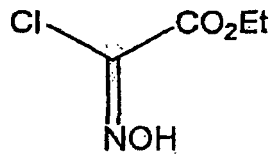

IV

in the presence of a base, preferably sodium hydrogen carbonate or lithium hydroxide, preferably in an organic solvent, in particular methanol, dichloromethane or THF, while forming the 1-oxa-2,8-diaza-spiro[4.5]dec-2-ene derivative of formula (V)

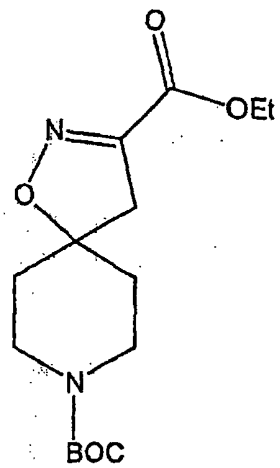

V

(c) compound (V) is reacted either directly or after previous saponification of the carboxylic acid ethyl ester function of compound (V) and optionally with activation of the carboxylic acid function thus formed with an amine of formula $HNR^1R^2$, wherein $R^1$ and $R^2$ are as defined in any of claims 1 to 8, to form the 1-oxa-2,8-diaza-spiro[4.5]dec-2-ene derivative of formula (VI)

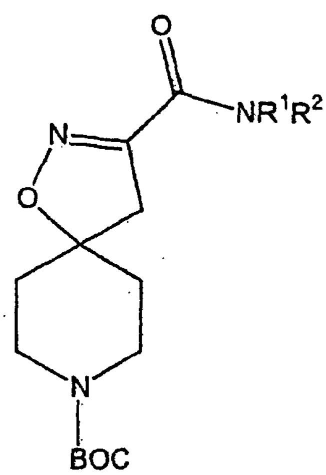

VI

(d) by removing the BOC protective group from compound (VI), compound (I) is obtained, where $R^3$ = H

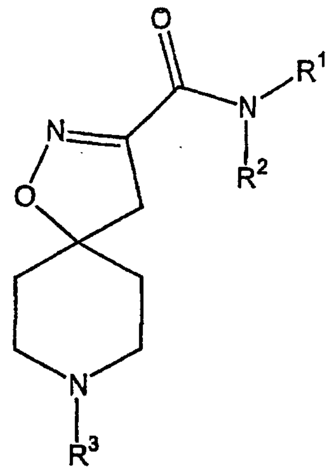

I

and

(e) optionally compound (I), where $R^3$ = H, is converted with an acid chloride of formula $R^{12}SO_2C1$ into a compound (I) where $R^3$ = $SO_2R^{12}$, wherein $R^{12}$ is as defined in any of claims 1 to 8, or is converted with a carboxylic acid chloride of formula $R^{13}COCl$ into a compound (I) where $R^3$ = $COR^{13}$, wherein $R^{13}$ is as defined in any of claims 1 to 8.

**11.** Pharmaceutical composition, containing at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates.

**12.** Use of at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates; for producing a pharmaceutical composition to treat pain, in particular neuropathic pain and/or chronic pain, and/or to treat and/or prevent migraines.

**13.** Use of at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates; for producing a pharmaceutical composition to treat and/or prevent urinary incontinence and/or irritation and/or Tinnitus aurium and/or diarrhoea.

**14.** Use of at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates; for producing a pharmaceutical composition for anaesthesia, in particular local anaesthesia, and/or to treat and/or prevent arrhythmia and/or emesis and/or cardiovascular diseases and/or cerebral ischemia and/or alcohol dependency and/or drug dependency and/or medication dependency and/or inflammations and/or vertigo

and/or as a nootropic (neurotropic) and/or a muscle relaxant.

**15.** Use of at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates; for producing a pharmaceutical composition to treat and/or prevent inflammatory and/or allergic reactions and/or gastritis and/or ulcers and/or depression and/or states of shock and/or narcolepsy and/or epilepsy and/or excess weight and/or asthma and/or glaucoma and/or hyperkinetic syndrome.

**16.** Use of at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates; for producing a pharmaceutical composition to treat and/or prevent inertia and/or bulimia and/or anorexia and/or catalepsy and/or for anxiolysis and/or to increase alertness and/or libido.

**17.** Use of at least one substituted 1-oxa-2,8-diaza-spiro[4.5]dec-2-ene derivative of general formula (I) according to claim 1 in the form of its racemates, its pure stereoisomers, in particular enantiomers or diastereomers, or in the form of blends of stereoisomers, in particular the enantiomers or diastereomers, in any mixing ratio; in the illustrated form or in the form of its acids or its bases or in the form of its salts, in particular the physiologically acceptable salts, more particularly preferably in the form of its hydrochlorides or in the form of its solvates, in particular the hydrates; for producing a pharmaceutical composition to treat and/or prevent bipolar disturbances and/or postmenopausal hot flushes and/or amyotropic lateral sclerosis (ALS) and/or reflex-sympathetic dystrophy (RSD) and/or spastic paralysis and/or restless leg syndrome and/or acquired nystagmus and/or multiple sclerosis and/or Parkinson's disease and/or Alzheimer's disease and/or Huntington's chorea.

## Revendications

**1.** Dérivé substitué de 1-oxa-2,8-diaza-spiro[4.5]déc-2-ène de formule générale (I)

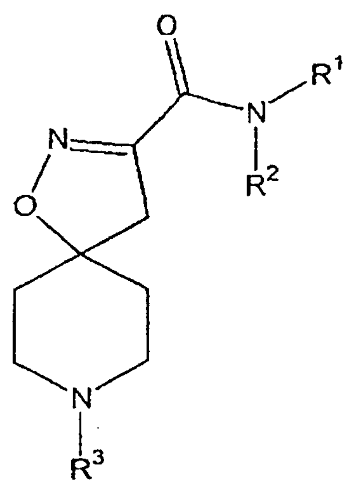

I

sous forme de ses racémates, de ses stéréoisomères purs, en particulier de ses énantiomères ou diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment

sous forme de ses chlorhydrates, ou sous forme de ses produits de solvatation, en particulier de ses hydrates ; formule dans laquelle

| | |
|---|---|
| $R^1$ et $R^2$ | représentent indépendamment l'un de l'autre H, un reste alkyle en $C_1$ à $C_{18}$, cycloalkyle en $C_3$ à $C_{10}$, (alkyle en $C_1$ à $C_{12}$)-(cycloalkyle en $C_3$ à $C_{10}$), aryle, (alkyle en $C_1$ à $C_{12}$)-aryle, hétérocyclyle, (alkyle en $C_1$ à $C_{12}$)-hétérocyclyle ou NH-C(=O)-aryle, l'un au moins des restes $R^1$ et $R^2$ ne représentant pas H, ou bien<br>forment ensemble un groupement $-(CR^4R^5)_m(CR^6R^7)_n$-Y- $(CR^8R^9)_p-(CR^{10}R^{11})_q-$, dans lequel m, n, p et q ont chacun la valeur 0, 1, 2, 3, 4 ou 5, sous réserve que la somme m + n soit supérieure ou égale à 1 et que la somme p + q soit supérieure ou égale à 1, ou bien un groupement $-CH_2-CH_2-C$(aryle)$=CH-CH_2-$ ; |
| $R^3$ | représente H, $SO_2R^{12}$ ou $COR^{13}$ ; |
| $R^4$, $R^5$, $R^6$, $R^7$, $R^8$, $R^9$, $R^{10}$ et $R^{11}$ | représentent indépendamment les uns des autres H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle, (alkyle en $C_1$ à $C_6$)-hétérocyclyle ou $C(=O)R^{14}$ ; |
| Y | représente $CR^{15}R^{16}$, $NR^{17}$ ou O ; |
| $R^{12}$ et $R^{13}$ | représentent indépendamment l'un de l'autre un reste alkyle en $C_1$ à $C_{10}$, cycloalkyle en $C_3$ à $C_{10}$, (alkyle en $C_1$ à $C_6$)-(cycloalkyle en $C_3$ à $C_{10}$), aryle, (alkyle en $C_1$ à $C_6$)-aryle, hétérocyclyle, (alkyle en $C_1$ à $C_6$)-hétérocyclyle ou $NR^{18}R^{19}$ ; |
| $R^{14}$ | représente H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle, (alkyle en $C_1$ à $C_6$)-hétérocyclyle ou $OR^{20}$ ; |
| $R^{15}$ et $R^{16}$ | représentent indépendamment l'un de l'autre H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle, (alkyle en $C_1$ à $C_6$)-hétérocyclyle ou $C(=O)R^{21}$ ; |
| $R^{17}$ | représente H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle, (alkyle en $C_1$ à $C_6$)-hétérocyclyle ou $C(=O)R^{22}$ ; |
| $R^{18}$ et $R^{19}$ | représentent, indépendamment l'un de l'autre, H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle ou (alkyle en $C_1$ à $C_6$)-hétérocyclyle ; |
| $R^{20}$ | représente H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle ou (alkyle en $C_1$ à $C_6$)-hétérocyclyle ; |
| $R^{21}$ | représente H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle, (alkyle en $C_1$ à $C_6$)-hétérocyclyle ou $OR^{23}$ ; |
| $R^{22}$ | représente H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle, (alkyle en $C_1$ à $C_6$)-hétérocyclyle ou $OR^{24}$ ; et |
| $R^{23}$ et $R^{24}$ | représentent, indépendamment l'un de l'autre, H, un reste alkyle en $C_1$ à $C_{10}$, cycloalkyle en $C_3$ à $C_8$, (alkyle en $C_1$ à $C_6$)-(cycloalkyle en $C_3$ à $C_8$), aryle, (alkyle en $C_1$ à $C_6$)-aryle, hétérocyclyle ou (alkyle en $C_1$ à $C_6$)-hétérocyclyle. |

**2.** Dérivé substitué de 1-oxa-2,8-diaza-spiro[4.5]déc-2-ène suivant la revendication 1, **caractérisé en ce que**

| | |
|---|---|
| $R^1$ et $R^2$ | représentent indépendamment l'un de l'autre H, un reste alkyle en $C_1$ à $C_8$, cycloalkyle en $C_3$ à $C_8$, (alkyle en $C_1$ à $C_4$)-(cycloalkyle en $C_3$ à $C_8$), aryle, (alkyle en $C_1$ à $C_4$)-aryle, hétérocyclyle, (alkyle en $C_1$ à $C_4$)-hétérocyclyle ou NH-C(=O)-aryle, l'un au moins des restes $R^1$ et $R^2$ ne représentant pas H, ou<br>forment conjointement un groupement $-(CR^4R^5)_m-(CR^6R^7)_n$-Y- $(CR^8R^9)_p-(CR^{10}R^{11})_q-$, avec m = 1, n = 0 ou 1, p = 1 ou 2 et q = 1 ou 2, ou un groupement |

| | |
|---|---|
| | $-CH_2-CH_2-C(aryle)=CH-CH_2-$ ; |
| $R^3$ | représente $SO_2R^{12}$ ou $COR^{13}$ ; |
| $R^4$, $R^5$, $R^6$, $R^7$, $R^8$, $R^9$, $R^{10}$ et $R^{11}$ | représentent, indépendamment les uns des autres, H, un reste alkyle en $C_1$ à $C_8$ ou $C(=O)R^{14}$; |
| Y | représente $CR^{15}R^{16}$ ou $NR^{17}$ ; |
| $R^{12}$ | représente un reste alkyle en $C_1$ à $C_4$, (alkyle en $C_1$ à $C_4$)-(cycloalkyle en $C_3$ à $C_8$), aryle, (alkyle en $C_1$ à $C_4$)-aryle, hétérocyclyle ou $NR^{18}R^{19}$ ; |
| $R^{13}$ | est un reste alkyle en $C_1$ à $C_6$, cycloalkyle en $C_3$ à $C_{10}$, aryle, (alkyle en $C_1$ à $C_4$)-aryle ou hétérocyclyle ; |
| $R^{14}$ | représente $OR^{20}$ ; |
| $R^{15}$ et $R^{16}$ | représentent indépendamment l'un de l'autre H, un reste aryle ou un reste (alkyle en $C_1$ à $C_4$)-aryle ; |
| $R^{17}$ | représente H, un reste cycloalkyle en $C_3$ à $C_8$, aryle, (alkyle en $C_1$ à $C_4$)-aryle, hétérocyclyle ou $C(=O)R^{22}$ ; |
| $R^{18}$ et $R^{19}$ | représentent, indépendamment l'un de l'autre, H ou un reste alkyle en $C_1$ à $C_6$ ; |
| $R^{20}$ | est un reste alkyle en $C_1$ à $C_6$ ; |
| $R^{22}$ | est un reste aryle, (alkyle en $C_1$ à $C_4$)-aryle, hétérocyclyle ou $OR^{24}$ ; et |
| $R^{24}$ | est un reste alkyle en $C_1$ à $C_6$ ou (alkyle en $C_1$ à $C_4$)-aryle. |

**3.** Dérivé substitué de 1-oxa-2,8-diaza-spiro[4.5]déc-2-ène suivant la revendication 1 ou 2, **caractérisé en ce que**

$R^1$ est un reste alkyle en $C_1$ à $C_6$, cycloalkyle en $C_3$ à $C_8$, (alkyle en $C_1$ à $C_3$)-(cycloalkyle en $C_3$ à $C_8$), aryle, (alkyle en $C_1$ à $C_3$)-aryle, hétérocyclyle, (alkyle en $C_1$ à $C_3$)-hétérocyclyle ou NH-C(=O)-aryle ; et

$R^2$ représente H, un reste alkyle en $C_1$ à $C_4$, (alkyle en $C_1$ à $C_3$)-aryle ou (alkyle en $C_1$ à $C_3$)-hétérocyclyle ;

**4.** Dérivé substitué de 1-oxa-2,8-diaza-spiro[4.5]déc-2-ène suivant la revendication 3, **caractérisé en ce que**

$R^1$ est un reste méthyle, éthyle, n-propyle, iso-propyle, 2-méthylpropyle, n-butyle, tertiobutyle, n-pentyle, 3-méthylbutyle ou $CH_2-C(CH_3)=CH_2$ non substitué ou substitué avec F, Cl, Br, I, un radical -CN, N-alkyl-N-arylamine, N,N-dialkylamine, amide, carboxyalkyle, carboxybenzyle, en particulier un reste méthyle, éthyle, $CH_2-C(CH_3)=CH_2$, $CH(C(=O)OCH_2CH=CH_2)-CH_2C(=O)O$-tertiobutyle, 2-cyanéthyle, $CH_2-CH_2-NH-C(=O)CH_3$, 2-(N-éthyl-N-(3-méthylphényl)amino)-éthyle, 2-(N,N-diméthylamino)-éthyle, 2-(C(=O)-NH-(β-naphtyl)-éthyle, 1,2-(di-(C(=O)O-tertiobutyl)éthyle, 3-(N-méthyl-N-phénylamino)-propyle, 1-(C(=O)O-benzyl)-3-méthyl-butyle, 1-(C(=O)O-butyl)-3-méthyl-butyle, $CH_2CO_2$éthyle, $CH_2-CH_2CO_2$éthyle, $CH_2-CH_2$-Ophényle, $CH_2-CH_2-S-CH_2-CH_3$ ;
un reste cyclopropyle, cyclobutyle, cyclopentyle, cyclohexyle, cycloheptyle, cyclooctyle, bicyclo-[3.1.1]heptane-3-yle non substitué ou substitué avec F, Cl, Br, I, un radical -CN, alkyle, aryle, carboxyalkyle, carboxybenzyle, O-alkyle, O-benzyle, en particulier un reste 2-phénylcyclopropyle, 2-(O-benzyl)-cyclopentyle, 2-(carboxyéthyl)cyclohexyle, 7,7-diméthyl-2-méthylbicyclo[3.1.1]heptane-3-yle, cyclopropylméthyle, 7,7-diméthyl-2-oxo-bicyclo-[2.2.1]hept-1-ylméthyle ;
un reste phényle, 1-naphtyle ou 2-naphtyle non substitué ou substitué avec un radical phénoxy, $-CH_2-P(=O)(Oéthyle)_2$ ;
un reste $CH_2$-aryle, $CH_2-CH_2$-aryle, $CH_2-CH_2-CH_2$-aryle, $CH_2-CH_2-CH_2-CH_2$-aryle, $CH(CH_3)$aryle, $CH(CH_3)-CH_2$-aryle, $CH_2-CH-(aryle)_2$, $CH(CO_2alkyle)-CH_2$-aryle, $CH_2-CH_2-CH-(aryle)_2$ où aryle désigne un radical phényle, 1-naphtyle ou 2-naphtyle non substitué ou un radical phényle à substituant F, Cl, Br, I, -CN, $-NO_2$, alkyle, $CF_3$, alkoxy, alkylènedioxy, en particulier un reste benzyle, $-CH_2$-napht-1-yle, 2-fluorobenzyle, 3-fluorobenzyle, 3-chlorobenzyle, 3-méthoxybenzyle, 2-éthoxybenzyle, 2,4-difluorobenzyle, 3,5-dichlorobenzyle, 3-fluoro-5-trifluorométhylbenzyle, 3-fluoro-4-trifluorométhylbenzyle, 2-chloro-6-fluorobenzyle, 2,5-diméthoxybenzyle, 2-chloro-6-méthyl-benzyle, 3,4-diméthoxybenzyle, 3,4-dioxyméthylènebenzyle, $CH(CH_3)$-phényle, $CH(CH_3)-(4-CH_3$-phényle), $CH(CH_3)$-(4-nitrophényle), $CH(CH_3)$-(2,3-dioxyéthylènephényle), $CH_2-CH_2$-phényle, $CH_2-CH_2$-(2-fluorophényle), $CH_2-CH_2$-(3-fluorophényle), $CH_2-CH_2$-(4-fluorophényle), $CH_2-CH_2$-(4-chlorophényle), $CH_2-CH_2$-(3,4-dichlorophényle), $CH_2-CH_2$-(3-méthoxyphényle), $CH_2-CH_2$-(2,5-diméthoxyphényle), $CH(CO_2$-tertiobutyl)-$CH_2$-phényle, $CH(CO_2$-méthyl)-$CH_2$-(4-chlorophényle), $CH_2-CH(phényle)_2$, CH $(CH_3)-CH_2$-(4-chlorophényle), $CH_2-CH_2CH(phényle)_2$, $CH_2-CH_2-CH_2$-phényle ;
un reste pyrrolidine ou pipéridine non substitué ou substitué avec un radical aryle, alkylaryle ou carboxyéthyle, en particulier un reste pyrrolidine-3-yle, N-(4-trifluorobenzyl)-pyrrolidine-3-yle, N-(3-méthoxybenzyl)-pyrrolidine-3-yle, N-($CH_2$-(β-naphtyl)-pyrrolidine-3-yle ou N-(carboxyéthyl)-pipéridine-4-yle ; un reste $(CH_2)_{1-3}$-hétérocyclyle non substitué ou substitué avec un radical alkyle, F, Cl, Br, I, -CN, aryle, alkylaryle, le reste hétérocyclyle étant représenté par un reste furannyle, benzofurannyle, 1,4-dioxannyle, benzo-1,4-dioxannyle, thiényle, pyridinyle, pyrrolidinyle, 1H-indolyle, imidazolyle, pipéridinyle, tétrahydrofurannyle, en particulier $CH_2$-furanne-2-yle, 5-méthylfuranne-2-yle, $CH_2$-benzofuranne-2-yle,

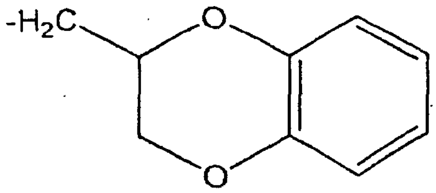
,

$CH_2$-thién-2-yle, $CH_2$-pyridine-3-yle, $CH_2$-pyridine-4-yle, $CH_2$-$CH_2$-pyridine-2-yle, $CH_2$-$CH_2$-(1H-indole-3-yle), $CH_2$-$CH_2$-pyrrolidine-1-yle, $CH_2$-(N-2,6-dichlorobenzyl-pyrrolidine-3-yle), $CH_2$-$CH_2$-(N-méthyl-pyrrolidine-2-yle), -$(CH_2)_3$-imidazole-1-yle, $CH_2$-(tétrahydrofuranne-2-yle) ou

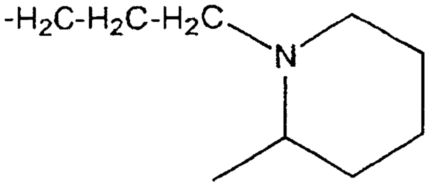
,

ou CH ($CO_2$méthyl)-$CH_2$-(1H-indole-3-yle) ;
NH-C(=O)-(4-diéthylaminophényle) ; et

$R^2$ représente H ;
un reste méthyle, éthyle ou $CH_2$-$C(CH_3)$=$CH_2$ non substitué ou substitué avec F, Cl, Br, I, -CN, en particulier un reste méthyle, éthyle, 2-cyanéthyle, $CH_2$-$C(CH_3)$=$CH_2$ ;
un reste benzyle ou phénéthyle non substitué ou substitué avec un radical F, Cl, Br, I, -CN, méthoxy, éthoxy, en particulier un reste benzyle, 4-fluorobenzyle, 2-chloro-6-fluorobenzyle, 2,5-diméthoxybenzyle, phénéthyle ; ou
un reste $CH_2$-furannyle, en particulier $CH_2$-furanne-2-yle, $CH_2$-benzofurannyle, en particulier $CH_2$-benzofuranne-2-yle, $CH_2$-pyridinyle, en particulier $CH_2$-pyridine-3-yle, $CH_2$-tétrahydrofurannyle, en particulier $CH_2$-tétrahydrofuranne-2-yle, $CH_2$-$CH_2$-pyridinyle, en particulier $CH_2$-$CH_2$-pyridine-2-yle.

**5.** Dérivé substitué de 1-oxa-2,8-diaza-spiro[4.5]déc-2-ène suivant la revendication 1 ou 2, **caractérisé en ce que**

$R^1$ et $R^2$ forment ensemble un groupe

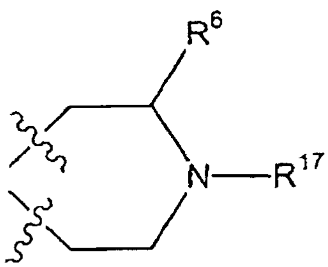
,

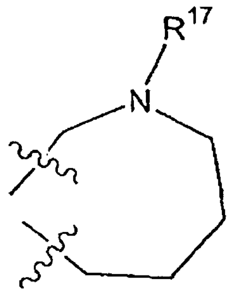,
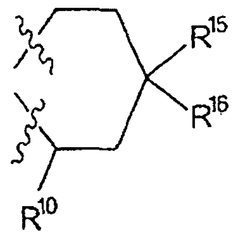
ou
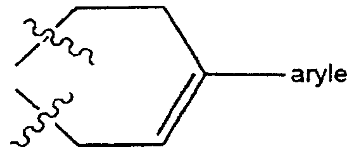,

dans lequel aryle désigne le reste phényle ou un reste phényle à substituant F, Cl, Br, I, en particulier 4-fluorophényle ;

$R^6$ représente H ou un reste alkyle en $C_1$ à $C_4$, en particulier méthyle ;

$R^{10}$ représente H, un reste C(=O)Ométhyle, C(=O)Oéthyle, C(=O)O-n-propyle, C(=O)O-iso-propyle, C (=O)O-n-butyle, C(=O)O-tertiobutyle ;

$R^{15}$ représente H ou un reste $CH_2$-aryle ;

$R^{16}$ représente H ;

$R^{17}$ représente H ;
un reste cycloalkyle en $C_3$ à $C_8$, en particulier cycloheptyle ;
un reste aryle, en particulier un reste phényle ou naphtyle non substitué ou substitué avec un radical méthyle, éthyle, n-propyle, iso-propyle, n-butyle, tertiobutyle, $CF_3$, F, Cl, Br, I, -CN, méthoxy, éthoxy, n-propoxy, iso-propoxy, n-butoxy, tertiobutoxy ;
un reste $((CH_2)_{1-3}$-alkyle) -aryle ou $CH(CH_3)$ -aryle, où aryle désigne un reste phényle ou naphtyle non substitué ou substitué avec un radical alkyle, $CF_3$, F, Cl, Br, I, -CN, alkoxy ;
un reste pyridinyle, pyrazinyle ou thiéno[2,3-d]pyrimidinyle non substitué ou substitué avec un radical alkyle, $CF_3$, F, Cl, Br, I, -CN, alkoxy ; ou un reste C(=O)$R^{22}$ ; et

$R^{22}$ est un reste phényle ou phényle à substituant alkoxy, un reste O-méthyle, O-éthyle, O-n-propyle, O-iso-propyle, O-n-butyle, O-tertiobutyle, O-benzyle, benzyle non substitué, benzyle substitué avec F, pyrazinyle non substitué ou pyrazinyle substitué avec un radical alkyle.

**6.** Dérivé substitué de 1-oxa-2,8-diaza-spiro[4.5]déc-2-ène suivant la revendication 5, **caractérisé en ce que**

$R^6$ représente H ou un reste méthyle ;

$R^{10}$ représente H ou un reste C(=O)Oéthyle ;

$R^{15}$ représente H ou un reste benzyle ;

$R^{16}$ représente H ; et

$R^{17}$ représente H ;
un reste cycloheptyle ;
un reste phényle, 2-méthylphényle, 3-méthylphényle, 2,4-diméthylphényle, 2-éthylphényle, 3-trifluorométhyle, 4-fluorophényle, 4-chlorophényle, 2-méthoxyphényle, 3,5-diméthoxyphényle, 3-chloro-6-méthylphényle ;
benzyle, $CH_2$-(4-tertiobutylphényle), $CH_2$-(β-naphtyle), $CH(CH_3)$-phényle, $(CH_2)_3$-phényle ;
pyridine-2-yle, (4-trifluorométhyl)-pyridine-2-yle, thiéno[2,3-d]pyrimidine-4-yle ; ou bien
C(=O)-(4-méthoxyphényle), C(=O)-benzyle, C(=O)-$CH_2$-(3,4-difluorophényle), C(=O)-(2-méthylpyrazine-5-yle), C(=O)O-tertiobutyle ou O-benzyle.

**7.** Dérivé substitué de 1-oxa-2,8-diaza-spiro[4.5]déc-2-ène suivant l'une quelconque des revendications 1 à 6, **caractérisé en ce que**

$R^3$ est un groupe $SO_2R^{12}$ ;

$R^{12}$ est un reste méthyle, éthyle, n-propyle, iso-propyle, en particulier n-propyle ;
un reste 7,7-diméthyl-2-oxo-bicyclo[2.2.1]hept-1-ylméthyle ;
un reste phényle ou un reste phényle substitué avec un radical méthyle, éthyle, n-propyle, iso-propyle, n-butyle, tertiobutyle, $CF_3$, F, Cl, Br, I, -CN, $NO_2$, méthoxy, éthoxy, n-propoxy, iso-propoxy, n-butoxy, tertiobutoxy, $OCF_3$, $CO_2$méthyle, en particulier un reste 4-méthylphényle, 3-trifluorométhylphényle, 4-fluorophényle, 2-chlorophényle, 3-chlorophényle, 4-chlorophényle, 4-méthoxyphényle,

4-trifluorométhoxyphényle, 4-nitrophényle, 2-$CO_2$méthyl-phényle, 2,5-dichlorophényle, 3-fluoro-6-méthylphényle, 3-bromo-6-méthoxyphényle, 2-méthyl-5-nitrophényle, 2,4,6-triméthylphényle ;
un reste benzyle ou benzyle substitué avec un radical méthyle, éthyle, n-propyle, iso-propyle, n-butyle, tertiobutyle, $CF_3$, F, Cl, Br, I, -CN, $NO_2$, méthoxy, éthoxy, n-propoxy, iso-propoxy, n-butoxy, tertiobutoxy, $OCF_3$ ;
un reste furannyle ou thiényle non substitué ou substitué avec un radical méthyle, éthyle, n-propyle, iso-propyle, n-butyle, tertiobutyle, $CF_3$, F, Cl, Br, I, -CN, $NO_2$, méthoxy, éthoxy, n-propoxy, iso-propoxy, n-butoxy, tertiobutoxy, en particulier un reste thién-2-yle, 5-chlorothién-2-yle ; ou $NR^{18}R^{19}$ ; et

$R^{18}$ et $R^{19}$ représentent, indépendamment l'un de l'autre, H, un radical méthyle ou éthyle.

8. Dérivé substitué de 1-oxa-2,8-diaza-spiro[4.5]déc-2-ène suivant l'une quelconque des revendications 1 à 6, **caractérisé en ce que**

$R^3$ est un groupe $COR^{13}$ ; et

$R^{13}$ est un reste méthyle, éthyle, C(=O)Ométhyle, n-propyle, iso-propyle, 2-méthylpropyle, n-butyle, tertiobutyle, n-pentyle ou 3-méthylbutyle non substitué ou substitué avec un radical O-méthyle, O-éthyle, O-$(CH_2)_2$-$OCH_3$, O-benzyle, O-phényle, le radical phényle étant non substitué ou substitué avec F, Cl, Br, I, -CN, en particulier un reste méthyle, éthyle, n-propyle, n-butyle, tertiobutyle, n-pentyle, $CH_2$-O-$CH_2$-$CH_2$-$OCH_3$, $CH(CH_3)$-O-phényle, $CH_2$-$CH_2$-C(=O)$OCH_3$, $C(CH_3)_2$-OC(=O)$CH_3$, $CH_2$-O-benzyle, $CH_2$-O-(3-chlorophényle), $CH_2$-$CH_2$-$CH_2$-O-phényle, CH(OC(=O)-méthyl)$CH_3$ ;
un reste cyclopropyle, 2-phénylcyclopropyle, 1-adamantyle ;
un reste phényle ou naphtyle non substitué ou substitué avec un radical F, Cl, Br, I, -CN, phényle, méthyle, éthyle, n-propyle, iso-propyle, n-butyle, tertiobutyle, $CF_3$, F, Cl, Br, I, -CN, $NO_2$, méthoxy, éthoxy, n-propoxy, iso-propoxy, n-butoxy, tertiobutoxy, $OCF_3$, $OCHF_2$, $OCH_2F$, $SCF_3$, $SCHF_2$, $SCH_2F$, $SCH_3$, $CH_2OC$(=O)phényle, -$N(CH_3)$, en particulier un reste 4-fluorophényle, 2-chlorophényle, 3-chlorophényle, 4-bromophényle, 4-phénylphényl(4-biphényle), 4-éthylphényle, 4-$CF_3$-phényle, 4-méthoxyphényle, 2-éthoxyphényle, 4-tertiobutyle, 3-$OCF_3$-phényle, 4-$OCF_3$-phényle, 4-$SCF_3$-phényle, 3-$SCF_2$-phényle, 2-$CH_2$-OC(=O)phényle, 3-diméthylaminophényle, 2,3-dichlorophényle, 2,4-difluorophényle, 3-chloro-4-fluorophényle, 3-chloro-2-fluorophényle, 4-$CF_3$-3-fluorophényle, 3-$CF_3$-6-fluorophényle, 4-bromo-3-méthylphényle, 2-chloro-4-nitrophényle, 2,3,4,5,6-pentafluorophényle, 2,6-difluoro-3-méthylphényle, 2,3-difluoro-4-méthylphényle, 2-chloro-5-méthyl-6-fluorophényle, 1-naphtyle, 2-naphtyle ;
un reste (alkyle en $C_1$ ou $C_2$)-aryle non substitué ou substitué, en particulier un reste benzyle, phénéthyle, $CH(C_2H_5)$-phényle, CH(NH-$SO_2$-(4-méthylphényl))-$CH_2$-phényle, CH=CH-phényle, CH=CH-(3-trifluorophényle) ; ou
un reste furannyle non substitué ou à substituant alkyle, un reste benzofurannyle, un reste thiényle, pyridinyle, pyrazolyle, benzodihydropyrannyle, isooxazolyle non substitué ou substitué avec un radical alkyle, $CF_3$, aryle, O-phényle, chloro, S-méthyle, S-éthyle, en particulier un reste 1,5-diméthylfuranne-3-yle, 2-méthyl-5-tertiobutyl-furanne-3-yle, 3-chlorothién-2-yle, 1-(4-chloro-phényl)-5-trifluorométhyl-pyrazole-4-yle, 1-méthyl-3-tertiobutyl-pyrazole-5-yle,

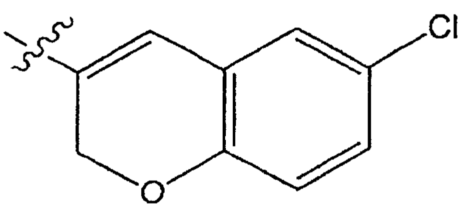

,

pyridine-4-yle, 2-méthylthiopyridine-3-yle, 2-éthylthiopyridine-3-yle, 2-phénoxypyridine-3-yle, 2-chloropyridine-3-yle, 5-méthyl-3-(2,6-dichloro-phényl)-isoxazole-4-yle, 5-méthyl-3-(2-chloro-6-fluorophényl)-isoxazole-4-yle.

9. Dérivé substitué de 1-oxa-2,8-diaza-spiro[4.5]déc-2-ène suivant la revendication 1, **caractérisé en ce qu'**il est choisi dans le groupe qui est constitué des composés :

- ester méthylique d'acide 3-(1H-indole-3-yl)-2-{[8-(4-trifluorométhoxy-benzènesulfonyl)-1-oxa-2,8-diaza-spiro[4.5]déc-2-ène-3-carbonyl]-amino}-propionique

- ester diéthylique d'acide {4-[(8-phénylméthanesulfonyl-1-oxa-2,8-diaza-spiro[4.5]déc-2-ène-3-carbonyl)-amino]-benzyl}-phosphonique
- (4-cycloheptyl-pipérazine-1-yl)-[8-(thiophène-2-sulfonyl)-1-oxa-2,8-diaza-spiro[4.5]déc-2-ène-3-yl]-méthanone
- [8-(5-chloro-thiophène-2-sulfonyl)-1-oxa-2,8-diaza-spiro[4.5]déc-2-ène-3-yl]-(4-phényl-pipérazine-1-yl)-méthanone
- cyclopentylamide d'acide 8-(2-chloro-4-nitro-benzoyl)-1-oxa-2,8-diaza-spiro[4.5]déc-2-ène-3-carboxylique
- (4-naphtalène-2-ylméthyl-pipérazine-1-yl)-(8-phényl-méthanesulfonyl-1-oxa-2,8-diaza-spiro[4.5]déc-2-ène-3-yl)-méthanone
- phénéthylamide d'acide 8-diméthylsulfamoyl-1-oxa-2,8-diaza-spiro[4.5]déc-2-ène-3-carboxylique
- (1-phényl-éthyl)-amide d'acide 8-(thiophène-2-sulfonyl)-1-oxa-2,8-diaza-spiro[4.5]déc-2-ène-3-carboxylique
- [2-(3,4-dichloro-phényl)-éthyl]-amide d'acide 8-(toluène-4-sulfonyl)-1-oxa-2,8-diaza-spiro[4.5]déc-2-ène-3-carboxylique
- N'-[8-(thiophène-2-sulfonyl)-1-oxa-2,8-diaza-spiro-[4.5]déc-2-ène-3-carbonyl]-hydrazide d'acide 4-diéthylamino-benzoïque
- (2-pyrrolidine-1-yl-éthyl)-amide d'acide 8-(3-chloro-4-fluoro-benzènesulfonyl)-1-oxa-2,8-diaza-spiro[4.5]-déc-2-ène-3-carboxylique
- phénéthyl-amide d'acide 8-phénylméthanesulfonyl-1-oxa-2,8-diaza-spiro[4.5]déc-2-ène-3-carboxylique
- (2-diméthylamino-éthyl)-amide d'acide 8-benzène-sulfonyl-1-oxa-2,8-diaza-spiro[4.5]déc-2-ène-3-carboxylique
- [4-(3-phényl-propyl)-pipérazine-1-yl]-[8-(thiophène-2-sulfonyl)-1-oxa-2,8-diaza-spiro[4.5]déc-2-ène-3-yl)-méthanone
- [3-(2-méthyl-pipéridine-1-yl)-propyl]-amide d'acide 8-(2,5-dichloro-benzoylsulfonyl-1-oxa-2,8-diaza-spiro[4.5]déc-2-ène-3-carboxylique
- [2-(1H-indole-3-yl)-éthyl]-méthyl-amide d'acide 8-(4-trifluorométhoxy-benzènesulfonyl)-1-oxa-2,8-diaza-spiro[4.5]déc-2-ène-3-carboxylique
- (2-acétylamino-éthyl)-amide d'acide 8-(5-fluoro-2-méthyl-benzènesulfonyl)-1-oxa-2,8-diaza-spiro[4.5]déc-2-ène-3-carboxylique
- benzyl-phénéthyl-amide d'acide 8-(toluène-4-sulfonyl)-1-oxa-2,8-diaza-spiro[4.5]déc-2-ène-3-carboxylique
- (2-diméthylamino-éthyl)-amide d'acide 8-(2-méthyl-5-nitro-benzènesulfonyl)-1-oxa-2,8-diaza-spiro[4.5]déc-2-ène-3-carboxylique
- (2-benzyloxy-cyclopentyl)-amide d'acide 8-phényl-méthanesulfonyl-1-oxa-2,8-diaza-spiro[4.5]déc-2-ène-3-carboxylique
- (2,3-dihydro-benzo[1,4]dioxine-2-ylméthyl)-amide d'acide 8-(thiophène-2-sulfonyl)-1-oxa-2,8-diaza-spiro[4.5]déc-2-ène-3-carboxylique
- (4-phénoxy-phényl)-amide d'acide 8-(toluène-4-sulfonyl)-1-oxa-2,8-diaza-spiro[4.5]déc-2-ène-3-carboxylique
- (1-p-tolyl-éthyl)-amide d'acide 8-(thiophène-2-sulfonyl)-1-oxa-2,8-diaza-spiro[4.5]déc-2-ène-3-carboxylique
- 3-fluoro-benzylamide d'acide 8-(thiophène-2-sulfonyl)-1-oxa-2,8-diaza-spiro[4.5]déc-2-ène-3-carboxylique
- 2-phényl-1-{4-[8-(toluène-4-sulfonyl)-1-oxa-2,8-diaza-spiro[4.5]déc-2-ène-3-carbonyl]-pipérazine-1-yl}-éthanone
- [1-(4-trifluorométhyl-benzyl)-pyrrolidine-3-yl]-amide d'acide 8-(thiophène-2-sulfonyl)-1-oxa-2,8-diaza-spiro[4.5]déc-2-ène-3-carboxylique
- [8-(thiophène-2-sulfonyl)-1-oxa-2,8-diaza-spiro[4.5]-déc-2-ène-3-yl]-(4-o-tolyl-pipérazine-1-yl)-méthanone
- benzyl-(2-cyano-éthyl)-amide d'acide 8-(toluène-4-sulfonyl)-1-oxa-2,8-diaza-spiro[4.5]déc-2-ène-3-carboxylique
- [8-(2,5-dichloro-benzènesulfonyl)-1-oxa-2,8-diaza-spiro[4.5]déc-2-ène-3-yl]-(4-thiéno[2,3-d]pyrimidine-4-yl-pipérazine-1-yl)-méthanone
- (benzo[1,3]dioxole-5-ylméthyl)-amide d'acide 8-(5-chloro-thiophène-2-sulfonyl)-1-oxa-2,8-diaza-spiro-[4.5]déc-2-ène-3-carboxylique
- (3-méthyl-4-m-tolyl-pipérazine-1-yl)-[8-(thiophène-2-sulfonyl)-1-oxa-2,8-diaza-spiro[4.5]déc-2-ène-3-yl]-méthanone
- [4-(1-phényl-éthyl)-pipérazine-1-yl]-[8-(toluène-4-sulfonyl)-1-oxa-2,8-diaza-spiro[4.5]déc-2-ène-3-yl]-méthanone
- [4-(2,4-diméthyl-phényl)-pipérazine-1-yl]-[8-(toluène-4-sulfonyl)-2-oxa-2,8-diaza-spiro[4.5]déc-2-ène-3-yl]-méthanone
- cyclopentylamide d'acide 8-(2-chloro-pyridine-3-carbonyl)-1-oxa-2,8-diaza-spiro[4.5]déc-2-ène-3-carboxylique

- (4-naphtalène-2-ylméthyl-pipérazine-1-yl)-[8-(3-trifluorométhyl-benzènesulfonyl)-1-oxa-2,8-diaza-spiro[4.5]déc-2-ène-3-yl]-méthanone
- (pyridine-3-ylméthyl)-amide d'acide 8-[3-phényl-2-(toluène-4-sulfonylamino)-propionyl]-1-oxa-2,8-diaza-spiro[4.5]déc-2-ène-3-carboxylique
- (2-diméthylamino-éthyl)-amide d'acide 8-(5-fluoro-2-méthyl-benzènesulfonyl)-1-oxa-2,8-diaza-spiro[4.5]déc-2-ène-3-carboxylique
- (thiophène-2-ylméthyl)-amide d'acide 8-(4-nitro-benzènesulfonyl)-1-oxa-2,8-diaza-spiro[4.5]déc-2-ène-3-carboxylique
- 3-fluoro-5-trifluorométïzyl-benzylamide d'acide 8-(2,5-dichloro-benzènesulfonyl)-1-oxa-2,8-diaza-spiro[4.5]-déc-2-ène-3-carboxylique
- benzhydryl-amide d'acide 8-(5-chloro-thiophène-2-sulfonyl)-1-oxa-2,8-diaza-spiro[4.5]déc-2-ène-3-carboxylique
- (2-cyano-éthyl)-amide d'acide 8-[3-phényl-2-(toluène-4-sulfonylamino)-propionyl]-1-oxa-2,8-diaza-spiro-[4.5]déc-2-ène-3-carboxylique
- ester éthylique d'acide [(8-phénylméthanesulfonyl-1-oxa-2,8-diaza-spiro[4.5]déc-2-ène-3-carbonyl)-amino]-acétique
- (2-cyano-éthyl)-amide d'acide 8-(3-diméthylamino-benzoyl)-1-oxa-2,8-diaza-spiro[4.5]déc-2-ène-3-carboxylique
- [1-(naphtalène-2-ylcarbamoyl)-éthyl]-amide d'acide 8-(2,5-dichloro-benzènesulfonyl)-1-oxa-2,8-diaza-spiro[4.5]déc-2-ène-3-carboxylique
- (1-p-tolyl-éthyl)-amide d'acide 8-(toluène-4-sulfonyl)-1-oxa-2,8-diaza-spiro[4.5]déc-2-ène-3-carboxylique
- [4-(4-chloro-phényl)-pipérazine-1-yl]-(8-phénylméthanesulfonyl-1-oxa-2,8-diaza-spiro[4.5]déc-2-ène-3-yl)-méthanone
- (2-benzyloxy-cyclopentyl)-amide d'acide 8-(thiophène-2-sulfonyl)-1-oxa-2,8-diaza-spiro[4.5]déc-2-ène-3-carboxylique
- benzylamide d'acide 8-acétyl-1-oxa-2,8-diaza-spiro-[4.5]déc-2-ène-3-carboxylique
- 2,5-difluoro-benzylamide d'acide 8-(5-chloro-thiophène-2-sulfonyl)-1-oxa-2,8-diaza-spiro[4.5]déc-2-ène-3-carboxylique
- (1-naphtalène-2-ylméthyl-pyrrolidine-3-yl)-amide d'acide 8-(5-chloro-thiophène-2-sulfonyl)-1-oxa-2,8-diaza-spiro[4.5]déc-2-ène-3-carboxylique
- 2-éthoxy-benzylamide d'acide 8-phénylméthanesulfonyl-1-oxa-2,8-diaza-spiro[4.5]déc-2-ène-3-carboxylique
- (thiophène-2-ylméthyl)-amide d'acide 8-(4-éthyl-benzoyl)-1-oxa-2,8-diaza-spiro[4.5]déc-2-ène-3-carboxylique
- ester de 1-allyle et 4-tertiobutyle d'acide 2-{[8-(2,5-dichloro-benzènesulfonyl)-1-oxa-2,8-diaza-spiro[4.5]déc-2-ène-3-carbonyl]-amino}-succinique
- naphtalène-2-ylamide d'acide 8-(4-trifluorométhoxy-benzènesulfonyl)-1-oxa-2,8-diaza-spiro[4.5]déc-2-ène-3-carboxylique
- ester éthylique d'acide 4-{[8-(2-phényl-cyclopropane-carbonyl)-1-oxa-2,8-diaza-spiro[4.5]déc-2-ène-3-carbonyl]-amino}-pipéridine-1-carboxylique
- ester éthylique d'acide 4-{[8-(2,4-difluoro-benzoyl)-1-oxa-2,8-diaza-spiro[4.5]déc-2-ène-3-carbonyl]-amino}-pipéridine-1-carboxylique
- (2-diméthylamino-éthyl)-amide d'acide 8-(toluène-4-sulfonyl)-1-oxa-2,8-diaza-spiro[4.5]déc-2-ène-3-carboxylique
- [2-(4-chloro-phényl)-éthyl]-amide d'acide 8-diméthyl-sulfamoyl-1-oxa-2,8-diaza-spiro[4.5]déc-2-ène-3-carboxylique
- (5-méthyl-furanne-2-ylméthyl)-amide d'acide 8-(4-méthoxy-benzoyl)-1-oxa-2,8-diaza-spiro[4.5]déc-2-ène-3-carboxylique
- [2-(4-chloro-phényl)-éthyl]-amide d'acide 8-(thiophène-2-sulfonyl)-1-oxa-2,8-diaza-spiro[4.5]déc-2-ène-3-carboxylique
- [2-(1-méthyl-pyrrolidine-2-yl)-éthyl]-amide d'acide 8-(5-chloro-thiophène-2-sulfonyl)-1-oxa-2,8-diaza-spiro[4.5]déc-2-ène-3-carboxylique
- ester éthylique d'acide 4-{[8-(4-bromo-benzoyl)-1-oxa-2,8-diaza-spiro[4.5]déc-2-ène-3-carbonyl]-amino}-pipéridine-1-carboxylique
- 3,5-dichloro-benzylamide d'acide 8-(toluène-4-sulfonyl)-1-oxa-2,8-diaza-spiro[4.5]déc-2-ène-3-carboxylique
- [1-(3-méthoxy-benzyl)-pyrrolidine-3-yl]-amide d'acide 8-(2,5-dichloro-benzènesulfonyl)-1-oxa-2,8-diaza-spiro[4.5]déc-2-ène-3-carboxylique
- diméthylamide d'acide 3-(4-naphtalène-2-ylméthyl-pipérazine-1-carbonyl)-1-oxa-2,8-diaza-spiro[4.5]déc-2-ène-8-sulfonique

- ester éthylique d'acide 3-{[8-(7,7-diméthyl-2-oxo-bicyclo[2.2.1]hept-1-ylméthanesulfonyl)-1-oxa-2,8-diaza-spiro[4.5]déc-2-ène-3-carbonyl]-amino}-propionique
- (2-benzyloxy-cyclopentyl)-amide d'acide 8-(toluène-4-sulfonyl)-1-oxa-2,8-diaza-spiro[4.5]déc-2-ène-3-carboxylique
- 3,4-diméthoxy-benzylamide d'acide 8-(2,5-dichloro-benzènesulfonyl)-1-oxa-2,8-diaza-spiro[4.5]déc-2-ène-3-carboxylique
- (8-phénylméthanesulfonyl-1-oxa-2,8-diaza-spiro[4.5]-déc-2-ène-3-yl)-[4-(3-phényl-propyl)-pipérazine-1-yl]-méthanone
- diméthylamide d'acide 3-(4-thiéno[2,3-d]pyrimidine-4-yl-pipérazine-1-carbonyl)-1-oxa-2,8-diaza-spiro[4.5]-déc-2-ène-8-sulfonique
- [8-(5-chloro-thiophène-2-sulfonyl)-1-oxa-2,8-diaza-spiro[4.5]déc-2-ène-3-yl)-[4-(3,5-diméthoxy-phényl)-pipérazine-1-yl]-méthanone
- 3-fluoro-4-trifluorométhyl-benzylamide d'acide 8-diméthylsulfamoyl-1-oxa-2,8-diaza-spiro[4.5]déc-2-ène-3-carboxylique
- (2-benzyloxy-cyclopentyl)-amide d'acide 8-(toluène-4-sulfonyl)-1-oxa-2,8-diaza-spiro[4.5]déc-2-ène-3-carboxylique
- ester éthylique d'acide 4-[(8-butyryl-1-oxa-2,8-diaza-spiro[4.5]déc-2-ène-3-carbonyl)-amino]-pipéridine-1-carboxylique
- (2-phényl-cyclopropyl)-amide d'acide 8-(toluène-4-sulfonyl)-1-oxa-2,8-diaza-spiro[4.5]déc-2-ène-3-carboxylique
- [8-(4-fluoro-benzènesulfonyl)-2-oxa-2,8-diaza-spiro-[4.5]déc-2-ène-3-yl]-[4-(5-méthyl-pyrazine-2-carbonyl)-pipérazine-1-yl]-méthanone
- 2,4-difluoro-benzylamide d'acide 8-diméthylsulfamoyl-1-oxa-2,8-diaza-spiro[9.5]déc-2-ène-3-carboxylique
- [2-(3-fluoro-phényl)-éthyl]-amide d'acide 8-phényl-méthanesulfonyl-1-oxa-2,8-diaza-spiro[4.5]déc-2-ène-3-carboxylique
- (2,2-diphényl-éthyl)-amide d'acide 8-(5-chloro-thiophène-2-sulfonyl)-1-oxa-2,8-diaza-spiro[4.5]déc-2-ène-3-carboxylique
- diméthylamide d'acide 3-[4-(3-phényl-propyl)-pipérazine-1-carbonyl]-1-oxa-2,8-diaza-spiro[4.5]déc-2-ène-8-sulfonique
- ester éthylique d'acide 3-{[8-(3,5-difluoro-benzoyl)-1-oxa-2,8-diaza-spiro[4.5]déc-2-ène-3-carbonyl]-amino}-propionique
- isobutyl-amide d'acide 8-(propane-1-sulfonyl)-1-oxa-2,8-diaza-spiro[4.5]déc-2-ène-3-carboxylique
- [4-(3-phényl-propyl)-pipérazine-1-yl]-[8-(3-trifluoro-méthyl-benzènesulfonyl)-2-oxa-2,8-diaza-spiro[4.5]-déc-2-ène-3-yl]-méthanone
- [1-(2,3-dihydro-benzo[1,4]dioxine-5-yl)-éthyl]-amide d'acide 8-(thiophène-2-sulfonyl)-1-oxa-2,8-diaza-spiro [4.5]déc-2-ène-3-carboxylique
- phénylamide d'acide 8-butyryl-1-oxa-2,8-diaza-spiro[4.5]déc-2-ène-3-carboxylique
- cyclooctylamide d'acide 8-(4-trifluorométhoxy-benzène-sulfonyl)-1-oxa-2,8-diaza-spiro[4.5]déc-2-ène-3-carboxylique
- benzylamide d'acide 8-[2-(2-méthoxy-éthoxy)-acétyl]-1-oxa-2,8-diaza-spiro[4.5]déc-2-ène-3-carboxylique
- isobutyl-amide d'acide 8-(4-bromo-3-méthyl-benzoyl)-1-oxa-2,8-diaza-spiro[4.5]déc-2-ène-3-carboxylique
- [3-(méthyl-phényl-amino)-propyl]-amide d'acide 8-(5-chloro-thiophène-2-sulfonyl)-1-oxa-2,8-diaza-spiro-[4.5] déc-2-ène-3-carboxylique
- ester éthylique d'acide 4-{[8-(2-méthyl-5-nitro-benzènesulfonyl)-1-oxa-2,8-diaza-spiro[4.5]déc-2-ène-3-carbonyl]-amino}-pipéridine-1-carboxylique
- [8-(5-chloro-thiophène-2-sulfonyl)-1-oxa-2,8-diaza-spiro[4.5]déc-2-ène-3-yl]-[4-(4-fluoro-phényl)-pipérazine-1-yl]-méthanone
- méthyl-pyridine-3-ylméthyl-amide d'acide 8-(2,5-dichloro-benzènesulfonyl)-1-oxa-2,8-diaza-spiro[4.5]déc-2-ène-3-carboxylique
- 2-(3,4-difluoro-phényl)-1-{4-[8-(3-trifluorométhyl-benzènesulfonyl)-1-oxa-2,8-diaza-spiro[4.5]déc-2-ène-3-carbonyl]-pipérazine-1-yl}-éthanone
- cyclopropylméthyl-amide d'acide 8-(3-trifluorométhyl-benzènesulfonyl)-1-oxa-2,8-diaza-spiro[4.5]déc-2-ène-3-carboxylique
- ester éthylique d'acide 3-{[8-(2-méthyl-5-nitro-benzènesulfonyl)-1-oxa-2,8-diaza-spiro[4.5]dèc-2-ène-3-carbonyl]-amino}-propionique
- (thiophène-2-ylméthyl)-amide d'acide 8-[2-(3-chloro-phénoxy)-acétyl]-1-oxa-2,8-diaza-spiro[4.5]déc-2-ène-3-carboxylique
- benzyl-phénéthyl-amide d'acide 8-(4-trifluorométhoxy-benzènesulfonyl)-1-oxa-2,8-diaza-spiro[4.5]déc-2-ène-3-carboxylique

- benzyl-phénéthyl-amide d'acide 8-(2,5-dichloro-benzènesulfonyl)-1-oxa-2,8-diaza-spiro[4.5]déc-2-ène-3-carboxylique
- (2-cyano-éthyl)-pyridine-3-ylméthyl-amide d'acide 8-(2,5-dichloro-benzènesulfonyl)-1-oxa-2,8-diaza-spiro-[4.5]déc-2-ène-3-carboxylique
- (2-cyano-éthyl)-(tétrahydro-furanne-2-ylméthyl)-amide d'acide 8-(2,5-dichloro-benzènesulfonyl)-1-oxa-2,8-diaza-spiro[4.5]déc-2-ène-3-carboxylique
- méthyl-pyridine-3-ylméthyl-amide d'acide 8-(3-trifluorométhyl-benzènesulfanyl)-1-oxa-2,8-diaza-spiro[4.5]déc-2-ène-3-carboxylique
- benzylamide d'acide 8-(pyridine-4-carbonyl)-1-oxa-2,8-diaza-spiro[4.5]déc-2-ène-3-carboxylique
- éthyl-(2-méthyl-allyl)-amide d'acide 8-(4-trifluoro-méthoxy-benzènesulfonyl)-1-oxa-2,8-diaza-spiro[4.5]-déc-2-ène-3-carboxylique
- [2-(2-fluoro-phényl)-éthyl]-amide d'acide 8-(4-méthoxy-benzènesulfonyl)-1-oxa-2,8-diaza-spiro[4.5]-déc-2-ène-3-carboxylique
- isobutyl-amide d'acide 8-(5-tertiobutyl-2-méthyl-furanne-3-carbonyl)-1-oxa-2,8-diaza-spiro[4.5]déc-2-ène-3-carboxylique
- (thiophène-2-ylméthyl)-amide d'acide 8-(3-phényl-acryloyl)-1-oxa-2,8-diaza-spiro[4.5]déc-2-ène-3-carboxylique
- cyclopropylméthyl-amide d'acide 8-(4-trifluorométhyl-benzoyl)-1-oxa-2,8-diaza-spiro[4.5]déc-2-ène-3-carboxylique
- cyclopropylméthyl-amide d'acide 8-(4-trifluorométhyl-sulfanyl-benzoyl)-1-oxa-2,8-diaza-spiro[4.5]déc-2-ène-3-carboxylique
- (5-méthyl-furanne-2-ylméthyl)-amide d'acide 8-(4-bromo-benzoyl)-1-oxa-2,8-diaza-spiro[4.5]déc-2-ène-3-carboxylique
- cyclopropylméthyl-amide d'acide 8-(3-fluoro-4-trifluorométhyl-benzoyl)-1-oxa-2,8-diaza-spiro[4.5]-déc-2-ène-3-carboxylique
- (2-éthylsulfanyl-éthyl)-amide d'acide 8-(7,7-diméthyl-2-oxo-bicyclo[2.2.1]hept-1-ylméthanesulfonyl)-1-oxa-2,8-diaza-spiro[4.5]déc-2-ène-3-carboxylique
- (tétrahydro-furanne-2-ylméthyl)-amide d'acide 8-(2,4-difluoro-benzoyl)-1-oxa-2,8-diaza-spiro[4.5]déc-2-ène-3-carboxylique
- ester éthylique d'acide {[8-(3-chloro-thiophène-2-carbonyl)-1-oxa-2,8-diaza-spiro[4.5]déc-2-ène-3-carbonyl]-amino}-acétique
- ester méthylique d'acide 4-oxo-4-{3-[(thiophène-2-ylméthyl)-carbamoyl]-1-oxa-2,8-diaza-spiro[4.5]déc-2-ène-8-yl}-butyrique
- benzylamide d'acide 8-(2-éthylsulfanyl-pyridine-3-carbonyl)-1-oxa-2,8-diaza-spiro[4.5]déc-2-ène-3-carboxylique
- ester éthylique d'acide 3-{[8-(2-chloro-benzène-sulfonyl)-1-oxa-2,8-diaza-spiro[4.5]déc-2-ène-3-carbonyl]-amino}-propionique
- cyclopropylméthyl-amide d'acide 8-(4-trifluorométhoxy-benzoyl)-1-oxa-2,8-diaza-spiro[4.5]déc-2-ène-3-carboxylique
- cyclopropylméthyl-amide d'acide 8-(4-phénoxy-butyryl)-1-oxa-2,8-diaza-spiro[4.5]déc-2-ène-3-carboxylique
- [8-(3-trifluorométhyl-benzènesulfonyl)-1-oxa-2,8-diaza-spiro[4.5]déc-2-ène-3-yl]-[4-(4-trifluoro-méthyl-pyridine-2-yl)-pipérazine-1-yl]-méthanone
- cyclopropylméthyl-amide d'acide 8-(2-chloro-5-trifluorométhyl-benzoyl)-1-oxa-2,8-diaza-spiro[4.5]-déc-2-ène-3-carboxylique
- (2-benzyloxy-cyclopentyl)-amide d'acide 8-(4-méthoxy-benzènesulfonyl)-1-oxa-2,8-diaza-spiro[4.5]déc-2-ène-3-carboxylique
- ester éthylique d'acide 4-{[8-(3-trifluorométhyl-benzènesulfonyl)-1-oxa-2,8-diaza-spiro[4.5]déc-2-ène-3-carbonyl]-amino}-pipéridine-1-carboxylique
- cyclopropylméthyl-amide d'acide 8-(2-phénoxy-propionyl)-1-oxa-2,8-diaza-spiro[4.5]déc-2-ène-3-carboxylique
- [2-(3-méthoxy-phényl)-éthyl]-amide d'acide 8-(4-fluorobenzènesulfonyl)-1-oxa-2,8-diaza-spiro[4.5]déc-2-ène-3-carboxylique
- isobutyl-amide d'acide 8-(2-méthylsulfanyl-pyridine-3-carbonyl)-1-oxa-2,8-diaza-spiro[4.5]déc-2-ène-3-carboxylique
- cyclopentylamide d'acide 8-(5-tertiobutyl-2-méthyl-furanne-3-carbonyl)-1-oxa-2,8-diaza-spiro[4.5]déc-2-ène-3-carboxylique
- (thiophène-2-ylméthyl)-amide d'acide 8-(4-chloro-benzènesulfonyl)-1-oxa-2,8-diaza-spiro[4.5]déc-2-ène-3-carboxylique
- (naphtalène-1-ylméthyl)-amide d'acide 8-(5-chloro-thiophène-2-sulfonyl)-1-oxa-2,8-diaza-spiro[4.5]déc-2-ène-3-carboxylique
- [2-(4-fluoro-phényl)-éthyl]-amide d'acide 8-(4-trifluorométhoxy-benzènesulfonyl)-1-oxa-2,8-diaza-spiro[4.5]déc-2-ène-3-carboxylique
- (pyridine-4-ylméthyl)-amide d'acide 8-(4-bromo-3-méthyl-benzoyl)-1-oxa-2,8-diaza-spiro[4.5]déc-2-ène-3-carboxylique
- benzylamide d'acide 8-(4-méthoxy-benzoyl)-1-oxa-2,8-diaza-spiro[4.5]déc-2-ène-3-carboxylique
- 3-chloro-benzylamide d'acide 8-(3-trifluorométhyl-benzènesulfonyl)-1-oxa-2,8-diaza-spiro[4.5]déc-2-ène-3-carboxylique
- (8-phénylméthanesulfonyl-1-oxa-2,8-diaza-spiro[4.5]-déc-2-ène-3-yl)-(4-o-tolyl-pipérazine-1-yl)-méthanone
- (8-phénylméthanesulfonyl-1-oxa-2,8-diaza-spiro[4.5]-déc-2-ène-3-yl)-(4-pyridine-2-yl-pipérazine-1-yl)-méthanone
- (thiophène-2-ylméthyl)-amide d'acide 8-(4-trifluorométhylsulfanyl-benzoyl)-1-oxa-2,8-diaza-spiro[4.5]déc-2-ène-3-carboxylique
- benzylamide d'acide 8-(thiophène-2-sulfonyl)-1-oxa-2,8-diaza-spiro[4.5]déc-2-ène-3-carboxylique
- isobutyl-amide d'acide 8-(4-fluoro-benzoyl)-1-oxa-2,8-diaza-spiro[4.5]déc-2-ène-3-carboxylique
- ester méthylique d'acide 2-(3-isobutylcarbamoyl-1-oxa-2,8-diaza-spiro[4.5]déc-2-ène-8-sulfonyl)-benzoïque
- ester tertiobutylique d'acide 2-[(8-diméthylsulfamoyl-1-oxa-2,8-diaza-spiro[4.5]déc-2-ène-3-carbonyl)-amino]-3-phényl-propionique
- [1-(2,3-dihydro-benzo[1,4]dioxine-5-yl)-éthyl]-amide d'acide 8-(5-chloro-thiophène-2-sulfonyl)-1-oxa-2,8-diaza-spiro[4.5]déc-2-ène-3-carboxylique
- (thiophène-2-ylméthyl)-amide d'acide 8-(4-bromo-benzoyl)-1-oxa-2,8-diaza-spiro[4.5]déc-2-ène-3-carboxylique
- (3-imidazole-1-yl-propyl)-amide d'acide 8-(4-fluoro-benzènesulfonyl)-1-oxa-2,8-diaza-spiro[4.5]déc-2-ène-3-carboxylique
- ester benzylique d'acide 4-[8-(4-fluoro-benzène-sulfonyl)-1-oxa-2,8-diaza-spiro[4.5]déc-2-ène-3-carbonyl]-pipérazine-1-carboxylique
- diméthylamide d'acide 3-(9-cycloheptyl-pipérazine-1-carbonyl)-1-oxa-2,8-diaza-spiro[4.5]déc-2-ène-8-sulfonique
- ester tertiobutylique d'acide 4-méthyl-2-{[8-(thiophène-2-sulfonyl)-1-oxa-2,8-diaza-spiro[4.5]déc-2-ène-3-carbonyl]-amino}-pentanoïque
- (thiophène-2-ylméthyl)-amide d'acide 8-(3-chloro-2-fluoro-benzoyl)-1-oxa-2,8-diaza-spiro[4.5]déc-2-ène-3-carboxylique
- ester tertiobutylique d'acide 2-{[8-(4-méthoxy-benzènesulfonyl)-1-oxa-2,8-diaza-spiro[4.5]déc-2-ène-3-carbonyl]-amino}-4-méthyl-pentanoïque
- ester éthylique d'acide 3-{[8-(6-chloro-2-fluoro-3-méthyl-benzoyl)-1-oxa-2,8-diaza-spiro[4.5]déc-2-ène-3-carbonyl]-amino}-propionique
- benzylamide d'acide 8-(2-phénoxy-propionyl)-1-oxa-2,8-diaza-spiro[4.5]déc-2-ène-3-carboxylique
- (thiophène-2-ylméthyl)-amide d'acide 8-(5-fluoro-2-méthyl-benzènesulfonyl)-1-oxa-2,8-diaza-spiro[4.5]déc-2-ène-3-carboxylique
- [8-(5-chloro-thiophène-2-sulfonyl)-1-oxa-2,8-diaza-spiro[4.5]déc-2-ène-3-yl]-[4-(2-méthoxy-phényl)-pipéridine-1-yl]-méthanone
- (thiophène-2-ylméthyl)-amide d'acide 8-(2-chloro-benzoyl)-1-oxa-2,8-diaza-spiro[4.5]déc-2-ène-3-carboxylique
- cyclopentylamide d'acide 8-(2-phénoxy-pyridine-3-carbonyl)-1-oxa-2,8-diaza-spiro[4.5]déc-2-ène-3-carboxylique
- (2-éthylsulfanyl-éthyl)-amide d'acide 8-(4-bromo-benzoyl)-1-oxa-2,8-diaza-spiro[4.5]déc-2-ène-3-carboxylique
- ester éthylique d'acide 1-[8-(5-chloro-thiophène-2-sulfonyl)-1-oxa-2,8-diaza-spiro[4.5]déc-2-ène-3-carbonyl]-pipéridine-2-carboxylique
- (thiophène-2-ylméthyl)-amide d'acide 8-(2-chloro-benzènesulfonyl)-1-oxa-2,8-diaza-spiro[4.5]déc-2-ène-3-carboxylique
- cyclopropylméthyl-amide d'acide 8-(2,3-dichloro-benzoyl)-1-oxa-2,8-diaza-spiro[4.5]déc-2-ène-3-carboxylique
- cyclopropylméthyl-amide d'acide 8-hexanoyl-1-oxa-2,8-diaza-spiro[4.5]déc-2-ène-3-carboxylique
- cyclopropylméthyl-amide d'acide 8-(2,3-difluoro-4-méthyl-benzoyl)-1-oxa-2,8-diaza-spiro[4.5]déc-2-ène-3-carboxylique

- (2,5-diméthoxy-benzyl)-furanne-2-ylméthyl-amide d'acide 8-(9-fluoro-benzènesulfonyl)-1-oxa-2,8-diaza-spiro [4.5]déc-2-ène-3-carboxylique
- (2-éthylsulfanyl-éthyl)-amide d'acide 8-diméthyl-sulfamoyl-1-oxa-2,8-diaza-spiro[4.5]déc-2-ène-3-carboxylique
- 3-méthoxy-benzylamide d'acide 8-(4-trifluorométhoxy-benzènesulfonyl)-1-oxa-2,8-diaza-spiro[4.5]déc-2-ène-3-carboxylique
- (2-benzyloxy-cyclopentyl)-amide d'acide 8-diméthyl-sulfamoyl-1-oxa-2,8-diaza-spiro[4.5]déc-2-ène-3-carboxylique
- [2-(4-chloro-phényl)-1-méthyl-éthyl]-amide d'acide 8-(4-méthoxy-benzènesulfonyl)-1-oxa-2,8-diaza-spiro-[4.5]déc-2-ène-3-carboxylique
- [2-(3,4-dichloro-phényl)-éthyl]-amide d'acide 8-(4-trifluorométhoxy-benzènesulfonyl)-1-oxa-2,8-diaza-spiro [4.5]déc-2-ène-3-carboxylique
- benzylamide d'acide 8-[2-(3-chloro-phénoxy)-acétyl]-1-oxa-2,8-diaza-spiro[4.5]déc-2-ène-3-carboxylique
- (2-cyano-éthyl)-(2-pyridine-2-yl-éthyl)-amide d'acide 8-(4-fluoro-benzènesulfonyl)-1-oxa-2,8-diaza-spiro-[4.5] déc-2-ène-3-carboxylique
- [2-(4-chloro-phényl)-1-méthyl-éthyl]-amide d'acide 8-(5-chloro-thiophène-2-sulfonyl)-1-oxa-2,8-diaza-spliro-[4.5]déc-2-ène-3-carboxylique
- (2-benzyloxy-cyclopentyl)-amide d'acide 8-(4-méthoxy-benzènesulfonyl)-1-oxa-2,8-diaza-spiro[4.5]déc-2-ène-3-carboxylique
- (thiophène-2-ylméthyl)-amide d'acide 8-(2,6-difluoro-3-méthyl-benzoyl)-1-oxa-2,8-diaza-spiro[4.5]déc-2-ène-3-carboxylique
- benzylamide d'acide 8-(2-benzyloxy-acétyl)-2-oxa-2,8-diaza-spiro[4.5]déc-2-ène-3-carboxylique
- phénylamide d'acide 8-(2,3-dichloro-benzoyl)-1-oxa-2,8-diaza-spiro[4.5]déc-2-ène-3-carboxylique
- cyclopentylamide d'acide 8-(5-bromo-2-méthoxy-benzène-sulfonyl)-1-oxa-2,8-diaza-spiro[4.5]déc-2-ène-3-carboxylique
- ester 2-(3-benzylcarbamoyl-1-oxa-2,8-diaza-spiro[4.5]-déc-2-ène-8-yl)-1,1-diméthyl-2-oxo-éthylique d'acide acétique
- (2-éthylsulfanyl-éthyl)-amide d'acide 8-[3-(2-chloro-6-fluoro-phényl)-5-méthyl-isoxazole-4-carbonyl]-1-oxa-2,8-diaza-spiro[4.5]déc-2-ène-3-carboxylique
- [4-(3-phényl-propyl)-pipérazine-1-yl]-[8-(4-trifluorométhoxy-benzènesulfonyl)-1-oxa-2,8-diaza-spiro[4.5]déc-2-ène-3-yl]-méthanone
- benzylamide d'acide 8-[3-(2-chloro-6-fluoro-phényl)-5-méthyl-isoxazole-4-carbonyl]-1-oxa-2,8-diaza-spiro [4.5]déc-2-ène-3-carboxylique
- isobutyl-amide d'acide 8-(naphtalènecarbonyl)-1-oxa-2,8-diaza-spiro[4.5]déc-2-ène-3-carboxylique
- ester éthylique d'acide 1-[8-(5-tertiobutyl-2-méthyl-furanne-3-carbonyl)-1-oxa-2,8-diaza-spiro[4.5]déc-2-ène-3-carbonyl]-pipéridine-2-carboxylique
- benzylamide d'acide 8-(3-difluorométhylsulfanyl-benzoyl)-1-oxa-2,8-diaza-spiro[4.5]déc-2-ène-3-carboxylique
- phénylamide d'acide 8-(4-bromo-benzoyl)-1-oxa-2,8-diaza-spiro[4.5]déc-2-ène-3-carboxylique
- (2-phényl-cyclopropyl)-amide d'acide 8-(4-méthoxy-benzènesulfonyl)-1-oxa-2,8-diaza-spiro[4.5]déc-2-ène-3-carboxylique
- (thiophène-2-ylméthyl)-amide d'acide 8-(4-phénoxy-butyryl)-1-oxa-2,8-diaza-spiro[4.5]déc-2-ène-3-carboxylique
- (thiophène-2-ylméthyl)-amide d'acide 8-(3-chloro-4-fluoro-benzoyl)-1-oxa-2,8-diaza-spiro[4.5]déc-2-ène-3-carboxylique
- (2-éthylsulfanyl-éthyl)-amide d'acide 8-(5-tertio-butyl-2-méthyl-2H-pyrazole-3-carbonyl)-1-oxa-2,8-diaza-spiro[4.5]déc-2-ène-3-carboxylique
- [8-(4-fluoro-benzènesulfonyl)-1-oxa-2,8-diaza-spiro-[4.5]déc-2-ène-3-yl]-[4-(3-phényl-propyl)-pipérazine-1-yl]-méthanone
- (pyridine-4-ylméthyl)-amide d'acide 8-(4-trifluoro-méthoxy-benzoyl)-1-oxa-2,8-diaza-spiro[4.5]déc-2-ène-3-carboxylique
- ester 2-(3-cyclopentylcarbamoyl-1-oxa-2,8-diaza-spiro[4.5]déc-2-ène-8-carbonyl)-benzylique d'acide benzoïque
- diméthylamide d'acide 3-[4-(4-tertiobutyl-benzyl)-pipérazine-1-carbonyl]-1-oxa-2,8-diaza-spiro[4.5]déc-2-ène-8-sulfonique
- ester éthylique d'acide {[8-(2-éthoxy-benzoyl)-1-oxa-2,8-diaza-spiro[4.5]déc-2-ène-3-carbonyl]-amino}-acétique
- (2-benzyloxy-cyclopentyl)-amide d'acide 8-(5-chloro-thiophène-2-sulfonyl)-1-oxa-2,8-diaza-spiro[4.5]déc-2-ène-3-carboxylique

- (thiophène-2-ylméthyl)-amide d'acide 8-(naphtalène-1-carbonyl)-1-oxa-2,8-diaza-spiro[4.5]déc-2-ène-3-carboxylique
- (3-phényl-propyl)-amide d'acide 8-(4-fluoro-benzène-sulfonyl)-1-oxa-2,8-diaza-spiro[9.5]déc-2-ène-3-carboxylique
- (2,3-dihydro-benzo[1,4]dioxine-2-ylméthyl)-amide d'acide 8-(5-chloro-thiophène-2-sulfonyl)-1-oxa-2,8-diaza-spiro[4.5]déc-2-ène-3-carboxylique
- ester éthylique d'acide 1-[8-(4-tertiobutyl-benzoyl)-1-oxa-2,8-diaza-spiro[4.5]déc-2-ène-3-carbonyl]-pipéridine-2-carboxylique
- [2-(3-trifluorométhyl-phényl)-éthyl]-amide d'acide 8-(4-fluoro-benzènesulfonyl)-1-oxa-2,8-diaza-spiro[4.5]-déc-2-ène-3-carboxylique
- (thiophène-2-ylméthyl)-amide d'acide 8-[3-(2,6-dichloro-phényl)-5-méthyl-isoxazole-4-carbonyl]-1-oxa-2,8-diaza-spiro[4.5]déc-2-ène-3-carboxylique
- [1-(naphtalène-2-ylcarbamoyl)-éthyl]-amide d'acide 8-(4-fluoro-benzènesulfonyl)-1-oxa-2,8-diaza-spiro[4.5] déc-2-ène-3-carboxylique
- (2-benzyloxy-cyclopentyl)-amide d'acide 8-(4-fluoro-benzènesulfonyl)-1-oxa-2,8-diaza-spiro[4.5]déc-2-ène-3-carboxylique
- ester méthylique d'acide 3-(4-chloro-phényl)-2-{[8-(4-méthoxy-benzènesulfonyl)-1-oxa-2,8-diaza-spiro[4.5] déc-2-ène-3-carbonyl]-amino}-propionique
- (2-benzyloxy-cyclopentyl)-amide d'acide 8-(3-trifluorométhyl-benzènesulfonyl)-1-oxa-2,8-diaza-spiro[4.5] déc-2-ène-3-carboxylique
- (2-phénoxy-éthyl)-amide d'acide 8-(3-trifluorométhyl-benzènesulfonyl)-1-oxa-2,8-diaza-spiro[4.5]déc-2-ène-3-carboxylique
- benzylamide d'acide 8-(2-phénoxy-pyridine-3-carbonyl)-1-oxa-2,8-diaza-spiro[4.5]déc-2-ène-3-carboxylique
- cyclopropylméthyl-amide d'acide 8-(naphtalène-2-carbonyl)-1-oxa-2,8-diaza-spiro[4.5]déc-2-ène-3-carboxylique
- benzylamide d'acide 8-(3-chloro-4-fluoro-benzoyl)-1-oxa-2,8-diaza-spiro[4.5]déc-2-ène-3-carboxylique
- (2-cyano-éthyl)-amide d'acide 8-(2-phényl-butyryl)-1-oxa-2,8-diaza-spiro[4.5]déc-2-ène-3-carboxylique
- 2-fluoro-benzylamide d'acide 8-(4-fluoro-benzène-sulfonyl)-1-oxa-2,8-diaza-spiro[4.5]déc-2-ène-3-carboxylique
- (thiophène-2-ylméthyl)-amide d'acide 8-(3-chloro-benzoyl)-1-oxa-2,8-diaza-spiro[4.5]déc-2-ène-3-carboxylique
- (4-benzyl-pipérazine-1-yl)-[8-(2,5-dichloro-benzènesulfonyl)-1-oxa-2,8-diaza-spiro[4.5]déc-2-ène-3-yl]-méthanone
- benzylamide d'acide 8-(3-chloro-benzènesulfonyl)-1-oxa-2,8-diaza-spiro[4.5]déc-2-ène-3-carboxylique
- (4-benzyl-pipéridine-1-yl)-[8-(3-trifluorométhyl-benzènesulfonyl)-1-oxa-2,8-diaza-spiro[4.5]déc-2-ène-3-yl]-méthanone
- benzylamide d'acide 8-(4-bromo-benzoyl)-1-oxa-2,8-diaza-spiro[4.5]déc-2-ène-3-carboxylique
- [1-(4-nitro-phényl)-éthyl]-amide d'acide 8-(4-fluoro-benzènesulfonyl)-1-oxa-2,8-diaza-spiro[4.5]déc-2-ène-3-carboxylique
- ester tertiobutylique d'acide 4-méthyl-2-{[8-(3-trifluorométhyl-benzènesulfonyl)-1-oxa-2,8-diaza-spiro[4.5] déc-2-ène-3-carbonyl]-amino}-pentanoïque
- [4-(4-fluoro-phényl)-3,6-dihydro-2H-pyridine-1-yl]-(8-phénylméthanesulfonyl-1-oxa-2,8-diaza-spiro[4.5]déc-2-ène-3-yl]-méthanone
- [2-(éthyl-m-tolyl-amino)-éthyl]-amide d'acide 8-diméthylsulfamoyl-1-oxa-2,8-diaza-spiro[4.5]déc-2-ène-3-carboxylique
- benzylamide d'acide 8-(2,5-diméthyl-furanne-3-carbonyl)-1-oxa-2,8-diaza-spiro[4.5]déc-2-ène-3-carboxylique
- (4-cycloheptyl-pipérazine-1-yl)-[8-(4-fluoro-benzènesulfonyl)-1-oxa-2,8-diaza-spiro[4.5]déc-2-ène-3-yl]-méthanone
- benzylamide d'acide 8-(2-benzyloxy-acétyl)-1-oxa-2,8-diaza-spiro[4.5]déc-2-ène-3-carboxylique
- (2-benzyloxy-cyclopentyl)-amide d'acide *R*,*R*-8-(5-chloro-thiophène-2-sulfonyl)-1-oxa-2,8-diaza-spiro-[4.5] déc-2-ène-3-carboxylique ;

ainsi que leurs chlorhydrates.

**10.** Procédé de production d'un dérivé substitué de 1-oxa-2,8-diaza-spiro[4.5]déc-2-ène de formule générale (I)

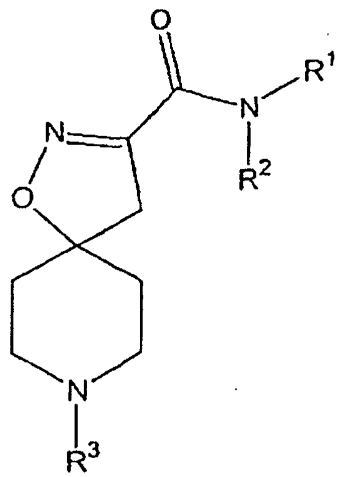

I

dans laquelle $R^1$, $R^2$ et $R^3$ sont tels que définis dans l'une quelconque des revendications 1 à 8, **caractérisé en ce que**

(a) le composé de formule (II)

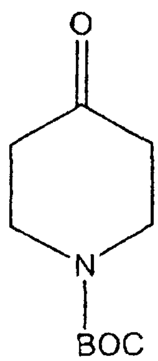

II

est amené à réagir avec un agent de méthylénation, avantageusement $Ph_3PCH_3Br$ en présence de tertiobutylate de potassium dans du THF pour former le composé (III)

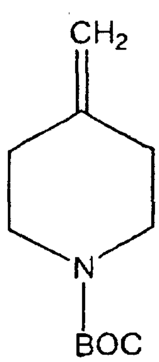

III

(b) le composé (III) est soumis à une réaction avec le chloroximidoacétate d'éthyle (IV)

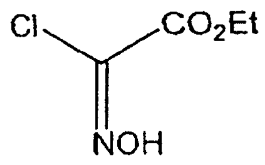

IV

en présence d'une base, avantageusement le bicarbonate de sodium ou l'hydroxyde de lithium, préférentiellement dans un solvant organique, en particulier le méthanol, le dichlorométhane ou le THF, avec formation du dérivé de 1-oxa-2,8-diaza-spiro[4.5]déc-2-ène de formule (V)

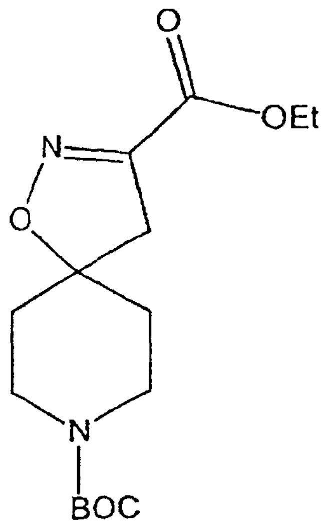

V

(c) le composé (V) est amené à réagir directement ou après saponification préalable de la fonction ester éthylique d'acide carboxylique du composé (V) et, le cas échéant, avec activation de la fonction acide carboxylique ainsi formée, avec une amine de formule $HNR^1R^2$, dans laquelle $R^1$ et $R^2$ sont tels que définis dans l'une quelconque des revendications 1 à 8, pour obtenir le dérivé de 1-oxa-2,8-diaza-spiro[4.5]déc-2-ène de formule (VI)

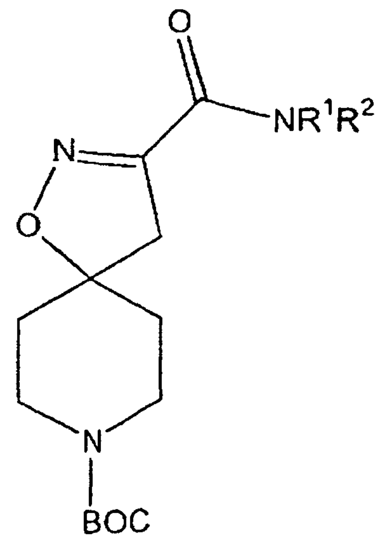

VI

(d) le composé (I) dans lequel $R^3$ représente H

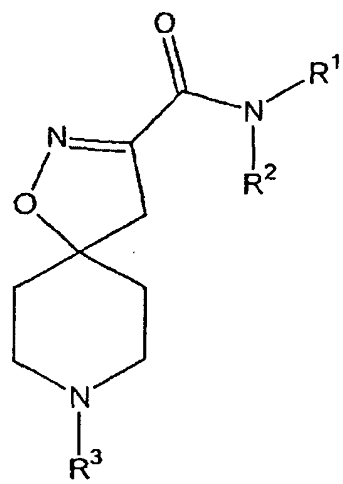

I

est obtenu par élimination du groupe protecteur BOC du composé (VI) ; et

(e) le cas échéant, le composé (I) dans lequel $R^3$ représente H est transformé avec un chlorure d'acide de formule $R^{12}SO_2Cl$ en un composé (I) dans lequel $R^3$ représente $SO_2R^{12}$, $R^{12}$ étant tel que défini dans l'une quelconque des revendications 1 à 8, ou avec un chlorure d'acide carboxylique de formule $R^{13}COCl$ en un composé (I) dans lequel $R^3$ représente $COR^{13}$, $R^{13}$ étant tel que défini dans l'une quelconque des revendications 1 à 8.

**11.** Médicament contenant au moins un dérivé substitué de 1-oxa-2,8-diaza-spiro[4.5]déc-2-ène de formule générale (I) suivant la revendication 1 sous forme de ses racémates, de ses stéréoisomères purs, en particulier de ses énantiomères ou diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates.

12. Utilisation d'au moins un dérivé substitué de 1-oxa-2,8-diaza-spiro[4.5]déc-2-ène de formule générale (I) suivant la revendication 1 sous forme de ses racémates, de ses stéréoisomères purs, en particulier ses énantiomères ou diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates ; pour la préparation d'un médicament destiné au traitement de la douleur, en particulier d'une douleur neuropathique et/ou d'une douleur chronique, et/ou pour le traitement et/ou pour la prophylaxie de la migraine.

13. Utilisation d'au moins un dérivé substitué de 1-oxa-2,8-diaza-spiro[4.5]déc-2-ène de formule générale (I) suivant la revendication 1, sous forme de ses racémates, de ses stéréoisomères purs, en particulier ses énantiomères ou ses diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates ; pour la préparation d'un médicament destiné au traitement et/ou à la prophylaxie de l'incontinence d'urine et/ou du prurit et/ou des acouphènes et/ou de la diarrhée.

14. Utilisation d'au moins un dérivé substitué de 1-oxa-2,8-diaza-spiro[4.5]déc-2-ène de formule générale (I) suivant la revendication 1 sous forme de ses racémates, de ses stéréoisomères purs, en particulier ses énantiomères ou ses diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates ; pour la préparation d'un médicament destiné à l'anesthésie, en particulier à l'anesthésie locale, et/ou au traitement et/ou à la prophylaxie d'arythmies et/ou de l'émèse et/ou de maladies cardiovasculaires et/ou d'ischémies cérébrales et/ou de la dépendance à l'égard de l'alcool et/ou de la dépendance à l'égard de la drogue et/ou de la dépendance à l'égard des médicaments et/ou d'inflammations et/ou du vertige et/ou comme neurotrope et/ou comme myorelaxant.

15. Utilisation d'au moins un dérivé substitué de 1-oxa-2,8-diaza-spiro[4.5]déc-2-ène de formule générale (I) suivant la revendication 1 sous forme de ses racémates, de ses stéréoisomères purs, en particulier ses énantiomères ou ses diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates ; pour la préparation d'un médicament destiné au traitement et/ou à la prophylaxie de réactions inflammatoires et/ou allergiques et/ou de la gastrite et/ou d'ulcères et/ou de dépressions et/ou d'états de choc et/ou de narcolepsie et/ou d'épilepsie et/ou de surcharge pondérale et/ou de l'asthme et/ou du glaucome et/ou du syndrome hypercinétique.

16. Utilisation d'au moins un dérivé substitué de 1-oxa-2,8-diaza-spiro[4.5]déc-2-ène de formule générale (I) suivant la revendication 1 sous forme de ses racémates, de ses stéréoisomères purs, en particulier ses énantiomères ou ses diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates ; pour la préparation d'un médicament destiné au traitement et/ou à la prophylaxie de l'absence de tonus et/ou de la boulimie et/ou de l'anorexie et/ou de la catalepsie et/ou pour l'anxiolyse et/ou pour accroître la vigilance et/ou la libido.

17. Utilisation d'au moins un dérivé substitué de 1-oxa-2,8-diaza-spiro[4.5]déc-2-ène de formule générale (I) suivant la revendication 1 sous forme de ses racémates, de ses stéréoisomères purs, en particulier ses énantiomères ou diastéréoisomères, ou sous forme de mélanges des stéréoisomères, en particulier des énantiomères ou des diastéréoisomères, dans un rapport de mélange quelconque ; sous la forme représentée ou sous forme de ses acides ou de ses bases ou sous forme de ses sels, en particulier des sels acceptables du point de vue physiologique, notamment sous forme de ses chlorhydrates, ou sous forme de ses produits de solvatation, en particulier des hydrates ; pour la préparation d'un médicament destiné au traitement et/ou à la prophylaxie de troubles bipolaires

et/ou de bouffées de chaleur postménopausiques et/ou de la sclérose latérale amyotrophique (ALS) et/ou d'algodystrophie sympathique réflexe (RSD) et/ou de la paralysie spastique et/ou du Restless Leg Syndrom et/ou du nystagmus acquis et/ou de la sclérose en plaques et/ou de la maladie de Parkinson et/ou de la maladie d'Alzheimer et/ou de la maladie de Huntington.